# EUROPEAN PATENT APPLICATION

(11) **EP 4 567 036 A1**
(43) Date of publication of application: **11.06.2025**
(21) Application number: 23849080.9
(22) Date of filing: 26.06.2023
(51) Int. Cl.: C07D 487/04, A61K 31/4353, A61P 35/00

(54) **NITROGEN-CONTAINING FUSED THREE RING PRMT5 INHIBITOR, AND PREPARATION METHOD THEREFOR AND PHARMACEUTICAL USE THEREOF**

(30) Priority: 03.08.2022 CN 202210928223; 21.11.2022 CN 202211462338; 22.02.2023 CN 202310153354
(71) Applicant: Abbisko Therapeutics Co., Ltd., Shanghai 201203 (CN)
(72) Inventor: DENG, Haibing, Shanghai 201203 (CN); LIU, Zhaomin, Shanghai 201203 (CN); YANG, Fei, Shanghai 201203 (CN); YU, Hongping, Shanghai 201203 (CN); CHEN, Zhui, Shanghai 201203 (CN); XU, Yaochang, Shanghai 201203 (CN)
(74) Representative: dompatent von Kreisler Selting Werner - Partnerschaft von Patent- und Rechtsanwälten mbB
(86) International application number: PCT/CN2023/102507
(87) International publication number: WO 2024/027370

(57) **Abstract**

A PRMTS inhibitor having a structure of formula (I), a preparation method therefor, a pharmaceutical composition containing same, the use thereof as a PRMTS inhibitor, and the use thereof in the treatment and/or prevention of PRMT5-mediated diseases.

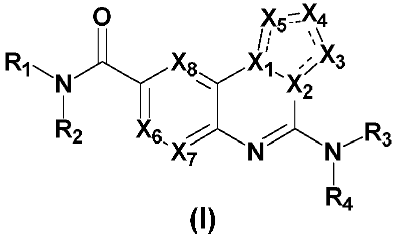

## Description

### TECHNICAL FIELD

The present invention belongs to the field of pharmaceutical synthesis and particularly relates to a nitrogen-containing fused three ring PRMT5 inhibitor and a preparation method therefor and pharmaceutical use thereof.

### BACKGROUND

Epigenetic gene regulation is an important biological regulatory mechanism for protein synthesis and cell differentiation and plays an important role in many human diseases.

Epigenetic regulation includes the regulation of genetic material that can be inherited without altering its nucleic acid sequence. Typically, epigenetic regulation controls the switch between the active and inactive states of transcription in chromatin conformation by selective reversible modifications (e.g., methylation) of DNA and proteins (e.g., histones). Modifications of these covalent bonds can be controlled by enzymes such as methyltransferases (e.g., PRMT5), many of which are associated with specific genetic alternations in many pathogenic genes of human diseases. PRMT5 plays an important role in many diseases such as tumors, metabolic diseases and hematological diseases.

Homozygous deletion of tumor suppressor genes is the driver of tumors and often leads to deletions of passenger genes near the suppressor genes. The deletion of these passenger genes results in tumor cell-specific weaknesses that can be targeted by targeted therapies. Homozygous deletion of the chromosome 9p21 locus, which harbors the well-known tumor suppressor CDKN2A, occurs in 15% of all tumors and often includes the deletion of the passenger gene MTAP. MTAP is a key enzyme in the methionine and adenine recycling pathway. Deletion of MTAP results in accumulation of its substrate, MTA. MTA and S-adenosylmethionine (SAM) are structurally similar, and the latter is a methyl substrate donor for the type II methyltransferase PRMT5. Due to the increase of MTA level caused by deletion of MTAP, it will selectively compete with SAM for binding of PRMT5, leaving methyltransferase in an inactivation state and more likely to be affected by PRMT5 inhibition. shRNA screening of a wide range of tumor cell lines across many different genomic ranges has shown a correlation between MTAP deletion and dependence of the cell line on PRMT5, thus putting the impact of this metabolic susceptibility in the spotlight. However, PRMT5 is an important gene for cells and conditional PRMT5 knockout and siRNA knockdown studies suggest that inhibition of PRMT5 in normal tissues will have significant side effects (e.g., cytopenia, infertility, decreased skeletal muscle, myocardial hypertrophy, etc.). Therefore, novel strategies are required to apply and explore this metabolic susceptibility and selectively target PRMT5 in MTAP-deleted tumors while sparing PRMT5 in normal tissues (MTAP wild-type).

Small-molecule inhibitors targeting PRMT5 that work together with MTA can selectively target PRMT5 only in the MTA-bound state, and this PRMT5 is only enriched in MTAP-deleted tumor cells. Therefore, PRMT5 will not be targeted when MTA levels are very low in normal cells with intact MTAP, thus providing a better treatment window.

### SUMMARY

The object of the present invention is to provide a nitrogen-containing fused three ring PRMT5 inhibitor, a preparation method therefor and pharmaceutical use thereof. The series of compounds of the present invention have a strong inhibitory effect on PRMT5, can be widely applied to the preparation of medicaments for treating and/or preventing PRMT5-mediated diseases, and are expected to be developed into a new generation of PRMTS inhibitors.

In a first aspect, the present invention provides a compound of formula (I), a stereoisomer or pharmaceutically acceptable salt thereof: 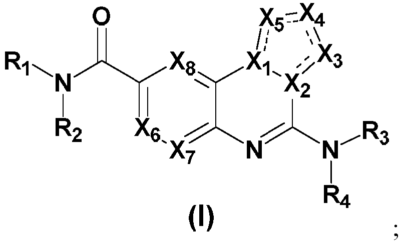
wherein, "---" is a double bond or a single bond;
X₁ is C or N, and X₂ is C or N, **provided that at least one of X₁ and X₂ is N;**
X₃ is CR₅ or N, X₄ is CR₆ or N, X₅ is CR₇ or N,
X₆ is CR₈ or N, X₇ is CR₉ or N, X₈ is CR₁₀ or N;
**R₁** is -(CR₁₁R₁₂)ₘ-R, **or R₁** and **R₂,** together with a nitrogen atom to which they are directly attached, form a **ring B,** wherein the **ring B** is a 4-10 membered nitrogen-containing heterocyclyl or 5-10 membered nitrogen-containing heteroaryl and optionally further substituted with one or more substituents selected from the group consisting of R, deuterium, halogen, cyano, nitro, azido, C₁₋₁₀ alkyl, C₂₋₁₀ alkenyl, C₂₋₁₀ alkynyl, =O, =S, -C₀₋₈ alkyl-SF₅, -C₀₋₈ alkyl-O-S(O)₂R₁₃, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, -C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(O)NR₁₆R₁₇ and -C₀₋₈ alkyl-N(R₁₆)-C(O)R₁₅;
**R is -ring A-(Ra)n,**
**ring A** is selected from the group consisting of C₃₋₁₂ cycloalkyl, 4-10 membered heterocyclyl, C₆₋₁₀ aryl and 5-10 membered heteroaryl, wherein the C₃₋₁₂ cycloalkyl or 4-10 membered heterocyclyl is optionally further fused to C₆₋₁₀ aryl or 5-10 membered heteroaryl, and the C₆₋₁₀ aryl or 5-10 membered heteroaryl is optionally further fused to 5-10 membered heteroaryl, C₃₋₁₂ cycloalkyl or 4-10 membered heterocyclyl,
**each Rₐ** is independently selected from the group consisting of hydrogen, deuterium, halogen, cyano, nitro, azido, C₁₋₁₀ alkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, -C₀₋₈ alkyl-SF₅, -C₀₋₈ alkyl-O-S(O)₂R₁₃, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, - C₀₋₈ alkyl-S-C(O)R₁₅, -C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(O)NR₁₆R₁₇ and -C₀₋₈ alkyl-N(R₁₆)-C(O)R₁₅, or, when n ≥ 2, two Rₐ on the same carbon, together with a carbon atom to which they are directly attached, form C(O), the above groups are independently optionally more further substituted with one or more substituents selected from the group consisting of deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, =O, =S, -C₀₋₈ alkyl-SF₅, -C₀₋₈ alkyl-O-S(O)₂R₁₃, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, -C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(O)NR₁₆R₁₇ and -C₀₋₈ alkyl-N(R₁₆)-C(O)R₁₅;
**R₂** is selected from the group consisting of hydrogen, deuterium, C₁₋₁₀ alkyl, C₂₋₁₀ alkenyl, C₂₋₁₀ alkynyl, C₃₋₁₂ cycloalkyl, 3-12 membered heterocyclyl, C₆₋₁₀ aryl, 5-10 member heteroaryl, -S(O)ᵣR₁₃, -O-R₁₄, -C(O)OR₁₄, -C(O)R₁₅ and -C(O)NR₁₆R₁₇, wherein the C₃₋₁₂ cycloalkyl or 3-12 membered heterocyclyl is optionally further fused to C₆₋₁₀ aryl or 5-10 membered heteroaryl, and the C₆₋₁₀ aryl or 5-10 membered heteroaryl is optionally further fused to C₃₋₁₂ cycloalkyl or 4-10 membered heterocyclyl, the above groups are independently optionally further substituted with one or more substituents selected from the group consisting of deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, halogen-substituted C₃₋₆ cycloalkyl, halogen-substituted 3-6 membered heterocyclyl, halogen-substituted C₆₋₈ aryl, halogen-substituted 5-8 membered heteroaryl, C₁₋₄ alkyl-substituted C₃₋₆ cycloalkyl, C₁₋₄ alkyl-substituted 3-6 membered heterocyclyl, C₁₋₄ alkyl-substituted C₆₋₈ aryl, C₁₋₄ alkyl-substituted 5-8 membered heteroaryl, (2-(trimethylsilyl)ethoxy)methyl, =O, =S, -C₀₋₈ alkyl-SF₅, -C₀₋₈ alkyl-O-S(O)₂R₁₃, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, - C₀₋₈ alkyl-S-C(O)R₁₅, -C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(O)NR₁₆R₁₇ and -C₀₋₈ alkyl-N(R₁₆)-C(O)R₁₅;
**R₃ and R₄** are each independently selected from the group consisting of hydrogen, deuterium, hydroxy, C₁₋₁₀ alkyl, C₂₋₁₀ alkenyl, C₂₋₁₀ alkynyl, C₃₋₁₂ cycloalkyl and 3-12 membered heterocyclyl, or R₃ and R₄, together with a nitrogen atom to which they are directly attached, form 4-10 membered nitrogen-containing heterocyclyl or 5-10 membered nitrogen-containing heteroaryl, the above groups are independently optionally further substituted with one or more substituents selected from the group consisting of deuterium, halogen, cyano, hydroxy, =O, =S, C₁₋₁₀ alkyl, C₁₋₁₀ haloalkyl, C₁₋₁₀ deuterioalkyl, C₂₋₁₀ alkenyl, C₂₋₁₀ alkynyl, C₁₋₁₀ alkoxy, C₃₋₁₂ cycloalkyl, C₃₋₁₂ cycloalkyloxy, 3-12 membered heterocyclyl, 3-12 membered heterocyclyloxy and -NR₁₆R₁₇;
**R₅**, **R₆ and R₇** are each independently selected from the group consisting of hydrogen, deuterium, halogen, cyano, nitro, azido, C₁₋₁₀ alkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, -C₀₋₈ alkyl-SF₅, -C₀₋₈ alkyl-O-S(O)₂R₁₃, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, -C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(O)NR₁₆R₁₇ and -C₀₋₈ alkyl-N(R₁₆)-C(O)R₁₅, the above groups are independently optionally more further substituted with one or more substituents selected from the group consisting of deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, =O, =S, -C₀₋₈ alkyl-SF₅, -C₀₋₈ alkyl-O-S(O)₂R₁₃, -C₀₋₈ alkylS(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, - C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(O)NR₁₆R₁₇ and -C₀₋₈ alkyl-N(R₁₆)-C(O)R₁₅;
**R₈**, **R₉ and R₁₀** are each independently selected from the group consisting of hydrogen, deuterium, halogen, cyano, nitro, azido, C₁₋₁₀ alkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, -C₀₋₈ alkyl-SF₅, -C₀₋₈ alkyl-O-S(O)₂R₁₃, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, -C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(O)NR₁₆R₁₇ and -C₀₋₈ alkyl-N(R₁₆)-C(O)R₁₅, the above groups are independently optionally more further substituted with one or more substituents selected from the group consisting of deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, =O, =S, -C₀₋₈ alkyl-SF₅, -C₀₋₈ alkyl-O-S(O)₂R₁₃, -C₀₋₈ alkylS(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, - C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(O)NR₁₆R₁₇ and -C₀₋₈ alkyl-N(R₁₆)-C(O)R₁₅;
**each R₁₁ and each R₁₂** are independently selected from the group consisting of hydrogen, deuterium, halogen, cyano, nitro, azido, C₁₋₁₀ alkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, -C₀₋₈ alkyl-SF₅, -C₀₋₈ alkyl-O-S(O)₂R₁₃, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, -C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(O)NR₁₆R₁₇ and -C₀₋₈ alkyl-N(R₁₆)-C(O)R₁₅, or R₁₁ and R₁₂, together with a carbon atom to which they are directly attached, form C₃₋₁₂ cycloalkyl, 4-10 membered heterocyclyl, C₆₋₁₀ aryl or 5-10 membered heteroaryl, the above groups are independently optionally more further substituted with one or more substituents selected from the group consisting of deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, =O, =S, -C₀₋₈ alkyl-SF₅, -C₀₋₈ alkyl-O-S(O)₂R₁₃, -C₀₋₈ alkylS(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, - C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(O)NR₁₆R₁₇ and -C₀₋₈ alkyl-N(R₁₆)-C(O)R₁₅;
each R₁₃ is independently selected from the group consisting of hydrogen, deuterium, hydroxy, C₁₋₁₀ alkyl, C₂₋₁₀ alkenyl, C₃₋₁₂ cycloalkyl, 3-12 membered heterocyclyl, C₆₋₁₀ aryl, 5-10 membered heteroaryl and -NR₁₆R₁₇, the above groups are independently optionally further substituted with one or more substituents selected from the group consisting of deuterium, halogen, hydroxy, =O, C₁₋₁₀ alkyl, C₁₋₁₀ alkoxy, C₃₋₁₂ cycloalkyl, C₃₋₁₂ cycloalkyloxy, 3-12 membered heterocyclyl, 3-12 membered heterocyclyloxy, C₆₋₁₀ aryl, C₆₋₁₀ aryloxy, 5-10 membered heteroaryl, 5-10 membered heteroaryloxy and -NR₁₆R₁₇;
each R₁₄ is independently selected from the group consisting of hydrogen, deuterium, C₁₋₁₀ alkyl, C₂₋₁₀ alkenyl, C₃₋₁₂ cycloalkyl, 3-12 membered heterocyclyl, C₆₋₁₀ aryl and 5-10 membered heteroaryl, the above groups are independently optionally further substituted with one or more substituents selected from the group consisting of deuterium, halogen, hydroxy, =O, cyano, C₁₋₁₀ alkyl, C₁₋₁₀ alkoxy, C₃₋₁₂ cycloalkyl, C₃₋₁₂ cycloalkyloxy, 3-12 membered heterocyclyl, 3-12 membered heterocyclyloxy, C₆₋₁₀ aryl, C₆₋₁₀ aryloxy, 5-10 membered heteroaryl, 5-10 membered heteroaryloxy and -NR₁₆R₁₇;
each R₁₅ is independently selected from the group consisting of hydrogen, deuterium, hydroxy, C₁₋₁₀ alkyl, C₁₋₁₀ alkoxy, C₂₋₁₀ alkenyl, C₂₋₁₀ alkynyl, C₃₋₁₂ cycloalkyl, C₃₋₁₂ cycloalkyloxy, 3-12 membered heterocyclyl, 3-12 membered heterocyclyloxy, C₆₋₁₀ aryl, C₆₋₁₀ aryloxy, 5-10 membered heteroaryl, 5-10 membered heteroaryloxy and -NR₁₆R₁₇, the above groups are independently optionally further substituted with one or more substituents selected from the group consisting of deuterium, halogen, hydroxy, =O, cyano, C₁₋₁₀ alkyl, C₁₋₁₀ alkoxy, C₃₋₁₂ cycloalkyl, C₃₋₁₂ cycloalkyloxy, 3-12 membered heterocyclyl, 3-12 membered heterocyclyloxy, C₆₋₁₀ aryl, C₆₋₁₀ aryloxy, 5-10 membered heteroaryl, 5-10 membered heteroaryloxy and -NR₁₆R₁₇;
each R₁₆ and each R₁₇ are independently selected from the group consisting of hydrogen, deuterium, hydroxy, C₁₋₁₀ alkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, sulfinyl, sulfonyl, methylsulfonyl, isopropylsulfonyl, cyclopropylsulfonyl, p-toluenesulfonyl, aminosulfonyl, dimethylaminosulfonyl and C₁₋₁₀ alkanoyl, the above groups are independently optionally further substituted with one or more substituents selected from the group consisting of deuterium, halogen, hydroxy, =O, C₁₋₁₀ alkyl, C₂₋₁₀ alkenyl, C₂₋₁₀ alkynyl, C₁₋₁₀ haloalkyl, C₁₋₁₀ deuterioalkyl, C₁₋₁₀ alkoxy, C₃₋₁₂ cycloalkyl, C₃₋₁₂ cycloalkyloxy, 3-12 membered heterocyclyl, 3-12 membered heterocyclyloxy, C₆₋₁₀ aryl, C₆₋₁₀ aryloxy, 5-10 membered heteroaryl, 5-10 membered heteroaryloxy, amino, mono-C₁₋₁₀ alkyl-substituted amino, di-C₁₋₁₀ alkyl-substituted amino and C₁₋₁₀ alkanoyl, or
R₁₆ and R₁₇, together with a nitrogen atom to which they are directly attached, form 4-10 membered heterocyclyl or 5-10 membered heteroaryl, the 4-10 membered heterocyclyl or 5-10 membered heteroaryl is optionally further substituted with one or more substituents selected from the group consisting of deuterium, halogen, hydroxy, =O, C₁₋₁₀ alkyl, C₂₋₁₀ alkenyl, C₂₋₁₀ alkynyl, C₁₋₁₀ haloalkyl, C₁₋₁₀ deuterioalkyl, C₁₋₁₀ alkoxy, C₃₋₁₂ cycloalkyl, C₃₋₁₂ cycloalkyloxy, 3-12 membered heterocyclyl, 3-12 membered heterocyclyloxy, C₆₋₁₀ aryl, C₆₋₁₀ aryloxy, 5-10 membered heteroaryl, 5-10 membered heteroaryloxy, amino, mono-C₁₋₁₀ alkyl-substituted amino, di-C₁₋₁₀ alkyl-substituted amino and C₁₋₁₀ alkanoyl;
**m is 0, 1 or 2;**
**n is 0, 1, 2, 3, 4, 5 or 6; and**
each r is independently 0, 1 or 2.

As a further preferred embodiment, in the compound of formula (I), the stereoisomer or pharmaceutically acceptable salt thereof, **R₃ and R₄** are each independently selected from the group consisting of hydrogen, deuterium, hydroxy, C₁₋₄ alkyl, C₂₋₄ alkenyl, C₂₋₄ alkynyl, C₃₋₆ cycloalkyl and 3-6 membered heterocyclyl, the above groups are independently optionally further substituted with one or more substituents selected from the group consisting of deuterium, halogen, cyano, hydroxy, =O, =S, C₁₋₄ alkyl, C₁₋₄ haloalkyl, C₁₋₄ deuterioalkyl, C₂₋₄ alkenyl, C₂₋₄ alkynyl, C₁₋₄ alkoxy, C₃₋₆ cycloalkyl, C₃₋₆ cycloalkyloxy, 3-6 membered heterocyclyl, 3-6 membered heterocyclyloxy and -NR₁₆R₁₇;
wherein, R₁₆ and R₁₇ are defined as those in the compound of formula (I).

As a more further preferred embodiment, in the compound of formula (I), the stereoisomer or pharmaceutically acceptable salt thereof, **R₃ and R₄** are each independently selected from the group consisting of hydrogen, deuterium, hydroxy, C₁₋₄ alkyl, C₁₋₄ haloalkyl, C₁₋₄ deuterioalkyl, C₂₋₄ alkenyl, C₂₋₄ alkynyl, C₃₋₆ cycloalkyl and 3-6 membered heterocyclyl.

As a further preferred embodiment, in the compound of formula (I), the stereoisomer or pharmaceutically acceptable salt thereof, **R₅**, **R₆ and R₇** are each independently selected from the group consisting of hydrogen, deuterium, halogen, cyano, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -C₀₋₄ alkyl-SF₅, -C₀₋₄ alkyl-O-S(O)₂R₁₃, -C₀₋₄ alkyl-S(O)ᵣR₁₃, -C₀₋₄ alkyl-O-R₁₄, -C₀₋₄ alkyl-C(O)OR₁₄, -C₀₋₄ alkyl-C(O)SR₁₄, -C₀₋₄ alkyl-S-C(O)R₁₅, -C₀₋₄ alkyl-C(O)R₁₅, -C₀₋₄ alkyl-O-C(O)R₁₅, -C₀₋₄ alkyl-P(O)(R₁₅)₂, -C₀₋₄ alkyl-NR₁₆R₁₇, -C₀₋₄ alkyl-C(O)NR₁₆R₁₇ and -C₀₋₄ alkyl-N(R₁₆)-C(O)R₁₅, the above groups are independently optionally more further substituted with one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -C₀₋₄ alkyl-SF₅, -C₀₋₄ alkyl-O-S(O)₂R₁₃, -C₀₋₄ alkyl-S(O)ᵣR₁₃, - C₀₋₄ alkyl-O-R₁₄, -C₀₋₄ alkyl-C(O)OR₁₄, -C₀₋₄ alkyl-C(O)SR₁₄, -C₀₋₄ alkyl-S-C(O)R₁₅, -C₀₋₄ alkyl-C(O)R₁₅, -C₀₋₄ alkyl-O-C(O)R₁₅, -C₀₋₄ alkyl-P(O)(R₁₅)₂, -C₀₋₄ alkyl-NR₁₆R₁₇, -C₀₋₄ alkyl-C(O)NR₁₆R₁₇ and -C₀₋₄ alkyl-N(R₁₆)-C(O)R₁₅;
wherein, R₁₃, R₁₄, R₁₅, R₁₆, R₁₇ and r are defined as those in the compound of formula (I).

As a more further preferred embodiment, in the compound of formula (I), the stereoisomer or pharmaceutically acceptable salt thereof, **R₅**, **R₆ and R₇** are each independently selected from the group consisting of hydrogen, deuterium, halogen, cyano, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -SF₅, -O-S(O)₂R₁₃, -S(O)ᵣR₁₃, -O-R₁₄, -C(O)OR₁₄, -C(O)SR₁₄, -S-C(O)R₁₅, -C(O)R₁₅, -O-C(O)R₁₅, - P(O)(R₁₅)₂, -NR₁₆R₁₇, -C(O)NR₁₆R₁₇ and -N(R₁₆)-C(O)R₁₅, the above groups are independently optionally more further substituted with one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -SF₅, -O-S(O)₂R₁₃, -S(O)ᵣR₁₃, -O-R₁₄, -C(O)OR₁₄, -C(O)SR₁₄, -S-C(O)R₁₅, -C(O)R₁₅, -O-C(O)R₁₅, -P(O)(R₁₅)₂, -NR₁₆R₁₇, -C(O)NR₁₆R₁₇ and -N(R₁₆)-C(O)R₁₅;
wherein, R₁₃, R₁₄, R₁₅, R₁₆, R₁₇ and r are defined as those in the compound of formula (I).

As a further preferred embodiment, in the compound of formula (I), the stereoisomer or pharmaceutically acceptable salt thereof, **R₈**, **R₉ and R₁₀** are each independently selected from the group consisting of hydrogen, deuterium, halogen, cyano, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -C₀₋₄ alkyl-SF₅, -C₀₋₄ alkyl-O-S(O)₂R₁₃, -C₀₋₄ alkyl-S(O)ᵣR₁₃, -C₀₋₄ alkyl-O-R₁₄, -C₀₋₄ alkyl-C(O)OR₁₄, -C₀₋₄ alkyl-C(O)SR₁₄, -C₀₋₄ alkyl-S-C(O)R₁₅, -C₀₋₄ alkyl-C(O)R₁₅, -C₀₋₄ alkyl-O-C(O)R₁₅, -C₀₋₄ alkyl-P(O)(R₁₅)₂, -C₀₋₄ alkyl-NR₁₆R₁₇, -C₀₋₄ alkyl-C(O)NR₁₆R₁₇ and -C₀₋₄ alkyl-N(R₁₆)-C(O)R₁₅, the above groups are independently optionally more further substituted with one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -C₀₋₄ alkyl-SF₅, -C₀₋₄ alkyl-O-S(O)₂R₁₃, -C₀₋₄ alkyl-S(O)ᵣR₁₃, - C₀₋₄ alkyl-O-R₁₄, -C₀₋₄ alkyl-C(O)OR₁₄, -C₀₋₄ alkyl-C(O)SR₁₄, -C₀₋₄ alkyl-S-C(O)R₁₅, -C₀₋₄ alkyl-C(O)R₁₅, -C₀₋₄ alkyl-O-C(O)R₁₅, -C₀₋₄ alkyl-P(O)(R₁₅)₂, -C₀₋₄ alkyl-NR₁₆R₁₇, -C₀₋₄ alkyl-C(O)NR₁₆R₁₇ and -C₀₋₄ alkyl-N(R₁₆)-C(O)R₁₅;
wherein, R₁₃, R₁₄, R₁₅, R₁₆, R₁₇ and r are defined as those in the compound of formula (I).

As a more further preferred embodiment, in the compound of formula (I), the stereoisomer or pharmaceutically acceptable salt thereof, **R₈**, **R₉ and R₁₀** are each independently selected from the group consisting of hydrogen, deuterium, halogen, cyano, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -SF₅, -O-S(O)₂R₁₃, -S(O)ᵣR₁₃, -O-R₁₄, -C(O)OR₁₄, -C(O)SR₁₄, -S-C(O)R₁₅, -C(O)R₁₅, -O-C(O)R₁₅, - P(O)(R₁₅)₂, -NR₁₆R₁₇, -C(O)NR₁₆R₁₇ and -N(R₁₆)-C(O)R₁₅, the above groups are independently optionally more further substituted with one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -SF₅, -O-S(O)₂R₁₃, -S(O)ᵣR₁₃, -O-R₁₄, -C(O)OR₁₄, -C(O)SR₁₄, -S-C(O)R₁₅, -C(O)R₁₅, -O-C(O)R₁₅, -P(O)(R₁₅)₂, -NR₁₆R₁₇, -C(O)NR₁₆R₁₇ and -N(R₁₆)-C(O)R₁₅;
wherein, R₁₃, R₁₄, R₁₅, R₁₆, R₁₇ and r are defined as those in the compound of formula (I).

As a further preferred embodiment, in the compound of formula (I), the stereoisomer or pharmaceutically acceptable salt thereof, **each R₁₁ and each R₁₂** are independently selected from the group consisting of hydrogen, deuterium, halogen, cyano, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -C₀₋₄ alkyl-SF₅, -C₀₋₄ alkyl-O-S(O)₂R₁₃, -C₀₋₄ alkyl-S(O)ᵣR₁₃, -C₀₋₄ alkyl-O-R₁₄, -C₀₋₄ alkyl-C(O)OR₁₄, -C₀₋₄ alkyl-C(O)SR₁₄, -C₀₋₄ alkyl-S-C(O)R₁₅, -C₀₋₄ alkyl-C(O)R₁₅, -C₀₋₄ alkyl-O-C(O)R₁₅, -C₀₋₄ alkyl-P(O)(R₁₅)₂, -C₀₋₄ alkyl-NR₁₆R₁₇, -C₀₋₄ alkyl-C(O)NR₁₆R₁₇ and -C₀₋₄ alkyl-N(R₁₆)-C(O)R₁₅, or, R₁₁ and R₁₂, together with a carbon atom to which they are directly attached, form C₃₋₆ cycloalkyl, 4-8 membered heterocyclyl, C₆₋₈ aryl or 5-8 membered heteroaryl, the above groups are independently optionally more further substituted with one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -C₀₋₄ alkyl-SF₅, -C₀₋₄ alkyl-O-S(O)₂R₁₃, -C₀₋₄ alkyl-S(O)ᵣR₁₃, -C₀₋₄ alkyl-OR₁₄, -C₀₋₄ alkyl-C(O)OR₁₄, -C₀₋₄ alkyl-C(O)SR₁₄, -C₀₋₄ alkyl-S-C(O)R₁₅, -C₀₋₄ alkyl-C(O)R₁₅, -C₀₋₄ alkyl-O-C(O)R₁₅, -C₀₋₄ alkyl-P(O)(R₁₅)₂, -C₀₋₄ alkyl-NR₁₆R₁₇, -C₀₋₄ alkyl-C(O)NR₁₆R₁₇ and -C₀₋₄ alkyl-N(R₁₆)-C(O)R₁₅;
wherein, R₁₃, R₁₄, R₁₅, R₁₆, R₁₇ and r are defined as those in the compound of formula (I).

As a more further preferred embodiment, in the compound of formula (I), the stereoisomer or pharmaceutically acceptable salt thereof, **R₁₁ and R₁₂** are each independently selected from the group consisting of hydrogen, deuterium, halogen, cyano, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -SF₅, -O-S(O)₂R₁₃, -S(O)ᵣR₁₃, -O-R₁₄, -C(O)OR₁₄, -C(O)SR₁₄, -S-C(O)R₁₅, -C(O)R₁₅, -O-C(O)R₁₅, - P(O)(R₁₅)₂, -NR₁₆R₁₇, -C(O)NR₁₆R₁₇ and -N(R₁₆)-C(O)R₁₅, the above groups are independently optionally more further substituted with one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -SF₅, -O-S(O)₂R₁₃, -S(O)ᵣR₁₃, -O-R₁₄, -C(O)OR₁₄, -C(O)SR₁₄, -S-C(O)R₁₅, -C(O)R₁₅, -O-C(O)R₁₅, -P(O)(R₁₅)₂, -NR₁₆R₁₇, -C(O)NR₁₆R₁₇ and -N(R₁₆)-C(O)R₁₅;
wherein, R₁₃, R₁₄, R₁₅, R₁₆, R₁₇ and r are defined as those in the compound of formula (I).

As a further preferred embodiment, in the compound of formula (I), the stereoisomer or pharmaceutically acceptable salt thereof, **R₁** is -(CR₁₁R₁₂)ₘ-R, **or R₁** and **R₂**, together with a nitrogen atom to which they are directly attached, form a **ring B,** wherein the ring B is 4-10 membered nitrogen-containing heterocyclyl or 5-10 membered nitrogen-containing heteroaryl and optionally further substituted with one or more substituents selected from the group consisting of R, deuterium, halogen, cyano, C₁₋₄ alkyl, C₂₋₄ alkenyl, C₂₋₄ alkynyl, =O, =S, -C₀₋₄ alkyl-SF₅, - C₀₋₄ alkyl-O-S(O)₂R₁₃, -C₀₋₄ alkyl-S(O)ᵣR₁₃, -C₀₋₄ alkyl-O-R₁₄, -C₀₋₄ alkyl-C(O)OR₁₄, -C₀₋₄ alkyl-C(O)SR₁₄, -C₀₋₄ alkyl-S-C(O)R₁₅, -C₀₋₄ alkyl-C(O)R₁₅, -C₀₋₄ alkyl-O-C(O)R₁₅, -C₀₋₄ alkyl-P(O)(R₁₅)₂, -C₀₋₄ alkyl-NR₁₆R₁₇, -C₀₋₄ alkyl-C(O)NR₁₆R₁₇ and -C₀₋₄ alkyl-N(R₁₆)-C(O)R₁₅;
**R is -ring A-(Rₐ)ₙ, ring A is selected from** the group consisting of C₃₋₆ cycloalkyl, 4-8 membered heterocyclyl, C₆₋₈ aryl and 5-8 membered heteroaryl, wherein the C₃₋₆ cycloalkyl or 4-8 membered heterocyclyl is optionally further fused to C₆₋₈ aryl or 5-8 membered heteroaryl, and the C₆₋₈ aryl or 5-8 membered heteroaryl is optionally fused to 5-8 membered heteroaryl, C₃₋₆ cycloalkyl or 4-8 membered heterocyclyl,
each **Rₐ** is independently selected from the group consisting of hydrogen, deuterium, halogen, cyano, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -C₀₋₄ alkyl-SF₅, -C₀₋₄ alkyl-O-S(O)₂R₁₃, -C₀₋₄ alkyl-S(O)ᵣR₁₃, -C₀₋₄ alkyl-O-R₁₄, -C₀₋₄ alkyl-C(O)OR₁₄, -C₀₋₄ alkyl-C(O)SR₁₄, -C₀₋₄ alkyl-S-C(O)R₁₅, -C₀₋₄ alkyl-C(O)R₁₅, -C₀₋₄ alkyl-O-C(O)R₁₅, -C₀₋₄ alkyl-P(O)(R₁₅)₂, -C₀₋₄ alkyl-NR₁₆R₁₇, -C₀₋₄ alkyl-C(O)NR₁₆R₁₇ and -C₀₋₄ alkyl-N(R₁₆)-C(O)R₁₅, the above groups are independently optionally more further substituted with one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -C₀₋₄ alkyl-SF₅, -C₀₋₄ alkyl-O-S(O)₂R₁₃, -C₀₋₄ alkyl-S(O)ᵣR₁₃, -C₀₋₄ alkyl-O-R₁₄, -C₀₋₄ alkyl-C(O)OR₁₄, -C₀₋₄ alkyl-C(O)SR₁₄, -C₀₋₄ alkyl-S-C(O)R₁₅, -C₀₋₄ alkyl-C(O)R₁₅, -C₀₋₄ alkyl-O-C(O)R₁₅, -C₀₋₄ alkyl-P(O)(R₁₅)₂, -C₀₋₄ alkyl-NR₁₆R₁₇, -C₀₋₄ alkyl-C(O)NR₁₆R₁₇ and -C₀₋₄ alkyl-N(R₁₆)-C(O)R₁₅;
**R₂** is selected from the group consisting of hydrogen, deuterium, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, - S(O)ᵣR₁₃, -O-R₁₄, -C(O)OR₁₄, -C(O)R₁₅ and -C(O)NR₁₆R₁₇, the above groups are independently optionally further substituted with one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, halogen-substituted C₃₋₆ cycloalkyl, halogen-substituted 3-6 membered heterocyclyl, halogen-substituted C₆₋₈ aryl, halogen-substituted 5-8 membered heteroaryl, C₁₋₄ alkyl-substituted C₃₋₆ cycloalkyl, C₁₋₄ alkyl-substituted 3-6 membered heterocyclyl, C₁₋₄ alkyl-substituted C₆₋₈ aryl, C₁₋₄ alkyl-substituted 5-8 membered heteroaryl, (2-(trimethylsilyl)ethoxy)methyl, =O, =S, -C₀₋₄ alkyl-SF₅, - C₀₋₄ alkyl-O-S(O)₂R₁₃, -C₀₋₄ alkyl-S(O)ᵣR₁₃, -C₀₋₄ alkyl-O-R₁₄, -C₀₋₄ alkyl-C(O)OR₁₄, -C₀₋₄ alkyl-C(O)SR₁₄, -C₀₋₄ alkyl-S-C(O)R₁₅, -C₀₋₄ alkyl-C(O)R₁₅, -C₀₋₄ alkyl-O-C(O)R₁₅, -C₀₋₄ alkyl-P(O)(R₁₅)₂, -C₀₋₄ alkyl-NR₁₆R₁₇, -C₀₋₄ alkyl-C(O)NR₁₆R₁₇ and -C₀₋₄ alkyl-N(R₁₆)-C(O)R₁₅;
wherein, R₁₁, R₁₂, R₁₃, R₁₄, R₁₅, R₁₆, R₁₇, m, n and r are defined as those in the compound of formula (I).

As a further preferred embodiment, in the compound of formula (I), the stereoisomer or pharmaceutically acceptable salt thereof, each R₁₃ is independently selected from the group consisting of hydrogen, deuterium, hydroxy, C₁₋₄ alkyl, C₂₋₄ alkenyl, C₃₋₆ cycloalkyl, 3-6 membered heterocyclyl, C₆₋₈ aryl, 5-8 membered heteroaryl and -NR₁₆R₁₇, the above groups are independently optionally further substituted with one or more substituents selected from the group consisting of deuterium, halogen, hydroxy, =O, C₁₋₄ alkyl, C₁₋₄ alkoxy, C₃₋₆ cycloalkyl, C₃₋₆ cycloalkyloxy, 3-6 membered heterocyclyl, 3-6 membered heterocyclyloxy, C₆₋₈ aryl, C₆₋₈ aryloxy, 5-8 membered heteroaryl, 5-8 membered heteroaryloxy and -NR₁₆R₁₇;
each R₁₄ is independently selected from the group consisting of hydrogen, deuterium, C₁₋₄ alkyl, C₂₋₄ alkenyl, C₃₋₆ cycloalkyl, 3-6 membered heterocyclyl, C₆₋₈ aryl and 5-8 membered heteroaryl, the above groups are independently optionally further substituted with one or more substituents selected from the group consisting of deuterium, halogen, hydroxy, =O, cyano, C₁₋₄ alkyl, C₁₋₄ alkoxy, C₃₋₆ cycloalkyl, C₃₋₆ cycloalkyloxy, 3-6 membered heterocyclyl, 3-6 membered heterocyclyloxy, C₆₋₈ aryl, C₆₋₈ aryloxy, 5-8 membered heteroaryl, 5-8 membered heteroaryloxy and -NR₁₆R₁₇;
each R₁₅ is independently selected from the group consisting of hydrogen, deuterium, hydroxy, C₁₋₄ alkyl, C₁₋₄ alkoxy, C₂₋₄ alkenyl, C₂₋₄ alkynyl, C₃₋₆ cycloalkyl, C₃₋₆ cycloalkyloxy, 3-6 membered heterocyclyl, 3-6 membered heterocyclyloxy, C₆₋₈ aryl, C₆₋₈ aryloxy, 5-8 membered heteroaryl, 5-8 membered heteroaryloxy and -NR₁₆R₁₇, the above groups are independently optionally further substituted with one or more substituents selected from the group consisting of deuterium, halogen, hydroxy, =O, cyano, C₁₋₄ alkyl, C₁₋₄ alkoxy, C₃₋₆ cycloalkyl, C₃₋₆ cycloalkyloxy, 3-6 membered heterocyclyl, 3-6 membered heterocyclyloxy, C₆₋₈ aryl, C₆₋₈ aryloxy, 5-8 membered heteroaryl, 5-8 membered heteroaryloxy and -NR₁₆R₁₇;
each R₁₆ and each R₁₇ are independently selected from the group consisting of hydrogen, deuterium, hydroxy, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, sulfinyl, sulfonyl, methylsulfonyl, isopropylsulfonyl, cyclopropylsulfonyl, p-toluenesulfonyl, aminosulfonyl, dimethylaminosulfonyl and C₁₋₄ alkanoyl, the above groups are independently optionally further substituted with one or more substituents selected from the group consisting of deuterium, halogen, hydroxy, =O, C₁₋₄ alkyl, C₂₋₄ alkenyl, C₂₋₄ alkynyl, C₁₋₄ haloalkyl, C₁₋₄ deuterioalkyl, C₁₋₄ alkoxy, C₃₋₆ cycloalkyl, C₃₋₆ cycloalkyloxy, 3-6 membered heterocyclyl, 3-6 membered heterocyclyloxy, C₆₋₈ aryl, C₆₋₈ aryloxy, 5-8 membered heteroaryl, 5-8 membered heteroaryloxy, amino, mono-C₁₋₄ alkyl-substituted amino, di-C₁₋₄ alkyl-substituted amino and C₁₋₄ alkanoyl, or
R₁₆ and R₁₇, together with a nitrogen atom to which they are directly attached, form 4-8 membered heterocyclyl or 5-8 membered heteroaryl, wherein the 4-8 membered heterocyclyl or 5-8 membered heteroaryl is optionally further substituted with one or more substituents selected from the group consisting of deuterium, halogen, hydroxy, =O, C₁₋₄ alkyl, C₂₋₄ alkenyl, C₂₋₄ alkynyl, C₁₋₄ haloalkyl, C₁₋₄ deuterioalkyl, C₁₋₄ alkoxy, C₃₋₆ cycloalkyl, C₃₋₆ cycloalkyloxy, 3-6 membered heterocyclyl, 3-6 membered heterocyclyloxy, C₆₋₈ aryl, C₆₋₈ aryloxy, 5-8 membered heteroaryl, 5-8 membered heteroaryloxy, amino, mono-C₁₋₄ alkyl-substituted amino, di-C₁₋₄ alkyl-substituted amino and C₁₋₄ alkanoyl.

As a further preferred embodiment, in the compound of formula (I), the stereoisomer or pharmaceutically acceptable salt thereof, the compound of formula (I) is a compound having formula (II): 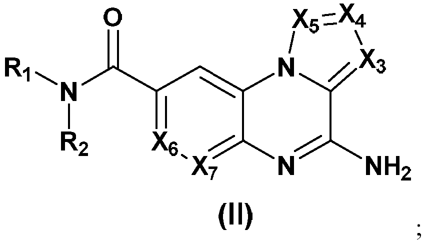 wherein, X₃ is CR₅ or N, X₄ is CR₆ or N, X₅ is CR₇ or N, X₆ is CR₈ or N, X₇ is CR₉ or N;
**R₁** is -(CR₁₁R₁₂)ₘ-R, **or, R₁** and **R₂**, together with a nitrogen atom to which they are directly attached, form a **ring B,** wherein the ring B is 4-10 membered nitrogen-containing heterocyclyl or 5-10 membered nitrogen-containing heteroaryl and optionally further substituted with one or more substituents selected from the group consisting of R, deuterium, halogen, cyano, C₁₋₄ alkyl, C₂₋₄ alkenyl, C₂₋₄ alkynyl, =O, =S, -SF₅, -O-S(O)₂R₁₃, -S(O)ᵣR₁₃, -O-R₁₄, -C(O)OR₁₄, -C(O)SR₁₄, -S-C(O)R₁₅, -C(O)R₁₅, -O-C(O)R₁₅, -P(O)(R₁₅)₂, -NR₁₆R₁₇, -C(O)NR₁₆R₁₇ and -N(R₁₆)-C(O)R₁₅;
**R** is 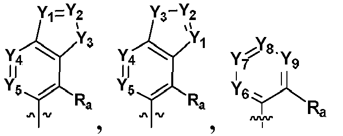 **or** 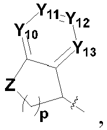
wherein, Y₁, Y₂, Y₄, Y₅, Y₆, Y₇, Y₈, Y₉, Y₁₀, Y₁₁, Y₁₂ and Y₁₃ are each independently CRₐ or N; Y₃ is -O-, -S- or -NRₐ₃-; Z is -O-, -S-, -CRₐ₁Rₐ₂-, -CRₐ₁Rₐ₂-O- or -NRₐ₃-; **p** is 0, 1, 2 or 3;
**each Rₐ** is independently selected from the group consisting of hydrogen, deuterium, halogen, cyano, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -SF₅, -O-S(O)₂R₁₃, -S(O)ᵣR₁₃, -O-R₁₄, -C(O)OR₁₄, -C(O)SR₁₄, -S-C(O)R₁₅, -C(O)R₁₅, -O-C(O)R₁₅, -P(O)(R₁₅)₂, -NR₁₆R₁₇, -C(O)NR₁₆R₁₇ and -N(R₁₆)-C(O)R₁₅, the above groups are independently optionally more further substituted with one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -SF₅, -O-S(O)₂R₁₃, -S(O)ᵣR₁₃, -O-R₁₄, -C(O)OR₁₄, -C(O)SR₁₄, -S-C(O)R₁₅, -C(O)R₁₅, -O-C(O)R₁₅, -P(O)(R₁₅)₂, -NR₁₆R₁₇, - C(O)NR₁₆R₁₇ and -N(R₁₆)-C(O)R₁₅;
**each Rₐ₁** and **each Rₐ₂** are independently selected from the group consisting of hydrogen, deuterium, halogen, cyano, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -SF₅, -O-S(O)₂R₁₃, -S(O)ᵣR₁₃, -O-R₁₄, - C(O)OR₁₄, -C(O)SR₁₄, -S-C(O)R₁₅, -C(O)R₁₅, -O-C(O)R₁₅, -P(O)(R₁₅)₂, -NR₁₆R₁₇, -C(O)NR₁₆R₁₇ and -N(R₁₆)-C(O)R₁₅, the above groups are independently optionally more further substituted with one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -SF₅, -O-S(O)₂R₁₃, -S(O)ᵣR₁₃, -O-R₁₄, -C(O)OR₁₄, -C(O)SR₁₄, -S-C(O)R₁₅, -C(O)R₁₅, -O-C(O)R₁₅, -P(O)(R₁₅)₂, -NR₁₆R₁₇, - C(O)NR₁₆R₁₇ and -N(R₁₆)-C(O)R₁₅;
**each Rₐ₃** is independently selected from the group consisting of hydrogen, deuterium, C₁₋₄ alkyl, C₃₋₆ cycloalkyl, 3-6 membered heterocyclyl, C₆₋₈ aryl, 5-8 membered heteroaryl, -SF₅, - C(O)OR₁₄, -C(O)SR₁₄, -C(O)R₁₅ and -C(O)NR₁₆R₁₇, the above groups are independently optionally more further substituted with one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -SF₅, -O-S(O)₂R₁₃, -S(O)ᵣR₁₃, -O-R₁₄, -C(O)OR₁₄, -C(O)SR₁₄, -S-C(O)R₁₅, -C(O)R₁₅, -O-C(O)R₁₅, -P(O)(R₁₅)₂, -NR₁₆R₁₇, -C(O)NR₁₆R₁₇ and -N(R₁₆)-C(O)R₁₅;
**each R₂** is independently selected from the group consisting of hydrogen, deuterium, C₁₋₄ alkyl, C₃₋₆ cycloalkyl, 3-6 membered heterocyclyl, C₆₋₈ aryl and 5-8 membered heteroaryl, the above groups are independently optionally further substituted with one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, halogen-substituted C₃₋₆ cycloalkyl, halogen-substituted 3-6 membered heterocyclyl, halogen-substituted C₆₋₈ aryl, halogen-substituted 5-8 membered heteroaryl, C₁₋₄ alkyl-substituted C₃₋₆ cycloalkyl, C₁₋₄ alkyl-substituted 3-6 membered heterocyclyl, C₁₋₄ alkyl-substituted C₆₋₈ aryl, C₁₋₄ alkyl-substituted 5-8 membered heteroaryl, (2-(trimethylsilyl)ethoxy)methyl, =O, =S, -SF₅, -O-S(O)₂R₁₃, -S(O)ᵣR₁₃, -O-R₁₄, -C(O)OR₁₄, - C(O)SR₁₄, -S-C(O)R₁₅, -C(O)R₁₅, -O-C(O)R₁₅, -P(O)(R₁₅)₂, -NR₁₆R₁₇, -C(O)NR₁₆R₁₇ and - N(R₁₆)-C(O)R₁₅;
**R₅, R₆ and R₇** are each independently selected from the group consisting of hydrogen, deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -SF₅, -O-S(O)₂R₁₃, -S(O)ᵣR₁₃, -O-R₁₄, -C(O)OR₁₄, -C(O)SR₁₄, -S-C(O)R₁₅, -C(O)R₁₅, -O-C(O)R₁₅, - P(O)(R₁₅)₂, -NR₁₆R₁₇, -C(O)NR₁₆R₁₇ and -N(R₁₆)-C(O)R₁₅;
**R₈ and R₉** are each independently selected from the group consisting of hydrogen, deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -SF₅, -O-S(O)₂R₁₃, -S(O)ᵣR₁₃, -O-R₁₄, -C(O)OR₁₄, -C(O)SR₁₄, -S-C(O)R₁₅, -C(O)R₁₅, -O-C(O)R₁₅, - P(O)(R₁₅)₂, -NR₁₆R₁₇, -C(O)NR₁₆R₁₇ and -N(R₁₆)-C(O)R₁₅;
**each R₁₁ and each R₁₂** are independently selected from the group consisting of hydrogen, deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -SF₅, -O-S(O)₂R₁₃, -S(O)ᵣR₁₃, -O-R₁₄, -C(O)OR₁₄, -C(O)SR₁₄, -S-C(O)R₁₅, -C(O)R₁₅, -O-C(O)R₁₅, - P(O)(R₁₅)₂, -NR₁₆R₁₇, -C(O)NR₁₆R₁₇ and -N(R₁₆)-C(O)R₁₅;
wherein, R₁₃, R₁₄, R₁₅, R₁₆, R₁₇, r and m are defined as those in the compound of formula (I).

As a further preferred embodiment, in the compound of formula (I), the stereoisomer or pharmaceutically acceptable salt thereof, the compound of formula (I) is a compound having formula (III₁) or formula (III₂): 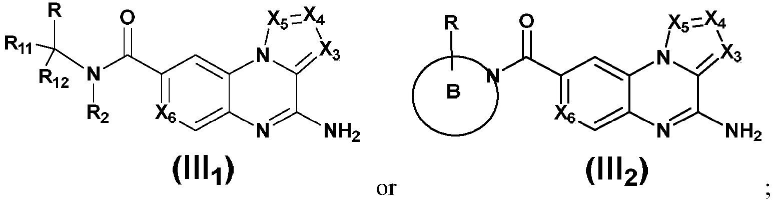
wherein, each X₃ is independently CR₅ or N, each X₄ is independently CR₆ or N, each X₅ is independently CR₇ or N, each X₆ is independently CR₈ or N;
in formula (III₂), ring B is 4-10 membered nitrogen-containing heterocyclyl, wherein the 4-10 membered nitrogen-containing heterocyclyl is optionally further substituted with one or more substituents selected from the group consisting of hydrogen, deuterium, halogen, cyano, hydroxy, carboxyl, C₁₋₄ alkyl, C₁₋₄ haloalkyl, C₁₋₄ deuterioalkyl, C₁₋₄ alkoxy, C₂₋₄ alkenyl, C₂₋₄ alkynyl, C₃₋₆ cycloalkyl, 3-6 membered heterocyclyl, -SF₅ and -NR₁₆R₁₇;
**each R** is independently 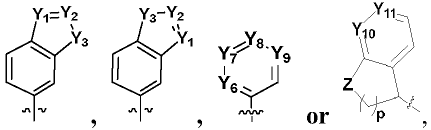
wherein, Y₁, Y₂, Y₆, Y₇, Y₈, Y₉, Y₁₀ and Y₁₁ are each independently CRₐ or N; Y₃ is -O- or - S-; Z is -O-, -S-, -CH₂- or -CH₂-O-; p is 0, 1, 2 or 3,
**each Rₐ** is independently selected from the group consisting of hydrogen, deuterium, halogen, cyano, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -SF₅, -O-R₁₄, -C(O)OR₁₄, -C(O)SR₁₄, -C(O)R₁₅, -O-C(O)R₁₅ and -NR₁₆R₁₇, the above groups are independently optionally more further substituted with one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -SF₅, -O-R₁₄, -C(O)OR₁₄, -C(O)R₁₅, - O-C(O)R₁₅ and -NR₁₆R₁₇;
**R₂** is selected from the group consisting of hydrogen, deuterium, C₁₋₄ alkyl, C₃₋₆ cycloalkyl, 3-6 membered heterocyclyl, C₆₋₈ aryl and 5-8 membered heteroaryl, the above groups are independently optionally further substituted with one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, halogen-substituted C₃₋₆ cycloalkyl, halogen-substituted 3-6 membered heterocyclyl, halogen-substituted C₆₋₈ aryl, halogen-substituted 5-8 membered heteroaryl, C₁₋₄ alkyl-substituted C₃₋₆ cycloalkyl, C₁₋₄ alkyl-substituted 3-6 membered heterocyclyl, C₁₋₄ alkyl-substituted C₆₋₈ aryl, C₁₋₄ alkyl-substituted 5-8 membered heteroaryl, (2-(trimethylsilyl)ethoxy)methyl, =O, =S, -SF₅, -OR₁₄, -C(O)OR₁₄, -C(O)R₁₅, -O-C(O)R₁₅ and -NR₁₆R₁₇;
**each R₅, each R₆ and each R₇** are independently selected from the group consisting of hydrogen, deuterium, halogen, cyano, hydroxy, carboxyl, C₁₋₄ alkyl, C₁₋₄ alkoxy, C₁₋₄ haloalkyl, C₁₋₄ haloalkoxy, C₁₋₄ deuterioalkyl, C₁₋₄ deuterioalkoxy, C₂₋₄ alkenyl, C₂₋₄ alkynyl, C₃₋₆ cycloalkyl, C₃₋₆ cycloalkyloxy, 3-6 membered heterocyclyl, 3-6 membered heterocyclyloxy, C₆₋₈ aryl, C₆₋₈ aryloxy, 5-8 membered heteroaryl, 5-8 membered heteroaryloxy, -SF₅ and -NR₁₆R₁₇;
**each R₈** is independently selected from the group consisting of hydrogen, deuterium, halogen, cyano, hydroxy, carboxyl, C₁₋₄ alkyl, C₁₋₄ alkoxy, C₁₋₄ haloalkyl, C₁₋₄ haloalkoxy, C₁₋₄ deuterioalkyl, C₁₋₄ deuterioalkoxy, C₂₋₄ alkenyl, C₂₋₄ alkynyl, C₃₋₆ cycloalkyl, C₃₋₆ cycloalkyloxy, 3-6 membered heterocyclyl, 3-6 membered heterocyclyloxy, C₆₋₈ aryl, C₆₋₈ aryloxy, 5-8 membered heteroaryl, 5-8 membered heteroaryloxy, -SF₅ and -NR₁₆R₁₇;
**each R₁₁ and each R₁₂** are independently selected from the group consisting of hydrogen, deuterium, halogen, cyano, hydroxy, carboxyl, C₁₋₄ alkyl, C₁₋₄ alkoxy, C₁₋₄ haloalkyl, C₁₋₄ haloalkoxy, C₁₋₄ deuterioalkyl, C₁₋₄ deuterioalkoxy, C₂₋₄ alkenyl, C₂₋₄ alkynyl, C₃₋₆ cycloalkyl, C₃₋₆ cycloalkyloxy, 3-6 membered heterocyclyl, 3-6 membered heterocyclyloxy, C₆₋₈ aryl, C₆₋₈ aryloxy, 5-8 membered heteroaryl, 5-8 membered heteroaryloxy, -SF₅ and -NR₁₆R₁₇;
wherein, R₁₄, R₁₅, R₁₆ and R₁₇ are defined as those in the compound of formula (I).

As a further preferred embodiment, in the compound of formula (I), the stereoisomer or pharmaceutically acceptable salt thereof, " 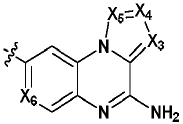 " is selected from the group consisting of the following structures: 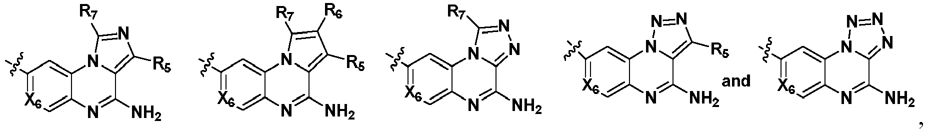
wherein, **each X₆** is independently CR₈ or N;
**each R₅, each R₆ and each R₇** are independently selected from the group consisting of hydrogen, deuterium, halogen, cyano, hydroxy, carboxyl, C₁₋₄ alkyl, C₁₋₄ alkoxy, C₁₋₄ haloalkyl, C₁₋₄ haloalkoxy, C₁₋₄ deuterioalkyl, C₁₋₄ deuterioalkoxy, C₂₋₄ alkenyl, C₂₋₄ alkynyl, C₃₋₆ cycloalkyl, C₃₋₆ cycloalkyloxy, 3-6 membered heterocyclyl, 3-6 membered heterocyclyloxy, C₆₋₈ aryl, C₆₋₈ aryloxy, 5-8 membered heteroaryl, 5-8 membered heteroaryloxy, -SF₅, amino, mono-C₁₋₄ alkyl-substituted amino and di-C₁₋₄ alkyl-substituted amino;
**each R₈** is independently selected from the group consisting of hydrogen, deuterium, halogen, cyano, hydroxy, carboxyl, C₁₋₄ alkyl, C₁₋₄ alkoxy, C₁₋₄ haloalkyl, C₁₋₄ haloalkoxy, C₁₋₄ deuterioalkyl, C₁₋₄ deuterioalkoxy, C₂₋₄ alkenyl, C₂₋₄ alkynyl, C₃₋₆ cycloalkyl, C₃₋₆ cycloalkyloxy, 3-6 membered heterocyclyl, 3-6 membered heterocyclyloxy, C₆₋₈ aryl, C₆₋₈ aryloxy, 5-8 membered heteroaryl, 5-8 membered heteroaryloxy, -SF₅, amino, mono-C₁₋₄ alkyl-substituted amino and di-C₁₋₄ alkyl-substituted amino.

As a further preferred embodiment, in the compound of formula (I), the stereoisomer or pharmaceutically acceptable salt thereof, the compound of formula (I) is a compound having formula (IV₁₋₁), (IV₁₋₂), (IV₂₋₁) or (IV₂₋₂): 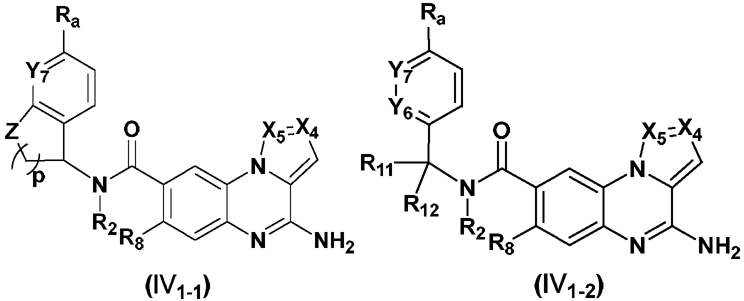 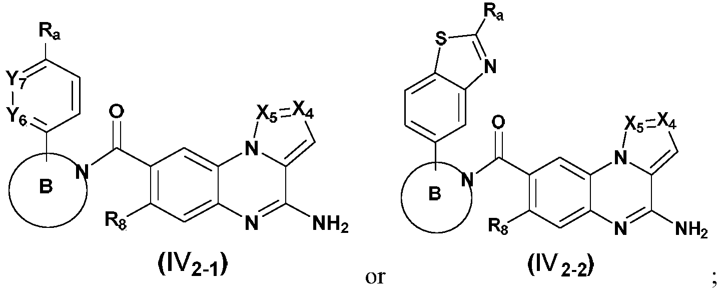
**wherein,** each X₄ is independently CR₆ or N; each X₅ is independently CR₇ or N;
each Y₆ and each Y₇ are independently CH or N; Z is O or CH₂; p is 1 or 2;
in formula (IV₂₋₁) or (IV₂₋₂), ring B, together with a substituent thereon, is selected from the following structures: 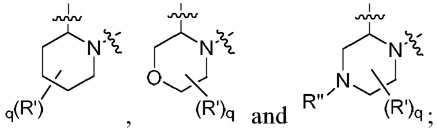 q is 0, 1 or 2;
**each R'** is independently selected from the group consisting of hydrogen, deuterium, fluoro, chloro, bromo, cyano, hydroxy, methyl, ethyl, *n*-propyl, isopropyl, trifluoromethyl, trideuteriomethyl, methoxy, ethoxy, cyclopropyl and -SF₅;
**each R"** is independently selected from the group consisting of hydrogen, deuterium, hydroxy, methyl, ethyl, *n*-propyl, isopropyl, trifluoromethyl, trideuteriomethyl and cyclopropyl;
**each R₂** is independently selected from the group consisting of hydrogen, deuterium, methyl, ethyl, *n*-propyl, isopropyl, *n*-butyl, isobutyl, cyclopropyl, cyclobutyl, bicyclo[1.1.1]pentyl, cyclohexyl, oxacyclobutyl, azacyclobutyl, tetrahydrofuryl, phenyl, pyrazolyl, imidazolyl, triazolyl, oxazolyl, thiazolyl, pyridyl, pyridazinyl, pyrimidinyl and triazinyl, the above groups are independently optionally further substituted with one or more substituents selected from the group consisting of deuterium, fluoro, chloro, bromo, cyano, methyl, ethyl, n-propyl, isopropyl, n-butyl, isobutyl, trifluoromethyl, difluoromethyl, trideuteriomethyl, dideuteriomethyl, vinyl, ethynyl, cyclopropyl, cyclobutyl, cyclopentyl, bicyclo[1.1.1]pentyl, cyclohexyl, oxacyclobutyl, azacyclopentyl, tetrahydrofuryl, phenyl, pyrazolyl, 2-(trimethylsilyl)ethoxy, methyl-substituted pyrazolyl, imidazolyl, triazolyl, oxazolyl, thiazolyl, pyridyl, fluoro-substituted pyridyl, pyridazinyl, pyrimidinyl, triazinyl, =O, =S, -SF₅, hydroxy, methoxy, ethoxy, cyclopropyloxy, cyclobutyloxy, amino, methylamino and dimethylamino;
**each Rₐ** is independently selected from the group consisting of hydrogen, deuterium, fluoro, chloro, bromo, cyano, methyl, ethyl, *n*-propyl, isopropyl, *n*-butyl, isobutyl, methoxy, ethoxy, trifluoromethyl, difluoromethyl, trideuteriomethyl, dideuteriomethyl, trifluoromethoxy, trideuteriomethoxy, vinyl, ethynyl, cyclopropyl, cyclobutyl, methyl-substituted cyclopropyl, methyl-substituted cyclobutyl, cyclopropyloxy, cyclobutyloxy, oxacyclobutyl, azacyclobutyl, tetrahydropyrrolyl, piperidinyl, methyl-substituted piperidinyl, phenyl, pyrazolyl, imidazolyl, triazolyl, oxazolyl, thiazolyl, pyrimidinyl, methyl-substituted pyrazolyl, -SF₅, hydroxy, amino, methylamino, dimethylamino, dimethylaminomethyl and dimethylaminoethyl;
**each R₆** is independently selected from the group consisting of hydrogen, deuterium, fluoro, chloro, bromo, cyano, hydroxy, methyl, ethyl, *n*-propyl, isopropyl, methoxy, trifluoromethyl, trifluoromethoxy, trideuteriomethyl, trideuteriomethoxy and cyclopropyl;
**each R₇** is independently selected from the group consisting of hydrogen, deuterium, fluoro, chloro, bromo, cyano, hydroxy, methyl, ethyl, *n*-propyl, isopropyl, methoxy, trifluoromethyl, trifluoromethoxy, trideuteriomethyl, trideuteriomethoxy and cyclopropyl;
**each R₈** is independently selected from the group consisting of hydrogen, deuterium, fluoro, chloro, bromo, cyano, hydroxy, methyl, ethyl, *n*-propyl, isopropyl, methoxy, trifluoromethyl, trifluoromethoxy, trideuteriomethyl, trideuteriomethoxy and cyclopropyl;
**R₁₁ and R₁₂** are each independently selected from the group consisting of hydrogen, deuterium, fluoro, chloro, bromo, cyano, methyl, trifluoromethyl, trideuteriomethyl and cyclopropyl.

As a further preferred embodiment, in the compound of formula (I), the stereoisomer or pharmaceutically acceptable salt thereof, " 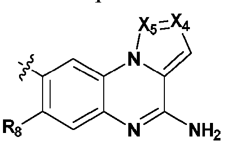 " is selected from the group consisting of the following structures: 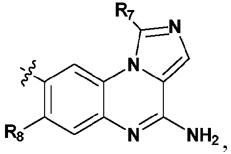 wherein
**R₇** is selected from the group consisting of hydrogen, deuterium, fluoro, chloro, methyl, trifluoromethyl, trideuteriomethyl and cyclopropyl;
**R₈** is selected from the group consisting of hydrogen, deuterium, fluoro, chloro, bromo, cyano, methyl, trifluoromethyl, trideuteriomethyl and cyclopropyl.

As a further preferred embodiment, in the compound of formula (I), the stereoisomer or pharmaceutically acceptable salt thereof, 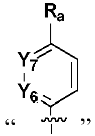 in the compound structures shown as formulas (IV₁₋₂) and (IV₂₋₁) is selected from the group consisting of : 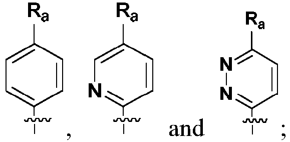 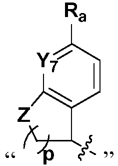 in the compound structure shown as formula (IV₁₋₁) is selected from the group consisting of: 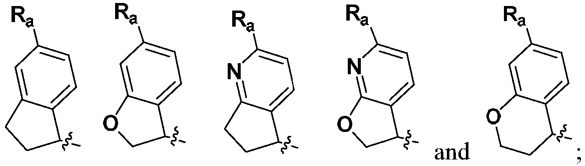 wherein
**each Rₐ** is independently selected from the group consisting of hydrogen, deuterium, fluoro, chloro, bromo, cyano, methyl, isopropyl, methoxy, trifluoromethyl, trideuteriomethyl, trifluoromethoxy, trideuteriomethoxy, cyclopropyl, pyrazolyl, methyl-substituted pyrazolyl and - SF₅.

As a further preferred embodiment, in the compound of formula (I), the stereoisomer or pharmaceutically acceptable salt thereof, **each R₂** is independently selected from the group consisting of hydrogen, deuterium, methyl, ethyl, *n*-propyl, isopropyl, cyclopropyl, cyclobutyl, bicyclo[1.1.1]pentyl, cyclohexyl, oxacyclobutyl, azacyclobutyl, tetrahydrofuryl, pyrazolyl, imidazolyl, thiazolyl, pyridyl and pyrimidinyl, the above groups are independently optionally further substituted with one or more substituents selected from the group consisting of deuterium, fluoro, chloro, bromo, cyano, methyl, ethyl, *n*-propyl, isopropyl, trifluoromethyl, difluoromethyl, trideuteriomethyl, dideuteriomethyl, cyclopropyl, cyclobutyl, cyclopentyl, bicyclo[1.1.1]pentyl, cyclohexyl, oxacyclobutyl, azacyclobutyl, tetrahydrofuryl, pyrazolyl, (2-(trimethylsilyl)ethoxy)methyl-substituted pyrazolyl, imidazolyl, thiazolyl, pyridyl, fluoro-substituted pyridyl, pyrimidinyl, methoxy, ethoxy and cyclopropyloxy.

As a further preferred embodiment, in the compound of formula (I), the stereoisomer thereof or pharmaceutically acceptable salt thereof, **each R₂** is independently selected from the group consisting of hydrogen, deuterium, methyl, ethyl, trifluoromethyl, trifluoroethyl, trideuteriomethyl, ethyl, *n*-propyl, isopropyl, cyclopropyl, cyclobutyl and following substituent structures:

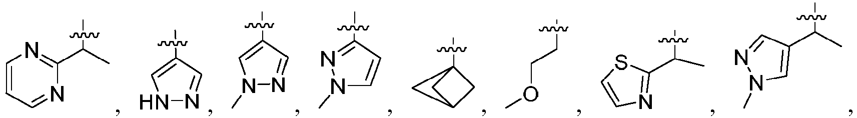

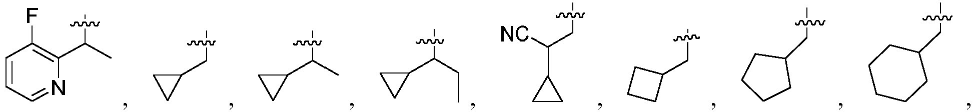

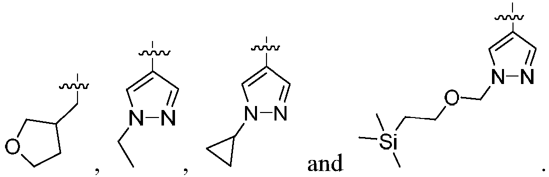

As a more further preferred embodiment, in the compound of formula (I), the stereoisomer or pharmaceutically acceptable salt thereof, the compound of formula (I) is a compound having formula (V₁₋₁₋₁) or formula (V₁₋₁₋₂): 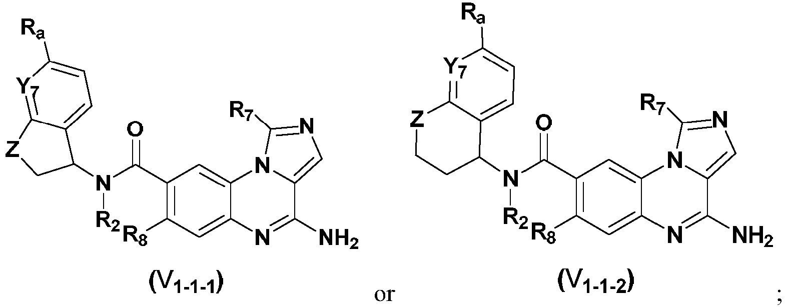
wherein, each Y₇ is independently CH or N; each Z is independently O or CH₂; **each Rₐ** is independently selected from the group consisting of hydrogen, deuterium, fluoro, chloro, bromo, cyano, methyl, methoxy, trifluoromethyl, trideuteriomethyl, trifluoromethoxy, trideuteriomethoxy, cyclopropyl, pyrazolyl, methyl-substituted pyrazolyl and -SF₅;
**each R₇** is independently selected from the group consisting of hydrogen, deuterium, fluoro, chloro, methyl, trifluoromethyl, trideuteriomethyl and cyclopropyl;
**each R₈** is independently selected from the group consisting of hydrogen, deuterium, fluoro, chloro, cyano, methyl, trifluoromethyl, trideuteriomethyl and cyclopropyl;
wherein, R₂ is defined as that in the compound of formula (I).

As a more further preferred embodiment, in the compound of formula (I), the stereoisomer or pharmaceutically acceptable salt thereof, the compound of formula (I) is a compound having formula (V₁₋₂₋₁) or formula (V₁₋₂₋₂): 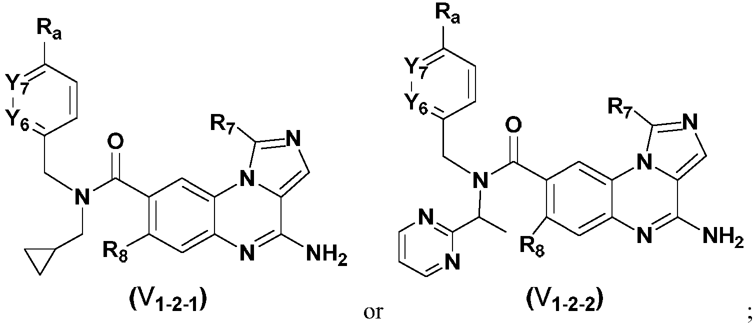
wherein, each Y₆ and each Y₇ are independently CH or N; **Rₐ** is selected from the group consisting of hydrogen, deuterium, fluoro, chloro, bromo, cyano, methyl, methoxy, trifluoromethyl, trideuteriomethyl, trifluoromethoxy, trideuteriomethoxy, cyclopropyl, pyrazolyl, methyl-substituted pyrazolyl and -SF₅;
**R₇** is selected from the group consisting of hydrogen, deuterium, fluoro, chloro, methyl, trifluoromethyl, trideuteriomethyl and cyclopropyl;
**R₈** is selected from the group consisting of hydrogen, deuterium, fluoro, chloro, cyano, methyl, trifluoromethyl, trideuteriomethyl and cyclopropyl.

As a more further preferred embodiment, in the compound of formula (I), the stereoisomer or pharmaceutically acceptable salt thereof, the compound of formula (I) is a compound having formula (V₂₋₁₋₁) or formula (V₂₋₁₋₂): 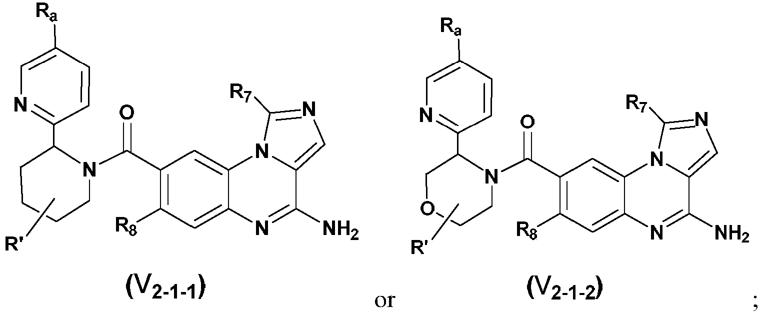
wherein, **each Rₐ** is independently selected from the group consisting of hydrogen, deuterium, fluoro, chloro, bromo, cyano, methyl, methoxy, trifluoromethyl, trideuteriomethyl, trifluoromethoxy, trideuteriomethoxy, cyclopropyl, pyrazolyl, methyl-substituted pyrazolyl and - SF₅;
**each R'** is independently selected from the group consisting of hydrogen, deuterium, fluoro, chloro, methyl, trifluoromethyl, trideuteriomethyl and cyclopropyl;
**each R₇** is independently selected from the group consisting of hydrogen, deuterium, fluoro, chloro, methyl, trifluoromethyl, trideuteriomethyl and cyclopropyl;
**each R₈** is independently selected from the group consisting of hydrogen, deuterium, fluoro, chloro, cyano, methyl, trifluoromethyl, trideuteriomethyl and cyclopropyl.

As a more further preferred embodiment, in the compound of formula (I), the stereoisomer or pharmaceutically acceptable salt thereof, the compound of formula (I) is a compound having formula (V₂₋₂₋₁) or formula (V₂₋₂₋₂): 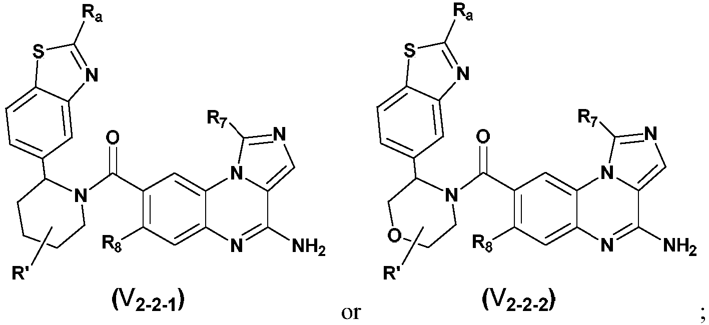
wherein, **each Rₐ** is independently selected from the group consisting of hydrogen, deuterium, fluoro, chloro, bromo, cyano, methyl, methoxy, trifluoromethyl, trideuteriomethyl, trifluoromethoxy, trideuteriomethoxy, cyclopropyl, pyrazolyl, methyl-substituted pyrazolyl, methyl-substituted piperidinyl, dimethylaminoethyl and -SF₅;
**each R'** is independently selected from the group consisting of hydrogen, deuterium, fluoro, chloro, methyl, trifluoromethyl, trideuteriomethyl and cyclopropyl;
**each R₇** is independently selected from the group consisting of hydrogen, deuterium, fluoro, chloro, methyl, trifluoromethyl, trideuteriomethyl and cyclopropyl;
**each R₈** is independently selected from the group consisting of hydrogen, deuterium, fluoro, chloro, cyano, methyl, trifluoromethyl, trideuteriomethyl and cyclopropyl.

As the most preferred embodiment, the compound of formula (I), the stereoisomer or pharmaceutically acceptable salt thereof include, but are not limited to, the following compounds:

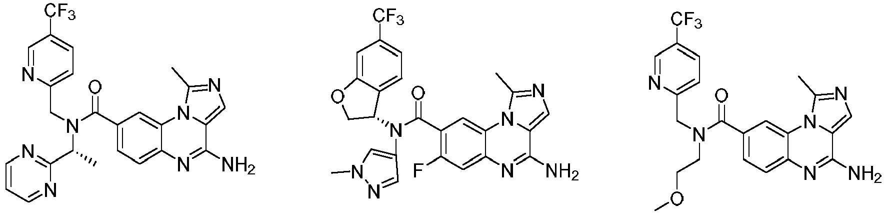

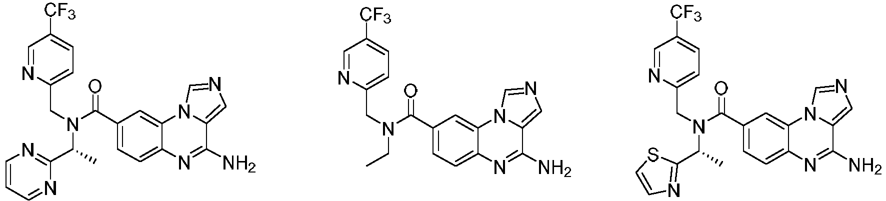

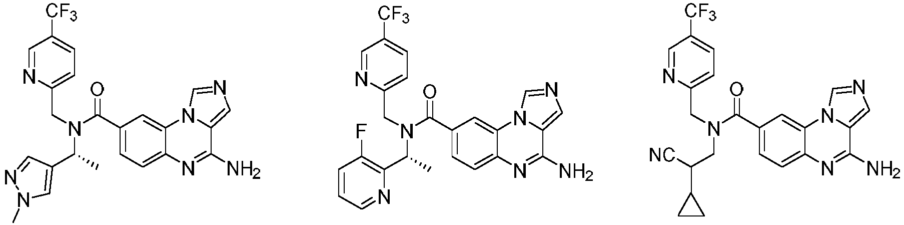

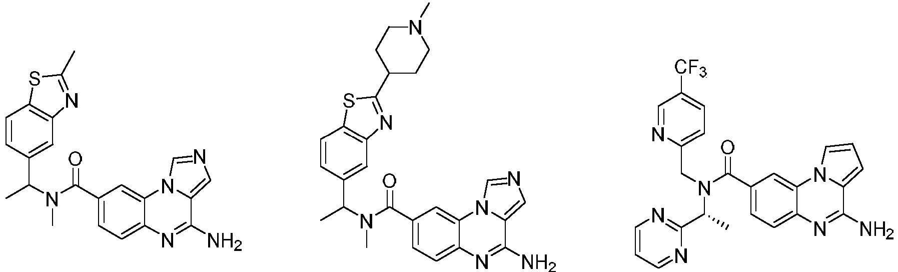

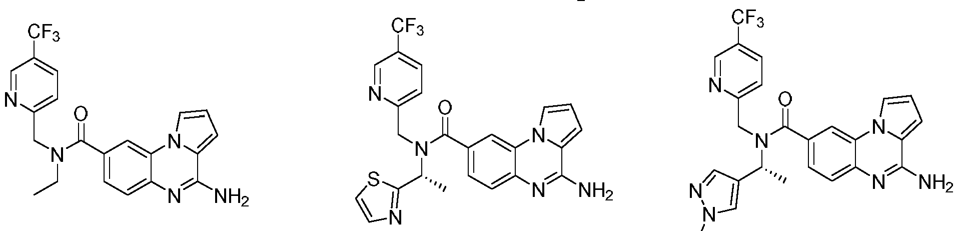

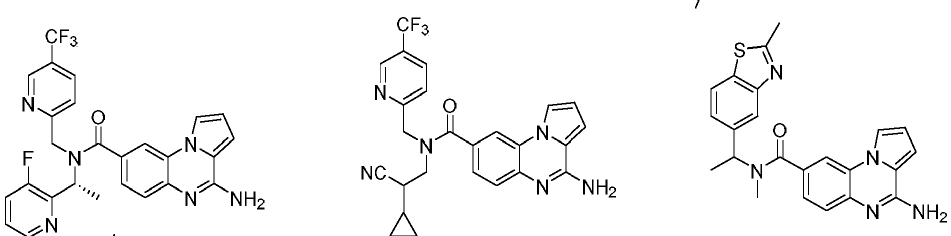

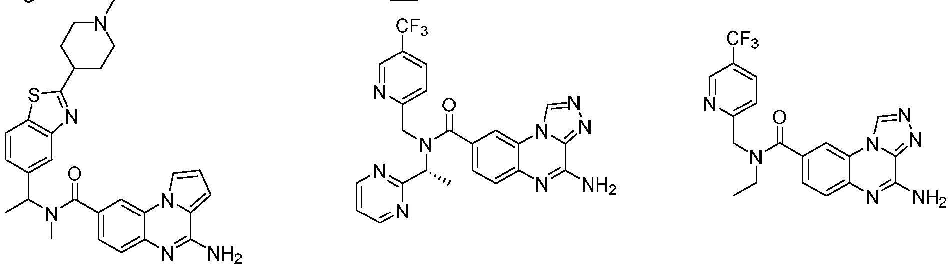

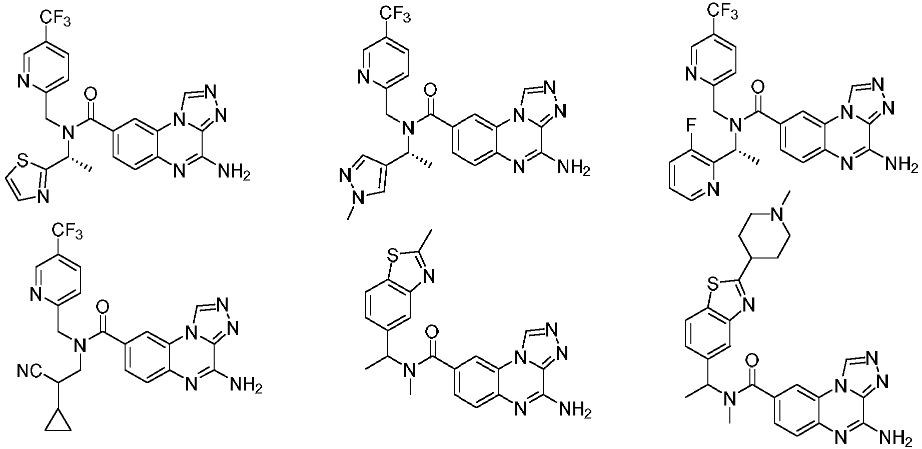

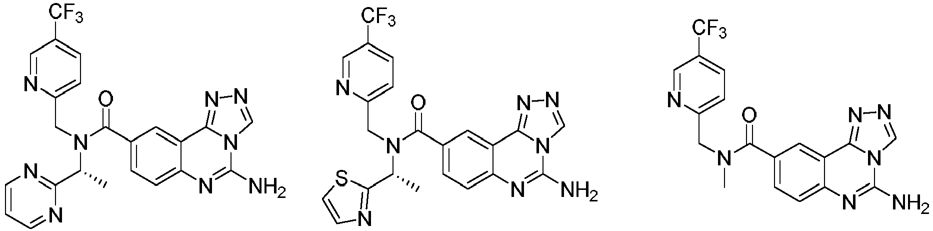

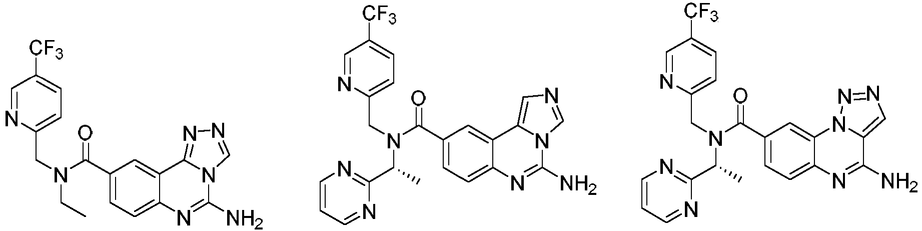

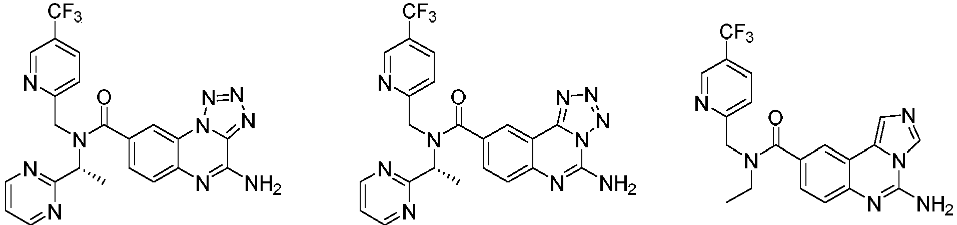

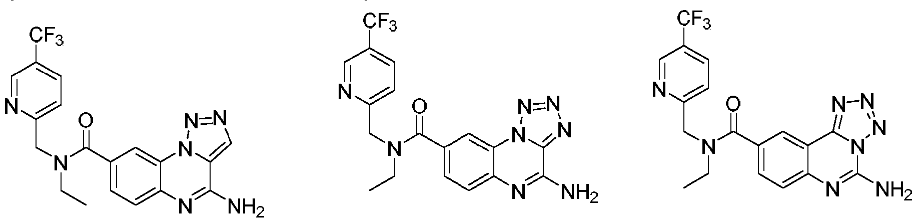

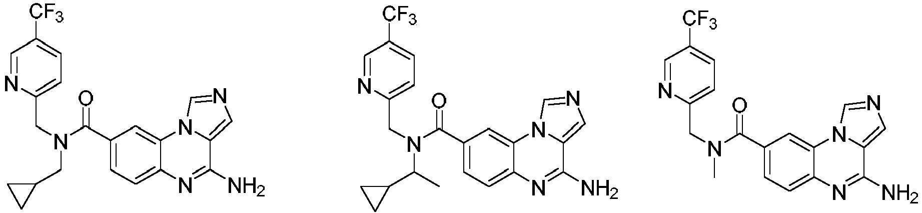

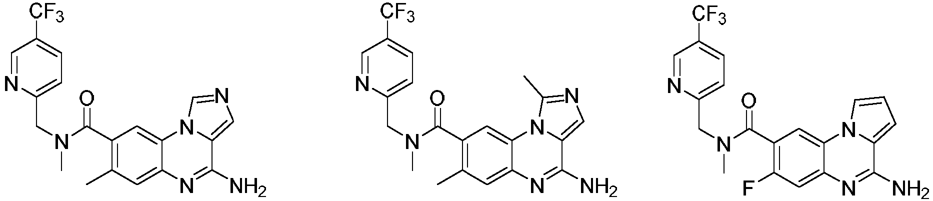

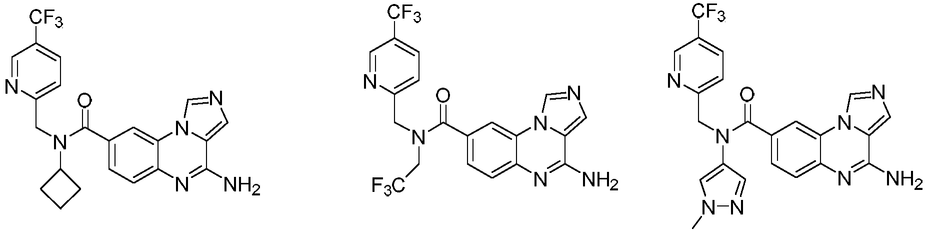

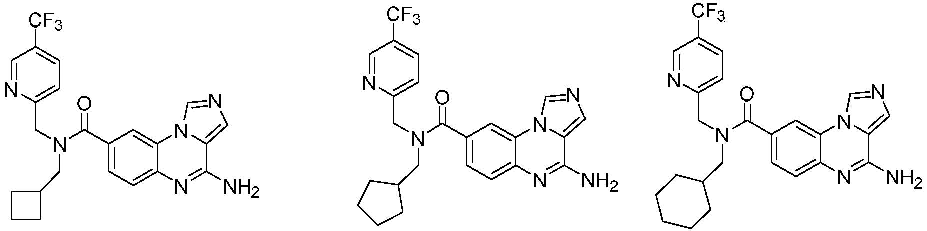

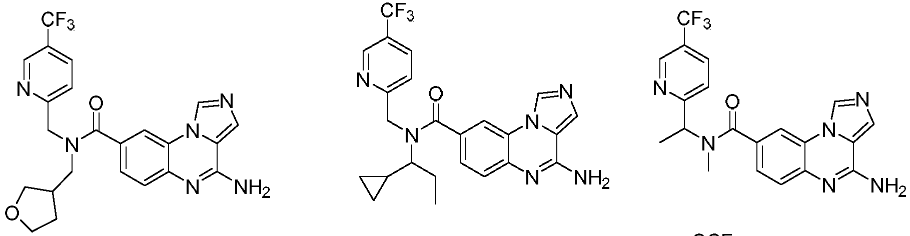

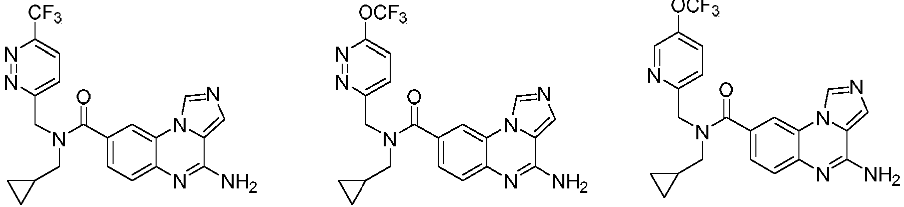

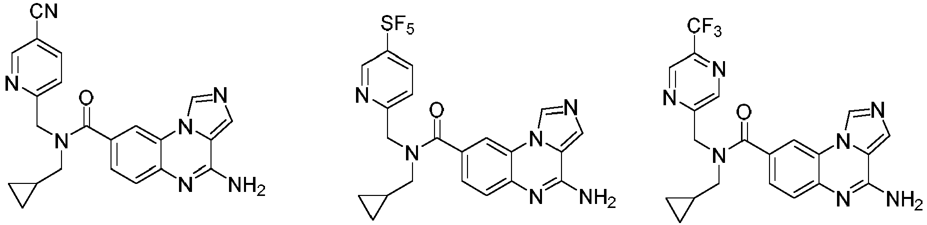

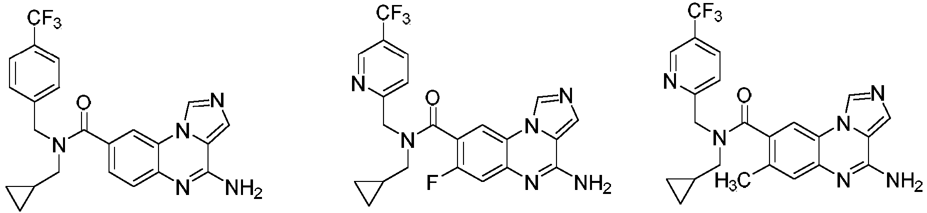

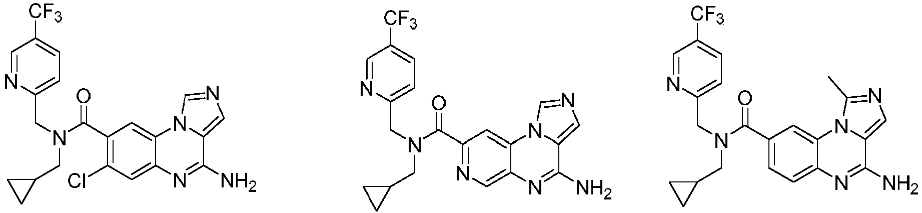

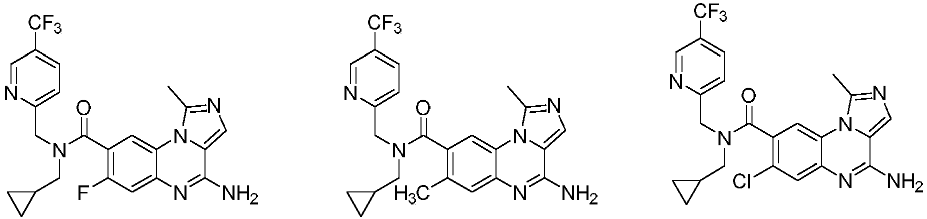

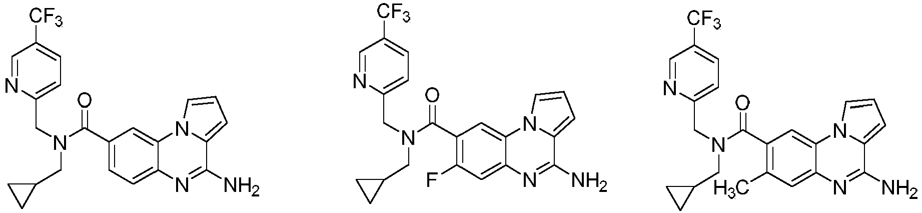

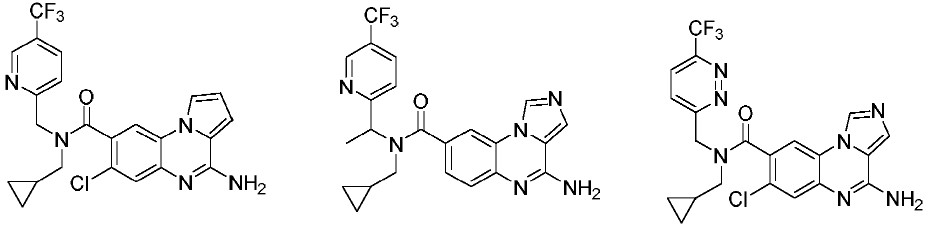

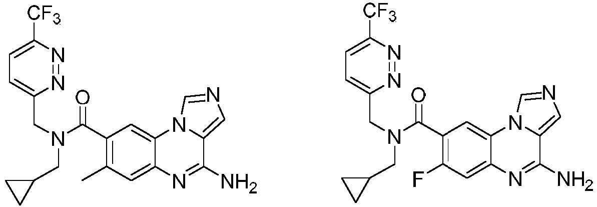

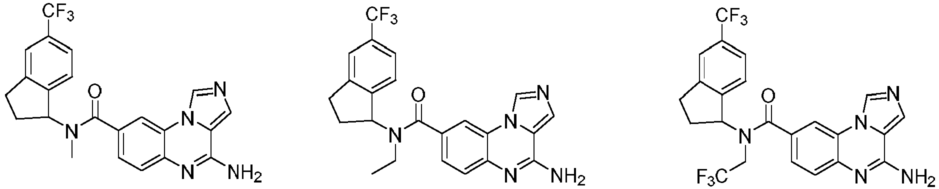

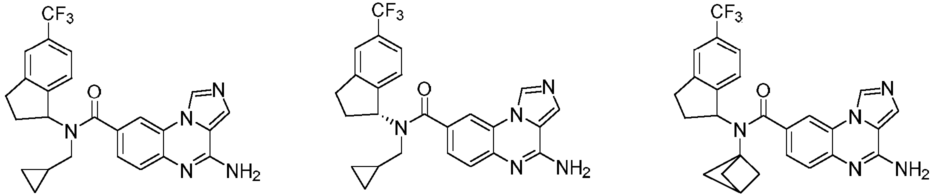

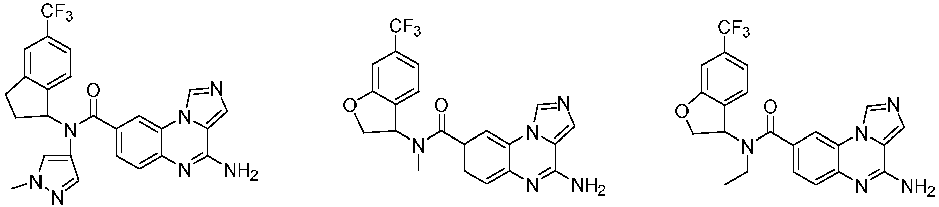

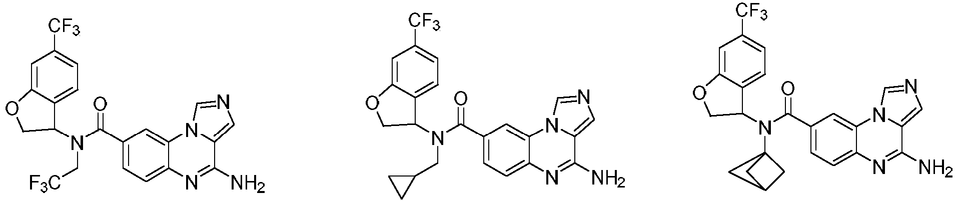

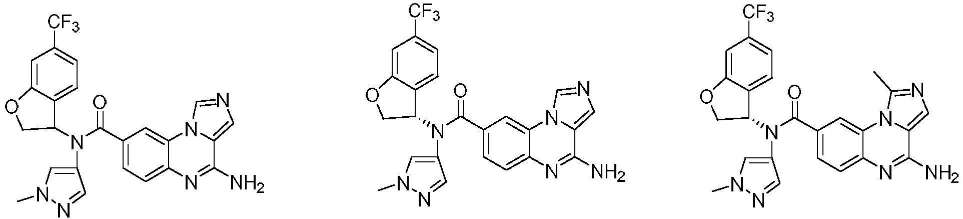

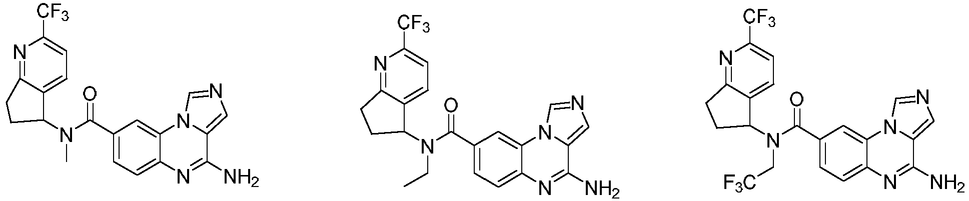

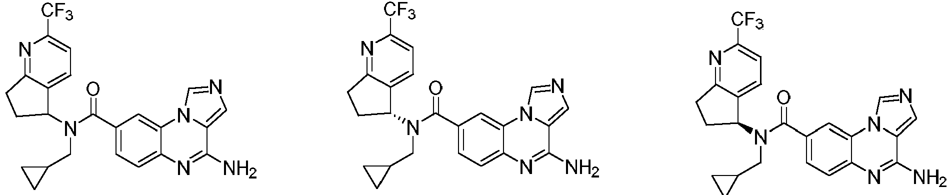

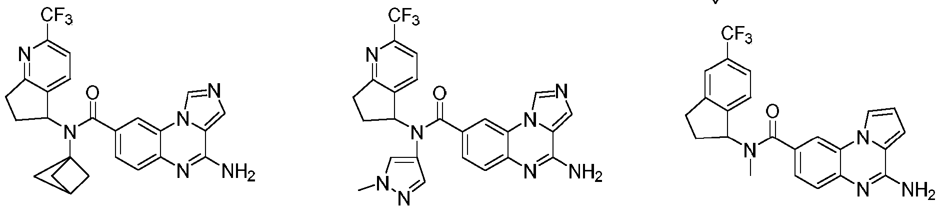

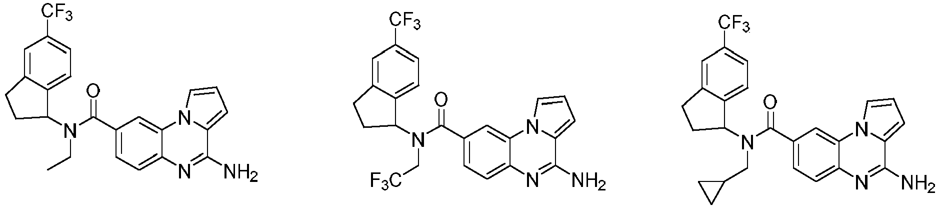

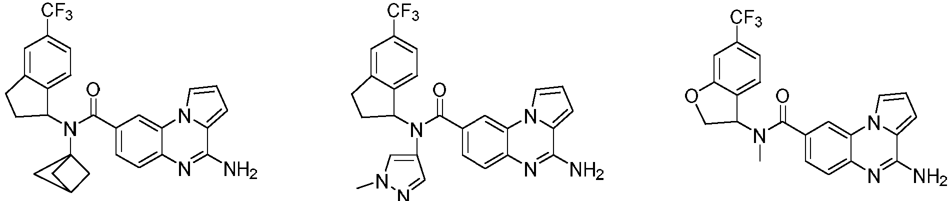

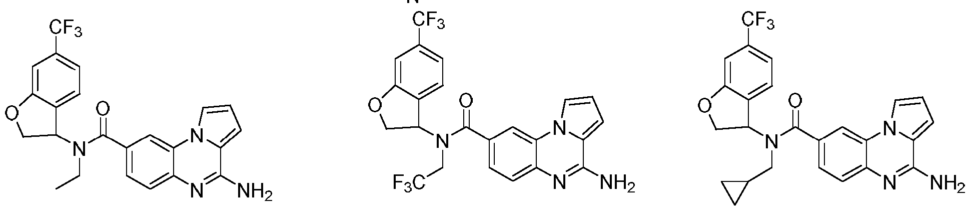

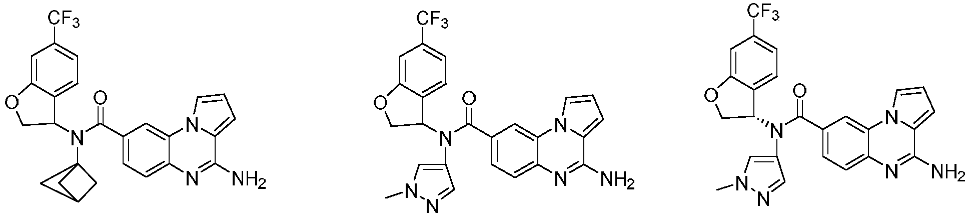

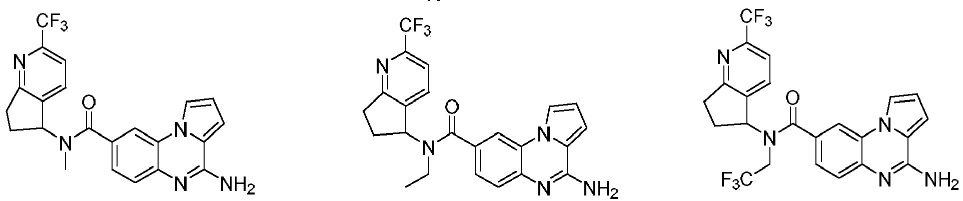

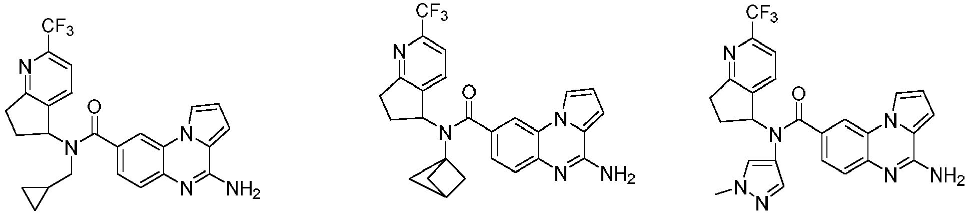

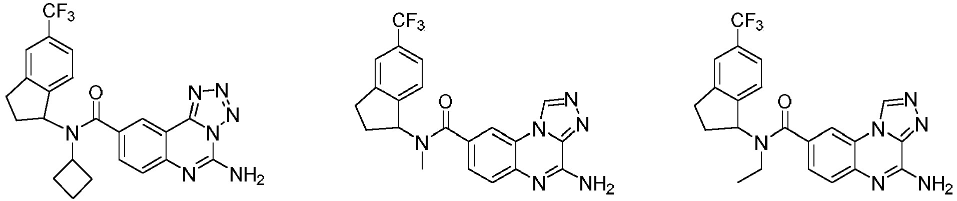

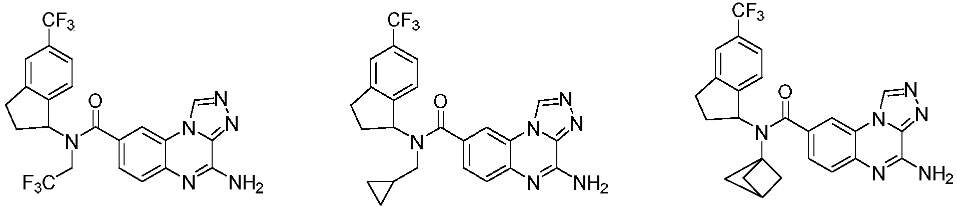

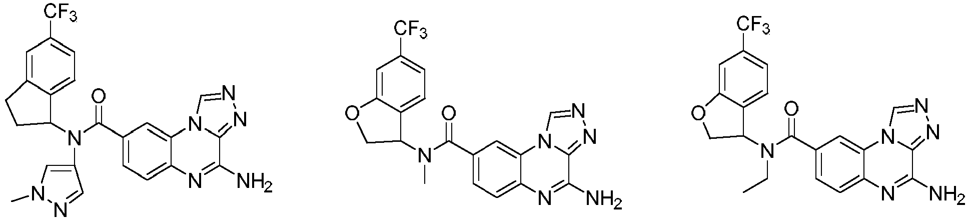

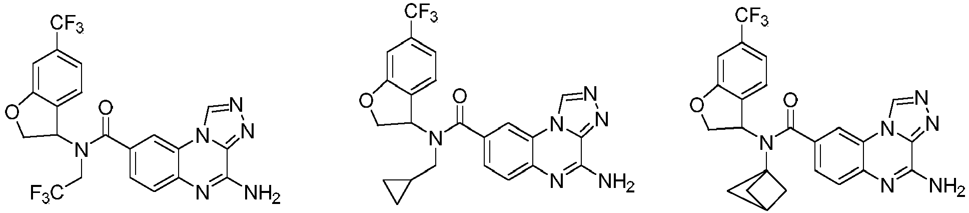

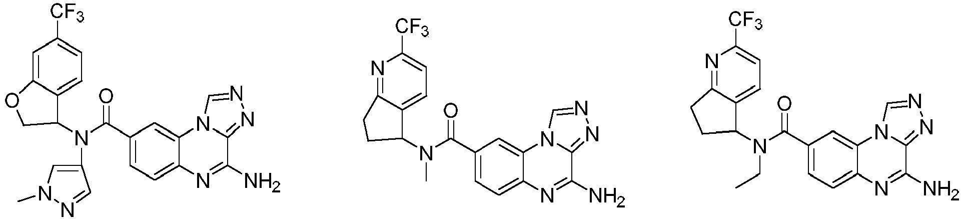

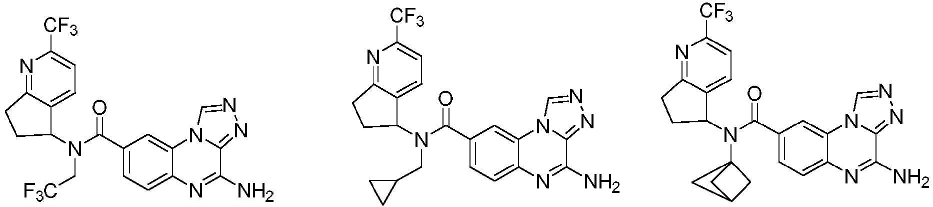

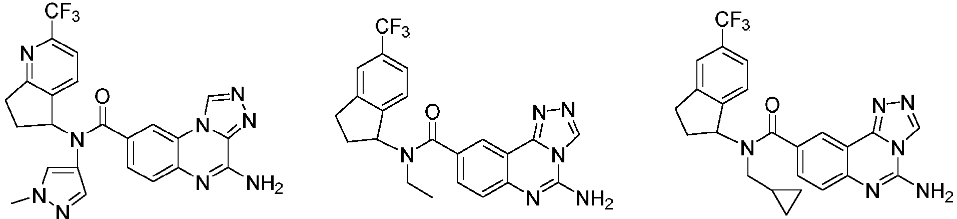

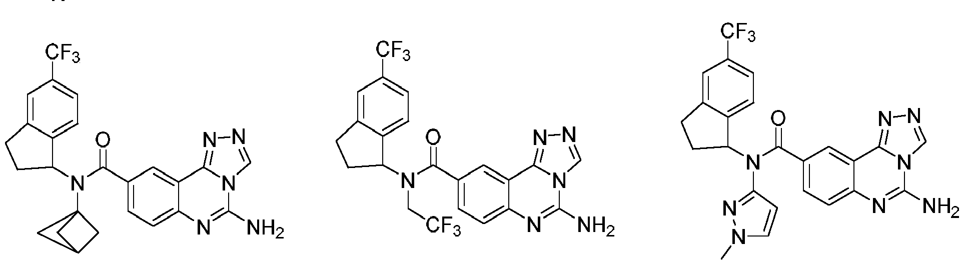

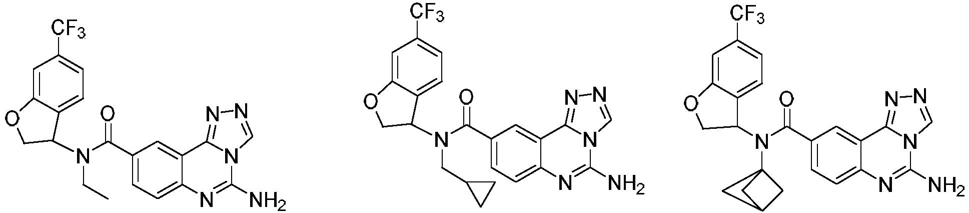

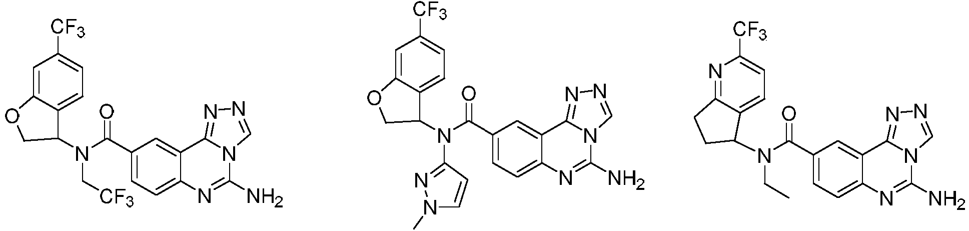

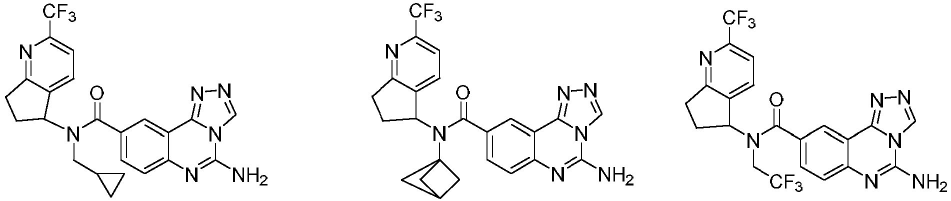

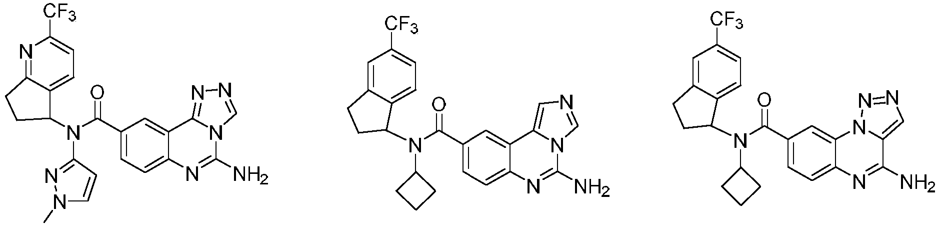

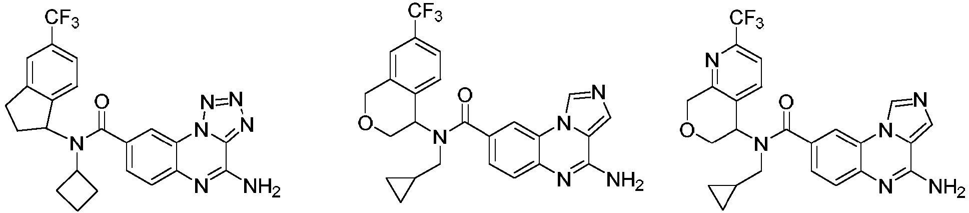

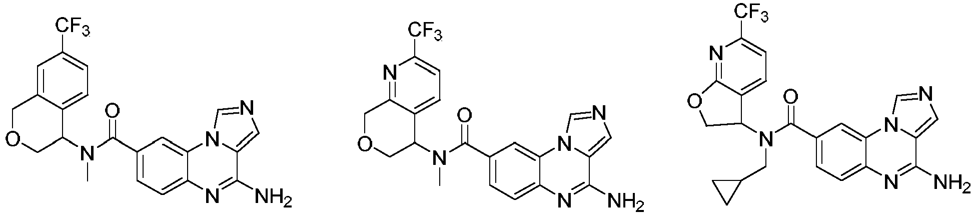

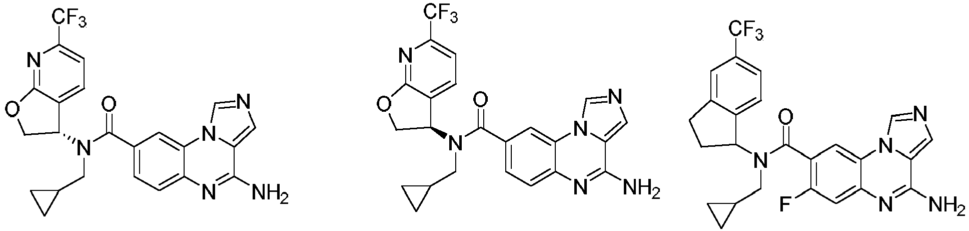

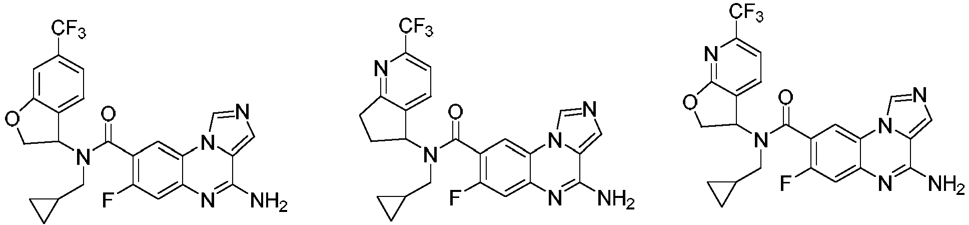

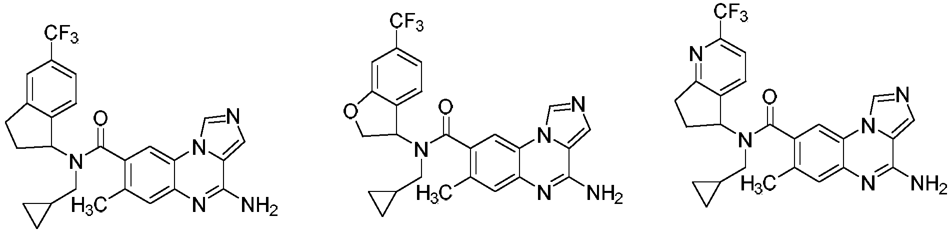

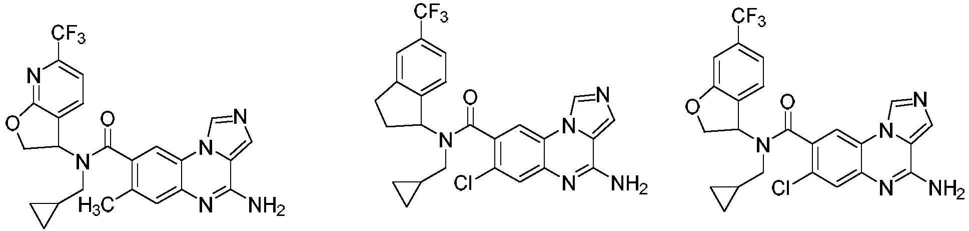

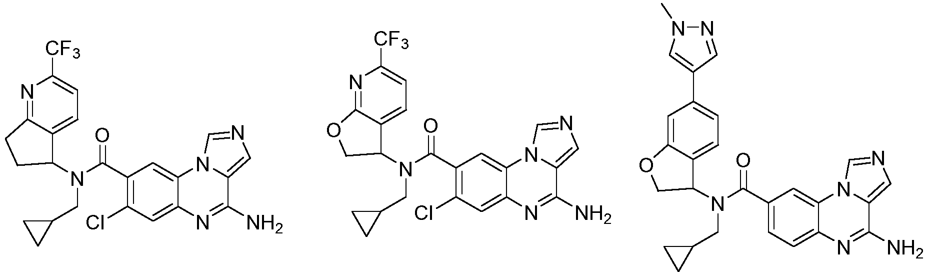

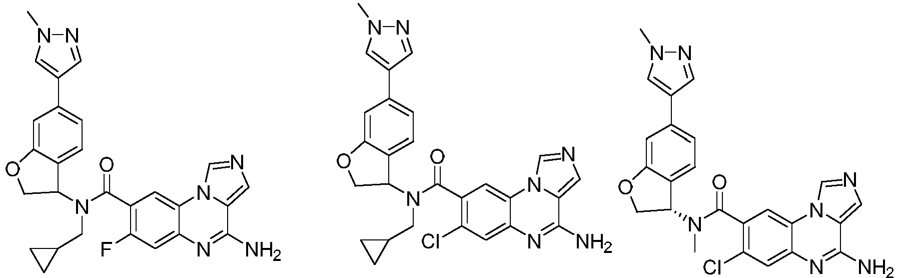

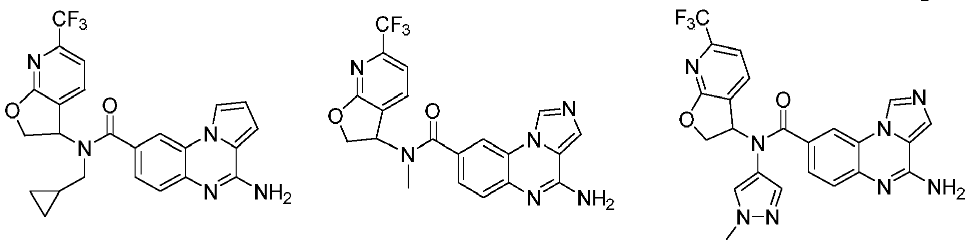

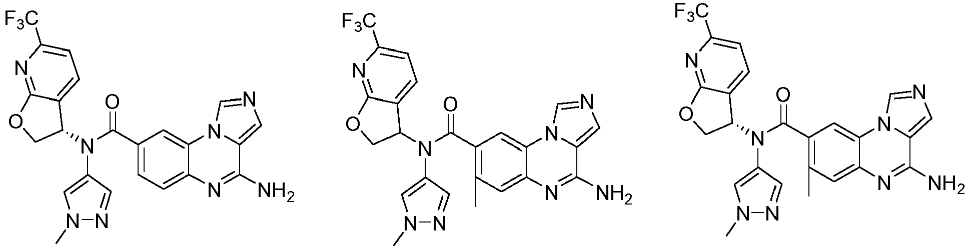

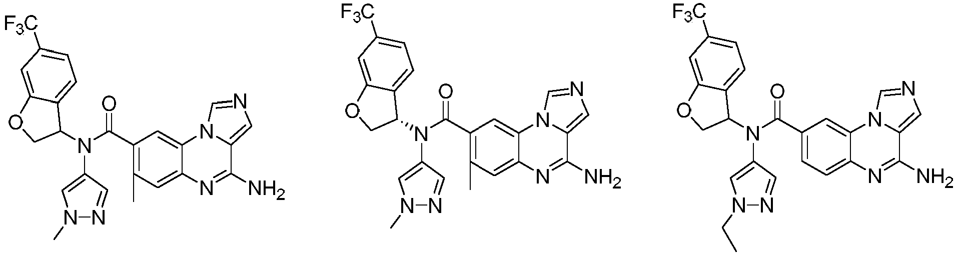

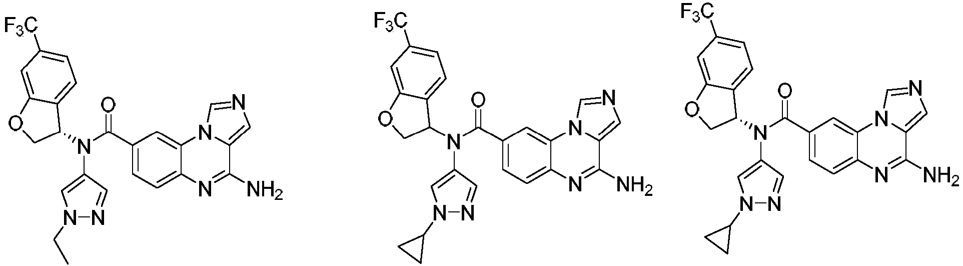

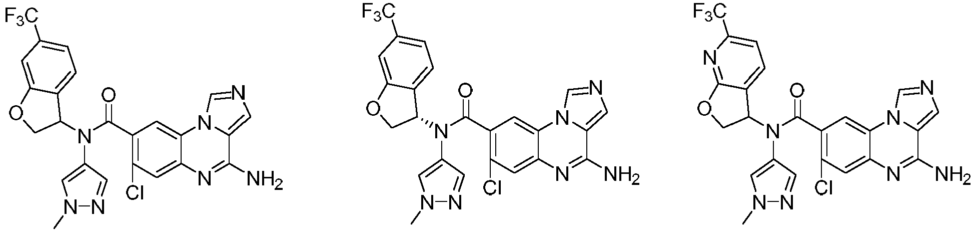

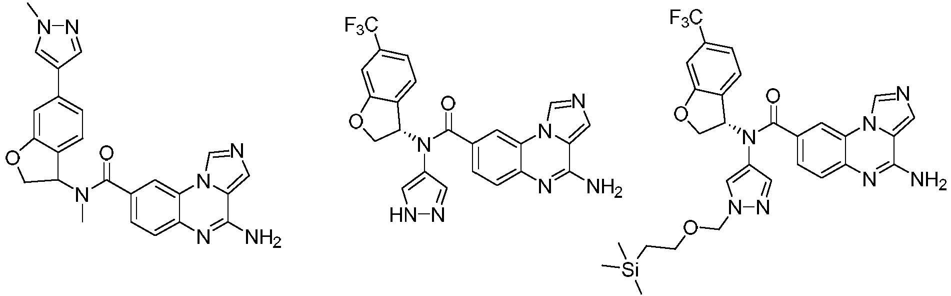

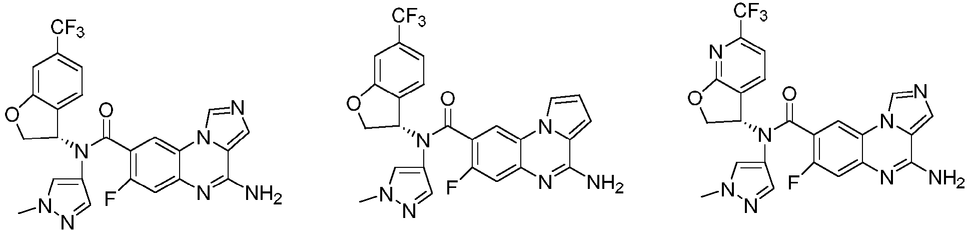

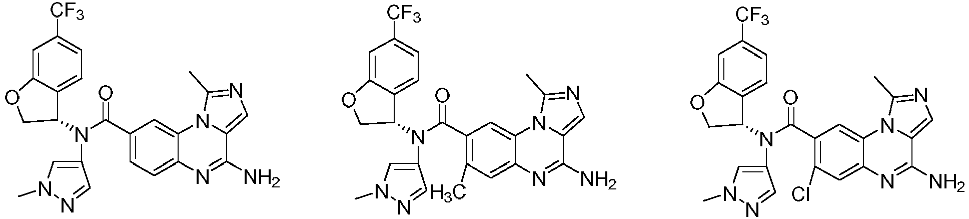

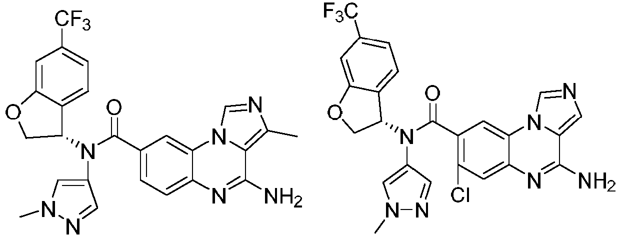

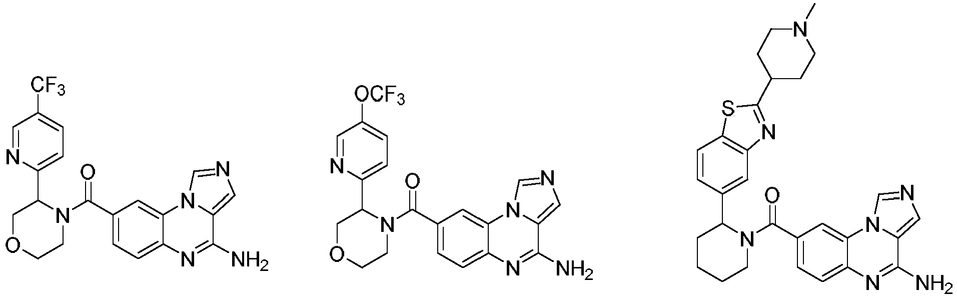

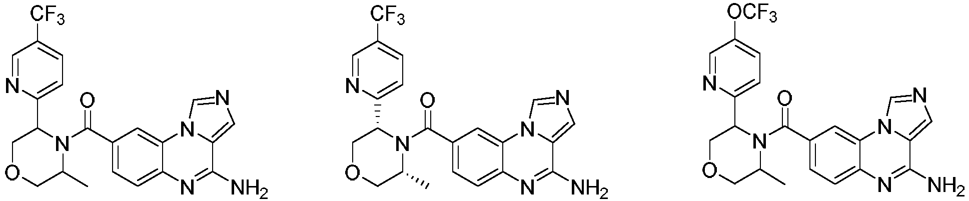

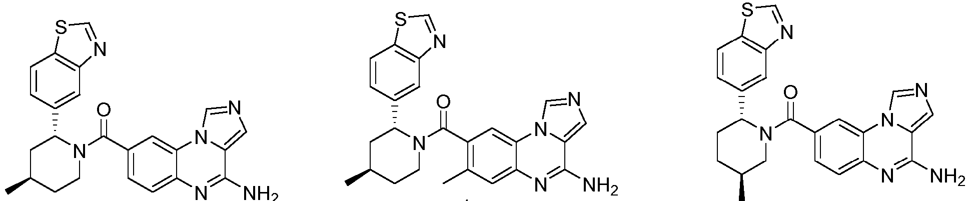

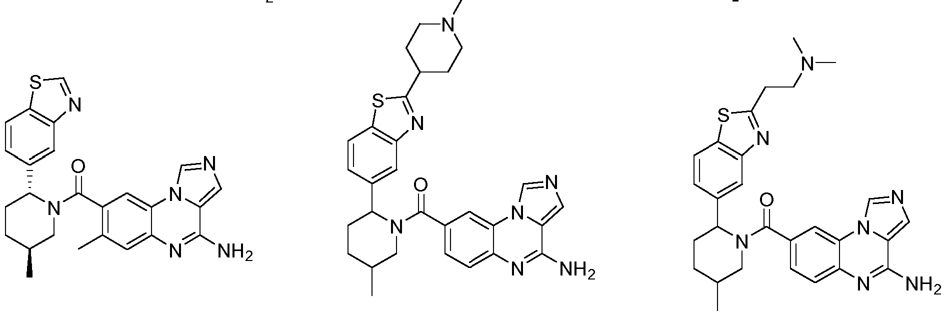

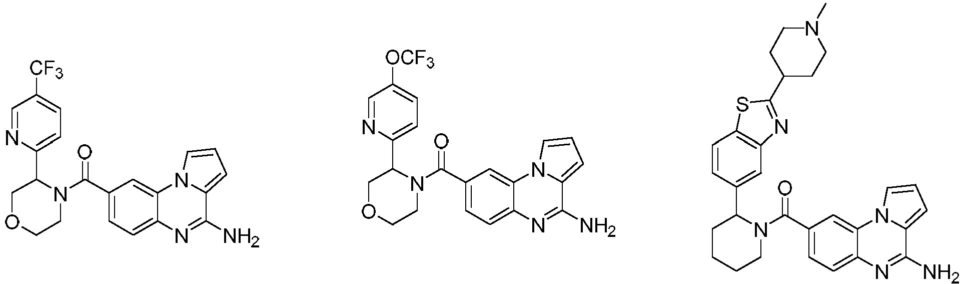

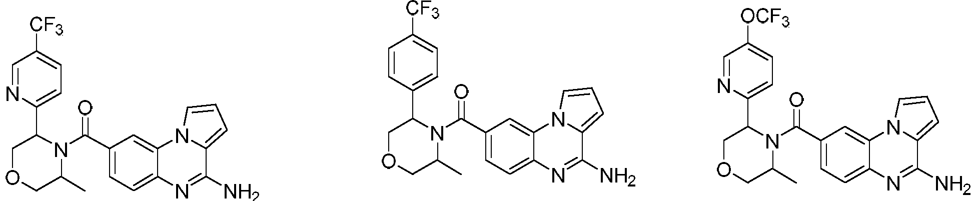

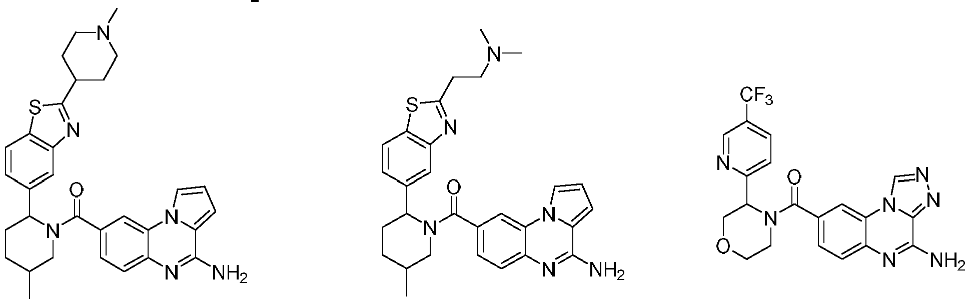

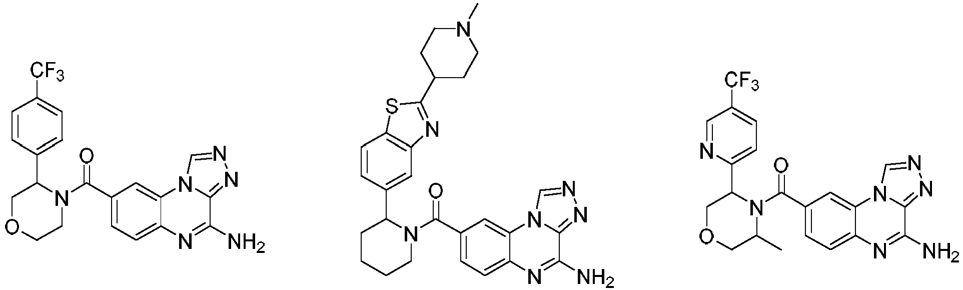

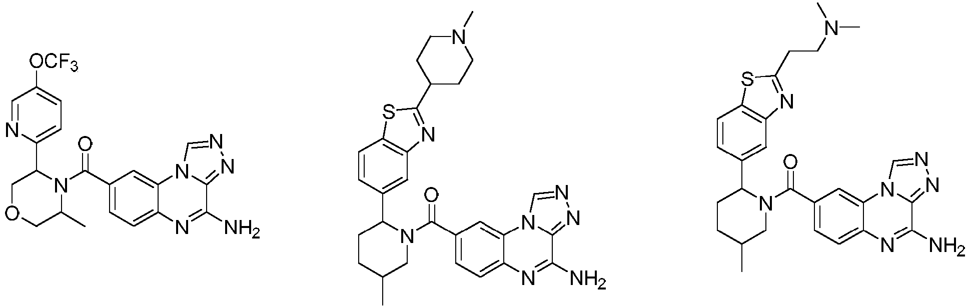

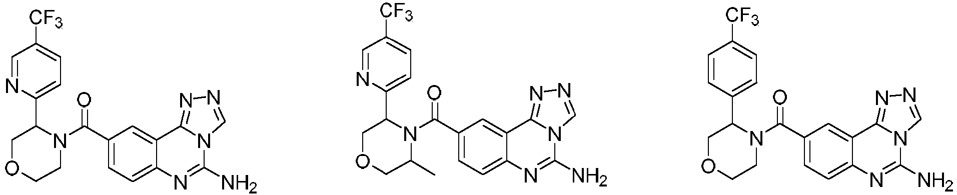

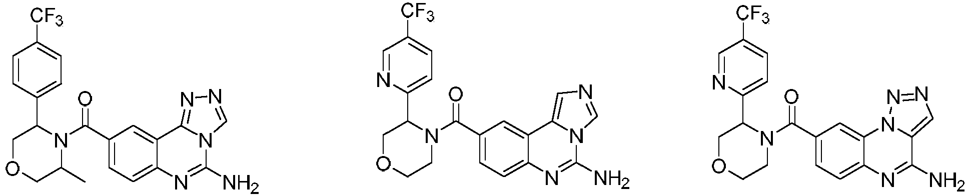

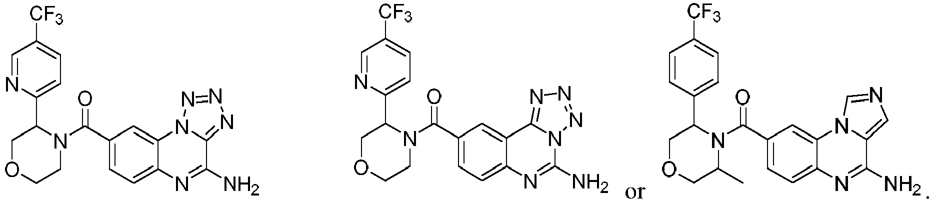

In a second aspect, the present invention provides a pharmaceutical composition, which comprises the compound of formula (I), the stereoisomer or pharmaceutically acceptable salt thereof, and a pharmaceutically acceptable carrier.

In a third aspect, the present invention provides a use of the compound of formula (I), the stereoisomer or pharmaceutically acceptable salt thereof in the preparation of a medicament for treating a MATP-related tumor.

As a further preferred embodiment, the tumor is cytoma, lymphoma, leukemia, osteoma, malignant teratoma, intraepithelial cancer, adenoma, fibroma, melanoma, fallopian tube cancer, bladder cancer, teratoma, embryonal carcinoma, choriocarcinoma, lipoma, liver cancer, cholangiocarcinoma, lung cancer, gastric cancer, hemangioma, gallbladder cancer, ampulla cancer, malignant melanoma, nevi, dysplastic nevi, myeloproliferative disease, Hodgkin's disease, chordoma, myxoma, rhabdomyoma, leiomyoma, hamartoma, mesothelioma, insulinoma, glucagonoma, gastrinoma, carcinoid tumor, vipoma, granuloma, xanthoma, osteitis deformans, ependymoma, schwanoma, congenital tumor, meningioma, glioma, skin cancer, head and neck cancer and sarcoma.

As a further preferred embodiment, the cytoma is granulosa-thecal cell tumor, sertoli cell tumor, germ cell tumor, nephroblastoma, seminoma, hepatoblastoma, malignant fibrous histiocytoma, chondroblastoma, giant cell tumor, astrocytoma, medulloblastoma, glioblastoma multiforme, oligodendroglioma, retinoblastoma, squamous cell carcinoma, clear cell carcinoma, transitional cell carcinoma, interstitial cell carcinoma and basal cell carcinoma;
the lymphoma is malignant lymphoma and non-Hodgkin lymphoma;
the leukemia is acute and chronic myeloid leukemia, acute lymphoblastic leukemia and chronic lymphoblastic leukemia;
the osteoma is osteochondroma, benign chondroma, osteoidosteoma, chondroma-like hamartoma, multiple myeloma and skull tumor;
the adenoma is fibroadenoma, adenomatoid tumor, hepatocellular adenoma, bronchial adenoma, tubular adenoma, villous adenoma, breast cancer, pancreatic cancer, endometrial adenocarcinoma, prostate cancer, ductal adenocarcinoma and colorectal adenocarcinoma;
the fibroma is fibroma, chondromyxoid fibroma, neurofibroma and spinal neurofibroma;
the myeloproliferative disease is multiple myeloma and myelodysplastic syndrome;
the lung cancer is bronchogenic carcinoma and alveolar carcinoma;
the sarcoma is fibrosarcoma, botryoid sarcoma, angiosarcoma, Kaposi's sarcoma, osteosarcoma, chondrosarcoma, Ewing's sarcoma, rhabdomyosarcoma, liposarcoma, leiomyosarcoma and meningosarcoma.

The present invention also relates to the compound of formula (I), the stereoisomer or pharmaceutically acceptable salt thereof for use as a PRMT5 inhibitor.

The present invention also relates to a use of the compound of formula (I), the stereoisomer or pharmaceutically acceptable salt thereof in the preparation of a medicament for treating and/or preventing PRMT5-mediated diseases.

The present invention also relates to a method for treating and/or preventing PRMT5-mediated diseases, which comprises administering to a patient in need thereof a therapeutically effective amount of the compound of formula (I), the stereoisomer or pharmaceutically acceptable salt thereof.

### DETAILED DESCRIPTION OF THE INVENTION

After an extensive and intensive research, the inventors of the present application have developed, for the first time, a PRMT5 inhibitor with a structure shown as formula (I). The series of compounds of the present invention can be widely applied to the preparation of medicaments for treating and/or preventing PRMT5-mediated diseases and are expected to be developed into a new generation of PRMT5 inhibitors. The present invention is achieved on this basis.

Detailed description: unless otherwise stated or specified, the following terms used in the specification and claims have the following meanings.

"Alkyl" refers to linear or branched saturated aliphatic alkyl groups, preferably linear alkyl or branched alkyl containing 1 to 10, 1 to 6 or 1 to 4 carbon atoms, including but not limited to methyl, ethyl, *n*-propyl, isopropyl, *n*-butyl, isobutyl, *tert*-butyl, *sec*-butyl, *n*-pentyl, 1,1-dimethylpropyl, 1,2-dimethylpropyl, 2,2-dimethylpropyl, 1-ethylpropyl, 2-methylbutyl, 3-methylbutyl, *n*-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, *n*-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, *n*-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl or various branched isomers thereof, and the like. "C₁₋₁₀ alkyl" refers to linear alkyl and branched alkyl containing 1 to 10 carbon atoms, "C₁₋₄ alkyl" refers to linear alkyl and branched alkyl containing 1 to 4 carbon atoms, "C₀₋₈ alkyl" refers to linear alkyl and branched alkyl containing 0 to 8 carbon atoms, and "C₀₋₄ alkyl" refers to linear alkyl and branched alkyl containing 0 to 4 carbon atoms.

Alkyl may be optionally substituted or unsubstituted, and when it is substituted, the substituent is preferably one or more (preferably 1, 2, 3 or 4) groups independently selected from the group consisting of deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, =O, =S, -C₀₋₈ alkyl-SF₅, -C₀₋₈ alkyl-O-S(O)₂R₁₃, -C₀₋₈ alkylS(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, - C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(O)NR₁₆R₁₇ and -C₀₋₈ alkyl-N(R₁₆)-C(O)R₁₅.

"Cycloalkyl" or "carbocycle" refers to a monocyclic or polycyclic hydrocarbon substituent that is saturated or partially unsaturated. The partially unsaturated cyclic hydrocarbon means that the cyclic hydrocarbon may contain one or more (preferably 1, 2 or 3) double bonds, but none of the rings has a fully conjugated π-electron system; cycloalkyl is classified into monocyclic cycloalkyl and polycyclic cycloalkyl, and is preferably cycloalkyl containing 3 to 12, 3 to 8, or 3 to 6 carbon atoms; for example, "C₃₋₁₂ cycloalkyl" refers to cycloalkyl containing 3 to 12 carbon atoms, "C₄₋₈ cycloalkyl" refers to cycloalkyl containing 4 to 8 carbon atoms, "C₃₋₈ cycloalkyl" refers to cycloalkyl containing 3 to 8 carbon atoms, and "C₃₋₆ cycloalkyl" refers to cycloalkyl containing 3 to 6 carbon atoms, wherein:
monocyclic cycloalkyl includes, but is not limited to, cyclopropyl, cyclobutyl, cyclopentyl, cyclopentenyl, cyclohexyl, cyclohexenyl, cyclohexadienyl, cycloheptyl, cycloheptatrienyl, cyclooctyl and the like;
polycyclic cycloalkyl includes spirocycloalkyl, fused cycloalkyl and bridged cycloalkyl. "Spirocycloalkyl" refers to a polycyclic group in which a carbon atom (called a spiro-atom) is shared among monocyclic rings, wherein those rings may contain one or more (preferably, 1, 2 or 3) double bonds, but none of them has a fully conjugated π-electron system. According to the number of the spiro-atoms shared among the rings, the spirocycloalkyl may be monospirocycloalkyl, bispirocycloalkyl or polyspirocycloalkyl, including but not limited to:

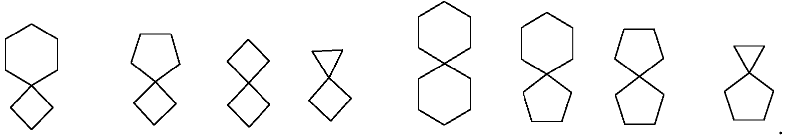

"Fused cycloalkyl" refers to an all-carbon polycyclic group in which each ring shares a pair of adjacent carbon atoms with the other rings in the system, wherein one or more of the rings may contain one or more (preferably, 1, 2 or 3) double bonds, but none of them has a fully conjugated π-electron system. According to the number of formed rings, the fused cycloalkyl may be bicyclic, tricyclic, tetracyclic or polycyclic, including but not limited to:

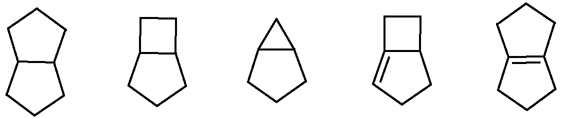

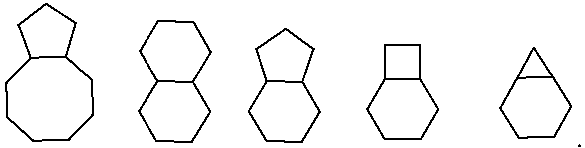

"Bridged cycloalkyl" refers to an all-carbon polycyclic group in which any two rings share two carbon atoms that are not directly connected to each other, wherein these rings may contain one or more (preferably, 1, 2 or 3) double bonds, but none of them has a fully conjugated π-electron system. According to the number of formed rings, the bridged cycloalkyl may be bicyclic, tricyclic, tetracyclic or polycyclic, including but not limited to:

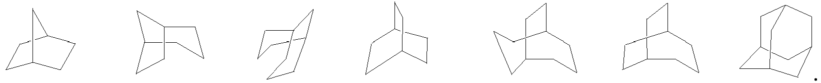

The cycloalkyl ring can be fused to an aryl, heteroaryl or heterocycloalkyl ring, wherein the ring attached to the parent structure is cycloalkyl, which includes, but is not limited to, indanyl, tetrahydronaphthyl, benzocycloheptyl and the like.

The "cycloalkyl" or "carbocycle" may be optionally substituted or unsubstituted, and when it is substituted, the substituent is preferably one or more (preferably 1, 2, 3 or 4) groups independently selected from the group consisting of deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, =O, =S, -C₀₋₈ alkyl-SF₅, -C₀₋₈ alkyl-O-S(O)₂R₁₃, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, -C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(O)NR₁₆R₁₇ and -C₀₋₈ alkyl-N(R₁₆)-C(O)R₁₅.

"Heterocyclyl" or "heterocycle" refers to a monocyclic or polycyclic hydrocarbon substituent that is saturated or partially unsaturated. The partially unsaturated cyclic hydrocarbon means that the cyclic hydrocarbon may contain one or more (preferably 1, 2 or 3) double bonds, but none of the rings has a fully conjugated π-electron system. One or more (preferably 1, 2, 3 or 4) ring atoms in the heterocyclyl are selected from heteroatoms N, O, N•O and S(O)ᵣ (where r is an integer of 0, 1 or 2), excluding ring moiety of -O-O-, -O-S- or -S-S-, and the remaining ring atoms are carbon atoms. Preferred is heterocyclyl comprising 3 to 12, 3 to 8 or 3 to 6 ring atoms. For example, "3-6 membered heterocyclyl" refers to heterocyclyl containing 3 to 6 ring atoms, "3-8 membered heterocyclyl" refers to heterocyclyl containing 3 to 8 ring atoms, "4-8 membered heterocyclyl" refers to heterocyclyl containing 4 to 8 ring atoms, "4-10 membered heterocyclyl" refers to heterocyclyl containing 4 to 10 ring atoms, "5-8 membered heterocyclyl" refers to heterocyclyl containing 5 to 8 ring atoms, and "3-12 membered heterocyclyl" refers to heterocyclyl containing 3 to 12 ring atoms.

Monocyclic heterocyclyl includes, but is not limited to, pyrrolidinyl, piperidinyl, piperazinyl, morpholinyl, thiomorpholinyl, homopiperazinyl, oxetanyl, tetrahydrofuryl and the like.

Polycyclic heterocyclyl includes spiroheterocyclyl, fused heterocyclyl and bridged heterocyclyl. "Spiroheterocyclyl" refers to a polycyclic heterocyclyl group in which an atom (called a spiro-atom) is shared among monocyclic rings, wherein one or more (preferably 1, 2, 3 or 4) ring atoms are heteroatoms selected from N, O, N•O and S(O)ᵣ (where r is an integer of 0, 1 or 2), and the remaining ring atoms are carbon atoms. These rings may contain one or more (preferably, 1, 2 or 3) double bonds, but none of them has a fully conjugated π-electron system. According to the number of spiro-atoms shared among the rings, the spiroheterocyclyl may be monospiroheterocyclyl, bispiroheterocyclyl or polyspiroheterocyclyl. Spiroheterocyclyl includes, but is not limited to:

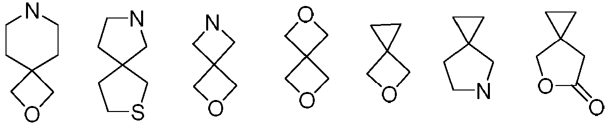

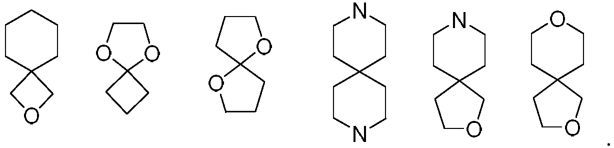

"Fused heterocyclyl" refers to a polycyclic heterocyclyl group in which each ring shares a pair of adjacent atoms with the other rings in the system, wherein one or more (preferably, 1, 2, 3 or 4) of the rings may contain one or more (preferably, 1, 2 or 3) double bonds, but none of them has a fully conjugated π-electron system, wherein one or more (preferably, 1, 2, 3 or 4) ring atoms are heteroatoms selected from N, O, N•O and S(O)ᵣ (where r is an integer of 0, 1 or 2), and the remaining ring atoms are carbon atoms. According to the number of formed rings, the fused heterocycloalkyl may be bicyclic, tricyclic, tetracyclic or polycyclic, including but not limited to:

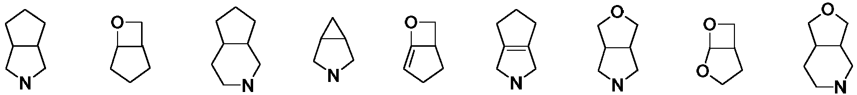

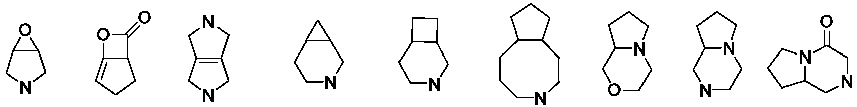

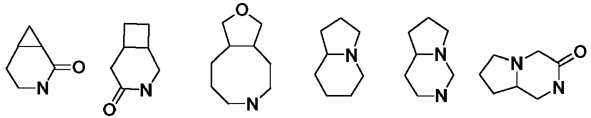

"Bridged heterocyclyl" refers to a polycyclic heterocyclyl group in which any two rings share two atoms that are not directly connected to each other, wherein these rings may contain one or more (preferably, 1, 2 or 3) double bonds, but none of them has a fully conjugated π-electron system, wherein one or more (preferably, 1, 2, 3 or 4) ring atoms are heteroatoms selected from N, O, N•O and S(O)ᵣ (where r is an integer of 0, 1 or 2), and the remaining ring atoms are carbon atoms. According to the number of formed rings, the bridged heterocyclyl may be bicyclic, tricyclic, tetracyclic or polycyclic, including but not limited to:

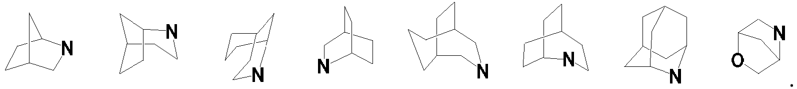

The heterocyclyl ring may be fused to an aryl, heteroaryl or cycloalkyl ring, wherein the ring attached to the parent structure is heterocyclyl, including but not limited to:

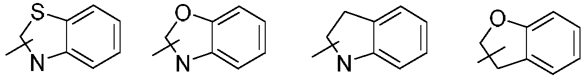

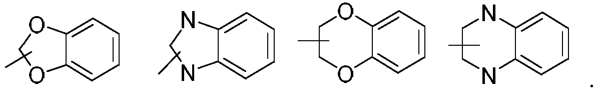

"Heterocyclyl" or "heterocycle" may be optionally substituted or unsubstituted, and when it is substituted, the substituent is preferably one or more (preferably 1, 2, 3 or 4) groups independently selected from the group consisting of deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, =O, =S, -C₀₋₈ alkyl-SF₅, -C₀₋₈ alkyl-O-S(O)₂R₁₃, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, -C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(O)NR₁₆R₁₇ and -C₀₋₈ alkyl-N(R₁₆)-C(O)R₁₅.

"Aryl" or "aromatic ring" refers to an all-carbon monocyclic or fused-polycyclic group (i.e., rings that share a pair of adjacent carbon atoms) and a polycyclic group having a conjugated π-electron system (i.e., rings with adjacent pairs of carbon atoms), and is preferably all-carbon aryl containing 6 to 10 or 6 to 8 carbon atoms. For example, "C₆₋₁₀ aryl" refers to all-carbon aryl containing 6 to 10 carbon atoms, including but not limited to phenyl and naphthyl, and "C₆₋₈ aryl" refers to all-carbon aryl containing 6 to 8 carbon atoms. The aryl ring can be fused to a heteroaryl, heterocyclyl or cycloalkyl ring, wherein the ring attached to the parent structure is the aryl ring, including but not limited to:

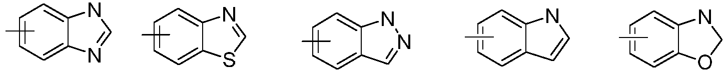

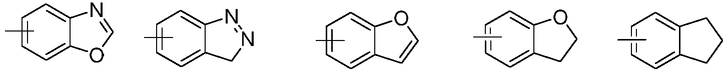

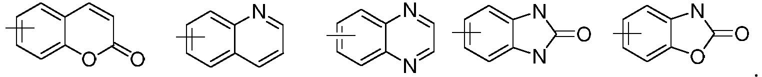

"Aryl" or "aromatic ring" may be substituted or unsubstituted, and when it is substituted, the substituent is preferably one or more (preferably 1, 2, 3 or 4) groups independently selected from the group consisting of deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, =O, =S, -C₀₋₈ alkyl-SF₅, -C₀₋₈ alkyl-O-S(O)₂R₁₃, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅,-C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(O)NR₁₆R₁₇ and -C₀₋₈ alkyl-N(R₁₆)-C(O)R₁₅.

"Heteroaryl" or "heteroaromatic ring" refers to a heteroaromatic system containing one or more (preferably 1, 2, 3 or 4) heteroatoms including N, O, N•O and S(O)ᵣ (where r is an integer of 0, 1 or 2), and is preferably a heteroaromatic system containing 5 to 10, 5 to 8, or 5 to 6 ring atoms. For example, "5-8 membered heteroaryl" refers to a heteroaromatic system containing 5 to 8 ring atoms, and "5-10 membered heteroaryl" refers to a heteroaromatic system containing 5 to 10 ring atoms. The heteroaryl includes, but is not limited to, furyl, thiophenyl, pyridyl, pyrrolyl, N-alkylpyrrolyl, pyrimidinyl, pyrazinyl, imidazolyl, tetrazolyl and the like. The heteroaryl ring can be fused to an aryl, heterocyclyl or cycloalkyl ring, wherein the ring attached to the parent structure is the heteroaryl ring, including but not limited to:

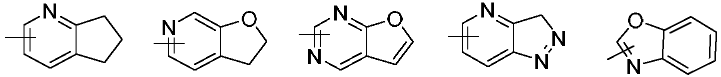

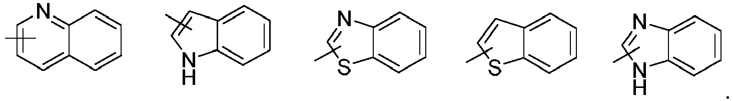

"Heteroaryl" or "heteroaromatic ring" may be optionally substituted or unsubstituted, and when it is substituted, the substituent is preferably one or more (preferably 1, 2, 3 or 4) groups independently selected from the group consisting of deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, =O, =S, -C₀₋₈ alkyl-SF₅, -C₀₋₈ alkyl-O-S(O)₂R₁₃, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, -C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(O)NR₁₆R₁₇ and -C₀₋₈ alkyl-N(R₁₆)-C(O)R₁₅.

"Alkenyl" refers to alkyl defined as above consisting of at least two carbon atoms and at least one carbon-carbon double bond, and is preferably linear or branched alkenyl containing 2 to 10 or 2 to 4 carbon atoms. For example, "C₂₋₁₀ alkenyl" refers to linear or branched alkenyl containing 2 to 10 carbon atoms, and "C₂₋₄ alkenyl" refers to linear or branched alkenyl containing 2 to 4 carbon atoms. Alkenyl includes, but is not limited to, vinyl, 1-propenyl, 2-propenyl, 1-, 2- or 3-butenyl, and the like.

"Alkenyl" may be substituted or unsubstituted, and when it is substituted, the substituent is preferably one or more (preferably 1, 2, 3 or 4) groups independently selected from the group consisting of deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, =O, =S, -C₀₋₈ alkyl-SF₅, -C₀₋₈ alkyl-O-S(O)₂R₁₃, -C₀₋₈ alkyl- S(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, - C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(O)NR₁₆R₁₇ and -C₀₋₈ alkyl-N(R₁₆)-C(O)R₁₅.

"Alkynyl" refers to alkyl defined as above consisting of at least two carbon atoms and at least one carbon-carbon triple bond, and is preferably linear or branched alkynyl containing 2 to 10 or 2 to 4 carbon atoms. For example, "C₂₋₁₀ alkynyl" refers to linear or branched alkynyl containing 2 to 10 carbon atoms, and "C₂₋₄ alkynyl" refers to linear or branched alkynyl containing 2 to 4 carbon atoms. Alkynyl includes, but is not limited to, ethynyl, 1-propynyl, 2-propynyl, 1-, 2- or 3-butynyl, and the like.

"Alkynyl" may be substituted or unsubstituted, and when it is substituted, the substituent is preferably one or more (preferably 1, 2, 3 or 4) groups independently selected from the group consisting of deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, =O, =S, -C₀₋₈ alkyl-SF₅, -C₀₋₈ alkyl-O-S(O)₂R₁₃, -C₀₋₈ alkyl- S(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, - C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(O)NR₁₆R₁₇ and -C₀₋₈ alkyl-N(R₁₆)-C(O)R₁₅.

"Alkoxy" refers to -O-alkyl, wherein the alkyl is defined as above. For example, "C₁₋₁₀ alkoxy" refers to alkoxy containing 1 to 10 carbon atoms, "C₁₋₄ alkoxy" refers to alkoxy containing 1 to 4 carbon atoms, and "C₁₋₂ alkoxy" refers to alkoxy containing 1 to 2 carbon atoms. The alkoxy includes, but is not limited to, methoxy, ethoxy, propoxy, butoxy and the like.

"Alkoxy" may be optionally substituted or unsubstituted, and when it is substituted, the substituent is preferably one or more (preferably 1, 2, 3 or 4) groups independently selected from the group consisting of deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, =O, =S, -C₀₋₈ alkyl-SF₅, -C₀₋₈ alkyl-O-S(O)₂R₁₃, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅,-C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(O)NR₁₆R₁₇ and -C₀₋₈ alkyl-N(R₁₆)-C(O)R₁₅.

"Cycloalkoxy" or "cycloalkyloxy" refers to -O-cycloalkyl, wherein the cycloalkyl is defined as above. For example, "C₃₋₁₂ cycloalkyloxy" refers to cycloalkyloxy containing 3 to 12 carbon atoms, and "C₃₋₆ cycloalkyloxy" refers to cycloalkyloxy containing 3 to 6 carbon atoms. The cycloalkyloxy includes, but is not limited to, cyclopropyloxy, cyclobutyloxy, cyclopentyloxy, cyclohexyloxy and the like.

"Cycloalkoxy" or "cycloalkyloxy" may be optionally substituted or unsubstituted, and when it is substituted, the substituent is preferably one or more (preferably 1, 2, 3 or 4) groups independently selected from the group consisting of deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, =O, =S, -C₀₋₈ alkyl-SF₅, -C₀₋₈ alkyl-O-S(O)₂R₁₃, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, -C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(O)NR₁₆R₁₇ and -C₀₋₈ alkyl-N(R₁₆)-C(O)R₁₅.

"Heterocycloxy" or "heterocyclyloxy" refers to -O-heterocyclyl, wherein the heterocyclyl is defined as above. Heterocyoxy or heterocyclyloxy includes, but is not limited to, azacyclobutyloxy, oxacyclobutyloxy, azacyclopentyloxy, nitrogen, oxacyclohexyloxy and the like.

"Heterocycloxy" or "heterocyclyloxy" may be optionally substituted or unsubstituted, and when it is substituted, the substituent is preferably one or more (preferably 1, 2, 3 or 4) groups independently selected from the group consisting of deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, =O, =S, -C₀₋₈ alkyl-SF₅, -C₀₋₈ alkyl-O-S(O)₂R₁₃, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, -C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(O)NR₁₆R₁₇ and -C₀₋₈ alkyl-N(R₁₆)-C(O)R₁₅.

"C₁₋₁₀ alkanoyl" refers to a monovalent atomic group which is obtained after hydroxy is removed from C₁₋₁₀ alkyl acid, and is also generally referred to as "C₀₋₉ alkyl-C(O)-". For example, "C₁ alkyl-C(O)-" refers to acetyl; "C₂ alkyl-C(O)-" refers to propionyl; and "C₃ alkyl-C(O)-" refers to butyryl or isobutyryl.

"-C₀₋₈ alkyl-O-S(O)₂R₁₃" means that the oxygen atom in -O-S(O)₂R₁₃ is connected to C₀₋₈ alkyl, wherein C₀₋₈ alkyl is defined as above.

"-C₀₋₈ alkyl-S(O)ᵣR₁₃" means that the sulfur atom in -S(O)ᵣR₁₃ is connected to C₀₋₈ alkyl, wherein C₀₋₈ alkyl is defined as above.

"-C₀₋₈ alkyl-O-R₁₄" means that the oxygen atom in -O-R₁₄ is connected to C₀₋₈ alkyl, wherein C₀₋₈ alkyl is defined as above.

"-C₀₋₈ alkyl-C(O)OR₁₄" means that the carbonyl in -C(O)OR₁₄ is connected to C₀₋₈ alkyl, wherein C₀₋₈ alkyl is defined as above.

"-C₀₋₈ alkyl-C(O)SR₁₄" means that the carbonyl in -C(O)SR₁₄ is connected to C₀₋₈ alkyl, wherein C₀₋₈ alkyl is defined as above.

"-C₀₋₈ alkyl-S-C(O)R₁₅" means that the sulfur atom in -S-C(O)R₁₅ is connected to C₀₋₈ alkyl, wherein C₀₋₈ alkyl is defined as above.

"-C₀₋₈ alkyl-C(O)R₁₅" means that the carbonyl in -C(O)R₁₅ is connected to C₀₋₈ alkyl, wherein C₀₋₈ alkyl is defined as above.

"-C₀₋₈ alkyl-O-C(O)R₁₅" means that the oxygen atom in -O-C(O)R₁₅ is connected to C₀₋₈ alkyl, wherein C₀₋₈ alkyl is defined as above.

"-C₀₋₈ alkyl-P(O)(R₁₅)₂" means that the phosphorus atom in -P(O)(R₁₅)₂ is connected to C₀₋₈ alkyl, wherein C₀₋₈ alkyl is defined as above.

"-C₀₋₈ alkyl-NR₁₆R₁₇" means that the nitrogen atom in -NR₁₆R₁₇ is connected to C₀₋₈ alkyl, wherein C₀₋₈ alkyl is defined as above.

"-C₀₋₈ alkyl-C(O)NR₁₆R₁₇" means that the carbonyl in -C(O)NR₁₆R₁₇ is connected to C₀₋₈ alkyl, wherein C₀₋₈ alkyl is defined as above.

"-C₀₋₈ alkyl-N(R₁₆)-C(O)R₁₅" means that the nitrogen atom in -N(R₁₆)-C(O)R₁₅ is connected to C₀₋₈ alkyl, wherein C₀₋₈ alkyl is defined as above.

"C₁₋₁₀ haloalkyl" refers to an alkyl group having 1 to 10 carbon atoms in which hydrogen on the alkyl is optionally substituted with a fluorine, chlorine, bromine or iodine atom, and includes, but is not limited to, difluoromethyl, dichloromethyl, dibromomethyl, trifluoromethyl, trichloromethyl, tribromomethyl and the like.

"C₁₋₁₀ haloalkoxy" refers to an alkoxy group having 1 to 10 carbon atoms in which hydrogen on the alkyl is optionally substituted with a fluorine, chlorine, bromine or iodine atom, and includes, but is not limited to, difluoromethoxy, dichloromethoxy, dibromomethoxy, trifluoromethoxy, trichloromethoxy, tribromomethoxy and the like.

"C₁₋₁₀ deuterioalkyl" refers to an alkyl group having 1 to 10 carbon atoms in which hydrogen on the alkyl is optionally substituted with a deuterium atom, and includes, but is not limited to, monodeuteriomethyl, dideuteriomethyl, trideuteriomethyl and the like.

"Halogen" means fluorine, chlorine, bromine or iodine, "EA" means ethanol, "PE" means petroleum ether, "EtOAc" means ethyl acetate, "DCM" means dichloromethane, "DMSO" means dimethyl sulfoxide, "Cs₂CO₃" means cesium carbonate, "ACN" means acetonitrile, "Pd(OH)₂" means palladium hydroxide on carbon, "MeOH" means methanol, "rt" means room temperature, "1,2-dichlorobenzene" means 1,2-dichlorobenzene, "N,N-dimethylaniline" means *N,N-*dimethylaniline, "POCl₃" means phosphorus oxychloride, "DIPEA" means *N,N-*diisopropylethylamine, "NMP" means N-methylpyrrolidone, "TFA" means trifluoroacetic acid, "LiOH" means lithium hydroxide, "THF" means tetrahydrofuran, "PyBrOP" means bromo-tris-pyrrolidino-phosphonium hexafluorophosphate, and "DMA" means *N*,*N*-dimethylacetamide.

The term "optional" or "optionally" means that the event or circumstance subsequently described may, but not necessarily, occur, and that the description includes instances where the event or circumstance occurs or does not occur, that is, instances where substitution occurs or does not occur. For example, "heterocyclyl group optionally substituted with alkyl" means that alkyl may be, but not necessarily, present, and that the description includes instances where the heterocyclyl group is or is not substituted with alkyl.

The term "substituted" means that one or more "hydrogen atoms" in the group are each independently substituted with a corresponding number of substituents. It goes without saying that a substituent is only in its possible chemical position and consistent with chemical valence bond theory, and those skilled in the art will be able to determine (by studies or theories) possible or impossible substitution without undue efforts. For example, it may be unstable when amino or hydroxy having free hydrogen is bound to a carbon atom having an unsaturated bond (such as olefin).

"Stereoisomers" refer to isomers produced by different spatial arrangements of atoms in molecules, and can be classified into *cis-trans* isomers and enantiomers and also into enantiomers and diastereomers. Stereoisomers resulting from rotation of single bonds are referred to as conformational stereo-isomers and sometimes also as rotamers. Stereoisomers resulting from bond lengths, bond angles, intramolecular double bonds, rings and the like are referred to as configuration stereo-isomers, and the configuration stereo-isomers are classified into two categories. Among them, isomers resulting from the fact that a double bond or a single bond of a ring-forming carbon atom cannot rotate freely are referred to as geometric isomers and also as *cis-trans* isomers, and the isomers are classified into *Z* and *E* configurations. For example, *cis*-2-butene and *trans*-2-butene are a pair of geometric isomers, and stereoisomers having different

optical rotation properties due to the absence of anti-axisymmetry in the molecule are referred to as optical isomers and are classified into *R* and *S* configurations. In the present invention, the term "stereoisomer" is understood to include one or more of the above enantiomers, configuration stereo-isomers and conformational stereo-isomers, unless otherwise specified.

"Pharmaceutically acceptable salt" used herein refers to pharmaceutically acceptable acid addition salts, including inorganic and organic acid salts, which may be prepared by methods known in the art.

"Pharmaceutical composition" refers to a mixture containing one or more of the compounds described herein or a physiologically/pharmaceutically acceptable salt or pro-drug thereof and other chemical components, for example, physiologically/pharmaceutically acceptable carriers and excipients. The purpose of the pharmaceutical composition is to promote the administration to an organism, facilitate the absorption of the active ingredient, and thereby exert biological activities.

**The present invention is further explained in detail below with reference to examples, which are not intended to limit the present invention, and the present invention is not merely limited to the contents of the examples.**

The compound structure of the present invention is determined by nuclear magnetic resonance (NMR) and/or liquid chromatography-mass spectrometry (LC-MS). The NMR chemical shift (δ) is given in parts per million (ppm). The NMR determination is conducted by using a Bruker AVANCE-400/500 nuclear magnetic resonance apparatus, with hexadeuterodimethyl sulfoxide (DMSO-*d₆*), tetradeuteromethanol (MeOH-*d*₄) and deuterated chloroform (CDCl₃) as determination solvents, and tetramethylsilane (TMS) as an internal standard.

The LC-MS determination is conducted by using an Agilent 6120 mass spectrometer. The HPLC determination is conducted by using an Agilent 1200 DAD high pressure liquid chromatograph (Sunfire C18 150 * 4.6 mm chromatographic column) and a Waters 2695-2996 high pressure liquid chromatograph (Gimini C18 150 * 4.6 mm chromatographic column).

Yantai Yellow Sea HSGF254 or Qingdao GF254 silica gel plate is adopted as a thin layer chromatography (TLC) silica gel plate. The specification adopted by the TLC is 0.15-0.20 mm, and the specification adopted by the thin layer chromatography for the separation and purification of products is 0.4-0.5 mm. The Yantai Yellow Sea silica gel of 200-300 mesh is generally utilized as a carrier in column chromatography.

Starting materials in the examples of the present invention are known and commercially available, or may be synthesized by using or according to methods known in the art.

Unless otherwise stated, all reactions of the present invention are carried out under a dry nitrogen or argon atmosphere with continuous magnetic stirring, the solvent is a dry solvent, and the reaction temperature is in degree centigrade (°C).

### I. Preparation of Intermediates

### Preparation of intermediate 1: 4-amino-1-methylimidazo[1,5-a]quinoxaline-8-carboxylic acid

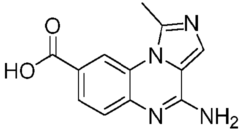

### Step 1: synthesis of methyl 3-(2-methyl-1H-imidazol-1-yl)-4-nitrobenzoate

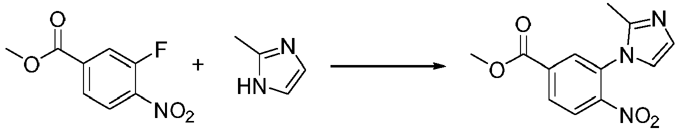

Methyl 3-fluoro-4-nitrobenzoate (1.5 g, 7.54 mmol) and 2-methyl-1*H*-imidazole (0.74 g, 9.05 mmol) were dissolved in acetonitrile (20 mL), and then cesium carbonate (3.69 g, 11.31 mmol) was added. The mixture was stirred at 80 °C overnight. After the reaction was completed, water was added to quench the reaction, and the mixture was extracted three times with ethyl acetate. The organic phases were combined, washed with saturated brine, dried over anhydrous sodium sulfate, filtered and concentrated to obtain methyl 3-(2-methyl-1*H*-imidazol-1-yl)-4-nitrobenzoate (1.97 g, 100%), which was used directly in the next step. MS m/z (ESI): 262 [M+H]⁺.

### Step 2: synthesis of methyl 4-amino-3-(2-methyl-1H-imidazol-1-yl)benzoate

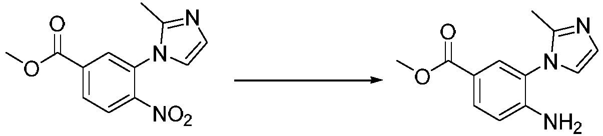

Methyl 3-(2-methyl-1*H*-imidazol-1-yl)-4-nitrobenzoate (1.97 g, 7.54 mmol) was dissolved in methanol (30 mL), and then palladium hydroxide (0.4 g, 20% w/w) was added. The mixture was stirred at room temperature overnight. After the reaction was completed, the reaction solution was filtered and concentrated, and the concentrate was separated by silica gel column chromatography [5% methanol-dichloromethane system ] to obtain methyl 4-amino-3-(2-methyl-1*H*-imidazol-1-yl)benzoate (800 mg, 46%). MS m/z (ESI): 232 [M+H]⁺.

### Step 3: synthesis of methyl 1-methyl-4-oxo-4,5-dihydroimidazo[1,5-a]quinoxaline-8-carboxylate

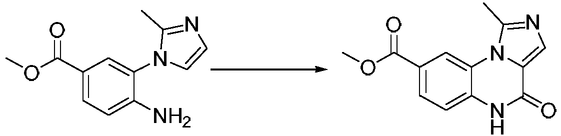

Methyl 4-amino-3-(2-methyl-1*H*-imidazol-1-yl)benzoate (800 mg, 3.46 mmol) and bis(1*H-*imidazol-1-yl)methanone (729 mg, 4.50 mmol) were placed in 1,2-dichlorobenzene (30 mL), and then the mixture was heated to 160-170 °C and stirred at that temperature for 3 hrs. After being cooled, the reaction solution was concentrated to remove the solvent, and the concentrate was slurried with methanol for 10 min, filtered and dried to obtain methyl 1-methyl-4-oxo-4,5-dihydroimidazo[1,5-*a*]quinoxaline-8-carboxylate (680 mg, 76%). MS m/z (ESI): 258 [M+H]⁺.

### Step 4: synthesis of methyl 4-chloro-1-methylimidazo[1,5-a]quinoxaline-8-carboxylate

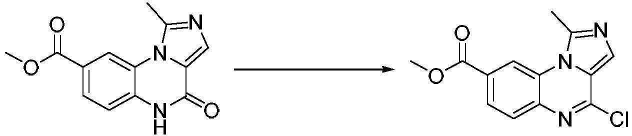

Methyl 1-methyl-4-oxo-4,5-dihydroimidazo[1,5-*a*]quinoxaline-8-carboxylate (680 mg, 2.64 mmol) was placed in phosphorus oxychloride (50 mL), and then *N*,*N*-dimethylaniline (115.9 mg, 0.53 mmol) was added. The mixture was heated to 120 °C and stirred overnight, and part of the starting materials were not reacted. The mixture was cooled and concentrated, and ice water was added to quench the reaction under an ice-water bath. The mixture was extracted three times with dichloromethane. The organic phases were combined, washed with a saturated aqueous sodium bicarbonate solution and saturated brine, dried over anhydrous sodium sulfate, filtered and concentrated, and the concentrate was separated by silica gel column chromatography [40-60% ethyl acetate-petroleum ether system] to obtain methyl 4-chloro-1-methylimidazo[1,5-*a*]quinoxaline-8-carboxylate (460 mg, 63%). MS m/z (ESI): 276 [M+H]⁺.

### Step 5: synthesis of methyl 4-((2,4-dimethoxybenzyl)amino)-1-methylimidazo[1,5-a]quinoxaline-8-carboxylate

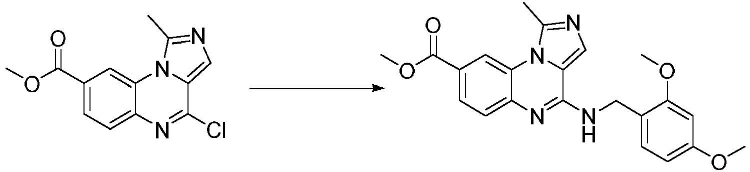

Methyl 4-chloro-1-methylimidazo[1,5-*a*]quinoxaline-8-carboxylate (460 mg, 1.67 mmol) was dissolved in *N*-methylpyrrolidone (8 mL), and then (2,4-dimethoxybenzyl)amino (646 mg, 5.01 mmol) was added. The mixture was heated to 110 °C and stirred for 3 hrs. After the reaction was completed, water was added to quench the reaction, and the mixture was extracted three times with ethyl acetate. The organic phases were combined, washed with saturated brine, dried over anhydrous sodium sulfate, filtered and concentrated, and the concentrate was separated by silica gel column chromatography [9% methanol-dichloromethane system] to obtain methyl 4-((2,4-dimethoxybenzyl)amino)-1-methylimidazo[1,5-*a*]quinoxaline-8-carboxylate (605 mg, 89%). MS m/z (ESI): 407 [M+H]⁺.

### Step 6: synthesis of methyl 4-amino-1-methylimidazo[1,5-a]quinoxaline-8-carboxylate

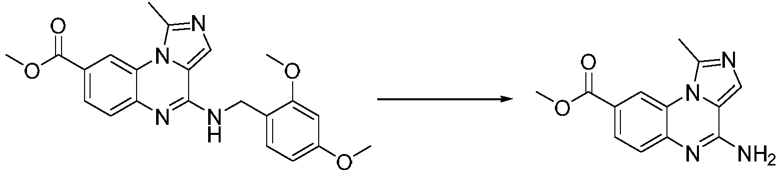

Methyl 4-((2,4-dimethoxybenzyl)amino)-1-methylimidazo[1,5-*a*]quinoxaline-8-carboxylate (330 mg, 0.81 mmol) was dissolved in trifluoroacetic acid (10 mL), and then the mixture was stirred at 60 °C for 6 hrs. After the reaction was completed, the reaction solution was concentrated. A suitable amount of water was added, and the pH was adjusted to about 6 with an aqueous sodium bicarbonate solution. The reaction solution was filtered and dried to obtain methyl 4-amino-1-methylimidazo[1,5-*a*]quinoxaline-8-carboxylate (370 mg, crude product). MS m/z (ESI): 257 [M+H]⁺.

### Step 7: synthesis of 4-amino-1-methylimidazo[1,5-a]quinoxaline-8-carboxylic acid

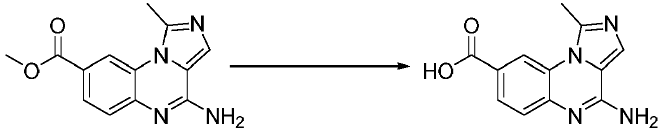

Methyl 4-amino-1-methylimidazo[1,5-*a*]quinoxaline-8-carboxylate (370 mg, 0.81 mmol) was dissolved in methanol (10 mL) and tetrahydrofuran (10 mL), and then lithium hydroxide monohydrate (102 mg, 2.43 mmol) was added. The mixture was stirred at 50 °C overnight. After the reaction was completed, the reaction solution was concentrated. A suitable amount of water was added, and the pH was adjusted to about 6 with diluted hydrochloric acid. The reaction solution was filtered, and the filter cake was washed several times with water and dried to obtain 4-amino-1-methylimidazo[1,5-*a*]quinoxaline-8-carboxylic acid (150 mg, 76%). MS m/z (ESI): 243 [M+H]⁺.

### Intermediates 2-7 can be prepared by selecting corresponding starting materials according to all or part of the synthesis method of Intermediate 1:

| **Intermediate No.** | **Structural formula** | **Chemical name** | **MS m/z [M+H]⁺** |
|---|---|---|---|
| **2** | 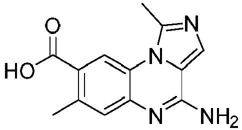 | 4-amino-1,7-dimethylimidazo[1,5-*a*]quinoxaline-8-carboxylic acid | **257** |
| **3** | 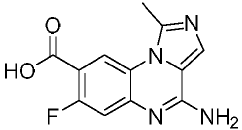 | 4-amino-7-fluoro-1-methylimidazo[1,5-*a*]quinoxaline-8-carboxylic acid | **261** |
| **4** | 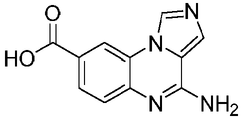 | 4-aminoimidazo[1,5-*a*]quinoxaline-8-carboxylic acid | **229** |
| **5** | 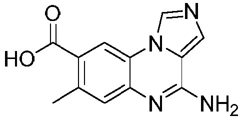 | 4-amino-7-methylimidazo[1,5-*a*]quinoxaline-8-carboxylic acid | **243** |
| **6** | 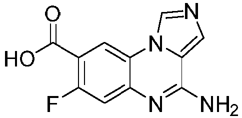 | 4-amino-7-fluoroimidazo[1,5-*a*]quinoxaline-8-carboxylic acid | **247** |
| **7** | 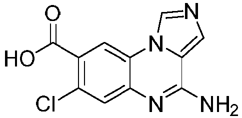 | 4-amino-7-chloroimidazo[1,5-*a*]quinoxaline-8-carboxylic acid | **263** |

### Preparation of intermediate 8: 4-amino-7-fluoropyrrolo[1,2-a]quinoxaline-8-carboxylic acid

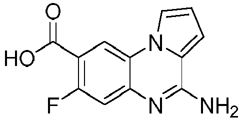

### Step 1: synthesis of methyl 5-(2-cyano-1H-pyrrol-1-yl)-2-fluoro-4-nitrobenzoate

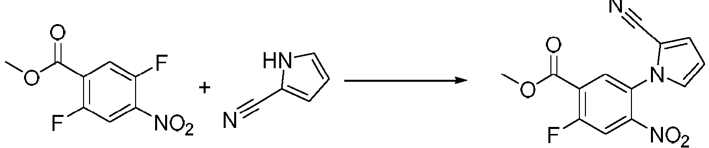

Methyl 2,5-difluoro-4-nitrobenzoate (2.0 g, 9.21 mmol) was dissolved in acetonitrile (20 mL), and then 1*H*-pyrrole-2-carbonitrile (0.78 mL, 9.21 mmol) and cesium fluoride (1.40 g, 9.21 mmol) were added. The mixture was stirred at 20 °C for 16 hrs. The reaction solution was concentrated by rotary evaporation, and the crude product was separated by a normal phase column to obtain methyl 5-(2-cyano-1*H*-pyrrol-1-yl)-2-fluoro-4-nitrobenzoate (500 mg, 19%).

¹H NMR (400 MHz, CDCl₃) δ 8.15 (d, *J* = 6.4 Hz, 1H), 7.92 (d, *J* = 8.8 Hz, 1H), 7.04 (dd, *J* = 4.0, 1.6 Hz, 1H), 6.93 (dd, *J* = 2.8, 1.6 Hz, 1H), 6.43 (dd, *J* = 4.0, 2.8 Hz, 1H), 4.01 (s, 3H).

### Step 2: synthesis of methyl 4-amino-7-fluoropyrrolo[1,2-a]quinoxaline-8-carboxylate

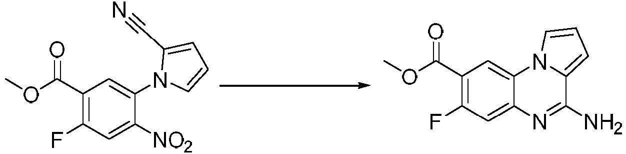

Methyl 5-(2-cyano-1*H*-pyrrol-1-yl)-2-fluoro-4-nitrobenzoate (1.5 g, 5.19 mmol) was dissolved in tetrahydrofuran (20 mL), and then iron powder (2.90 g, 51.86 mmol) and glacial acetic acid (20 mL) were added. The mixture was stirred at 20 °C for 16 hrs. The reaction solution was concentrated. The crude product was separated by a normal phase column to obtain methyl 4-amino-7-fluoropyrrolo[1,2-*a*]quinoxaline-8-carboxylate (650 mg, 48%).

¹H NMR (400 MHz, DMSO-*d₆*) δ 8.47 (d, *J* = 7.2 Hz, 1H), 8.36 (s, 1H), 7.52 (s, 2H), 7.25-7.06 (m, 2H), 6.75 (m, 1H), 3.88 (s, 3H).

### Step 3: synthesis of 4-amino-7-fluoropyrrolo[1,2-a]quinoxaline-8-carboxylic acid

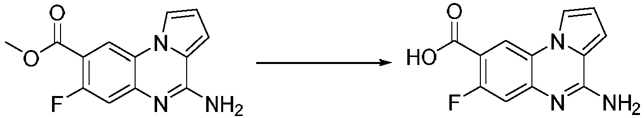

Methyl 4-amino-7-fluoropyrrolo[1,2-*a*]quinoxaline-8-carboxylate (600 mg, 2.31 mmol) was dissolved in tetrahydrofuran (10 mL) and water (3 mL), and then lithium hydroxide monohydrate (583 mg, 13.89 mmol) was added. The mixture was reacted at 50 °C for 16 hrs. After the organic solvent was removed by evaporation under reduced pressure, water (5 mL) was added thereto. The pH was adjusted to 3-4 with 1 M hydrochloric acid, and the reaction solution was filtered.

The filter cake was washed with water (20 mL) and acetonitrile (20 mL) and dried to obtain 4-amino-7-fluoropyrrolo[1,2-*a*]quinoxaline-8-carboxylic acid (341 mg, 60%). MS m/z (ESI): 246 [M+H]⁺.

¹H NMR (400 MHz, DMSO-*d₆*) δ 9.99 (s, 1H), 9.15 (s, 1H), 8.83-8.53 (m, 2H), 7.79 (d, *J* = 3.6 Hz, 1H), 7.57 (d, *J* = 10.8 Hz, 1H), 6.94 (d, *J* = 3.6 Hz, 1H).

### Preparation of intermediate 9: 1-cyclopropyl-N-((5-(trifluoromethyl)pyridin-2-yl)methyl) methanamine

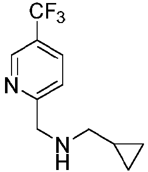

### Step 1: synthesis of 1-cyclopropyl-N-((5-(trifluoromethyl)pyridin-2-yl)methyl)methanamine

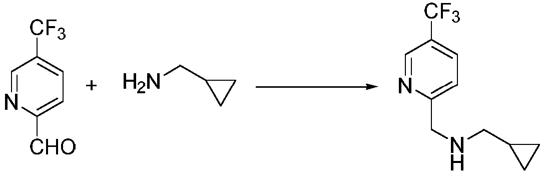

5-(Trifluoromethyl)picolinaldehyde (350 mg, 2.0 mmol) was dissolved in dichloromethane (10 mL), and then cyclopropylmethylamine (156 mg, 2.2 mmol) and acetic acid (132 mg, 2.2 mmol) were added. The mixture was stirred at room temperature for half an hour. Sodium triacetoxyborohydride (355 mg, 1.68 mmol) was added to the reaction solution, and the mixture was stirred at room temperature for 1 hr. The reaction solution was diluted with dichloromethane and a saturated sodium bicarbonate solution, and liquid separation was performed. The organic phase was concentrated by rotary evaporation, and the crude product was separated by column chromatography (methanol/dichloromethane = 1/19) to obtain 1-cyclopropyl-N-((5-(trifluoromethyl)pyridin-2-yl)methyl)methanamine (400 mg, 84%). MS m/z (ESI): 231 [M+H]⁺.

**Intermediates 10-19 can be prepared by selecting corresponding starting materials according to all or part of the synthesis method of Intermediate 9:**

| **Intermediate No.** | **Structural formula** | **Chemical name** | **MS m/z [M+H]⁺** |
|---|---|---|---|
| **10** | 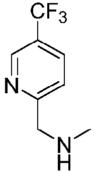 | *N*-methyl-1-(5-(trifluoromethyl)pyridin-2-yl)methanamine | **191** |
| **11** | 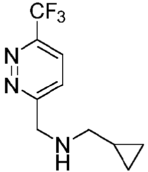 | 1-cyclopropyl-*N*-((6-(trifluoromethyl)pyridazin-3-yl)methyl)methanamine | **232** |
| **12** | 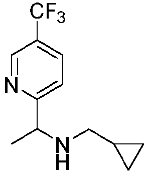 | *N-(cyclopropylmethyl)-1-(5-*(trifluoromethyl)pyridin-2-yl)ethan-1-amine | **245** |
| **13** | 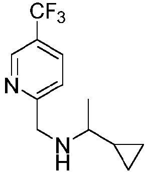 | 1-cyclopropyl-*N*-((5-(trifluoromethyl)pyridin-2-yl)methyl)ethan-1-amine | **245** |
| **14** | 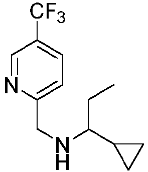 | 1-cyclopropyl-*N*-((5-(trifluoromethyl)pyridin-2-yl)methyl)propan-1-amine | **259** |
| **15** | 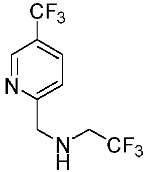 | 2,2,2-trifluoro-*N*-((5-(trifluoromethyl)pyridin-2-yl)methyl)ethan-1-amine | **259** |
| **16** | 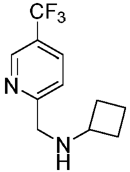 | *N*-((5-(trifluoromethyl)pyridin-2-yl)methyl)cyclobutanamine | **231** |
| **17** | 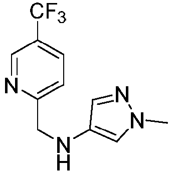 | 1-methyl-*N*-((5-(trifluoromethyl)pyridin-2-yl)methyl)-1*H*-pyrazol-4-amine | **257** |
| **18** | 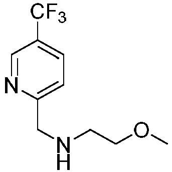 | 2-methoxy-*N*-((5-(trifluoromethyl)pyridin-2-yl)methyl)ethan-1-amine | **235** |
| **19** | 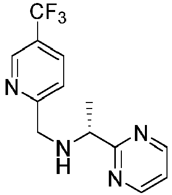 | (*R*)-1-(pyrimidin-2-yl)-*N*-((5-(trifluoromethyl)pyridin-2-yl)methyl)ethan-1-amine | **283** |

### Preparation of intermediate 20: 6-(trifluoromethyl)benzofuran-3(2H)-one

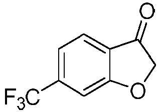

### Step 1: synthesis of methyl 2-(2-methoxy-2-oxoethoxy)-4-(trifluoromethyl)benzoate

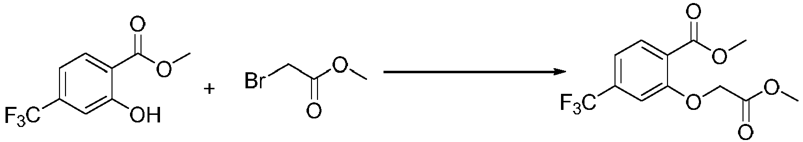

Methyl 2-hydroxy-4-(trifluoromethyl)benzoate (15.0 g, 68.1 mmol) was dissolved in acetone (200 mL), and then potassium carbonate (12.24 g, 88.6 mmol) was added. Methyl bromoacetate (8.42 mL, 88.6 mmol) was added dropwise to the reaction solution, and then the mixture was warmed to 55 °C and reacted for 18 hrs. After the reaction was completed, the reaction solution was cooled to room temperature and filtered to remove solids, and the filter cake was washed with ethyl acetate. The filtrate was washed with saturated brine, dried and concentrated to obtain methyl 2-(2-methoxy-2-oxoethoxy)-4-(trifluoromethyl)benzoate (22.0 g, 94%). MS m/z (ESI): 293 [M+H]⁺.

### Step 2: synthesis of 2-(carboxymethoxy)-4-(trifluoromethyl)benzoic acid

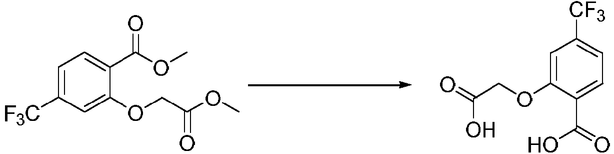

Methyl 2-(2-methoxy-2-oxoethoxy)-4-(trifluoromethyl)benzoate (74.0 g, 253.2 mmol) was dissolved in a mixture solvent of methanol (500 mL) and water (250 mL), and then lithium hydroxide monohydrate (31.91 g, 759.7 mmol) was added. The mixture was reacted at room temperature for 18 hrs. The reaction solution was acidified to pH = 2 with 1 N hydrochloric acid and concentrated to remove methanol. The aqueous phase was filtered, and the solid was washed with water. The filter cake was dried to obtain 2-(carboxymethoxy)-4-(trifluoromethyl)benzoic acid (61.0 g, 91%). MS m/z (ESI): 265 [M+H]⁺.

### Step 3: synthesis of 6-(trifluoromethyl)benzofuran-3(2H)-one

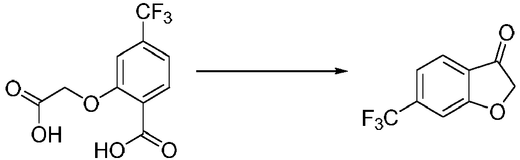

A mixture of 2-(carboxymethoxy)-4-(trifluoromethyl)benzoic acid (61 g, 230.9 mmol), acetic anhydride (377 mL, 4.02 mol), sodium acetate (28.41 g, 346.4 mmol) and acetic acid (110 mL) was heated to 150 °C and reacted for 18 hrs. After the reaction was completed, the reaction solution was cooled to room temperature, diluted with ethyl acetate, washed with water and saturated brine, dried over anhydrous sodium sulfate and concentrated. The concentrate was separated by silica gel column chromatography to obtain an intermediate (42 g). This intermediate was dissolved in methanol (300 mL), and then 1 N hydrochloric acid (300 mL) was added. The mixture was warmed to 100 °C and reacted for 6 hrs. The reaction solution was cooled to room temperature, and then water was added. The resulting mixture was extracted with ethyl acetate. The organic phase obtained by extraction was washed with saturated brine, dried over anhydrous sodium sulfate and concentrated. The concentrate was slurried with methyl *tert-butyl* ether to obtain 6-(trifluoromethyl)benzofuran-3(2H)-one (17.0 g, 36%).

¹H NMR (400 MHz, *CDCl₃*) δ 7.80 (d, *J* = 8.0 Hz, 1H), 7.43 (s, 1H), 7.35 (dd, *J* = 8.1, 1.4 Hz, 1H), 4.72 (s, 2H).

### Preparation of intermediate 21: 2-(trifluoromethyl)-6,7-dihydro-5H-cyclopenta[b] pyridin-5-one

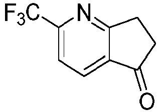

### Step 1: synthesis of methyl 2-bromo-6-(trifluoromethyl)nicotinate

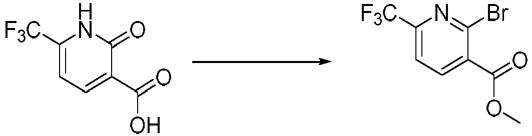

2-Oxo-6-(trifluoromethyl)-1,2-dihydropyridine-3-carboxylic acid (5.0 g, 24.14 mmol) and pyridine (1.95 mL, 24.14 mmol) were dissolved in chlorobenzene (50 mL), and then phosphorus oxybromide (4.91 mL, 48.28 mmol) was added slowly in portions at room temperature. The mixture was warmed to 120 °C and stirred for 16 hrs. After the reaction was completed, the reaction solution was concentrated under vacuum. The concentrate was cooled to 0 °C under an ice bath, and then dry methanol (20 mL) cooled in advance with dry ice was added dropwise and slowly. The mixture was stirred at room temperature for 1 hr. The reaction solution was concentrated under vacuum to obtain a brown oil. The oil was diluted with water, the pH of the solution was adjusted to 8 with a saturated sodium bicarbonate solution, and the mixture was extracted three times with ethyl acetate (50 mL * 3). The organic phase was washed with saturated brine, dried over anhydrous sodium sulfate, filtered and concentrated. The concentrate was separated by silica gel column chromatography to obtain methyl 2-bromo-6-(trifluoromethyl)nicotinate (5.7 g, 83%). MS m/z (ESI): 284, 286 [M+H]⁺.

¹H NMR (400 MHz, CDCl₃) *δ* 8.21 (d, *J* = 8.0 Hz, 1H), 7.72 (d, *J* = 8.0 Hz, 1H), 4.00 (s, 3H).

### Step 2: synthesis of methyl (E)-2-(3-methoxy-3-oxoprop-1-en-1-yl)-6-(trifluoromethyl)nicotinate

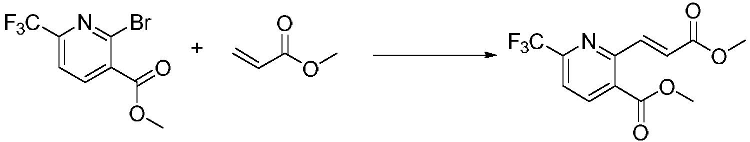

Allylpalladium(II) chloride dimer (0.37 g, 1.00 mmol), tris(o-methylphenyl)phosphorus (0.61 g, 2.01 mmol) and sodium carbonate (6.38 g, 60.20 mmol) were added to a solution of methyl 2-bromo-6-(trifluoromethyl)nicotinate (5.7 g, 20.07 mmol) and methyl acrylate (4.52 mL, 50.17 mmol) in toluene (100 mL) and *N,N*-dimethylformamide (15 mL), and then the mixture was stirred at 120 °C for 16 hrs under nitrogen atmosphere. The reaction solution was filtered, and the filter cake was washed three times with ethyl acetate (3 * 30 mL). The organic phases were combined and concentrated under vacuum, and the concentrate was separated by silica gel column chromatography to obtain methyl (*E*)-2-(3-methoxy-3-oxoprop-1-en-1-yl)-6-(trifluoromethyl)nicotinate (5.1 g, 88%). MS m/z (ESI): 290 [M+H]⁺.

¹H NMR (400 MHz, CDCl₃) *δ* 8.46 (d, *J* = 15.2 Hz, 1H), 8.40 (d, *J* = 8.0 Hz, 1H), 7.70 (d, *J* = 8.4 Hz, 1H), 7.26 (d, *J= 2.8* Hz, 1H), 4.01 (s, 3H), 3.84 (s, 3H).

### Step 3: synthesis of methyl 2-(3-methoxy-3-oxopropyl)-6-(trifluoromethyl)nicotinate

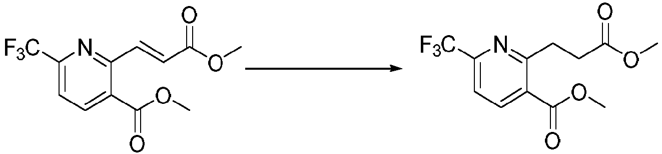

A solution of methyl (*E*)-2-(3-methoxy-3-oxoprop-1-en-1-yl)-6-(trifluoromethyl)nicotinate (5.1 g, 17.63 mmol) and tris(triphenylphosphine)rhodium chloride (1.31 g, 1.41 mmol) in ethanol (50 mL) was stirred under hydrogen atmosphere (15 atm) at room temperature for 48 hrs. The reaction solution was filtered, and the filtrate was concentrated under vacuum. The crude product was separated by silica gel column chromatography to obtain methyl 2-(3-methoxy-3-oxopropyl)-6-(trifluoromethyl)nicotinate (4.9 g, 95%). MS m/z (ESI): 292 [M+H]⁺.

¹H NMR (400 MHz, CDCl₃) *δ* 8.34 (d, *J* = 8.0 Hz, 1H), 7.58 (d, *J* = 8.0 Hz, 1H), 3.96 (s, 3H), 3.67 (s, 3H), 3.57 (t, *J* = 7.2 Hz, 2H), 2.85 (t, *J =* 7.2 Hz, 2H).

### Step 4: synthesis of 2-(trifluoromethyl)-6,7-dihydro-5H-cyclopenta[b]pyridin-5-one

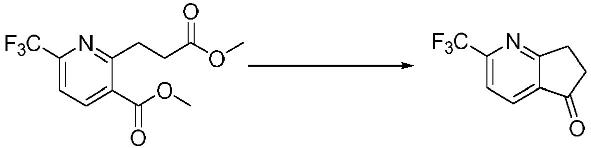

Sodium methoxide (1.36 g, 25.23 mmol) was added in portions to a solution of methyl 2-(3-methoxy-3-oxopropyl)-6-(trifluoromethyl)nicotinate (4.9 g, 16.82 mmol) in methanol (60 mL), and then the mixture was stirred at 80 °C overnight. After the reaction was completed, the reaction solution was cooled to room temperature and concentrated to obtain a brown solid. The brown solid was dispersed in concentrated hydrochloric acid (60 mL), and the mixture was stirred at 80 °C for 3 hrs. The reaction solution was cooled to room temperature, and the pH was adjusted to 6 with a 1 M sodium hydroxide solution. The mixture was extracted with dichloromethane (2 * 100 mL). The organic phase was washed with saturated brine, dried over anhydrous sodium sulfate and filtered, and the filtrate was concentrated to obtain a brown solid. The brown solid was separated by silica gel column chromatography to obtain 2-(trifluoromethyl)-6,7-dihydro-5H-cyclopenta[b]pyridin-5-one (2.07 g, 43%). MS m/z (ESI): 202 [M+H]⁺.

¹H NMR (400 MHz, CDCl₃) δ 8.21 (d, *J* = 8.0 Hz, 1H), 7.73 (d, *J* = 8.0 Hz, 1H), 3.42-3.36 (m, 2H), 2.92-2.85 (m, 2H).

### Preparation of intermediate 22: 6-(trifluoromethyl)furo[2,3-b]pyridin-3(2H)-one

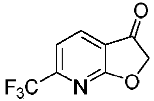

### Step 1: synthesis of ethyl 2-chloro-6-(trifluoromethyl)nicotinate

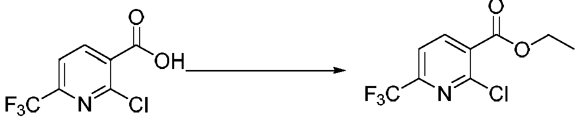

Thionyl chloride (3.62 mL, 49.9 mmol) was added dropwise to a solution of 2-chloro-6-(trifluoromethyl)nicotinic acid (7.5 g, 33.25 mmol) in ethanol (150 mL) under nitrogen protection at room temperature. The reaction solution was then heated to 70 °C and stirred for 14 hrs. After the reaction was completed, the reaction solution was concentrated to remove ethanol, and the residue was dissolved in ethyl acetate (200 mL). The organic phase was washed with water (100 mL * 3) and saturated brine, dried over anhydrous sodium sulfate, filtered and concentrated. the concentrate was separated by column chromatography [85% ethyl acetate-petroleum ether system] to obtain ethyl 2-chloro-6-(trifluoromethyl)nicotinate (7.72 g, 92%). MS m/z (ESI): 254 [M+H]⁺.

¹H NMR (400 MHz, CDCl₃) δ 8.30 (d, *J* = 8.0 Hz, 1H), 7.70 (d, *J* = 8.0 Hz, 1H), 4.46 (d, *J* = 7.2 Hz, 2H), 1.43 (d, *J* = 7.2 Hz, 3H).

### Step 2: synthesis of ethyl 3-hydroxy-6-(trifluoromethyl)furo[2,3-b]pyridine-2-carboxylate

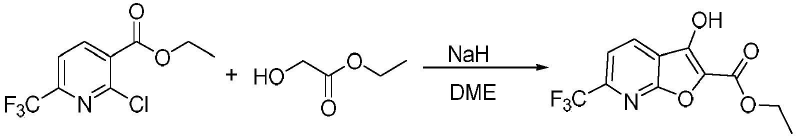

Sodium hydride (15.22 g, 380.5 mmol, 60% purity) was added in portions to a solution of ethyl glycolate (36.7 mL, 380.5 mmol) in ethylene glycol dimethyl ether (530 mL) at 0 °C. The reaction solution was warmed to room temperature and stirred for 30 min. Ethyl 2-chloro-6-(trifluoromethyl)nicotinate (38.6 g, 152.2 mmol) was then dissolved in ethylene glycol dimethyl ether (530 mL), and the solution was added dropwise to the reaction solution above. The mixture was reacted at 35 °C for 2 hrs. After the reaction was completed, the reaction solution was diluted with ethyl acetate, and a saturated sodium bicarbonate solution was added to quench the reaction. The organic phase was obtained by extraction, washed with saturated brine, dried over anhydrous sodium sulfate and concentrated. The concentrate was slurried with acetonitrile (300 mL) to obtain ethyl 3-hydroxy-6-(trifluoromethyl)furo[2,3-b]pyridine-2-carboxylate (29.5 g, 70%).

¹H NMR (400 MHz, DMSO-*d*₆) δ 8.24 (d, *J* = 7.6 Hz, 1H), 7.59 (d, *J* = 7.6 Hz, 1H), 4.17 (q, *J* = 7.2 Hz,2H), 1.26 (t, *J* = 7.2 Hz, 3H).

### Step 3: synthesis of 6-(trifluoromethyl)furo[2,3-b]pyridin-3(2H)-one

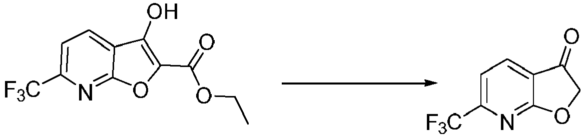

Ethyl 3-hydroxy-6-(trifluoromethyl)furo[2,3-b]pyridine-2-carboxylate (2.83 g, 10.28 mmol) was dissolved in a mixture solvent of tetrahydrofuran (32.0 mL) and water (8.0 mL), and then lithium hydroxide monohydrate (1.73 g, 41.1 mmol) was added. The mixture was warmed to 40 °C and reacted for 2 days. After the reaction was completed, the reaction solution was cooled to room temperature and filtered, and the filter cake was washed with tetrahydrofuran. The filter cake was then dissolved in a mixture solvent of ethyl acetate and water, and the system was acidified to pH = 6-7 with 1 M hydrochloric acid under an ice-water bath. Liquid separation was performed. The aqueous phase was extracted with ethyl acetate, and the organic phases were combined, dried over anhydrous sodium sulfate and filtered. The filtrate was concentrated by rotary evaporation to obtain 6-(trifluoromethyl)furo[2,3-*b*]pyridin-3(2*H*)-one (1.43 g, 68%). MS m/z (ESI): 204 [M+H]⁺.

¹H NMR (400 MHz, CDCl₃) δ 8.23 (d, *J* = 7.6 Hz, 1H), 7.52 (d, *J* = 7.6 Hz, 1H), 4.85 (s, 2H).

### Preparation of intermediate 23: 7-(trifluoromethyl)isochroman-4-one

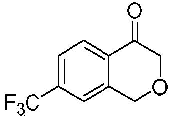

### Step 1: synthesis of 2-((allyloxy)methyl)-1-bromo-4-(trifluoromethyl)benzene

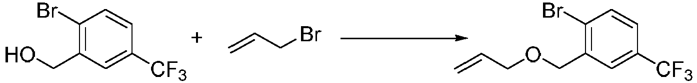

(2-Bromo-5-(trifluoromethyl)phenyl)methanol (7.39 g, 29.0 mmol) was dissolved in tetrahydrofuran (100 mL), and then sodium hydride (1.74 g, 43.5 mmol) was added at 0 °C. After reaction at 0 °C for 0.5 hrs, 3-bromoprop-1-ene (2.52 mL, 29.0 mmol) was added to the reaction solution. The mixture was reacted at 25 °C for 2 hrs. Water (50 mL) was added to the reaction solution, and the mixture was extracted three times with ethyl acetate (30 mL). The organic phases were combined and concentrated. The crude product was separated by a normal phase column to obtain 2-((allyloxy)methyl)-1-bromo-4-(trifluoromethyl)benzene (8.1 g, 95%).

¹H NMR (400 MHz, CDCl₃) δ 7.98-7.73 (m, 1H), 7.65 (d, *J =* 8.4 Hz, 1H), 7.40 (dd, *J =* 8.4, 2.4 Hz, 1H), 6.13-5.90 (m, 1H), 5.49-5.33 (m, 1H), 5.33-5.18 (m, 1H), 4.59 (s, 2H), 4.15 (dt, *J=* 5.6, 1.6 Hz, 2H).

### Step 2: synthesis of 4-methylene-7-(trifluoromethyl)isochromane

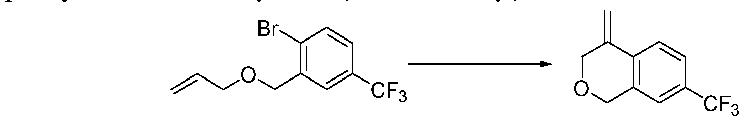

2-((Allyloxy)methyl)-1-bromo-4-(trifluoromethyl)benzene (8.1 g, 27.4 mmol) was dissolved in *N,N-*dimethylformamide (160 mL), and then cesium carbonate (10.73 g, 32.9 mmol), triphenylphosphine (3.24 g, 12.4 mmol) and palladium acetate (0.92 g, 4.1 mmol) were added. The mixture was reacted at 90 °C for 16 hrs. The reaction solution was concentrated, and the crude product was separated by a normal phase column to obtain 4-methylene-7-(trifluoromethyl)isochromane (3.8 g, 65%).

¹H NMR (400 MHz, CDCl₃) δ 7.78 (d, *J* = 8.4 Hz, 1H), 7.48 (d, *J* = 8.4, 2.0 Hz, 1H), 7.30 (s, 1H), 5.72 (s, 1H), 5.15 (t, *J* = 1.2 Hz, 1H), 4.84 (s, 2H), 4.47 (t, *J* = 1.2 Hz, 2H).

### Step 3: synthesis of 4-(hydroxymethyl)-7-(trifluoromethyl)isochroman-4-ol

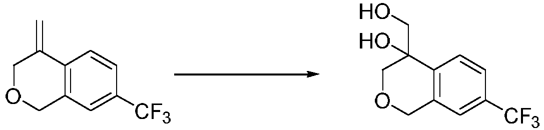

4-Methylene-7-(trifluoromethyl)isochromane (3.8 g, 17.74 mmol) was dissolved in water (10 mL) and acetone (40 mL), and then potassium osmate dihydrate (0.65 g, 1.77 mmol) was added. After reaction at 25 °C for 5 min, N-methylmorpholine oxide (7.27 g, 62.1 mmol) was added. The mixture was reacted at 25 °C for 16 hrs. A saturated sodium thiosulfate solution (50 mL) was added to the reaction solution, and the mixture was extracted with ethyl acetate (100 mL). The organic phase was concentrated, and the crude product was separated by a normal phase column to obtain 4-(hydroxymethyl)-7-(trifluoromethyl)isochroman-4-ol (3.57 g, 81%).

¹H NMR (400 MHz, *CDCl₃*) δ 7.72 *(d, J=* 8.4 Hz, 1H), 7.60-7.51 (m, 1H), 7.28 (s, 1H), 4.83 (s, 2H), 4.16 (d, *J* = 11.2 Hz, 1H), 3.90 (dd, *J* = 11.2, 6.4 Hz, 1H), 3.70 (ddd, *J* = 11.2, 4.8, 1.2 Hz, 1H), 3.63 (dd, *J* = 11.2, 1.2 Hz, 1H), 2.87 (s, 1H).

### Step 4: synthesis of 7-(trifluoromethyl)isochroman-4-one

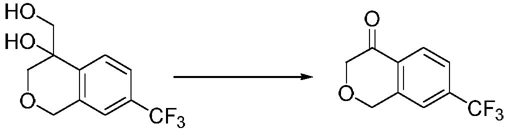

4-(Hydroxymethyl)-7-(trifluoromethyl)isochroman-4-ol (3.57 g, 14.38 mmol) was dissolved in tetrahydrofuran (50 mL) and water (5 mL), and then sodium periodate (10.46 g, 48.90 mmol) was added. The mixture was reacted at 25 °C for 16 hrs. A saturated sodium thiosulfate solution (40 mL) was added to the reaction solution, and the mixture was extracted three times with ethyl acetate (40 mL). The organic phase was concentrated, and the crude product was separated by a normal phase column to obtain 7-(trifluoromethyl)isochroman-4-one (2.88 g, 93%).

¹H NMR (400 MHz, CDCl₃) δ 8.16 (d, *J* = 8.4 Hz, 1H), 7.68 (dd, *J* = 8.4, 1.6 Hz, 1H), 7.52 (d, *J* = 2.0 Hz, 1H), 4.95 (s, 2H), 4.42 (s, 2H).

### Preparation of intermediate 24: 6-(1-methyl-1H-pyrazol-4-yl)benzofuran-3(2H)-one

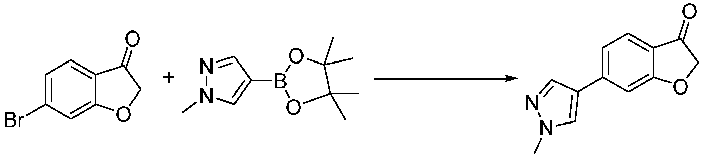

6-Bromobenzofuran-3(2*H*)-one (1.00 g, 4.69 mmol), 1-methyl-4-(4,4,5,5-tetramethyl-1,3,2-dioxaborolan-2-yl)-1*H*-pyrazole (1.17 g, 5.63 mmol), [1,1'-bis(diphenylphosphino) ferrocene]palladium dichloride dichloromethane complex (343 mg, 0.469 mmol) and potassium carbonate (1.94 g, 14.07 mmol) were dissolved in dioxane (15 mL) and water (5 mL), and then the mixture was heated to 85 °C and stirred overnight under nitrogen protection. After the reaction was completed, water was added to quench the reaction, and the mixture was extracted with ethyl acetate. The organic phase was dried over anhydrous sodium sulfate and filtered, and the resulting crude product was separated by silica gel column chromatography to obtain 6-(1-methyl-1*H-*pyrazol-4-yl)benzofuran-3(2H)-one (0.8 g, 80%). MS m/z (ESI): 215 [M+H]⁺.

### Preparation of intermediate 25: (S)-6-(trifluoromethyl)-2,3-dihydrofuro[2,3-b]pyridin-3-amine

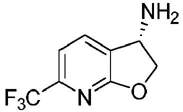

### Step 1: synthesis of (R)-6-(trifluoromethyl)-2,3-dihydrofuro[2,3-b]pyridin-3-ol

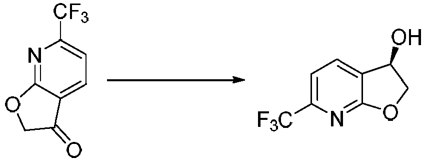

(*R*)-2-methyl-CBS-oxazolylborane (5.94 g, 21.4 mmol) was dissolved in tetrahydrofuran (1100 mL), and the mixture was cooled to 0 °C. Borane dimethylsulfide complex (53.5 mL, 107.1 mmol, 2 M in THF) was then added to the above solution, and the mixture was stirred for 1 hr. The reaction system was cooled to -70 °C. 6-(Trifluoromethyl)furo[2,3-*b*]pyridin-3(2*H*)-one (14.5 g, 71.4 mmol) was dissolved in tetrahydrofuran (140 mL), and the solution was added dropwise to the reaction system. The mixture was stirred for 1 hr with the temperature maintained at that value. Then the mixture was heated to -10 °C and stirred for 2 hrs. After the reaction was completed, as detected, methanol was added to quench the reaction, and the mixture was stirred at room temperature for 30 min. 70 mL of aqua ammonia was added, and the mixture was stirred. The supernate was poured out and concentrated, and the residue was separated by silica gel column chromatography (dichloromethane/ethyl acetate = 10/1) to obtain (*R*)-6-(trifluoromethyl)-2,3-dihydrofuro[2,3-*b*]pyridin-3-ol (12.0 g, 82%). MS m/z (ESI): 206 [M+H]⁺.

### Step 2: synthesis of (S)-3-azido-6-(trifluoromethyl)-2,3-dihydrofuro[2,3-b]pyridine

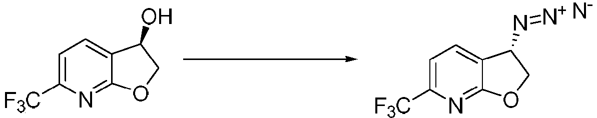

(*R*)-6-(trifluoromethyl)-2,3-dihydrofuro[2,3-*b*]pyridin-3-ol (12.0 g, 58.5 mmol) was dissolved in tetrahydrofuran (100 mL), and then the mixture was cooled to 0 °C. Diphenylphosphoryl azide (15.2 mL, 70.2 mmol) and 1,8-diazabicyclo[5.4.0]undec-7-ene (10.5 mL, 70.2 mmol) were then added. The mixture was warmed to room temperature and reacted for 18 hrs. After the reaction was completed, the reaction solution was diluted with water and extracted with ethyl acetate. The organic phase was dried and concentrated, and the concentrate was separated by silica gel column chromatography to obtain (S)-3-azido-6-(trifluoromethyl)-2,3-dihydrofuro[2,3-b]pyridine (12.5 g, 93%). MS m/z (ESI): 231 [M+H]⁺.

### Step 3: synthesis of (S)-6-(trifluoromethyl)-2,3-dihydrofuro[2,3-b]pyridin-3-amine hydrochloride

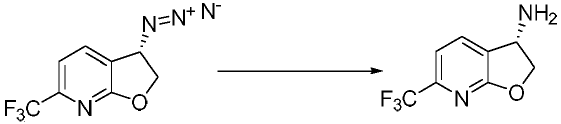

(S)-3-azido-6-(trifluoromethyl)-2,3-dihydrofuro[2,3-b]pyridine (12.5 g, 54.3 mmol) was dissolved in tetrahydrofuran (200 mL) and water (6 mL), and then triphenylphosphine (42.74 g, 162.9 mmol) was added. The mixture was warmed to 50 °C and reacted for 18 hrs. 0.5M hydrochloric acid was added, and the mixture was extracted three times with ethyl acetate. The aqueous phase obtained by liquid separation was concentrated to obtain (S)-6-(trifluoromethyl)-2,3-dihydrofuro[2,3-b]pyridin-3-amine hydrochloride (11.0 g, 84%). MS m/z (ESI): 205 [M+H]⁺.

### Intermediates 26-32 can be prepared by selecting corresponding starting materials according to all or part of the synthesis method of Intermediate 25:

| **Intermediate No.** | **Structural formula** | **Chemical name** | **MS m/z [M+H]⁺** |
|---|---|---|---|
| **26** | 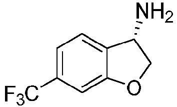 | (*S*)-6-(trifluoromethyl)-2,3-dihydrobenzofuran-3-amine | **204** |
| **27** | 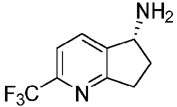 | (*R*)-2-(trifluoromethyl)-6,7-dihydro-5*H-*cyclopenta[*b*]pyridin-5-amine | **203** |
| **28** | 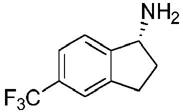 | (*R*)-5-(trifluoromethyl)-2,3-dihydro-1*H*-inden-1-amine | **202** |
| **29** | 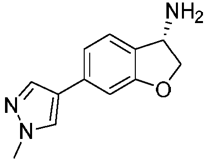 | (*S*)-6-(1-methyl-1*H*-pyrazol-4-yl)-2,3-dihydrobenzofuran-3-amine | **216** |
| **30** | 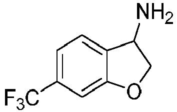 | 6-(trifluoromethyl)-2,3-dihydrobenzofuran-3-amine | **204** |
| **31** | 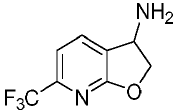 | 6-(trifluoromethyl)-2,3-dihydrofuro[2,3-*b*]pyridin-3-amine | **205** |
| **32** | 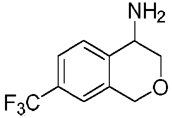 | 7-(trifluoromethyl)isochroman-4-amine | **218** |

### Preparation of intermediate 33: (S)-N-(1-methyl-1H-pyrazol-4-yl)-6-(trifluoromethyl)-2,3-dihydrofuro [2,3-b] pyridin-3-amine

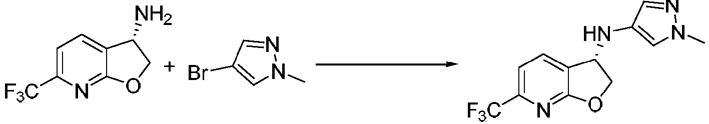

(S)-6-(trifluoromethyl)-2,3-dihydrofuro[2,3-*b*]pyridin-3-amine hydrochloride (5.5 g, 22.9 mmol) was dissolved in dioxane (500 mL), and then 4-bromo-1-methyl-1*H*-pyrazole (4.23 g, 26.3 mmol), methanesulfonic acid (2-di-*tert*-butylphosphino-2',4',6'-triisopropyl-1,1'-biphenyl)(2'-amino-1,1'-biphenyl-2-yl)palladium (II) (5.45 g, 6.86 mmol) and sodium *tert*-butoxide (6.59 g, 68.58 mmol) were added. The nitrogen was charged to replace three times by evacuation, and then the mixture was warmed to 95 °C and reacted for 2 hrs. After the reaction solution was cooled to room temperature, water was added, and the mixture was extracted with ethyl acetate. The organic phase obtained by liquid separation was dried and concentrated, and the concentrate was separated by silica gel column chromatography (PE/EA = 1/10) and C18 reversed-phase column (water (10 mM NH₄HCO₃)-ACN: 21%-51%) successively to obtain (*S*)-*N*-(1-methyl-1*H*-pyrazol-4-yl)-6-(trifluoromethyl)-2,3-dihydrofuro[2,3-b]pyridin-3-amine (2.40 g, 37%). MS m/z (ESI): 285 [M+H]⁺.

¹H NMR (400 MHz, DMSO-*d*₆) δ 7.88 (d, *J* = 7.4 Hz, 1H), 7.41 (d, *J* = 7.4 Hz, 1H), 7.22 (d, *J* = 1.0 Hz, 1H), 7.02 (d, *J* = 1.0 Hz, 1H), 5.19 (d, *J* = 8.9 Hz, 1H), 5.02 (td, *J* = 8.5, 4.5 Hz, 1H), 4.84 (dd, *J* = 9.6, 8.1 Hz, 1H), 4.38 (dd, *J =* 9.6, 4.6 Hz, 1H), 3.71 (s, 3H).

### Intermediates 34-38 can be prepared by selecting corresponding starting materials according to all or part of the synthesis method of Intermediate 33:

| **Intermediate No.** | **Structural formula** | **Chemical name** | **MS m/z [M+H]⁺** |
|---|---|---|---|
| **34** | 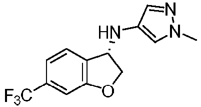 | (*S*)-1-methyl-*N*-(6-(trifluoromethyl)-2,3-dihydrobenzofuran-3-yl)-1*H-*pyrazol-4-amine | **284** |
| **35** | 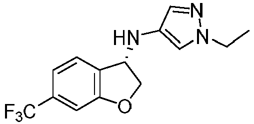 | (*S*)-1-ethyl-*N*-(6-(trifluoromethyl)-2,3-dihydrobenzofuran-3-yl)-1*H-*pyrazol-4-amine | **298** |
| **36** | 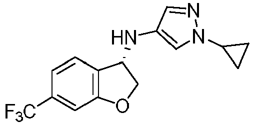 | (*S*)-1-cyclopropyl-*N*-(6-(trifluoromethyl)-2,3-dihydrobenzofuran-3-yl)-1*H-*pyrazol-4-amine | **310** |
| **37** | 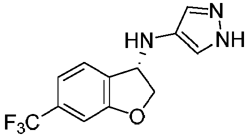 | (*S*)-*N*-(6-(trifluoromethyl)-2,3-dihydrobenzofuran-3-yl)-1*H-*pyrazol-4-amine | **270** |
| **38** | 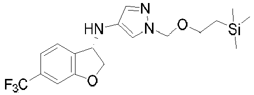 | (*S*)-*N*-(6-(trifluoromethyl)-2,3-dihydrobenzofuran-3-yl)-1-((2-(trimethylsilyl)ethoxy)methyl)-1*H*-pyrazol-4-amine | **400** |

### Preparation of intermediate 39: (R)-N-(cyclopropylmethyl)-5-(trifluoromethyl)-2,3-dihydro-1H-inden-1-amine

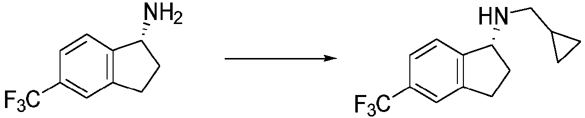

(*R*)-5-(Trifluoromethyl)-2,3-dihydro-1*H*-inden-1-amine hydrochloride (200 mg, 0.84 mmol) was dissolved in a mixed solution of dichloromethane (3 mL) and ethanol (3 mL), and then potassium acetate (82 mg, 0.84 mmol), cyclopropanecarboxaldehyde (59 mg, 0.84 mmol), acetic acid (5 mg, 0.084 mmol) and sodium triacetoxyborohydride (355 mg, 1.68 mmol) were added. The mixture was stirred at room temperature overnight. The reaction solution was diluted with dichloromethane and a saturated sodium bicarbonate solution, and liquid separation was performed. The organic phase was concentrated by rotary evaporation, and the crude product was separated by column chromatography (methanol/dichloromethane = 1/19) to obtain (*R*)-*N-*(cyclopropylmethyl)-5-(trifluoromethyl)-2,3-dihydro-1*H*-inden-1-amine (108 mg, 48%). MS m/z (ESI): 256 [M+H]⁺.

### Intermediates 40-43 can be prepared by selecting corresponding starting materials according to all or part of the synthesis method of Intermediate 39:

| **Intermediate No.** | **Structural formula** | **Chemical name** | **MS m/z [M+H]⁺** |
|---|---|---|---|
| **40** | 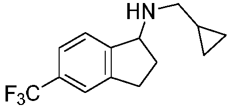 | *N*-(cyclopropylmethyl)-5-(trifluoromethyl)-2,3-dihydro-1*H-*inden-1-amine | **256** |
| **41** | 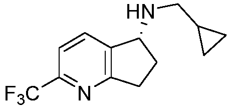 | (*R*)-*N*-(cyclopropylmethyl)-2-(trifluoromethyl)-6,7-dihydro-5*H-*cyclopenta[*b*]pyridin-5-amine | **257** |
| **42** | 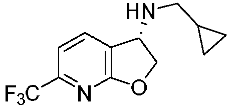 | (*S*)-*N*-(cyclopropylmethyl)-6-(trifluoromethyl)-2,3-dihydrofuro[2,3-*b*]pyridin-3-amine | **259** |
| **43** | 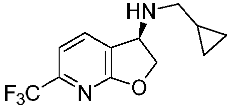 | (*R*)-*N*-(cyclopropylmethyl)-6-(trifluoromethyl)-2,3-dihydrofuro[2,3-*b*]pyridin-3-amine | **259** |

### Preparation of intermediate 44: N-methyl-6-(trifluoromethyl)-2,3-dihydrobenzofuran-3-amine

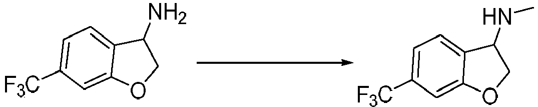

6-(Trifluoromethyl)-2,3-dihydrobenzofuran-3-amine (600 mg, 2.95 mmol) was dissolved in hexafluoroisopropanol (5 mL), and then methyl trifluoromethanesulfonate (727 mg, 4.43 mmol) was added. The mixture was stirred at room temperature for 2 hrs. The reaction solution was diluted with ethyl acetate and a saturated sodium bicarbonate solution, and liquid separation was performed. The organic phase was concentrated by rotary evaporation, and the crude product was separated by column chromatography to obtain N-methyl-6-(trifluoromethyl)-2,3-dihydrobenzofuran-3-amine (450 mg, 70%). MS m/z (ESI): 218 [M+H]⁺.

### Intermediates 45-47 can be prepared by selecting corresponding starting materials according to all or part of the synthesis method of Intermediate 44:

| **Intermediate No.** | **Structural formula** | **Chemical name** | **MS m/z [M+H]⁺** |
|---|---|---|---|
| **45** | 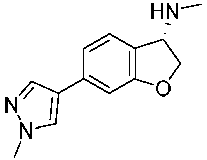 | (*S*)-*N*-methyl-6-(1-methyl-1*H-*pyrazol-4-yl)-2,3-dihydrobenzofuran-3-amine | **230** |
| **46** | 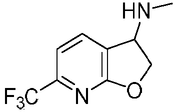 | *N*-methyl-6-(trifluoromethyl)-2,3-dihydrofuro[2,3-*b*]pyridin-3-amine | **219** |
| **47** | 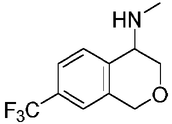 | *N*-methyl-7-(trifluoromethyl)isochroman-4-amine | **232** |

### Intermediate 48: preparation of 5-((2R,5S)-5-methylpiperidin-2-yl)benzo[d]thiazole

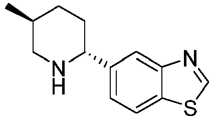

### Step 1: synthesis of methyl 4-methyl-5-oxopentanoate

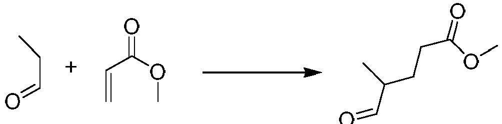

Propionaldehyde (5.0 g, 86.09 mmol) was added to piperidine (14.66 g, 172.2 mmol) and potassium carbonate (4.76 g, 34.43 mmol) under an ice-water bath, and then the mixture was stirred vigorously at room temperature for 18 hrs. The reaction solution was filtered, and the mother solution was dried over anhydrous sodium sulfate and filtered again. The filtrate was concentrated and dissolved in acetonitrile (50 mL), and methyl acrylate (14.82 g, 172.2 mmol) was added dropwise and slowly. The reaction solution was heated to reflux for 24 hrs. Glacial acetic acid (10.34 g, 172.2 mmol) and water (50 mL) were added, and the reaction solution was refluxed for 24 hrs. The reaction solution was diluted with water and extracted with *tert-butyl* methyl ether. The organic phase was washed with saturated brine, dried over anhydrous sodium sulfate and filtered, and the filtrate was concentrated by rotary evaporation. The crude product was separated by a normal phase column to obtain methyl 4-methyl-5-oxopentanoate (6.22 g, 48%).

¹H NMR (400 MHz, CDCl₃) δ 9.63 (d, *J =* 1.6 Hz, 1H), 3.68 (s, 3H), 2.48-2.40 (m, 1H), 2.41-2.35 (m, 2H), 2.10-2.02 (m, 1H), 1.71 (dq, *J* = 14.3, 7.3 Hz, 1H), 1.14 (d, *J* = 7.2 Hz, 3H).

### Step 2: synthesis of (3R,8S)-8-methyl-3-phenylhexahydro-5H-oxazolo[3,2-a]pyridin-5-one

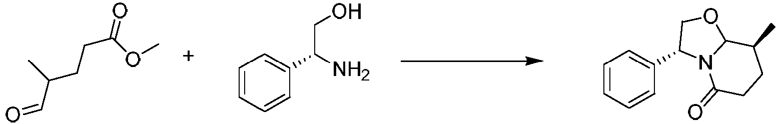

(*R*)-2-amino-2-phenylethan-1-ol (5.92 g, 43.16 mmol) was added to a solution of methyl 4-methyl-5-oxopentanoate (6.222 g, 43.16 mmol) in toluene (100 mL), and then the mixture was heated to reflux for 24 hrs. Water was removed by using a water knockout trap. The reaction solution was concentrated, and the crude product was separated by a normal phase column to obtain (3*R*,8*S*)-8-methyl-3-phenylhexahydro-5*H*-oxazolo[3,2-*a*]pyridin-5-one (4.866 g, 46%). MS m/z (ESI): 232 [M+H]⁺.

¹H NMR (400 MHz, CDCl*₃*) δ 7.33-7.20 (m, 5H), 4.93 (dd, *J* = 6.8, 1.3 Hz, 1H), 4.44 (d, *J* = 8.8 Hz, 1H), 4.17-4.08 (m, 2H), 4.01 (dd, *J* = 9.0, 1.3 Hz, 1H), 2.46-2.28 (m, 2H), 2.02-1.87 (m, 2H), 1.60-1.45 (m, 1H), 1.21 (d, *J* = 6.4 Hz, 3H).

### Step 3: synthesis of (S)-1-((R)-2-hydroxy-1-phenylethyl)-5-methylpiperidin-2-one

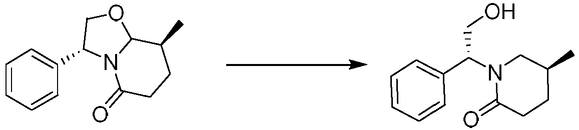

Triethylsilane (7.34 g, 63.12 mmol) and titanium tetrachloride (17.96 g, 94.68 mmol) were added to a solution of (3*R*,8*S*)-8-methyl-3-phenylhexahydro-5*H*-oxazolo[3,2-*a*]pyridin-5-one (4.866 g, 21.04 mmol) in an anhydrous dichloromethane solution (200 mL), and then the mixture was reacted at 50 °C for 24 hrs. Triethylsilane (7.34 g, 63.12 mmol) and titanium tetrachloride (17.96 g, 94.68 mmol) were then added, and the mixture was reacted at 50 °C for 24 hrs. The reaction solution was slowly poured into a saturated sodium bicarbonate solution (500 mL), and the aqueous phase was filtered and extracted with dichloromethane. The organic phases were dried over anhydrous sodium sulfate and filtered, and the filtrate was concentrated. The crude product was separated by a normal phase column to obtain (*S*)-1-((*R*)-2-hydroxy-1-phenylethyl)-5-methylpiperidin-2-one (1.54 g, 31%). MS m/z (ESI): 234 [M+H]⁺.

### Step 4: synthesis of (5)-5-methyl-1-(1-phenylvinyl)piperidin-2-one

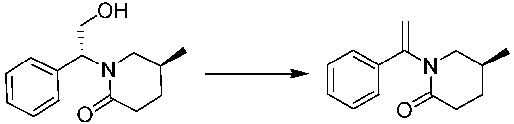

Lithium hydroxide monohydrate (37.05 g, 882.9 mmol) was added to a solution of (*S*)-1-((R)-2-hydroxy-1-phenylethyl)-5-methylpiperidin-2-one (10.3 g, 44.15 mmol) in dimethyl sulfoxide (150 mL), and the mixture was reacted at 135 °C for 4 days. The reaction solution was poured into water and extracted with ethyl acetate. The organic phase was dried over anhydrous sodium sulfate and filtered, and the filtrate was concentrated to obtain (S)-5-methyl-1-(1-phenylvinyl)piperidin-2-one (7.383 g, 66%). MS m/z (ESI): 216 [M+H]⁺.

### Step 5: synthesis of (S)-5-methylpiperidin-2-one

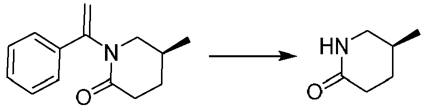

TFA (30 mL) and water (3 mL) were added to a solution of (*S*)-5-methyl-1-(1-phenylvinyl) piperidin-2-one (7.28 g, 33.81 mmol) in dichloromethane (30 mL), and the mixture was reacted at 40 °C overnight. The reaction solution was concentrated, and the concentrate was separated by a reversed-phase column to obtain a crude product. The crude product was lyophilized, and then aqua ammonia (3 mL) was added. The mixture was concentrated to dryness, and the concentrate was slurried with ethyl acetate and filtered to obtain (S)-5-methylpiperidin-2-one (2.52 g, 63%). MS m/z (ESI): 114 [M+H]⁺.

¹H NMR (400 MHz, MeOH-*d*₄) δ 3.30-3.24 (m, 1H), 2.86 (dd, *J* = 12.2, 10.1 Hz, 1H), 2.39-2.26 (m, 2H), 1.96-1.80 (m, 2H), 1.54-1.40 (m, 1H),1.01 (d, *J* = 6.5 Hz, 3H).

### Step 6: synthesis of tert-butyl (S)-5-methyl-2-oxopiperidine-1-carboxylate

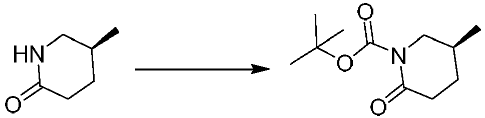

4-Dimethylaminopyridine (327 mg, 2.68 mmol) and di-*tert*-butyl dicarbonate (6.22 g, 26.78 mmol) were added to a solution of (*S*)-5-methylpiperidin-2-one (2.02 g, 17.85 mmol) in tetrahydrofuran (25 mL), and the mixture was stirred at 25 °C overnight. The reaction solution was concentrated, diluted with dichloromethane and washed with a 5% potassium hydrogen sulfate solution. The organic phase was dried over anhydrous sodium sulfate and filtered, and the filtrate was concentrated to obtain *tert-*butyl (*S*)-5-methyl-2-oxopiperidine-1-carboxylate (3.48 g, 70%). MS m/z (ESI): 214 [M+H]⁺.

¹H NMR (400 MHz, *CDCl₃*) δ 3.83-3.75 (m, 1H), 3.11 (dd, *J* = 12.7, 10.4 Hz, 1H), 2.63-2.53 (m, 1H), 2.53-2.41 (m, 1H), 2.01-1.93 (m, 1H), 1.92-1.83 (m, 1H), 1.53 (s, 9H), 1.46 -1.40 (m, 1H),1.04 (d, *J* = 6.6 Hz, 3H).

### Step 7: synthesis of tert-butyl (S)-3-methyl-6-(((trifluoromethyl)sulfonyl)oxy)-3,4-dihydropyridine-1(2H)-carboxylate

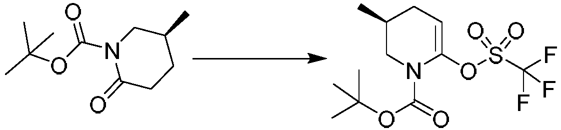

Sodiobis(trimethylsilyl)amine (3.52 mL, 7.03 mmol) was added to a solution of *tert-butyl* (*S*)-5-methyl-2-oxopiperidine-1-carboxylate (1.00 g, 4.69 mmol) in anhydrous tetrahydrofuran (25 mL) at -78 °C. The reaction solution was reacted at this temperature for 1 hr. N-phenylbis(trifluoromethanesulphonimide) (2.18 g, 6.10 mmol) was then added. The reaction solution was slowly warmed and stirred at room temperature for 3.5 hrs. The reaction solution was diluted with ethyl acetate and washed with saturated brine. The organic phase was dried over anhydrous sodium sulfate and filtered, and the filtrate was concentrated. The crude product was separated by a normal phase column to obtain *tert-butyl* (S)-3-methyl-6-(((trifluoromethyl) sulfonyl)oxy)-3,4-dihydropyridine-1(2*H*)-carboxylate (1.05 g, 62%). MS m/z (ESI): 290 [M+H-56]⁺.

¹H NMR (400 MHz, *CDCl*₃) δ 5.25 (t, *J* = 3.8 Hz, 1H), 3.87 (dd, *J* = 12.8, 3.3 Hz, 1H), 3.00 (dd, *J* = 12.7, 9.1 Hz, 1H), 2.44-2.34 (m, 1H), 1.98-1.88 (m, 1H), 1.88-1.78 (m, 1H), 1.49 (s, 9H), 0.99 (d, *J* = 6.6 Hz, 3H).

### Step 8: synthesis of tert-butyl (S)-6-(benzo[d]thiazol-5-yl)-3-methyl-3,4-dihydropyridine-1(2H)-carboxylate

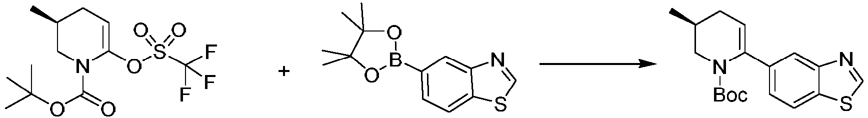

*Tert*-butyl (*S*)-3-methyl-6-(((trifluoromethyl)sulfonyl)oxy)-3,4-dihydropyridine-1(2*H*)-carboxylate (1.39 g, 4.02 mmol), 5-(4,4,5,5-tetramethyl-1,3,2-dioxaborolan-2-yl)benzo[d]thiazole (1.00 g, 3.83 mmol), sodium carbonate (1.01 g, 9.57 mmol) and palladium 1,1'-didiphenylphosphineferrocenedichloride (140 mg, 0.191 mmol) were added to 1,4-dioxane (30 mL) and water (10 mL), and the mixture was reacted at 90 °C for 18 hrs under nitrogen protection. The reaction solution was diluted with ethyl acetate and washed with saturated brine. The organic phase was dried over anhydrous sodium sulfate and filtered, and the filtrate was concentrated. The crude product was separated by a normal phase column to obtain *tert-*butyl (*S*)-6-(benzo[d]thiazol-5-yl)-3-methyl-3,4-dihydropyridine-1(2H)-carboxylate (1.01 g, 75%). MS m/z (ESI): 331 [M+H]⁺.

### Step 9: synthesis of (S)-5-(5-methyl-3,4,5,6-tetrahydropyridin-2-yl)benzo[d]thiazole

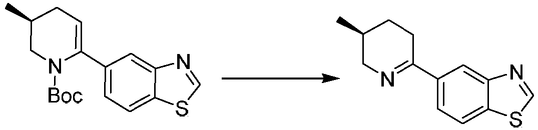

Trifluoroacetic acid (3 mL, 2.68 mmol) was added to a solution of *tert*-butyl (*S*)-6-(benzo[*d*]thiazol-5-yl)-3-methyl-3,4-dihydropyridine-1(2*H*)-carboxylate (1.01 g, 3.05 mmol) in dichloromethane (9 mL), and the mixture was reacted overnight at 25 °C. The reaction solution was concentrated and diluted with water. The pH was adjusted to 8 with aqua ammonia, and the mixture was extracted with dichloromethane. The organic phase was dried over anhydrous sodium sulfate and filtered, and the filtrate was concentrated to obtain (*S*)-5-(5-methyl-3,4,5,6-tetrahydropyridin-2-yl)benzo[*d*]thiazole (0.70 g, 92%). MS m/z (ESI): 231 [M+H]⁺.

### Step 10: synthesis of 5-((2R,5S)-5-methylpiperidin-2-yl)benzo[d]thiazole

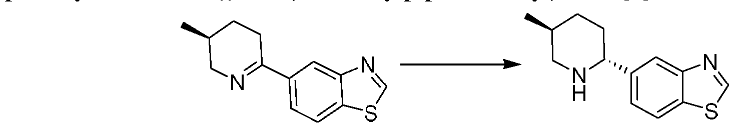

Sodium borohydride (308 mg, 9.12 mmol) was added to a solution of (*S*)-5-(5-methyl-3,4,5,6-tetrahydropyridin-2-yl)benzo[d]thiazole (0.70 g, 3.04 mmol) in methanol (5 mL) under an ice-water bath, and the reaction solution was stirred at room temperature for 45 min. The reaction solution was concentrated and diluted with water. The pH was adjusted to 8 with aqua ammonia, and the mixture was extracted with dichloromethane. The organic phase was dried over anhydrous sodium sulfate and filtered, and the filtrate was concentrated to obtain 5-((2*R*,5*S*)-5-methylpiperidin-2-yl)benzo[d]thiazole (0.69 g, 98%). MS m/z (ESI): 233 [M+H]⁺.

¹H NMR (400 MHz, *CDCl*₃) δ 8.98 (s, 1H), 8.15 (d, *J* = 1.7 Hz, 1H), 7.88 (d, *J* = 8.3 Hz, 1H), 7.56 (dd, *J* = 8.3, 1.7 Hz, 1H), 3.78 (dd, *J* = 11.4, 2.6 Hz, 1H), 3.22-3.13 (m, 1H), 2.48 (t, *J* = 11.4 Hz, 1H), 1.97-1.87 (m, 2H), 1.86-1.68 (m, 2H), 1.30-1.19 (m, 1H), 0.92 (d, *J* = 6.6 Hz, 3H).

### Intermediate 49 can be prepared by selecting corresponding starting materials according to all or part of the synthesis method of Intermediate 48:

| **Intermediate No.** | **Structural formula** | **Chemical name** | **MS m/z [M+H]⁺** |
|---|---|---|---|
| **49** | 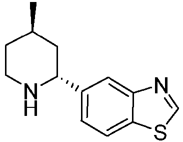 | 5-((2*R*,4*R*)-4-methylpiperidin-2-yl)benzo[*d*]thiazole | **233** |

### Preparation of intermediate 50: (3R,5S)-3-methyl-5-(5-(trifluoromethyl)pyridin-2-yl) morpholine

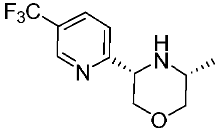

### Step 1: synthesis of (R)-1-((tributylstannyl)methoxy)propan-2-amine

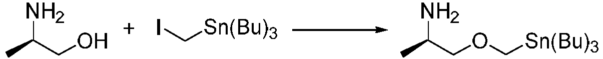

Sodium hydride (204 mg, 5.10 mmol, 60% in kerosene) was dissolved in *N,N-*dimethylformamide (20 mL), and the mixture was cooled under an ice-water bath. (*R*)-2-Aminopropan-1-ol (0.36 mL, 4.64 mmol) was added dropwise and slowly. After the dropwise addition was completed, the mixture was stirred at room temperature for 1 hr. The reaction solution was cooled under an ice-water bath, and a solution of tributyl(iodomethyl)stannane (2.00 g, 4.64 mmol) in *N,N-*dimethylformamide (20 mL) was added dropwise and slowly. The mixture was stirred at room temperature overnight. The reaction solution was cooled under an ice bath, and a saturated ammonium chloride solution was slowly added thereto to quench the reaction. Liquid separation was performed, and the organic phase was washed with saturated brine. The organic phase was concentrated by rotary evaporation, and the crude product was separated by column chromatography to obtain (R)-1-((tributylstannyl)methoxy)propan-2-amine (1.30 g, 71%). MS m/z (ESI): 380 [M+H]⁺.

### Step 2: synthesis of (R,E)-N-(1-((tributylstannyl)methoxy)propan-2-yl)-1-(5-(trifluoromethyl)pyridin-2-yl)methanimine

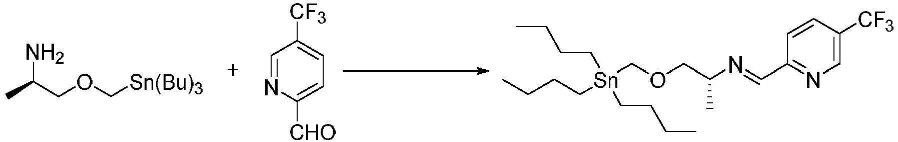

(*R*)-1-((tributylstannyl)methoxy)propan-2-amine (800 mg, 2.11 mmol) was dissolved in dichloromethane (20 mL), and then 5-(trifluoromethyl)picolinaldehyde (370 mg, 2.11 mmol) and molecular sieves (1.50 g, 3.41 mmol) were added, and then the mixture was stirred at room temperature overnight. The reaction solution was filtered and washed with dichloromethane, and the filtrate was concentrated by rotary evaporation to obtain (*R,E*)-*N*-(1-((tributylstannyl) methoxy)propan-2-yl)-1-(5-(trifluoromethyl)pyridin-2-yl)methanimine (1100 mg, 97%).

¹H NMR (400 MHz, CDCl₃) δ 8.90 (dt, *J* = 1.9, 0.9 Hz, 1H), 8.40 (s, 1H), 8.14 (dt, *J* = 8.3, 0.9 Hz, 1H), 7.95 (dd, *J* = 8.3, 2.3 Hz, 1H), 3.79-3.63 (m, 3H), 3.43 (d, *J* = 6.2 Hz, 2H), 1.48-1.36 (m, 6H), 1.28-1.18 (m, 9H), 0.95-0.81 (m, 15H).

### Step 3: synthesis of (3R,5S)-3-methyl-5-(5-(trifluoromethyl)pyridin-2-yl)morpholine

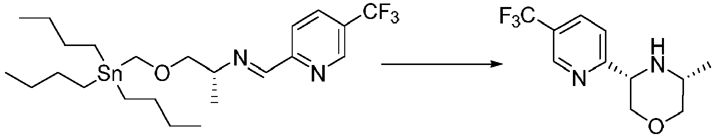

(*S,S*)-2,2'-isopropylidenebis(4-phenyl-2-oxazoline) (70 mg, 0.21 mmol) was dissolved in hexafluoroisopropanol (10 mL), and then copper trifluoromethanesulfonate (151 mg, 0.42 mmol) was added. The mixture was stirred at room temperature overnight. A solution of (*R,E*)-*N*-(1-((tributylstannyl)methoxy)propan-2-yl)-1-(5-(trifluoromethyl)pyridin-2-yl)methanimine (1113 mg, 2.08 mmol) in hexafluoroisopropanol (5 mL) was added to the reaction solution via a syringe, and the mixture was stirred at room temperature for 24 hrs. The reaction solution was diluted with dichloromethane and aqua ammonia, and liquid separation was performed. The organic phase was concentrated by rotary evaporation, and the crude product was separated by reversed-phase column chromatography to obtain (3*R*,5*S*)-3-methyl-5-(5-(trifluoromethyl) pyridin-2-yl)morpholine (195 mg, 37%). MS m/z (ESI): 247 [M+H]⁺.

### II. Preparation of Examples

### Example 1: preparation of (R)-4-amino-1-methyl-N-(1-(pyrimidin-2-yl)ethyl)-N-((5-(trifluoromethyl)pyridin-2-yl)methyl)imidazo[1,5-a]quinoxaline-8-carboxamide

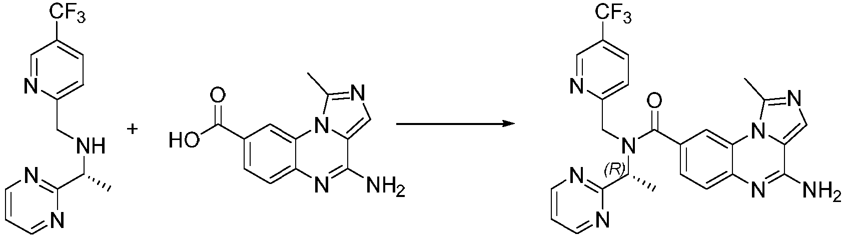

4-Amino-1-methylimidazo[1,5-*a*]quinoxaline-8-carboxylic acid (30.0 mg, 0.12 mmol) and 2-methoxy-*N*-((5-(trifluoromethyl)pyridin-2-yl)methyl)ethan-1-amine (34.9 mmol,0.12 mmol) were dissolved in *N,N*-dimethylacetamide (3 mL), and then bromo-tris-pyrrolidino-phosphonium hexafluorophosphate (75.1 mmol, 0.16 mmol) and *N,N*-diisopropylethylamine (48.0 mg, 0.37 mmol) were added. The mixture was stirred overnight at room temperature. After the reaction was completed, water was added to quench the reaction, and the mixture was extracted three times with ethyl acetate. The organic phases were combined, washed with saturated brine, dried over anhydrous sodium sulfate, filtered and concentrated, and the concentrate was separated by silica gel column chromatography [9% methanol-dichloromethane system] to obtain a crude product, which was then separated by a reversed-phase column [50% acetonitrile-10 nM aqueous ammonium bicarbonate solution system] and lyophilized to obtain (*R*)-4-amino-1-methyl-*N*-(1-(pyrimidin-2-yl)ethyl)-*N*-((5-(trifluoromethyl)pyridin-2-yl)methyl)imidazo[1,5-*a*]quinoxaline-8-carboxamide (18 mg, 28%). MS m/z (ESI): 507 [M+H]⁺.

¹H NMR (400 MHz, DMSO-*d*₆) δ 8.88-8.76 (m, 3H), 8.29 (s, 1H), 8.11 (dd, *J* = 8.5, 2.4 Hz, 1H), 7.80 (s, 1H), 7.61-7.47 (m, 3H), 7.41 (t, *J* = 4.9 Hz, 1H), 7.30 (s, 2H), 5.56 (s, 1H), 5.00-4.47 (m, 2H), 2.89 (s, 3H), 1.60 (d, *J* = 7.0 Hz, 3H).

### Example 2: Preparation of (S)-4-amino-7-fluoro-1-methyl-N-(1-methyl-1H-pyrazol-4-yl)-N-(6-(trifluoromethyl)-2,3-dihydrobenzofuran-3-yl)imidazo[1,5-a]quinoxaline-8-carboxamide

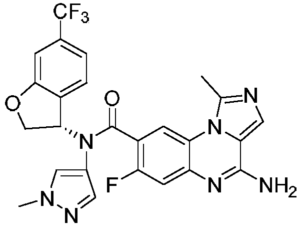

### Step 1: synthesis of 4-amino-7-fluoro-1-methylimidazo[1,5-a]quinoxaline-8-carbonyl chloride hydrochloride

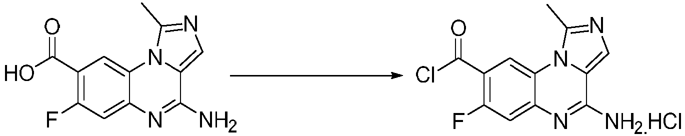

4-Amino-7-fluoro-1-methylimidazo[1,5-*a*]quinoxaline-8-carboxylic acid (100 mg, 0.38 mmol) was placed in dichloromethane (10 mL), and then a solution of hydrochloric acid in 1,4-dioxane (0.5 mL, 2.0 mmol, 4 mol/L) was added. The mixture was stirred at room temperature for half an hour, and the reaction solution was concentrated to dryness by rotary evaporation under reduced pressure. Thionyl chloride (1.0 mL, 13.79 mmol) was added to the residue, and the mixture was stirred at 65 °C overnight. The reaction solution was concentrated by rotary evaporation under reduced pressure, and dry dichloromethane (5 mL) was added to the residue. The mixture was concentrated by rotary evaporation to obtain 4-amino-7-fluoro-1-methylimidazo[1,5-*a*]quinoxaline-8-carbonyl chloride hydrochloride (118 mg, 99%). MS m/z (ESI): 275 [M+H]⁺.

### Step 2: synthesis of (S)-4-amino-7-fluoro-1-methyl-N-(1-methyl-1H-pyrazol-4-yl)-N-(6-(trifluoromethyl)-2,3-dihydrobenzofuran-3-yl)imidazo[1,5-a]quinoxaline-8-carboxamide

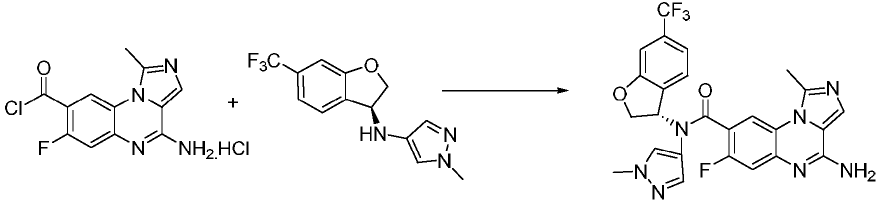

4-Amino-7-fluoro-1-methylimidazo[1,5-*a*]quinoxaline-8-carbonyl chloride hydrochloride (77 mg, 0.24 mmol) was placed in dichloroethane (6 mL), and then pyridine (0.10 mL, 1.22 mmol) and (*S*)-1-methyl-*N*-(6-(trifluoromethyl)-2,3-dihydrobenzofuran-3-yl)-1*H*-pyrazol-4-amine (69 mg, 0.24 mmol) were added. The mixture was stirred at 60 °C for 5 hrs. After the reaction was completed, the reaction solution was concentrated by rotary evaporation, and the crude product was separated by reversed-phase column chromatography to obtain (*S*)-4-amino-7-fluoro-1-methyl-*N*-(1-methyl-1*H*-pyrazol-4-yl)-*N*-(6-(trifluoromethyl)-2,3-dihydrobenzofuran-3-yl)imidazo[1,5-*a*]quinoxaline-8-carboxamide (32 mg, 25%). MS m/z (ESI): 526 [M+H]⁺.

¹H NMR (400 MHz, DMSO-*d*₆) δ 7.96 (d, *J* = 6.2 Hz, 1H), 7.80 (s, 1H), 7.71 (d, *J* = 7.9 Hz, 1H), 7.46 (s, 2H), 7.41 (s, 1H), 7.33 (d, *J* = 7.9 Hz, 1H), 7.11 (s, 1H), 6.99 (d, *J* = 10.6 Hz, 1H), 6.87 (s, 1H), 6.61 (s, 1H), 4.88 (t, *J* = 10.0 Hz, 1H), 4.67 (dd, *J =* 10.6, 4.0 Hz, 1H), 3.50 (s, 3H), 2.93 (s, 3H).

**Examples 3-233** can be prepared by selecting corresponding starting materials according to all or part of the synthesis method of **Example 1** or **Example 2.**

| **Example No.** | **Structural formula** | **Chemical name** | **M/Z [M+H]⁺** |
|---|---|---|---|
| 3 | 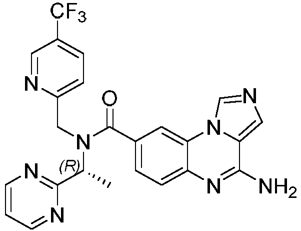 | (*R*)-4-amino-*N*-(1-(pyrimidin-2-yl)ethyl)-*N*-((5-(trifluoromethyl)pyridin-2-yl)methyl)imidazo[1,5-*a*]quinoxaline-8-carboxamide | **493** |
| 4 | 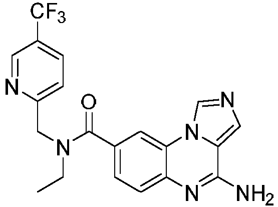 | 4-amino-N-ethyl-*N*-((5-(trifluoromethyl)pyridin-2-yl)methyl)imidazo[1,5-*a*]quinoxaline-8-carboxamide | **415** |
| 5 | 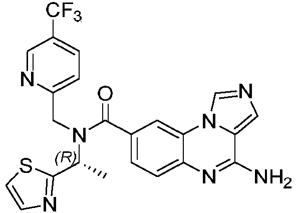 | (*R*)-4-amino-*N*-(1-(thiazol-2-yl)ethyl)-*N*-((5-(trifluoromethyl)pyridin-2-yl)methyl)imidazo[1,5-*a*]quinoxaline-8-carboxamide | **498** |
| 6 | 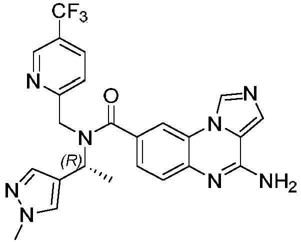 | (*R*)-4-amino-*N*-(1-(1-methyl-1*H*-pyrazol-4-yl)ethyl)-*N*-((5-(trifluoromethyl)pyridin-2-yl)methyl)imidazo[1,5-*a*]quinoxaline-8-carboxamide | **495** |
| 7 | 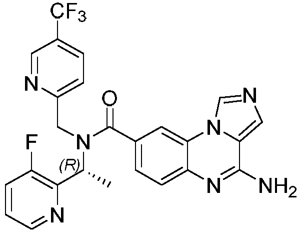 | (*R*)-4-amino-*N*-(1-(3-fluoropyridin-2-yl)ethyl)-*N*-((5-(trifluoromethyl)pyridin-2-yl)methyl)imidazo[1,5-*a*]quinoxaline-8-carboxamide | **510** |
| 8 | 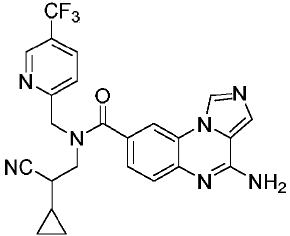 | 4-amino-*N*-(2-cyano-2-cyclopropylethyl)-*N*-((5-(trifluoromethyl)pyridin-2-yl)methyl)imidazo[1,5-*a*]quinoxaline-8-carboxamide | **480** |
| 9 | 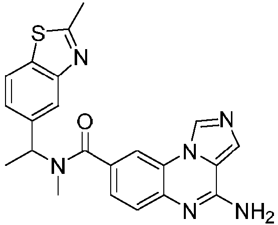 | 4-amino-*N*-methyl-*N*-(1-(2-methylbenzo[*d*]thiazol-5-yl)ethyl)imidazo[1,5-*a*]quinoxaline-8-carboxamide | **416** |
| 10 | 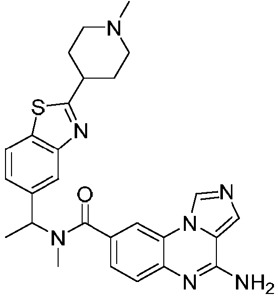 | 4-amino-*N*-methyl-*N*-(1-(2-(1-methylpiperidin-4-yl)benzo[*d*]thiazol-5-yl)ethyl)imidazo[1,5-*a*]quinoxaline-8-carboxamide | **500** |
| 11 | 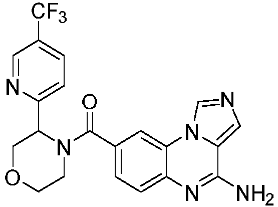 | (4-aminoimidazo[1,5-*a*]quinoxalin-8-yl)(3-(5-(trifluoromethyl)pyridin-2-yl)morpholino)methanone | **443** |
| 12 | 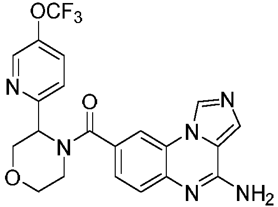 | (4-aminoimidazo[1,5-*a*]quinoxalin-8-yl)(3-(5-(trifluoromethoxy)pyridin-2-yl)morpholino)methanone | **459** |
| 13 | 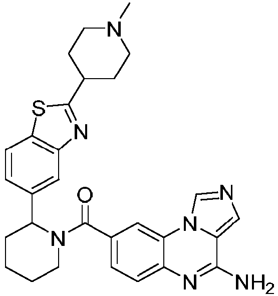 | (4-aminoimidazo[1,5-*a*]quinoxalin-8-yl)(2-(2-(1-methylpiperidin-4-yl)benzo[*d*]thiazol-5-yl)piperidin-1-yl)methanone | **526** |
| 14 | 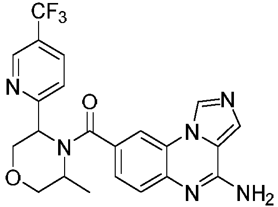 | (4-aminoimidazo[1,5-*a*]quinoxalin-8-yl)(3-methyl-5-(5-(trifluoromethyl)pyridin-2-yl)morpholino)methanone | **457** |
| 14a | 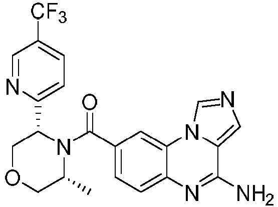 | (4-aminoimidazo[1,5-*a*]quinoxalin-8-yl)((3*R*,5*S*)-3-methyl-5-(5-(trifluoromethyl)pyridin-2-yl)morpholino)methanone | **457** |
| 15 | 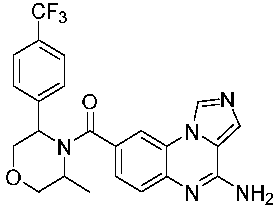 | (4-aminoimidazo[1,5-*a*]quinoxalin-8-yl)(3-methyl-5-(4-(trifluoromethyl)phenyl)morphol ino)methanone | **456** |
| 16 | 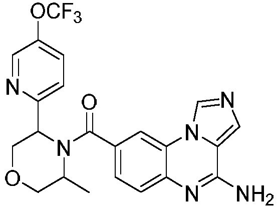 | (4-aminoimidazo[1,5-*a*]quinoxalin-8-yl)(3-methyl-5-(5-(trifluoromethoxy)pyridin-2-yl)morpholino)methanone | **473** |
| 17 | 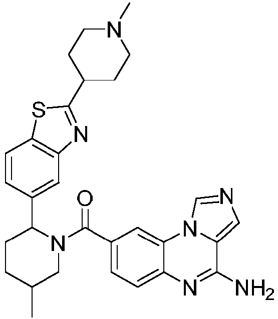 | (4-aminoimidazo[1,5-*a*]quinoxalin-8-yl)(5-methyl-2-(2-(1-methylpiperidin-4-yl)benzo[*d*]thiazol-5-yl)piperidin-1-yl)methanone | **540** |
| 18 | 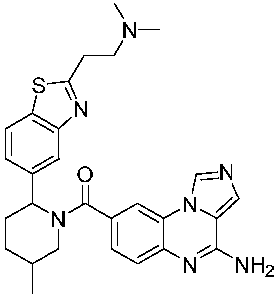 | (4-aminoimidazo[1,5-*a*]quinoxalin-8-yl)(2-(2-(2-(dimethylamino)ethyl)benzo[d]t hiazol-5-yl)-5-methylpiperidin-1-yl)methanone | **514** |
| 19 | 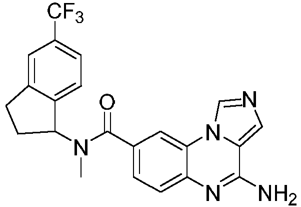 | 4-amino-*N*-methyl-*N*-(5-(trifluoromethyl)-2,3-dihydro-1*H*-inden-1-yl)imidazo[1,5-*a*]quinoxaline-8-carboxamide | **426** |
| 20 | 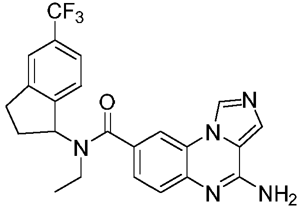 | 4-amino-*N*-ethyl-*N*-(5-(trifluoromethyl)-2,3-dihydro-1*H*-inden-1-yl)imidazo[1,5-*a*]quinoxaline-8-carboxamide | **440** |
| 21 | 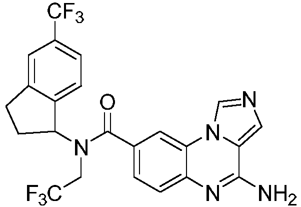 | 4-amino-*N*-(2,2,2-trifluoroethyl)-*N*-(5-(trifluoromethyl)-2,3-dihydro-1*H*-inden-1-yl)imidazo[1,5-*a*]quinoxaline-8-carboxamide | **494** |
| 22 | 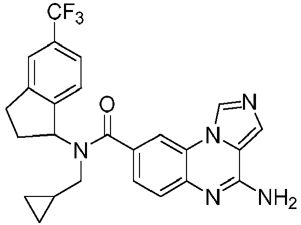 | 4-amino-*N*-(cyclopropylmethyl)-*N*-(5-(trifluoromethyl)-2,3-dihydro-1*H*-inden-1-yl)imidazo[1,5-*a*]quinoxaline-8-carboxamide | **466** |
| 22a | 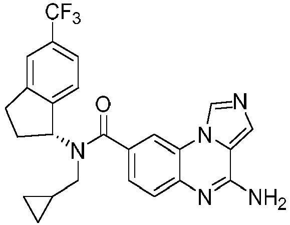 | (*R*)-4-amino-*N-*(cyclopropylmethyl)-N-(5-(trifluoromethyl)-2,3-dihydro-1*H*-inden-1-yl)imidazo[1,5-*a*]quinoxaline-8-carboxamide | **466** |
| 23 | 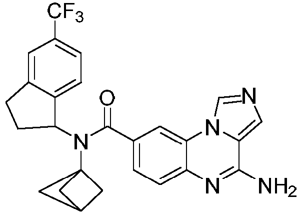 | 4-amino-*N-*(bicyclo[1.1.1]pentan-1-yl)-*N-*(5-(trifluoromethyl)-2,3-dihydro-1*H*-inden-1-yl)imidazo[1,5-*a*]quinoxaline-8-carboxamide | **478** |
| 24 | 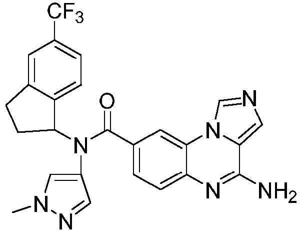 | 4-amino-*N*-(1-methyl-1*H-*pyrazol-4-yl)-*N*-(5-(trifluoromethyl)-2,3-dihydro-1*H*-inden-1-yl)imidazo[1,5-*a*]quinoxaline-8-carboxamide | **492** |
| 25 | 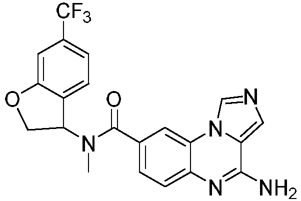 | 4-amino-N-methyl-*N*-(6-(trifluoromethyl)-2,3-dihydrobenzofuran-3-yl)imidazo[1,5-*a*]quinoxaline-8-carboxamide | **428** |
| 26 | 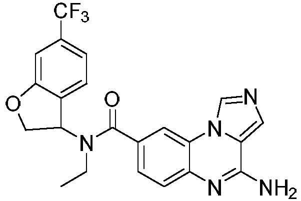 | 4-amino-*N*-ethyl-*N*-(6-(trifluoromethyl)-2,3-dihydrobenzofuran-3-yl)imidazo[1,5-*a*]quinoxaline-8-carboxamide | **441** |
| 27 | 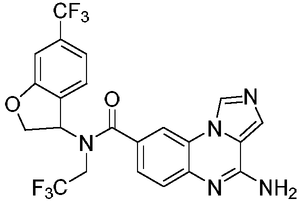 | 4-amino-*N*-(2,2,2-trifluoroethyl)-*N*-(6-(trifluoromethyl)-2,3-dihydrobenzofuran-3-yl)imidazo[1,5-*a*]quinoxaline-8-carboxamide | **496** |
| 28 | 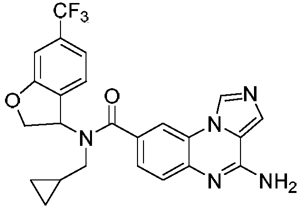 | 4-amino-*N*-(cyclopropylmethyl)-*N*-(6-(trifluoromethyl)-2,3-dihydrobenzofuran-3-yl)imidazo[1,5-*a*]quinoxaline-8-carboxamide | **468** |
| 29 | 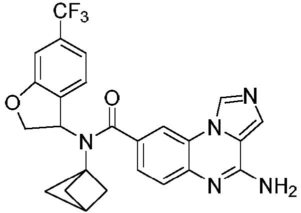 | 4-amino-*N-*(bicyclo[1.1.1]pentan-1-yl)-*N-*(6-(trifluoromethyl)-2,3-dihydrobenzofuran-3-yl)imidazo[1,5-*a*]quinoxaline-8-carboxamide | **480** |
| 30 | 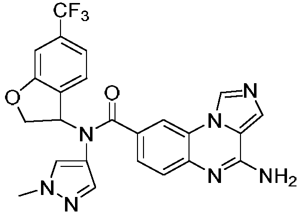 | 4-amino-*N*-(1-methyl-1*H-*pyrazol-4-yl)-*N*-(6-(trifluoromethyl)-2,3-dihydrobenzofuran-3-yl)imidazo[1,5-*a*]quinoxaline-8-carboxamide | **494** |
| 30a | 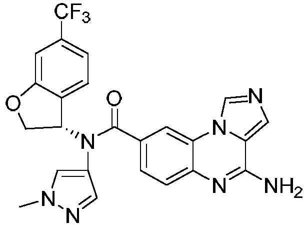 | (*S*)-4-amino-*N*-(1-methyl-1*H-*pyrazol-4-yl)-*N*-(6-(trifluoromethyl)-2,3-dihydrobenzofuran-3-yl)imidazo[1,5-*a*]quinoxaline-8-carboxamide | **494** |
| 31 | 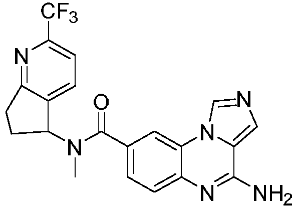 | 4-amino-*N*-methyl-*N*-(2-(trifluoromethyl)-6,7-dihydro-5*H*-cyclopenta[*b*]pyridin-5-yl)imidazo[1,5-*a*]quinoxaline-8-carboxamide | **427** |
| 32 | 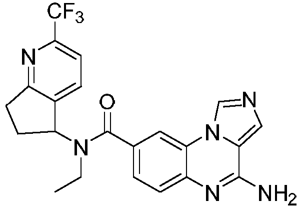 | 4-amino-*N*-ethyl-*N*-(2-(trifluoromethyl)-6,7-dihydro-5*H*-cyclopenta[*b*]pyridin-5-yl)imidazo[1,5-*a*]quinoxaline-8-carboxamide | **441** |
| 33 | 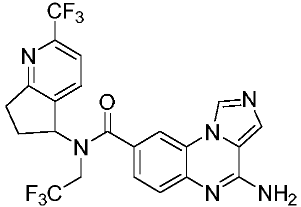 | 4-amino-*N*-(2,2,2-trifluoroethyl)-*N*-(2-(trifluoromethyl)-6,7-dihydro-5*H*-cyclopenta[*b*]pyridin-5-yl)imidazo[1,5-*a*]quinoxaline-8-carboxamide | **495** |
| 34 | 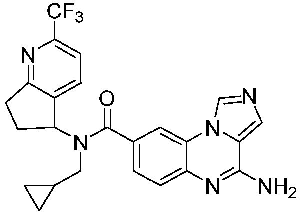 | 4-amino-*N*-(cyclopropylmethyl)-*N*-(2-(trifluoromethyl)-6,7-dihydro-5*H-*cyclopenta[*b*]pyridin-5-yl)imidazo[1,5-*a*]quinoxaline-8-carboxamide | **467** |
| 34a | 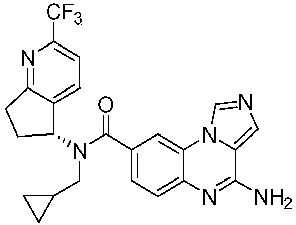 | (*R*)-4-amino-*N-*(cyclopropylmethyl)-*N*-(2-(trifluoromethyl)-6,7-dihydro-5*H*-cyclopenta[*b*]pyridin-5-yl)imidazo[1,5-*a*]quinoxaline-8-carboxamide | **467** |
| 34b | 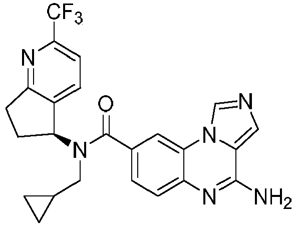 | (*S*)-4-amino-*N-*(cyclopropylmethyl)-*N*-(2-(trifluoromethyl)-6,7-dihydro-5*H*-cyclopenta[*b*]pyridin-5-yl)imidazo[1,5-*a*]quinoxaline-8-carboxamide | **467** |
| 35 | 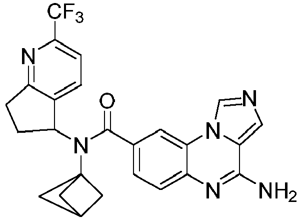 | 4-amino-*N-*(bicyclo[1.1.1]pentan-1-yl)-*N-*(2-(trifluoromethyl)-6,7-dihydro-5*H-*cyclopenta[b]pyridin-5-yl)imidazo[1,5-*a*]quinoxaline-8-carboxamide | **479** |
| 36 | 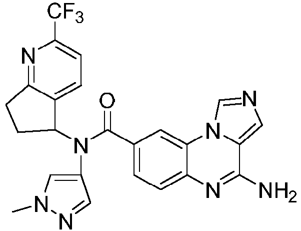 | 4-amino-*N*-(1-methyl-1*H-*pyrazol-4-yl)-*N*-(2-(trifluoromethyl)-6,7-dihydro-5*H-*cyclopenta[*b*]pyridin-5-yl)imidazo[1 ,5-*a*]quinoxaline-8-carboxamide | **493** |
| 37 | 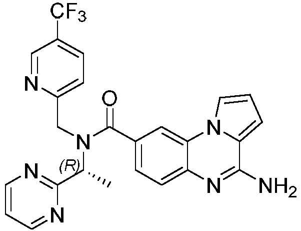 | (*R*)-4-amino-*N*-(1-(pyrimidin-2-yl)ethyl)-*N*-((5-(trifluoromethyl)pyridin-2-yl)methyl)pyrrolo[1,2-*a*]quinoxaline-8-carboxamide | **492** |
| 38 | 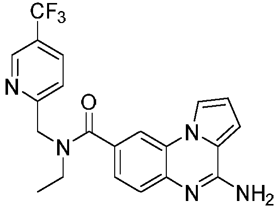 | 4-amino-*N*-ethyl-*N*-((5-(trifluoromethyl)pyridin-2-yl)methyl)pyrrolo[1,2-*a*]quinoxaline-8-carboxamide | **414** |
| 39 | 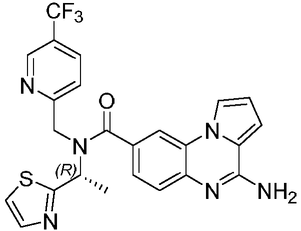 | (*R*)-4-amino-*N*-(1-(thiazol-2-yl)ethyl)-*N*-((5-(trifluoromethyl)pyridin-2-yl)methyl)pyrrolo[1,2-*a*]quinoxaline-8-carboxamide | **497** |
| 40 | 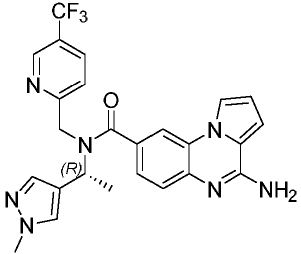 | (*R*)-4-amino-*N*-(1-(1-methyl-1*H*-pyrazol-4-yl)ethyl)-*N*-((5-(trifluoromethyl)pyridin-2-yl)methyl)pyrrolo[1,2-*a*]quinoxaline-8-carboxamide | **494** |
| 41 | 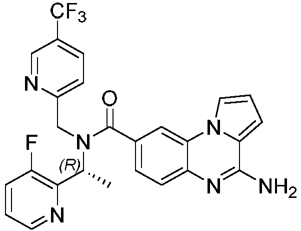 | (*R*)-4-amino-*N*-(1-(3-fluoropyridin-2-yl)ethyl)-*N*-((5-(trifluoromethyl)pyridin-2-yl)methyl)pyrrolo[1,2-*a*]quinoxaline-8-carboxamide | **509** |
| 42 | 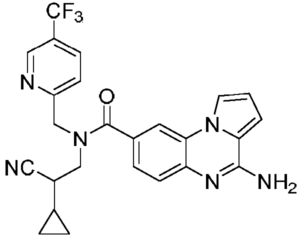 | 4-amino-*N*-(2-cyano-2-cyclopropylethyl)-*N*-((5-(trifluoromethyl)pyridin-2-yl)methyl)pyrrolo[1,2-*a*]quinoxaline-8-carboxamide | **479** |
| 43 | 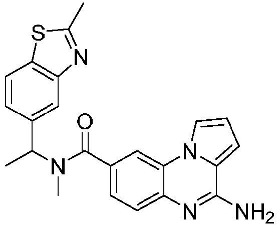 | 4-amino-*N*-methyl-*N*-(1-(2-methylbenzo[*d*]thiazol-5-yl)ethyl)pyrrolo[1,2-*a*]quinoxaline-8-carboxamide | **416** |
| 44 | 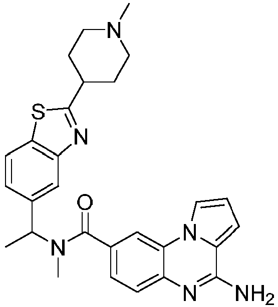 | 4-amino-*N*-methyl-*N*-(1-(2-(1-methylpiperidin-4-yl)benzo[*d*]thiazol-5-yl)ethyl)pyrrolo[1,2-*a*]quinoxaline-8-carboxamide | **499** |
| 45 | 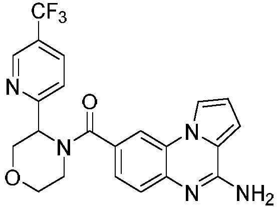 | (4-aminopyrrolo[1,2-*a*]quinoxalin-8-yl)(3-(5-(trifluoromethyl)pyridin-2-yl)morpholino)methanone | **442** |
| 46 | 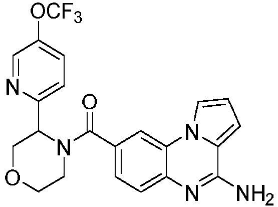 | (4-aminopyrrolo[1,2-*a*]quinoxalin-8-yl)(3-(5-(trifluoromethoxy)pyridin-2-yl)morpholino)methanone | **458** |
| 47 | 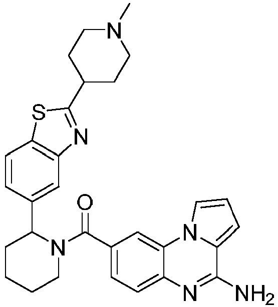 | (4-aminopyrrolo[1,2-*a*]quinoxalin-8-yl)(2-(2-(1-methylpiperidin-4-yl)benzo[*d*]thiazol-5-yl)piperidin-1-yl)methanone | **525** |
| 48 | 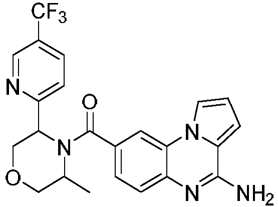 | (4-aminopyrrolo[1,2-*a*]quinoxalin-8-yl)(3-methyl-5-(5-(trifluoromethyl)pyridin-2-yl)morpholino)methanone | **456** |
| 49 | 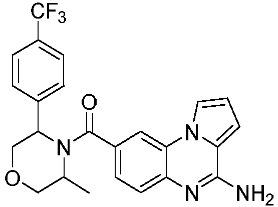 | (4-aminopyrrolo[1,2-*a*]quinoxalin-8-yl)(3-methyl-5-(4-(trifluoromethyl)phenyl)morphol ino)methanone | **455** |
| 50 | 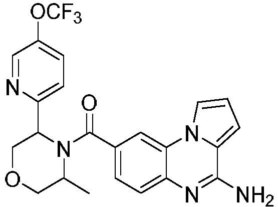 | (4-aminopyrrolo[1,2-*a*]quinoxalin-8-yl)(3-methyl-5-(5-(trifluoromethoxy)pyridin-2-yl)morpholino)methanone | **472** |
| 51 | 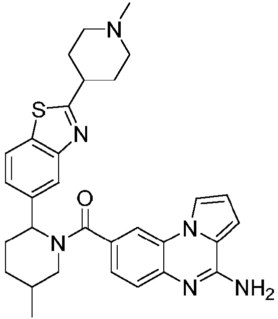 | (4-aminopyrrolo[1,2-*a*]quinoxalin-8-yl)(5-methyl-2-(2-(1-methylpiperidin-4-yl)benzo[d]thiazol-5-yl)piperidin-1-yl)methanone | **539** |
| 52 | 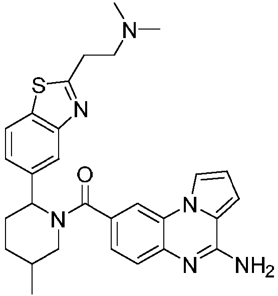 | (4-aminopyrrolo[1,2-*a*]quinoxalin-8-yl)(2-(2-(2-(dimethylamino)ethyl)benzo[d]t hiazol-5-yl)-5-methylpiperidin-1-yl)methanone | **513** |
| 53 | 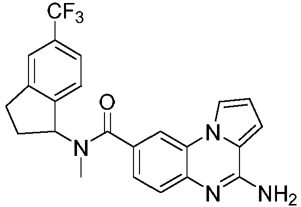 | 4-amino-*N*-methyl-*N*-(5-(trifluoromethyl)-2,3-dihydro-1*H*-inden-1-yl)pyrrolo[1,2-*a*]quinoxaline-8-carboxamide | **425** |
| 54 | 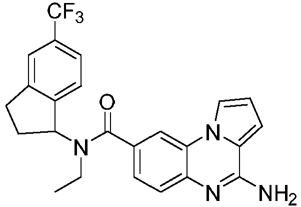 | 4-amino-*N*-ethyl-*N*-(5-(trifluoromethyl)-2,3-dihydro-1*H*-inden-1-yl)pyrrolo[1,2-*a*]quinoxaline-8-carboxamide | **439** |
| 55 | 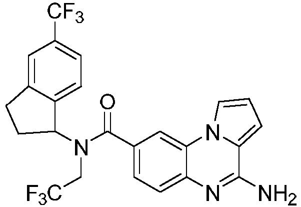 | 4-amino-*N*-(2,2,2-trifluoroethyl)-*N*-(5-(trifluoromethyl)-2,3-dihydro-1*H*-inden-1-yl)pyrrolo[1,2-*a*]quinoxaline-8-carboxamide | **493** |
| 56 | 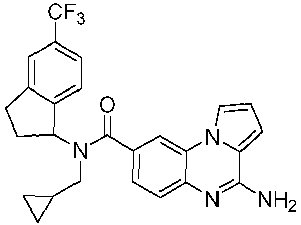 | 4-amino-*N*-(cyclopropylmethyl)-*N*-(5-(trifluoromethyl)-2,3-dihydro-1*H*-inden-1-yl)pyrrolo[1,2-*a*]quinoxaline-8-carboxamide | **465** |
| 57 | 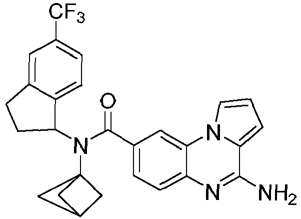 | 4-amino-*N-*(bicyclo[1.1.1]pentan-1-yl)-*N-*(5-(trifluoromethyl)-2,3-dihydro-1*H*-inden-1-yl)pyrrolo[1,2-*a*]quinoxaline-8-carboxamide | **477** |
| 58 | 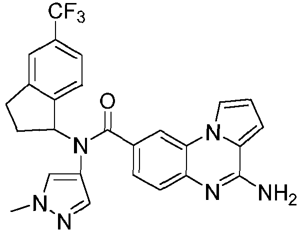 | 4-amino-*N*-(1-methyl-1*H-*pyrazol-4-yl)-*N*-(5-(trifluoromethyl)-2,3-dihydro-1*H*-inden-1-yl)pyrrolo[1,2-*a*]quinoxaline-8-carboxamide | **491** |
| 59 | 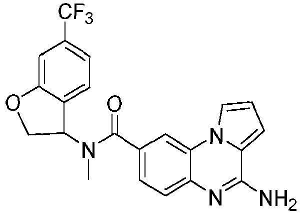 | 4-amino-*N*-methyl-*N*-(6-(trifluoromethyl)-2,3-dihydrobenzofuran-3-yl)pyrrolo[1,2-*a*]quinoxaline-8-carboxamide | **427** |
| 60 | 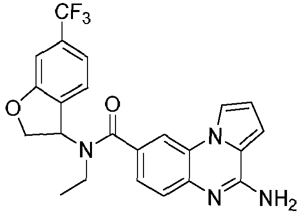 | 4-amino-*N*-ethyl-*N*-(6-(trifluoromethyl)-2,3-dihydrobenzofuran-3-yl)pyrrolo[1,2-*a*]quinoxaline-8-carboxamide | **441** |
| 61 | 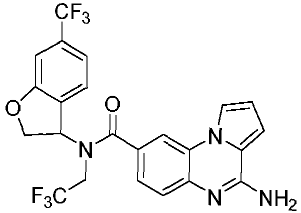 | 4-amino-*N*-(2,2,2-trifluoroethyl)*-N*-(6-(trifluoromethyl)-2,3-dihydrobenzofuran-3-yl)pyrrolo[1,2-*a*]quinoxaline-8-carboxamide | **495** |
| 62 | 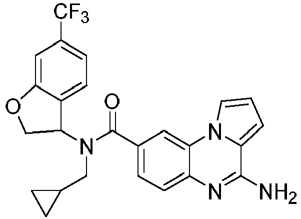 | 4-amino-*N*-(cyclopropylmethyl)-*N*-(6-(trifluoromethyl)-2,3-dihydrobenzofuran-3-yl)pyrrolo[1,2-*a*]quinoxaline-8-carboxamide | **467** |
| 63 | 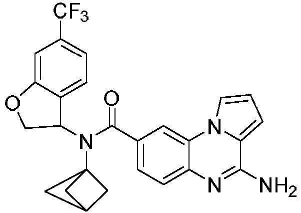 | 4-amino-*N-*(bicyclo[1.1.1]pentan-1-yl)-*N-*(6-(trifluoromethyl)-2,3-dihydrobenzofuran-3-yl)pyrrolo[1,2-*a*]quinoxaline-8-carboxamide | **479** |
| 64 | 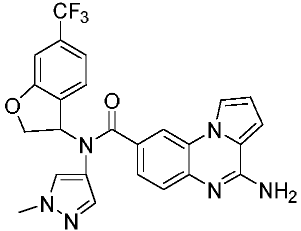 | 4-amino-*N*-(1-methyl-1*H-*pyrazol-4-yl)-*N*-(6-(trifluoromethyl)-2,3-dihydrobenzofuran-3-yl)pyrrolo[1,2-*a*]quinoxaline-8-carboxamide | **493** |
| 64a | 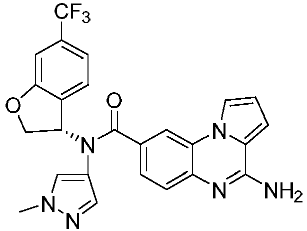 | (*S*)-4-amino-*N*-(1-methyl-1*H-*pyrazol-4-yl)-*N*-(6-(trifluoromethyl)-2,3-dihydrobenzofuran-3-yl)pyrrolo[1,2-*a*]quinoxaline-8-carboxamide | **493** |
| 65 | 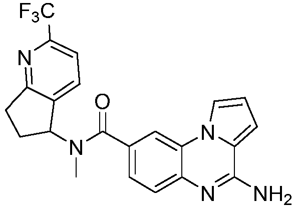 | 4-amino-*N*-methyl-*N*-(2-(trifluoromethyl)-6,7-dihydro-5*H-*cyclopenta[b]pyridin-5-yl)pyrrolo[1,2-*a*]quinoxaline-8-carboxamide | **426** |
| 66 | 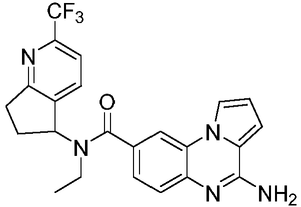 | 4-amino-*N*-ethyl-*N*-(2-(trifluoromethyl)-6,7-dihydro-5*H*-cyclopenta[b]pyridin-5-yl)pyrrolo[1,2-*a*]quinoxaline-8-carboxamide | **440** |
| 67 | 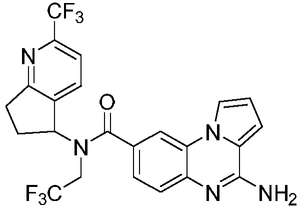 | 4-amino-*N*-(2,2,2-trifluoroethyl)-*N*-(2-(trifluoromethyl)-6,7-dihydro-5*H*-cyclopenta[*b*]pyridin-5-yl)pyrrolo[1,2-*a*]quinoxaline-8-carboxamide | **494** |
| 68 | 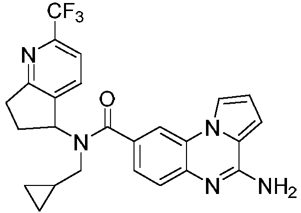 | 4-amino-*N*-(cyclopropylmethyl)-*N*-(2-(trifluoromethyl)-6,7-dihydro-5*H-*cyclopenta[*b*]pyridin-5-yl)pyrrolo[1,2-*a*]quinoxaline-8-carboxamide | **466** |
| 69 | 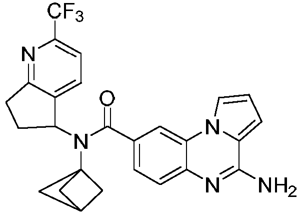 | 4-amino-*N-*(bicyclo[1.1.1]pentan-1-yl)-*N-*(2-(trifluoromethyl)-6,7-dihydro-5*H-*cyclopenta[*b*]pyridin-5-yl)pyrrolo[1,2-*a*]quinoxaline-8-carboxamide | **478** |
| 70 | 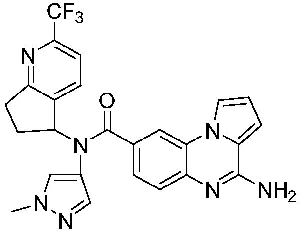 | 4-amino-*N*-(1-methyl-1*H-*pyrazol-4-yl)-*N*-(2-(trifluoromethyl)-6,7-dihydro-5*H*-cyclopenta[*b*]pyridin-5-yl)pyrrolo[1,2-*a*]quinoxaline-8-carboxamide | **492** |
| 71 | 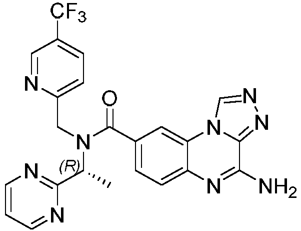 | (*R*)-4-amino-*N*-(1-(pyrimidin-2-yl)ethyl)-*N*-((5-(trifluoromethyl)pyridin-2-yl)methyl)-[1,2,4]triazolo[4,3-*a*]quinoxaline-8-carboxamide | **494** |
| 73 | 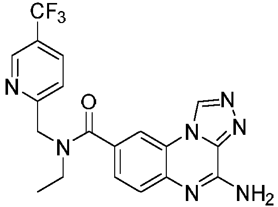 | 4-amino-*N*-ethyl-*N*-((5-(trifluoromethyl)pyridin-2-yl)methyl)-[1,2,4]triazolo[4,3-*a*]quinoxaline-8-carboxamide | **415** |
| 74 | 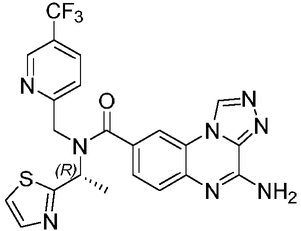 | (*R*)-4-amino-*N*-(1-(thiazol-2-yl)ethyl)-N-((5-(trifluoromethyl)pyridin-2-yl)methyl)-[1,2,4]triazolo[4,3-*a*]quinoxaline-8-carboxamide | **499** |
| 75 | 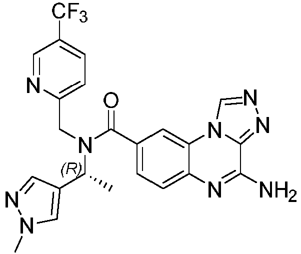 | (*R*)-4-amino-*N*-(1-(1-methyl-1*H*-pyrazol-4-yl)ethyl)-*N*-((5-(trifluoromethyl)pyridin-2-yl)methyl)-[1,2,4]triazolo[4,3-*a*]quinoxaline-8-carboxamide | **496** |
| 76 | 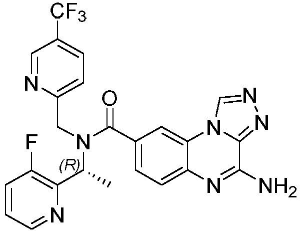 | (*R*)-4-amino-*N*-(1-(3-fluoropyridin-2-yl)ethyl)-*N*-((5-(trifluoromethyl)pyridin-2-yl)methyl)-[1,2,4]triazolo[4,3-*a*]quinoxaline-8-carboxamide | **511** |
| 77 | 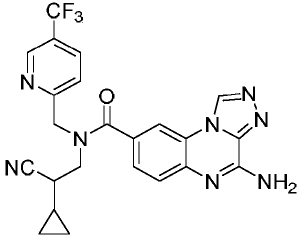 | 4-amino-*N*-(2-cyano-2-cyclopropylethyl)-*N*-((5-(trifluoromethyl)pyridin-2-yl)methyl)-[1,2,4]triazolo[4,3-*a*]quinoxaline-8-carboxamide | **481** |
| 78 | 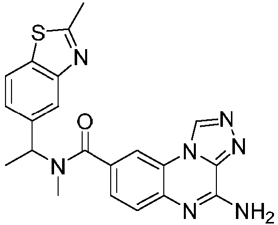 | 4-amino-*N*-methyl-*N*-(1-(2-methylbenzo[*d*]thiazol-5-yl)ethyl)-[1,2,4]triazolo[4,3-*a*]quinoxaline-8-carboxamide | **418** |
| 79 | 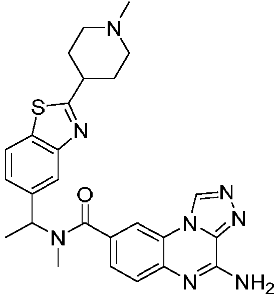 | 4-amino-*N*-methyl-*N*-(1-(2-(1-methylpiperidin-4-yl)benzo[*d*]thiazol-5-yl)ethyl)-[1,2,4]triazolo[4,3-*a*]quinoxaline-8-carboxamide | **501** |
| 80 | 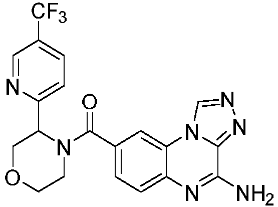 | (4-amino-[1,2,4]triazolo[4,3-*a*]quinoxalin-8-yl)(3-(5-(trifluoromethyl)pyridin-2-yl)morpholino)methanone | **444** |
| 81 | 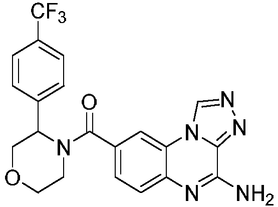 | (4-amino-[1,2,4]triazolo[4,3-*a*]quinoxalin-8-yl)(3-(4-(trifluoromethyl)phenyl)morphol ino)methanone | **443** |
| 82 | 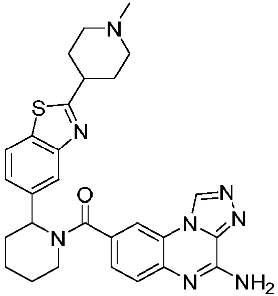 | (4-amino-[1,2,4]triazolo[4,3-*a*]quinoxalin-8-yl)(2-(2-(1-methylpiperidin-4-yl)benzo[*d*]thiazol-5-yl)piperidin-1-yl)methanone | **527** |
| 83 | 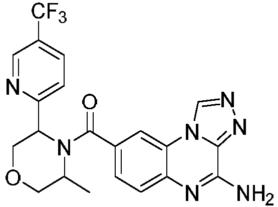 | (4-amino-[1,2,4]triazolo[4,3-*a*]quinoxalin-8-yl)(3-methyl-5-(5-(trifluoromethyl)pyridin-2-yl)morpholino)methanone | **457** |
| 84 | 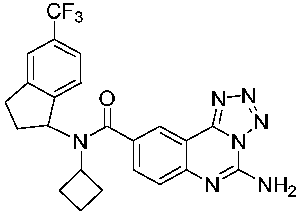 | 5-amino-*N*-cyclobutyl-*N*-(5-(trifluoromethyl)-2,3-dihydro-1*H*-inden-1-yl)tetrazolo[1,5-*c*]quinazoline-9-carboxamide | **468** |
| 85 | 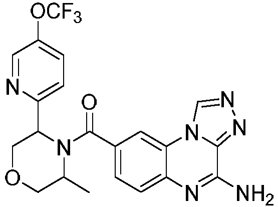 | (4-amino-[1,2,4]triazolo[4,3-*a*]quinoxalin-8-yl)(3-methyl-5-(5-(trifluoromethoxy)pyridin-2-yl)morpholino)methanone | **474** |
| 86 | 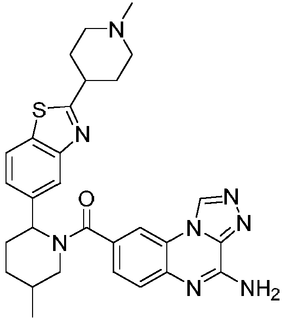 | (4-amino-[1,2,4]triazolo[4,3-*a*]quinoxalin-8-yl)(5-methyl-2-(2-(1-methylpiperidin-4-yl)benzo[*d*]thiazol-5-yl)piperidin-1-yl)methanone | **541** |
| 87 | 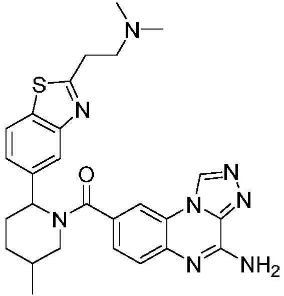 | (4-amino-[1,2,4]triazolo[4,3-*a*]quinoxalin-8-yl)(2-(2-(2-(dimethylamino)ethyl)benzo[*d*]t hiazol-5-yl)-5-methylpiperidin-1-yl)methanone | **515** |
| 88 | 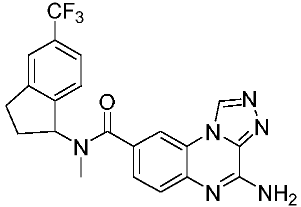 | 4-amino-*N*-methyl-*N*-(5-(trifluoromethyl)-2,3-dihydro-1*H*-inden-1-yl)-[1,2,4]triazolo[4,3-*a*]quinoxaline-8-carboxamide | **427** |
| 89 | 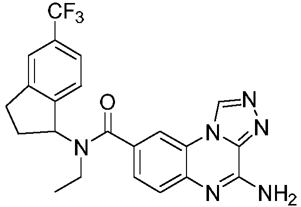 | 4-amino-*N*-ethyl-*N*-(5-(trifluoromethyl)-2,3-dihydro-1*H*-inden-1-yl)-[1,2,4]triazolo[4,3-*a*]quinoxaline-8-carboxamide | **441** |
| 90 | 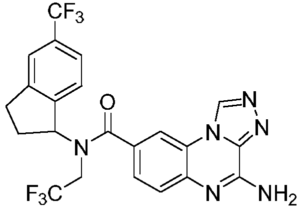 | 4-amino-*N*-(2,2,2-trifluoroethyl)-*N*-(5-(trifluoromethyl)-2,3-dihydro-1*H*-inden-1-yl)-[1,2,4]triazolo[4,3-*a*]quinoxaline-8-carboxamide | **495** |
| 91 | 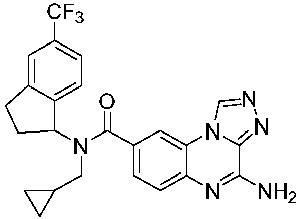 | 4-amino-*N*-(cyclopropylmethyl)-*N*-(5-(trifluoromethyl)-2,3-dihydro-1*H*-inden-1-yl)-[1,2,4]triazolo[4,3-*a*]quinoxaline-8-carboxamide | **467** |
| 92 | 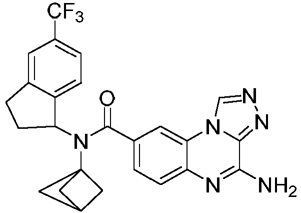 | 4-amino-*N-*(bicyclo[1.1.1]pentan-1-yl)-*N-*(5-(trifluoromethyl)-2,3-dihydro-1*H*-inden-1-yl)-[1,2,4]triazolo[4,3-*a*]quinoxaline-8-carboxamide | **479** |
| 93 | 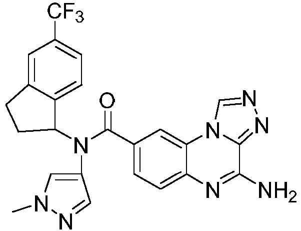 | 4-amino-*N*-(1-methyl-1*H-*pyrazol-4-yl)-*N*-(5-(trifluoromethyl)-2,3-dihydro-1*H*-inden-1-yl)-[1,2,4]triazolo[4,3-*a*]quinoxaline-8-carboxamide | **493** |
| 94 | 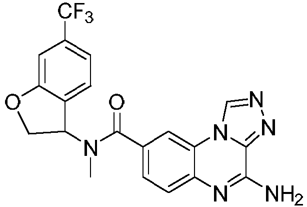 | 4-amino-*N*-methyl-*N*-(6-(trifluoromethyl)-2,3-dihydrobenzofuran-3-yl)-[1,2,4]triazolo[4,3-*a*]quinoxaline-8-carboxamide | **429** |
| 95 | 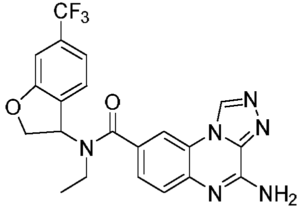 | 4-amino-*N*-ethyl-*N*-(6-(trifluoromethyl)-2,3-dihydrobenzofuran-3-yl)-[1,2,4]triazolo[4,3-*a*]quinoxaline-8-carboxamide | **443** |
| 96 | 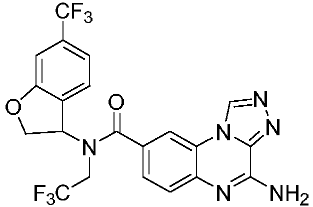 | 4-amino-*N*-(2,2,2-trifluoroethyl)-*N*-(6-(trifluoromethyl)-2,3-dihydrobenzofuran-3-yl)-[1,2,4]triazolo[4,3-*a*]quinoxaline-8-carboxamide | **497** |
| 97 | 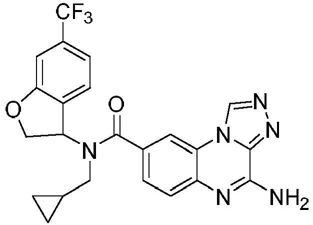 | 4-amino-*N*-(cyclopropylmethyl)-*N*-(6-(trifluoromethyl)-2,3-dihydrobenzofuran-3-yl)-[1,2,4]triazolo[4,3-*a*]quinoxaline-8-carboxamide | **469** |
| 98 | 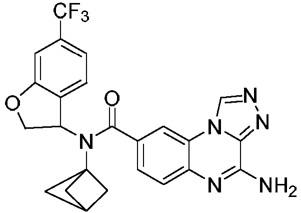 | 4-amino-*N-*(bicyclo[1.1.1]pentan-1-yl)-*N-*(6-(trifluoromethyl)-2,3-dihydrobenzofuran-3-yl)-[1,2,4]triazolo[4,3-*a*]quinoxaline-8-carboxamide | **481** |
| 99 | 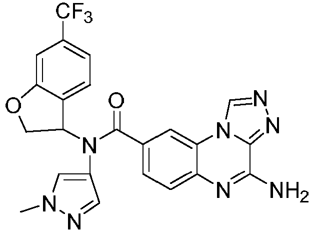 | 4-amino-*N*-(1-methyl-1*H-*pyrazol-4-yl)-*N*-(6-(trifluoromethyl)-2,3-dihydrobenzofuran-3-yl)-[1,2,4]triazolo[4,3-*a*]quinoxaline-8-carboxamide | **495** |
| 100 | 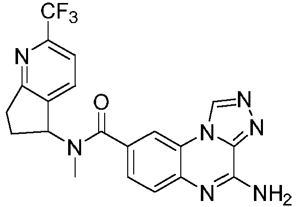 | 4-amino-*N*-methyl-*N*-(2-(trifluoromethyl)-6,7-dihydro-5*H*-cyclopenta[*b*]pyridin-5-yl)-[1,2,4]triazolo[4,3-*a*]quinoxaline-8-carboxamide | **428** |
| 101 | 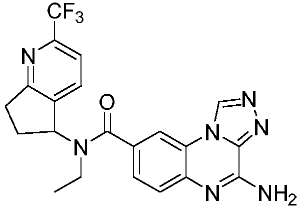 | 4-amino-*N*-ethyl-*N*-(2-(trifluoromethyl)-6,7-dihydro-5*H*-cyclopenta[*b*]pyridin-5-yl)-[1,2,4]triazolo[4,3-*a*]quinoxaline-8-carboxamide | **442** |
| 102 | 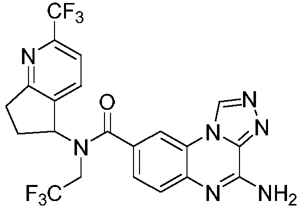 | 4-amino-*N*-(2,2,2-trifluoroethyl)-*N*-(2-(trifluoromethyl)-6,7-dihydro-5*H*-cyclopenta[*b*]pyridin-5-yl)-[1,2,4]triazolo[4,3-*a*]quinoxaline-8-carboxamide | **496** |
| 103 | 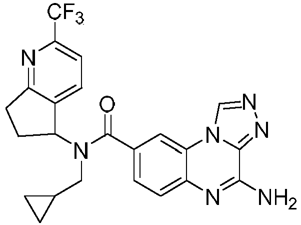 | 4-amino-*N*-(cyclopropylmethyl)-*N*-(2-(trifluoromethyl)-6,7-dihydro-5*H-*cyclopenta[*b*]pyridin-5-yl)-[1,2,4]triazolo[4,3-*a*]quinoxaline-8-carboxamide | **468** |
| 104 | 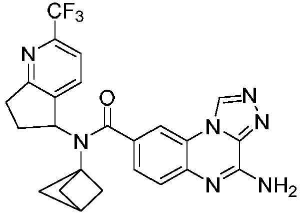 | 4-amino-*N-*(bicyclo[1.1.1]pentan-1-yl)-*N-*(2-(trifluoromethyl)-6,7-dihydro-5*H-*cyclopenta[*b*]pyridin-5-yl)-[1,2,4]triazolo[4,3-*a*]quinoxaline-8-carboxamide | **480** |
| 105 | 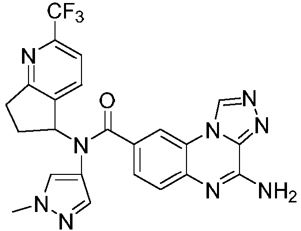 | 4-amino-*N*-(1-methyl-1*H-*pyrazol-4-yl)-*N*-(2-(trifluoromethyl)-6,7-dihydro-5*H*-cyclopenta[*b*]pyridin-5-yl)-[1,2,4]triazolo[4,3-*a*]quinoxaline-8-carboxamide | **494** |
| 106 | 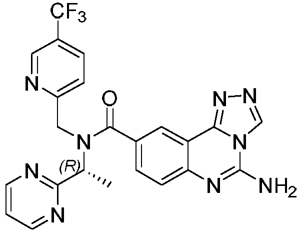 | (*R*)-5-amino-*N*-(1-(pyrimidin-2-yl)ethyl)-*N*-((5-(trifluoromethyl)pyridin-2-yl)methyl)-[1,2,4]triazolo[4,3-c]quinazoline-9-carboxamide | **494** |
| 107 | 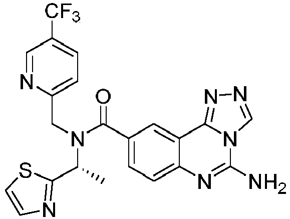 | (*R*)-5-amino-*N*-(1-(thiazol-2-yl)ethyl)-*N*-((5-(trifluoromethyl)pyridin-2-yl)methyl)-[1,2,4]triazolo[4,3-*c*]quinazoline-9-carboxamide | **499** |
| 108 | 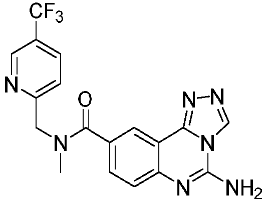 | 5-amino-*N*-methyl-*N*-((5-(trifluoromethyl)pyridin-2-yl)methyl)-[1,2,4]triazolo[4,3-*c*]quinazoline-9-carboxamide | **402** |
| 109 | 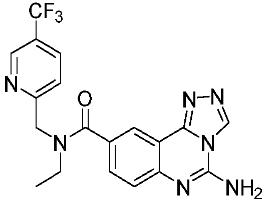 | 5-amino-*N*-ethyl-*N*-((5-(trifluoromethyl)pyridin-2-yl)methyl)-[1,2,4]triazolo[4,3-*c*]quinazoline-9-carboxamide | **416** |
| 110 | 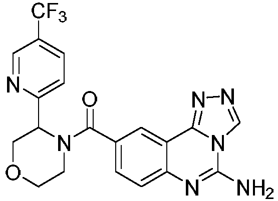 | (5-amino-[1,2,4]triazolo[4,3-c]quinazolin-9-yl)(3-(5-(trifluoromethyl)pyridin-2-yl)morpholino)methanone | **444** |
| 111 | 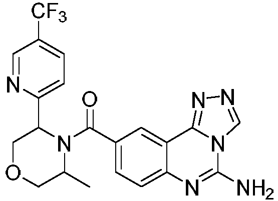 | (5-amino-[1,2,4]triazolo[4,3-c]quinazolin-9-yl)(3-methyl-5-(5-(trifluoromethyl)pyridin-2-yl)morpholino)methanone | **458** |
| 112 | 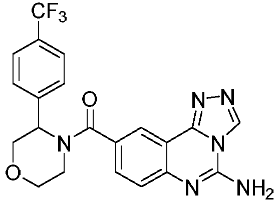 | (5-amino-[1,2,4]triazolo[4,3-*c*]quinazolin-9-yl)(3-(4-(trifluoromethyl)phenyl)morphol ino)methanone | **443** |
| 113 | 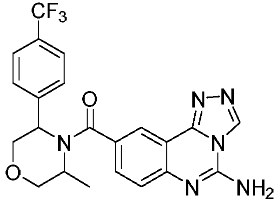 | (5-amino-[1,2,4]triazolo[4,3-*c*]quinazolin-9-yl)(3-methyl-5-(4-(trifluoromethyl)phenyl)morphol ino)methanone | **457** |
| 114 | 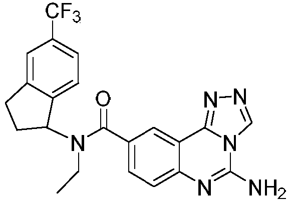 | 5-amino-*N*-ethyl-*N*-(5-(trifluoromethyl)-2,3-dihydro-1*H*-inden-1-yl)-[1,2,4]triazolo[4,3-c]quinazoline-9-carboxamide | **441** |
| 115 | 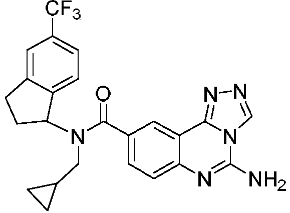 | 5-amino-*N*-(cyclopropylmethyl)-*N*-(5-(trifluoromethyl)-2,3-dihydro-1*H*-inden-1-yl)-[1,2,4]triazolo[4,3-*c*]quinazoline-9-carboxamide | **467** |
| 116 | 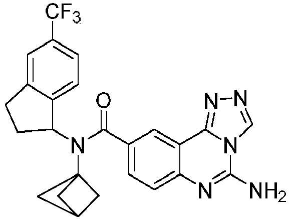 | 5-amino-*N-*(bicyclo[1.1.1]pentan-1-yl)-*N-*(5-(trifluoromethyl)-2,3-dihydro-1*H*-inden-1-yl)-[1,2,4]triazolo[4,3-*c*]quinazoline-9-carboxamide | **479** |
| 117 | 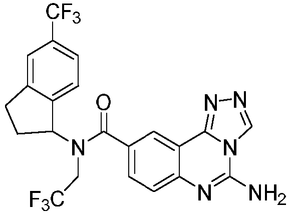 | 5-amino-*N*-(2,2,2-trifluoroethyl)-*N*-(5-(trifluoromethyl)-2,3-dihydro-1*H*-inden-1-yl)-[1,2,4]triazolo[4,3-*c*]quinazoline-9-carboxamide | **495** |
| 118 | 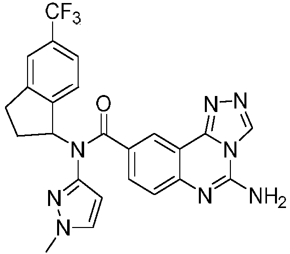 | 5-amino-*N*-(1-methyl-1*H-*pyrazol-3-yl)-*N*-(5-(trifluoromethyl)-2,3-dihydro-1*H*-inden-1-yl)-[1,2,4]triazolo[4,3-*c*]quinazoline-9-carboxamide | **493** |
| 119 | 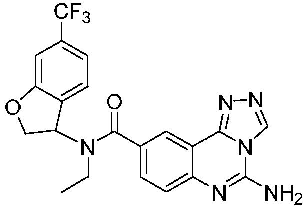 | 5-amino-*N*-ethyl-*N*-(6-(trifluoromethyl)-2,3-dihydrobenzofuran-3-yl)-[1,2,4]triazolo[4,3-*c*]quinazoline-9-carboxamide | **443** |
| 120 | 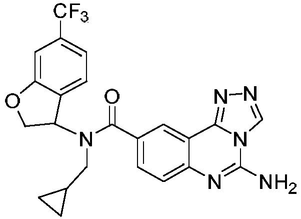 | 5-amino-*N*-(cyclopropylmethyl)-*N*-(6-(trifluoromethyl)-2,3-dihydrobenzofuran-3-yl)-[1,2,4]triazolo[4,3-*c*]quinazoline-9-carboxamide | **469** |
| 121 | 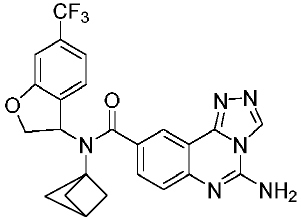 | 5-amino-*N-*(bicyclo[1.1.1]pentan-1-yl)-*N-*(6-(trifluoromethyl)-2,3-dihydrobenzofuran-3-yl)-[1,2,4]triazolo[4,3-*c*]quinazoline-9-carboxamide | **481** |
| 122 | 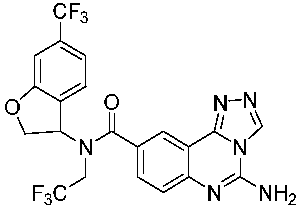 | 5-amino-*N*-(2,2,2-trifluoroethyl)-*N*-(6-(trifluoromethyl)-2,3-dihydrobenzofuran-3-yl)-[1,2,4]triazolo[4,3-*c*]quinazoline-9-carboxamide | **497** |
| 123 | 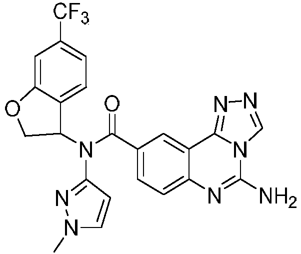 | 5-amino-*N*-(1-methyl-1*H-*pyrazol-3-yl)-*N*-(6-(trifluoromethyl)-2,3-dihydrobenzofuran-3-yl)-[1,2,4]triazolo[4,3-*c*]quinazoline-9-carboxamide | **495** |
| 124 | 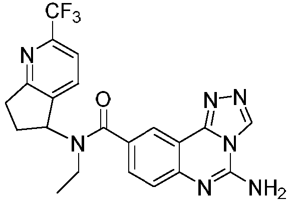 | 5-amino-*N*-ethyl-*N*-(2-(trifluoromethyl)-6,7-dihydro-5*H*-cyclopenta[*b*]pyridin-5-yl)-[1,2,4]triazolo[4,3-*c*]quinazoline-9-carboxamide | **442** |
| 125 | 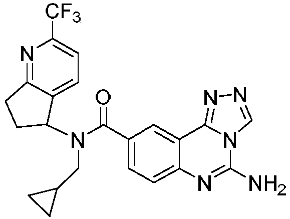 | 5-amino-*N*-(cyclopropylmethyl)-*N*-(2-(trifluoromethyl)-6,7-dihydro-5*H-*cyclopenta[*b*]pyridin-5-yl)-[1,2,4]triazolo[4,3-*c*]quinazoline-9-carboxamide | **468** |
| 126 | 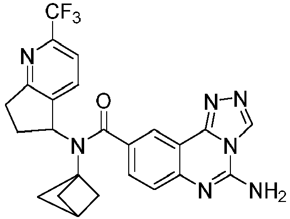 | 5-amino-*N-*(bicyclo[1.1.1]pentan-1-yl)-*N-*(2-(trifluoromethyl)-6,7-dihydro-5*H-*cyclopenta[*b*]pyridin-5-yl)-[1,2,4]triazolo[4,3-*c*]quinazoline-9-carboxamide | **480** |
| 127 | 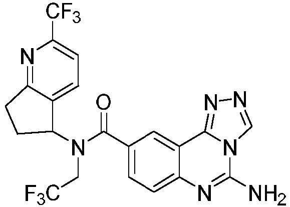 | 5-amino-*N*-(2,2,2-trifluoroethyl)-*N*-(2-(trifluoromethyl)-6,7-dihydro-5*H*-cyclopenta[*b*]pyridin-5-yl)-[1,2,4]triazolo[4,3-*c*]quinazoline-9-carboxamide | **496** |
| 128 | 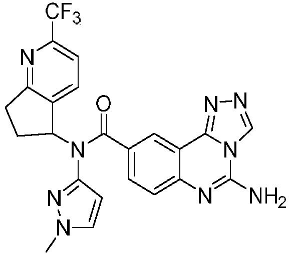 | 5-amino-*N*-(1-methyl-1*H-*pyrazol-3-yl)-*N*-(2-(trifluoromethyl)-6,7-dihydro-5*H*-cyclopenta[*b*]pyridin-5-yl)-[1,2,4]triazolo[4,3-*c*]quinazoline-9-carboxamide | **494** |
| 129 | 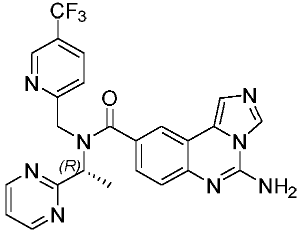 | (*R*)-5-amino-*N*-(1-(pyrimidin-2-yl)ethyl)-*N*-((5-(trifluoromethyl)pyridin-2-yl)methyl)imidazo[1,5-*c*]quinazoline-9-carboxamide | **493** |
| 130 | 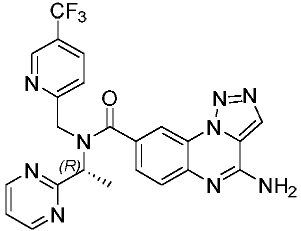 | (*R*)-4-amino-*N*-(1-(pyrimidin-2-yl)ethyl)-*N*-((5-(trifluoromethyl)pyridin-2-yl)methyl)-[1,2,3]triazolo[1,5-*a*]quinoxaline-8-carboxamide | **494** |
| 131 | 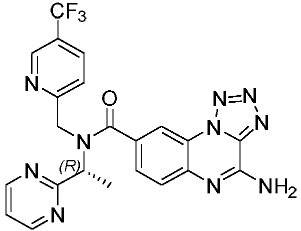 | (*R*)-4-amino-*N*-(1-(pyrimidin-2-yl)ethyl)-*N*-((5-(trifluoromethyl)pyridin-2-yl)methyl)tetrazolo[1,5-*a*]quinoxaline-8-carboxamide | **495** |
| 132 | 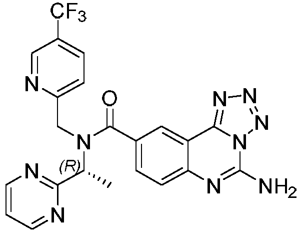 | (*R*)-5-amino-*N*-(1-(pyrimidin-2-yl)ethyl)-*N*-((5-(trifluoromethyl)pyridin-2-yl)methyl)tetrazolo[1,5-*c*]quinazoline-9-carboxamide | **495** |
| 133 | 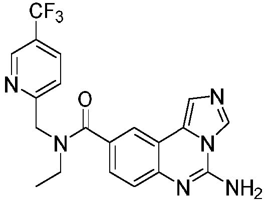 | 5-amino-*N*-ethyl-*N*-((5-(trifluoromethyl)pyridin-2-yl)methyl)imidazo[1,5-*c*]quinazoline-9-carboxamide | **415** |
| 134 | 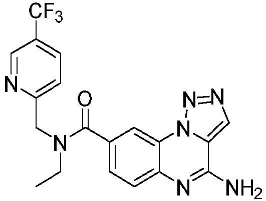 | 4-amino-*N*-ethyl-*N*-((5-(trifluoromethyl)pyridin-2-yl)methyl)-[1,2,3]triazolo[1,5-*a*]quinoxaline-8-carboxamide | **416** |
| 135 | 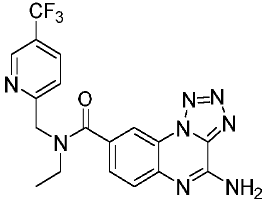 | 4-amino-*N*-ethyl-*N*-((5-(trifluoromethyl)pyridin-2-yl)methyl)tetrazolo[1,5-*a*]quinoxaline-8-carboxamide | **417** |
| 136 | 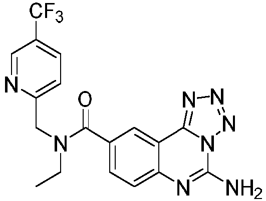 | 5-amino-*N*-ethyl-*N*-((5-(trifluoromethyl)pyridin-2-yl)methyl)tetrazolo[1,5-*c*]quinazoline-9-carboxamide | **417** |
| 137 | 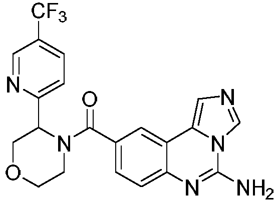 | (5-aminoimidazo[1,5-c]quinazolin-9-yl)(3-(5-(trifluoromethyl)pyridin-2-yl)morpholino)methanone | **443** |
| 138 | 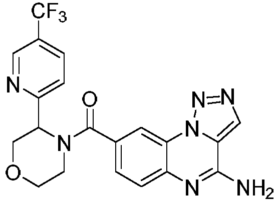 | (4-amino-[1,2,3]triazolo[1,5-*a*]quinoxalin-8-yl)(3-(5-(trifluoromethyl)pyridin-2-yl)morpholino)methanone | **444** |
| 139 | 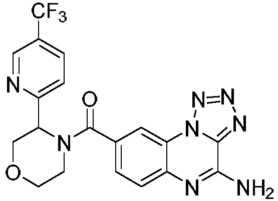 | (4-aminotetrazolo[1,5-*a*]quinoxalin-8-yl)(3-(5-(trifluoromethyl)pyridin-2-yl)morpholino)methanone | **445** |
| 140 | 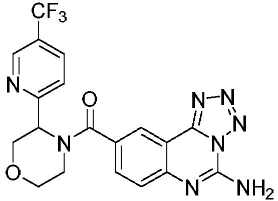 | (5-aminotetrazolo[1,5-*c*]quinazolin-9-yl)(3-(5-(trifluoromethyl)pyridin-2-yl)morpholino)methanone | **445** |
| 141 | 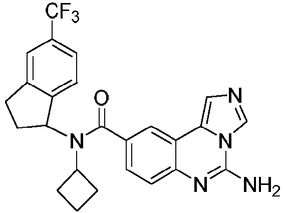 | 5-amino-*N*-cyclobutyl-*N*-(5-(trifluoromethyl)-2,3-dihydro-1*H*-inden-1-yl)imidazo[1,5-*c*]quinazoline-9-carboxamide | **466** |
| 142 | 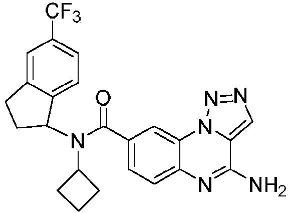 | 4-amino-*N*-cyclobutyl-*N*-(5-(trifluoromethyl)-2,3-dihydro-1*H*-inden-1-yl)-[1,2,3]triazolo[1,5-*a*]quinoxaline-8-carboxamide | **467** |
| 143 | 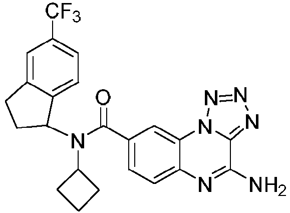 | 4-amino-*N*-cyclobutyl*-N*-(5-(trifluoromethyl)-2,3-dihydro-1*H*-inden-1-yl)tetrazolo[1,5-*a*]quinoxaline-8-carboxamide | **468** |
| 144 | 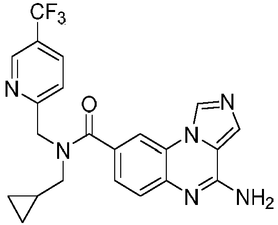 | 4-amino-*N*-(cyclopropylmethyl)-*N*-((5-(trifluoromethyl)pyridin-2-yl)methyl)imidazo[1,5-*a*]quinoxaline-8-carboxamide | **441** |
| 145 | 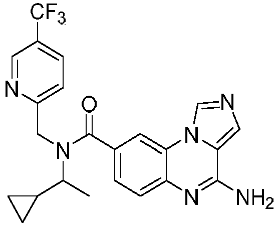 | 4-amino-*N*-(1-cyclopropylethyl)-*N*-((5-(trifluoromethyl)pyridin-2-yl)methyl)imidazo[1,5-*a*]quinoxaline-8-carboxamide | **455** |
| 146 | 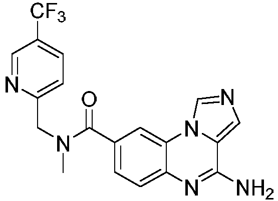 | 4-amino-*N*-methyl-N-((5-(trifluoromethyl)pyridin-2-yl)methyl)imidazo[1,5-*a*]quinoxaline-8-carboxamide | **401** |
| 147 | 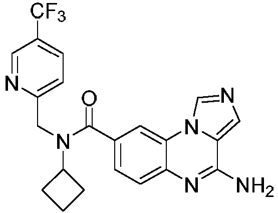 | 4-amino-*N*-cyclobutyl-*N*-((5-(trifluoromethyl)pyridin-2-yl)methyl)imidazo[1,5-*a*]quinoxaline-8-carboxamide | **441** |
| 148 | 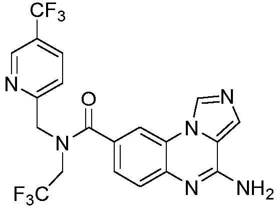 | 4-amino-*N*-(2,2,2-trifluoroethyl)-*N*-((5-(trifluoromethyl)pyridin-2-yl)methyl)imidazo[1,5-*a*]quinoxaline-8-carboxamide | **469** |
| 149 | 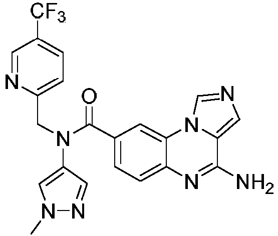 | 4-amino-*N*-(1-methyl-1*H-*pyrazol-4-yl)-*N*-((5-(trifluoromethyl)pyridin-2-yl)methyl)imidazo[1,5-*a*]quinoxaline-8-carboxamide | **468** |
| 150 | 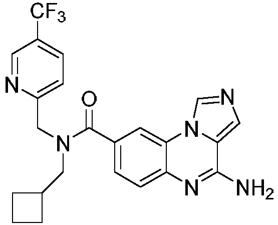 | 4-amino-*N*-(cyclobutylmethyl)-*N*-((5-(trifluoromethyl)pyridin-2-yl)methyl)imidazo[1,5-*a*]quinoxaline-8-carboxamide | **455** |
| 151 | 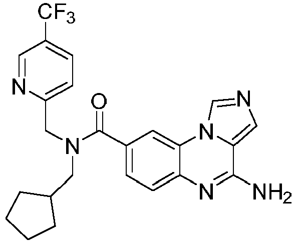 | 4-amino-*N*-(cyclopentylmethyl)-*N*-((5-(trifluoromethyl)pyridin-2-yl)methyl)imidazo[1,5-*a*]quinoxaline-8-carboxamide | **469** |
| 152 | 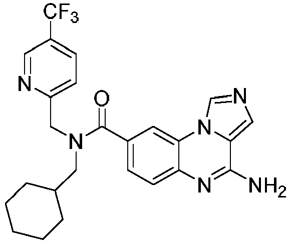 | 4-amino-*N*-(cyclohexylmethyl)-*N*-((5-(trifluoromethyl)pyridin-2-yl)methyl)imidazo[1,5-*a*]quinoxaline-8-carboxamide | **483** |
| 153 | 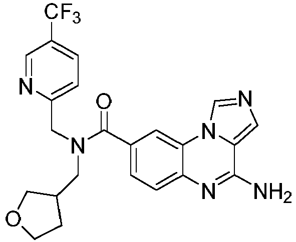 | 4-amino-*N*-((tetrahydrofuran-3-yl)methyl)-*N*-((5-(trifluoromethyl)pyridin-2-yl)methyl)imidazo[1,5-*a*]quinoxaline-8-carboxamide | **471** |
| 154 | 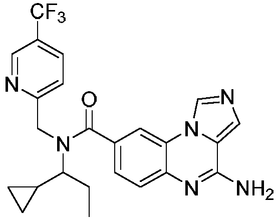 | 4-amino-*N*-(1-cyclopropylpropyl)-*N*-((5-(trifluoromethyl)pyridin-2-yl)methyl)imidazo[1,5-*a*]quinoxaline-8-carboxamide | **469** |
| 155 | 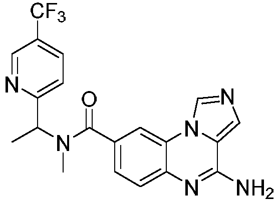 | 4-amino-N-methyl-*N*-(1-(5-(trifluoromethyl)pyridin-2-yl)ethyl)imidazo[1,5-*a*]quinoxaline-8-carboxamide | **415** |
| 156 | 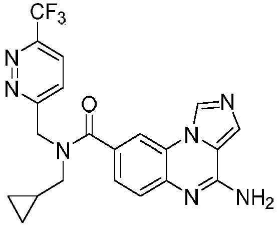 | 4-amino-*N*-(cyclopropylmethyl)-*N-((6-*(trifluoromethyl)pyridazin-3-yl)methyl)imidazo[1,5-*a*]quinoxaline-8-carboxamide | **442** |
| 157 | 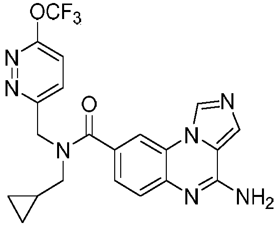 | 4-amino-*N*-(cyclopropylmethyl)-*N-((6-*(trifluoromethoxy)pyridazin-3-yl)methyl)imidazo[1,5-*a*]quinoxaline-8-carboxamide | **458** |
| 158 | 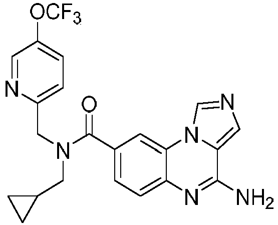 | 4-amino-*N*-(cyclopropylmethyl)-*N*-((5-(trifluoromethoxy)pyridin-2-yl)methyl)imidazo[1,5-*a*]quinoxaline-8-carboxamide | **457** |
| 159 | 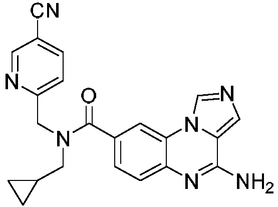 | 4-amino-*N*-((5-cyanopyridin-2-yl)methyl)-*N-*(cyclopropylmethyl)imidazo[1,5 -a]quinoxaline-8-carboxamide | **398** |
| 160 | 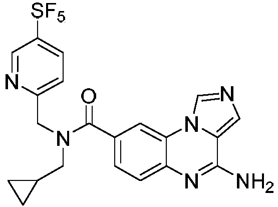 | 4-amino-*N*-(cyclopropylmethyl)-*N*-((5-(pentafluoro-λ⁶-sulfaneyl)pyridin-2-yl)methyl)imidazo[1,5-*a*]quinoxaline-8-carboxamide | **499** |
| 161 | 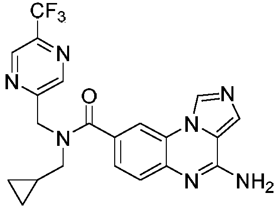 | 4-amino-*N*-(cyclopropylmethyl)-*N*-((5-(trifluoromethyl)pyrazin-2-yl)methyl)imidazo[1,5-*a*]quinoxaline-8-carboxamide | **440** |
| 162 | 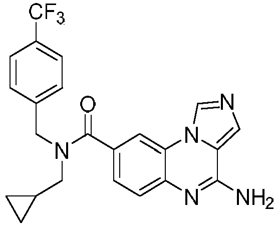 | 4-amino-*N*-(cyclopropylmethyl)-*N-*(4-(trifluoromethyl)benzyl)imidazo [1,5-*a*]quinoxaline-8-carboxamide | **440** |
| 163 | 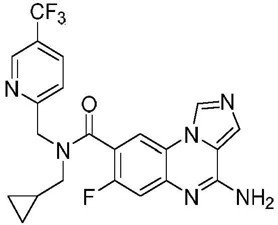 | 4-amino-*N*-(cyclopropylmethyl)-7-fluoro-*N*-((5-(trifluoromethyl)pyridin-2-yl)methyl)imidazo[1,5-*a*]quinoxaline-8-carboxamide | **459** |
| 164 | 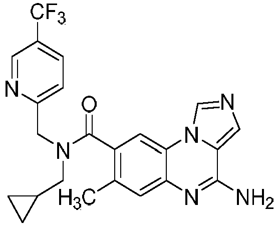 | 4-amino-*N*-(cyclopropylmethyl)-7-methyl-*N*-((5-(trifluoromethyl)pyridin-2-yl)methyl)imidazo[1,5-*a*]quinoxaline-8-carboxamide | **455** |
| 165 | 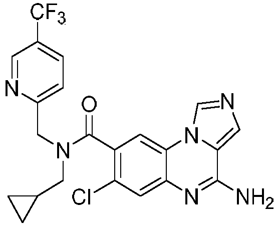 | 4-amino-7-chloro-*N-*(cyclopropylmethyl)-*N*-((5-(trifluoromethyl)pyridin-2-yl)methyl)imidazo[1,5-*a*]quinoxaline-8-carboxamide | **475** |
| 166 | 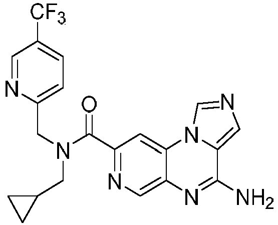 | 4-amino-*N*-(cyclopropylmethyl)-*N*-((5-(trifluoromethyl)pyridin-2-yl)methyl)imidazo[1,5-*a*]pyrido[3,4-*e*]pyrazine-8-carboxamide | **442** |
| 167 | 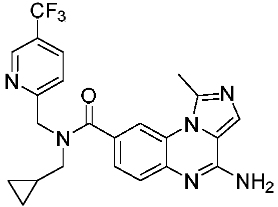 | 4-amino-*N*-(cyclopropylmethyl)-1-methyl-*N*-((5-(trifluoromethyl)pyridin-2-yl)methyl)imidazo[1,5-*a*]quinoxaline-8-carboxamide | **455** |
| 168 | 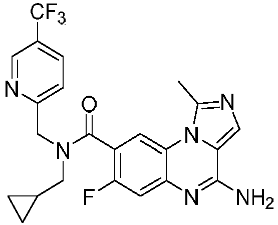 | 4-amino-*N*-(cyclopropylmethyl)-7-fluoro-1-methyl-*N*-((5-(trifluoromethyl)pyridin-2-yl)methyl)imidazo[1,5-*a*]quinoxaline-8-carboxamide | **473** |
| 169 | 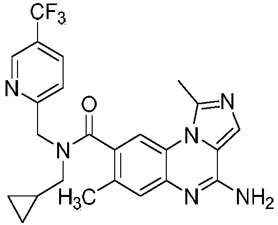 | 4-amino-*N*-(cyclopropylmethyl)-1,7-dimethyl-*N*-((5-(trifluoromethyl)pyridin-2-yl)methyl)imidazo[1,5-*a*]quinoxaline-8-carboxamide | **469** |
| 170 | 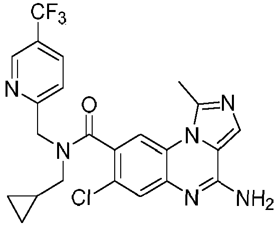 | 4-amino-7-chloro-*N-*(cyclopropylmethyl)-1-methyl-*N*-((5-(trifluoromethyl)pyridin-2-yl)methyl)imidazo[1,5-*a*]quinoxaline-8-carboxamide | **489** |
| 171 | 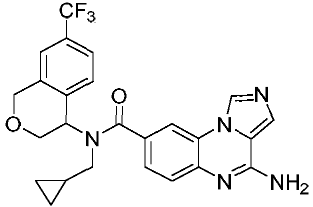 | 4-amino-*N*-(cyclopropylmethyl)-*N*-(7-(trifluoromethyl)isochroman-4-yl)imidazo[1,5-*a*]quinoxaline-8-carboxamide | **482** |
| 178 | 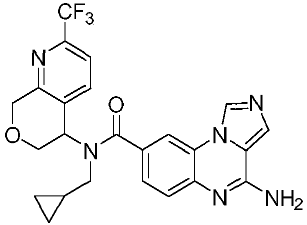 | 4-amino-*N-*(cyclopropylmethyl)-*N*-(2-(trifluoromethyl)-5,8-dihydro-6*H*-pyrano[3,4-*b*]pyridin-5-yl)imidazo[1,5-*a*]quinoxaline-8-carboxamide | **483** |
| 179 | 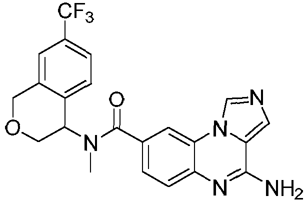 | 4-amino-*N*-methyl-*N*-(7-(trifluoromethyl)isochroman-4-yl)imidazo[1,5-*a*]quinoxaline-8-carboxamide | **442** |
| 180 | 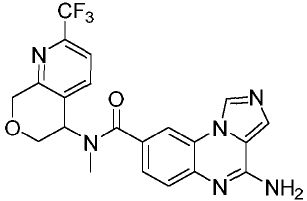 | 4-amino-*N*-methyl-*N*-(2-(trifluoromethyl)-5,8-dihydro-6*H*-pyrano[3,4-b]pyridin-5-yl)imidazo[1,5-*a*]quinoxaline-8-carboxamide | **443** |
| 181 | 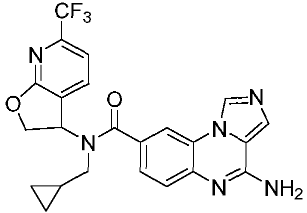 | 4-amino-*N*-(cyclopropylmethyl)-*N*-(6-(trifluoromethyl)-2,3-dihydrofuro[2,3-*b*]pyridin-3-yl)imidazo[1,5-*a*]quinoxaline-8-carboxamide | **469** |
| 181a | 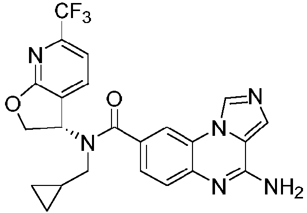 | (*S*)-4-amino-*N-*(cyclopropylmethyl)-*N*-(6-(trifluoromethyl)-2,3-dihydrofuro[2,3-*b*]pyridin-3-yl)imidazo[1,5-*a*]quinoxaline-8-carboxamide | **469** |
| 181b | 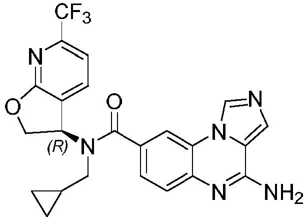 | (*R*)-4-amino-*N-*(cyclopropylmethyl)-*N*-(6-(trifluoromethyl)-2,3-dihydrofuro[2,3-*b*]pyridin-3-yl)imidazo[1,5-*a*]quinoxaline-8-carboxamide | **469** |
| 182 | 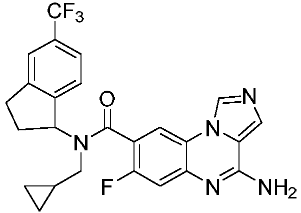 | 4-amino-*N*-(cyclopropylmethyl)-7-fluoro-*N*-(5-(trifluoromethyl)-2,3-dihydro-1*H*-inden-1-yl)imidazo[1,5-*a*]quinoxaline-8-carboxamide | **484** |
| 183 | 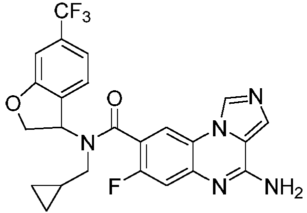 | 4-amino-*N*-(cyclopropylmethyl)-7-fluoro-*N*-(6-(trifluoromethyl)-2,3-dihydrobenzofuran-3-yl)imidazo[1,5-*a*]quinoxaline-8-carboxamide | **486** |
| 184 | 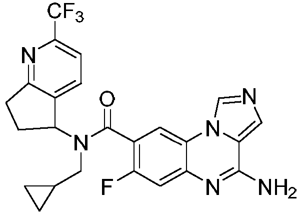 | 4-amino-*N*-(cyclopropylmethyl)-7-fluoro-*N*-(2-(trifluoromethyl)-6,7-dihydro-5*H-*cyclopenta[*b*]pyridin-5-yl)imidazo[1,5-*a*]quinoxaline-8-carboxamide | **485** |
| 185 | 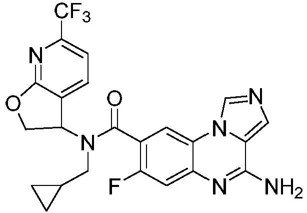 | 4-amino-*N*-(cyclopropylmethyl)-7-fluoro-*N*-(6-(trifluoromethyl)-2,3-dihydrofuro[2,3-*b*]pyridin-3-yl)imidazo[1,5-*a*]quinoxaline-8-carboxamide | **487** |
| 186 | 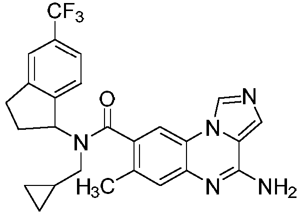 | 4-amino-*N*-(cyclopropylmethyl)-7-methyl-*N*-(5-(trifluoromethyl)-2,3-dihydro-1*H*-inden-1-yl)imidazo[1,5-*a*]quinoxaline-8-carboxamide | **480** |
| 187 | 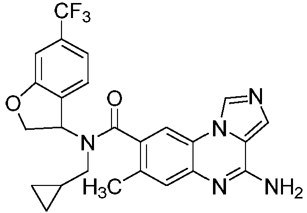 | 4-amino-*N*-(cyclopropylmethyl)-7-methyl-*N*-(6-(trifluoromethyl)-2,3-dihydrobenzofuran-3-yl)imidazo[1,5-*a*]quinoxaline-8-carboxamide | **482** |
| 188 | 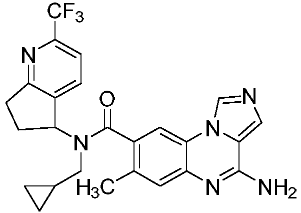 | 4-amino-*N*-(cyclopropylmethyl)-7-methyl-*N*-(2-(trifluoromethyl)-6,7-dihydro-5*H-*cyclopenta[*b*]pyridin-5-yl)imidazo[1,5-*a*]quinoxaline-8-carboxamide | **481** |
| 189 | 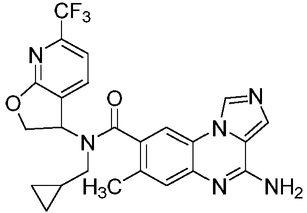 | 4-amino-*N*-(cyclopropylmethyl)-7-methyl-*N*-(6-(trifluoromethyl)-2,3-dihydrofuro[2,3-*b*]pyridin-3-yl)imidazo[1,5-*a*]quinoxaline-8-carboxamide | **483** |
| 190 | 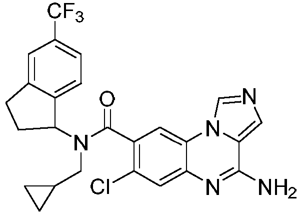 | 4-amino-7-chloro-*N-*(cyclopropylmethyl)-*N*-(5-(trifluoromethyl)-2,3-dihydro-1*H*-inden-1-yl)imidazo[1,5-*a*]quinoxaline-8-carboxamide | **500** |
| 191 | 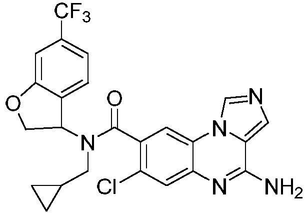 | 4-amino-7-chloro-*N-*(cyclopropylmethyl)-*N*-(6-(trifluoromethyl)-2,3-dihydrobenzofuran-3-yl)imidazo[1,5-*a*]quinoxaline-8-carboxamide | **502** |
| 192 | 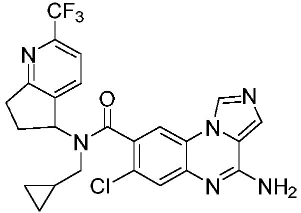 | 4-amino-7-chloro-*N-*(cyclopropylmethyl)-*N*-(2-(trifluoromethyl)-6,7-dihydro-5H-cyclopenta[*b*]pyridin-5-yl)imidazo[1,5-*a*]quinoxaline-8-carboxamide | **501** |
| 193 | 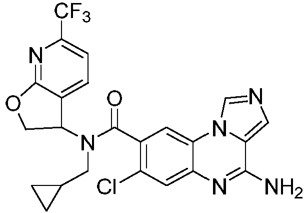 | 4-amino-7-chloro-*N-*(cyclopropylmethyl)-*N*-(6-(trifluoromethyl)-2,3-dihydrofuro[2,3-*b*]pyridin-3-yl)imidazo[1,5-*a*]quinoxaline-8-carboxamide | **503** |
| 194 | 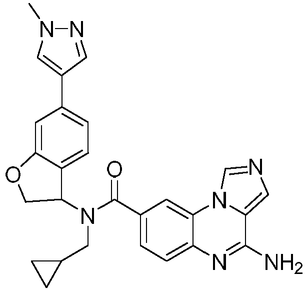 | 4-amino-*N*-(cyclopropylmethyl)-*N*-(6-(1-methyl-1*H*-pyrazol-4-yl)-2,3-dihydrobenzofuran-3-yl)imidazo[1 ,5*-a*]quinoxaline-8-carboxamide | **480** |
| 195 | 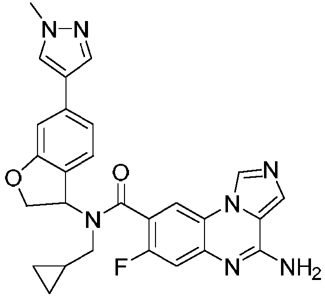 | 4-amino-*N*-(cyclopropylmethyl)-7-fluoro-*N*-(6-(1-methyl-1*H-*pyrazol-4-yl)-2,3-dihydrobenzofuran-3-yl)imidazo[1,5*-a*]quinoxaline-8-carboxamide | **498** |
| 196 | 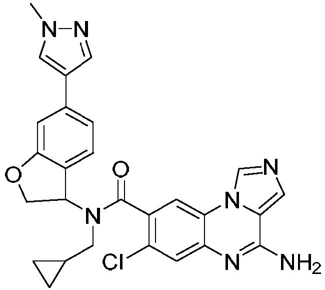 | 4-amino-7-chloro-*N-*(cyclopropylmethyl)-*N*-(6-(1-methyl-1*H*-pyrazol-4-yl)-2,3-dihydrobenzofuran-3-yl)imidazo[1 ,5*-a*] quinoxaline-8-carboxamide | **514** |
| 197 | 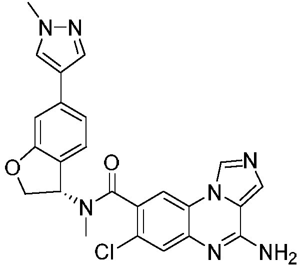 | (*S*)-4-amino-7-chloro-*N*-methyl-*N*-(6-(1-methyl-1*H*-pyrazol-4-yl)-2,3-dihydrobenzofuran-3-yl)imidazo[1,5*-a*]quinoxaline-8-carboxamide | **474** |
| 198 | 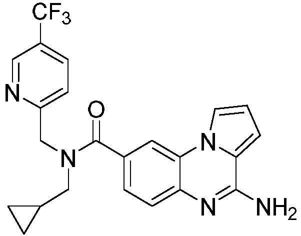 | 4-amino-*N*-(cyclopropylmethyl)-*N*-((5-(trifluoromethyl)pyridin-2-yl)methyl)pyrrolo[1,2-*a*]quinoxaline-8-carboxamide | **440** |
| 199 | 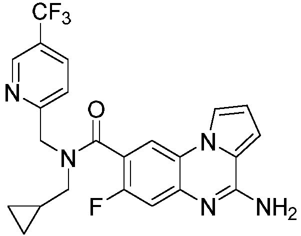 | 4-amino-*N*-(cyclopropylmethyl)-7-fluoro-*N*-((5-(trifluoromethyl)pyridin-2-yl)methyl)pyrrolo[1,2-*a*]quinoxaline-8-carboxamide | **458** |
| 200 | 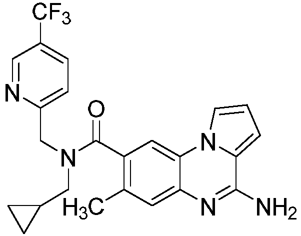 | 4-amino-*N*-(cyclopropylmethyl)-7-methyl-*N*-((5-(trifluoromethyl)pyridin-2-yl)methyl)pyrrolo[1,2-*a*]quinoxaline-8-carboxamide | **454** |
| 201 | 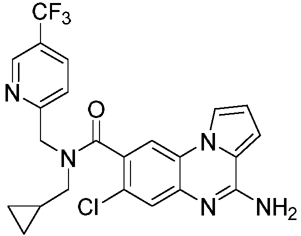 | 4-amino-7-chloro-*N-*(cyclopropylmethyl)-*N*-((5-(trifluoromethyl)pyridin-2-yl)methyl)pyrrolo[1,2-*a*]quinoxaline-8-carboxamide | **474** |
| 202 | 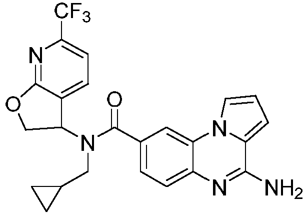 | 4-amino-*N*-(cyclopropylmethyl)-*N*-(6-(trifluoromethyl)-2,3-dihydrofuro[2,3-*b*]pyridin-3-yl)pyrrolo[1,2-*a*]quinoxaline-8-carboxamide | **468** |
| 203 | 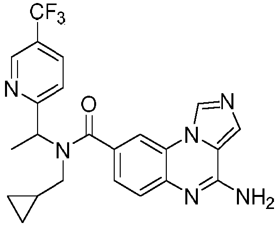 | 4-amino-*N*-(cyclopropylmethyl)-*N*-(1-(5-(trifluoromethyl)pyridin-2-ylethyl)imidazo[1,5-*a*]quinoxaline-8-carboxamide | **455** |
| 204 | 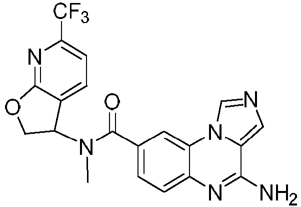 | 4-amino-*N*-methyl-*N*-(6-(trifluoromethyl)-2,3-dihydrofuro[2,3-*b*]pyridin-3-yl)imidazo[1,5-*a*]quinoxaline-8-carboxamide | **429** |
| 205 | 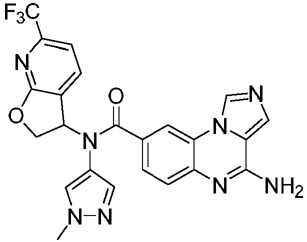 | 4-amino-*N*-(1-methyl-1*H-*pyrazol-4-yl)-*N*-(6-(trifluoromethyl)-2,3-dihydrofuro[2,3-*b*]pyridin-3-yl)imidazo[1,5-*a*]quinoxaline-8-carboxamide | **495** |
| 205a | 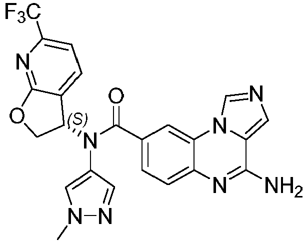 | (*S*)-4-amino-*N*-(1-methyl-1*H-*pyrazol-4-yl)-*N*-(6-(trifluoromethyl)-2,3-dihydrofuro[2,3-*b*]pyridin-3-yl)imidazo[1,5*-a*]quinoxaline-8-carboxamide | **495** |
| 206 | 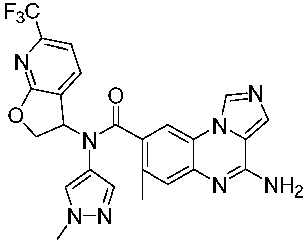 | 4-amino-7-methyl-*N*-(1-methyl-1*H*-pyrazol-4-yl)-*N*-(6-(trifluoromethyl)-2,3-dihydrofuro[2,3-*b*]pyridin-3-yl)imidazo[1,5-*a*]quinoxaline-8-carboxamide | **509** |
| 206a | 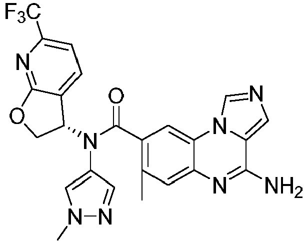 | (*S*)-4-amino-7-methyl-*N*-(1-methyl-1*H*-pyrazol-4-yl)-*N*-(6-(trifluoromethyl)-2,3-dihydrofuro[2,3-*b*]pyridin-3-yl)imidazo[1,5*-a*]quinoxaline-8-carboxamide | **509** |
| 207 | 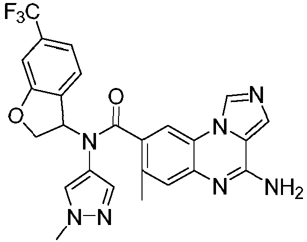 | 4-amino-7-methyl-*N*-(1-methyl-1*H*-pyrazol-4-yl)-*N*-(6-(trifluoromethyl)-2,3-dihydrobenzofuran-3-yl)imidazo[1,5-*a*]quinoxaline-8-carboxamide | **508** |
| 207a | 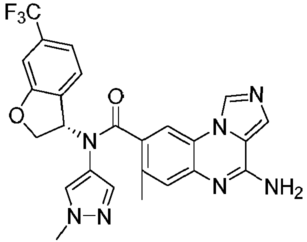 | (*S*)-4-amino-7-methyl-*N*-(1-methyl-1*H*-pyrazol-4-yl)-*N*-(6-(trifluoromethyl)-2,3-dihydrobenzofuran-3-yl)imidazo[1,5-*a*]quinoxaline-8-carboxamide | **508** |
| 208 | 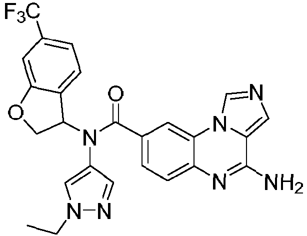 | 4-amino-*N*-(1-ethyl-1*H*-pyrazol-4-yl)-*N*-(6-(trifluoromethyl)-2,3-dihydrobenzofuran-3-yl)imidazo[1,5-*a*]quinoxaline-8-carboxamide | **508** |
| 208a | 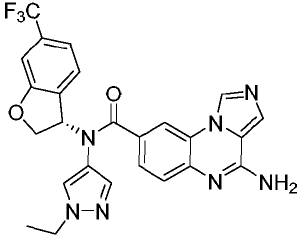 | (*S*)-4-amino-*N*-(1-ethyl-1*H-*pyrazol-4-yl)-*N*-(6-(trifluoromethyl)-2,3-dihydrobenzofuran-3-yl)imidazo[1,5-*a*]quinoxaline-8-carboxamide | **508** |
| 209 | 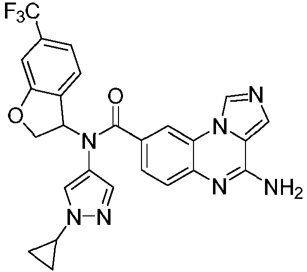 | 4-amino-*N*-(1-cyclopropyl-1*H-*pyrazol-4-yl)-*N*-(6-(trifluoromethyl)-2,3-dihydrobenzofuran-3-yl)imidazo[1,5*-a*]quinoxaline-8-carboxamide | **520** |
| 209a | 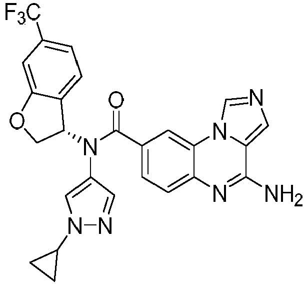 | (*S*)-4-amino-*N*-(1-cyclopropyl-1*H*-pyrazol-4-yl)-N-(6-(trifluoromethyl)-2,3-dihydrobenzofuran-3-yl)imidazo[1,5-*a*]quinoxaline-8-carboxamide | **520** |
| 210 | 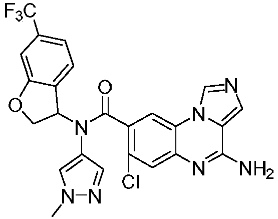 | 4-amino-7-chloro-*N*-(1-methyl-1*H*-pyrazol-4-yl)-*N*-(6-(trifluoromethyl)-2,3-dihydrobenzofuran-3-yl)imidazo[1,5-*a*]quinoxaline-8-carboxamide | **528** |
| 210a | 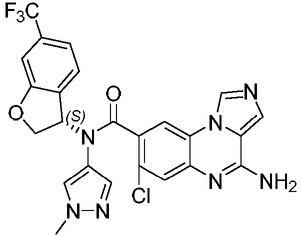 | (*S*)-4-amino-7-chloro-*N*-(1-methyl-1*H*-pyrazol-4-yl)-*N*-(6-(trifluoromethyl)-2,3-dihydrobenzofuran-3-yl)imidazo[1,5-*a*]quinoxaline-8-carboxamide | **528** |
| 211 | 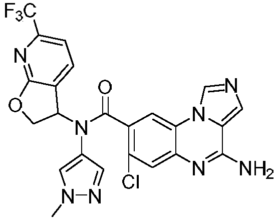 | 4-amino-7-chloro-*N*-(1-methyl-1*H*-pyrazol-4-yl)-*N*-(6-(trifluoromethyl)-2,3-dihydrofuro[2,3-*b*]pyridin-3-yl)imidazo[1,5*-a*]quinoxaline-8-carboxamide | **529** |
| 211a | 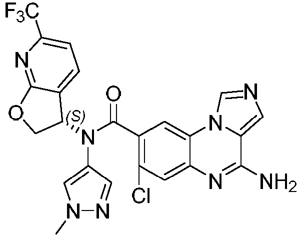 | (*S*)-4-amino-7-chloro-*N*-(1-methyl-1*H*-pyrazol-4-yl)-*N*-(6-(trifluoromethyl)-2,3-dihydrofuro[2,3-*b*]pyridin-3-yl)imidazo[1,5-*a*]quinoxaline-8-carboxamide | **529** |
| 212 | 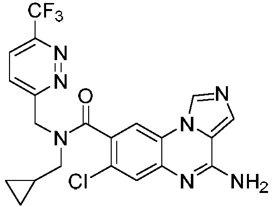 | 4-amino-7-chloro-*N-*(cyclopropylmethyl)-N-((6-(trifluoromethyl)pyridazin-3-yl)methyl)imidazo[1,5-*a*]quinoxaline-8-carboxamide | **476** |
| 213 | 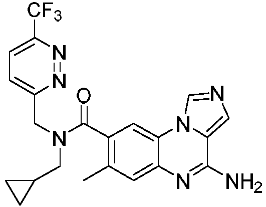 | 4-amino-*N*-(cyclopropylmethyl)-7-methyl-*N*-((6-(trifluoromethyl)pyridazin-3-yl)methyl)imidazo[1,5-*a*]quinoxaline-8-carboxamide | **456** |
| 214 | 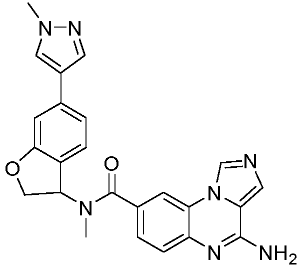 | 4-amino-*N*-methyl-N-(6-(1-methyl-1*H*-pyrazol-4-yl)-2,3-dihydrobenzofuran-3-yl)imidazo[1,5-*a*]quinoxaline-8-carboxamide | **440** |
| 215 | 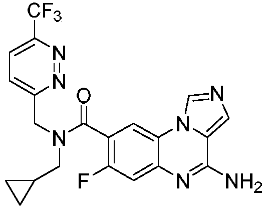 | 4-amino-*N*-(cyclopropylmethyl)-7-fluoro-*N*-((6-(trifluoromethyl)pyridazin-3-yl)methyl)imidazo[1,5-*a*]quinoxaline-8-carboxamide | **460** |
| 216 | 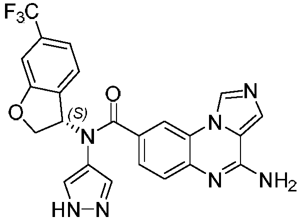 | (*S*)-4-amino-*N*-(1H-pyrazol-4-yl)-*N*-(6-(trifluoromethyl)-2,3-dihydrobenzofuran-3-yl)imidazo[1,5-*a*]quinoxaline-8-carboxamide | **480** |
| 217 | 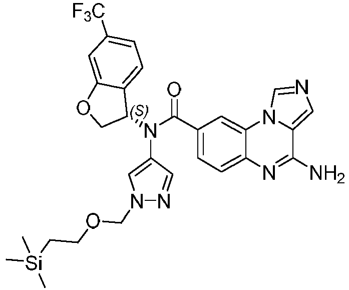 | (*S*)-4-amino-*N*-(6-(trifluoromethyl)-2,3-dihydrobenzofuran-3-yl)-*N*-(1-((2-(trimethylsilyl)ethoxy)methyl)-1*H*-pyrazol-4-yl)imidazo[1,5-*a*]quinoxaline-8-carboxamide | **610** |
| 218 | 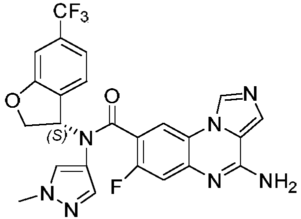 | (*S*)-4-amino-7-fluoro*-N*-(1-methyl-1*H*-pyrazol-4-yl)-*N*-(6-(trifluoromethyl)-2,3-dihydrobenzofuran-3-yl)imidazo[1,5-*a*]quinoxaline-8-carboxamide | **512** |
| 219 | 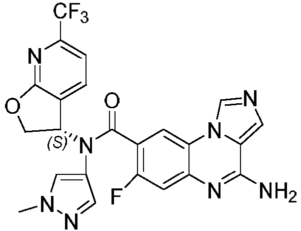 | (*S*)-4-amino-7-fluoro-*N*-(1-methyl-1*H*-pyrazol-4-yl)-*N*-(6-(trifluoromethyl)-2,3-dihydrofuro[2,3-*b*]pyridin-3-yl)imidazo[1,5-*a*]quinoxaline-8-carboxamide | **513** |
| 220 | 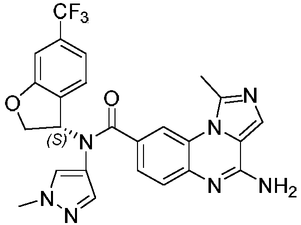 | (*S*)-4-amino-1-methyl-*N*-(1-methyl-1*H*-pyrazol-4-yl)-*N*-(6-(trifluoromethyl)-2,3-dihydrobenzofuran-3-yl)imidazo[1,5-*a*]quinoxaline-8-carboxamide | **508** |
| 221 | 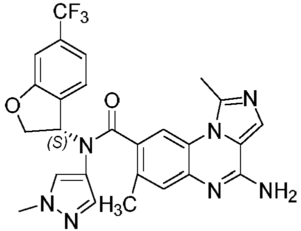 | (*S*)-4-amino-1,7-dimethyl-*N*-(1-methyl-1H-pyrazol-4-yl)-*N*-(6-(trifluoromethyl)-2,3-dihydrobenzofuran-3-yl)imidazo[1,5-*a*]quinoxaline-8-carboxamide | **522** |
| 222 | 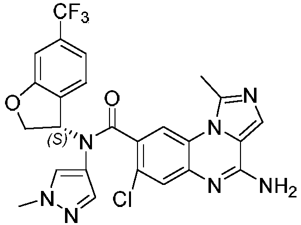 | (*S*)-4-amino-7-chloro-1-methyl-*N*-(1-methyl-1*H*-pyrazol-4-yl)-*N*-(6-(trifluoromethyl)-2,3-dihydrobenzofuran-3-yl)imidazo[1,5-*a*]quinoxaline-8-carboxamide | **542** |
| 223 | 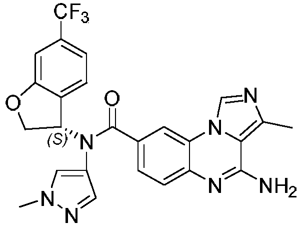 | (*S*)-4-amino-3-methyl-*N*-(1-methyl-1*H*-pyrazol-4-yl)-*N*-(6-(trifluoromethyl)-2,3-dihydrobenzofuran-3-yl)imidazo[1,5-*a*]quinoxaline-8-carboxamide | **508** |
| 224 | 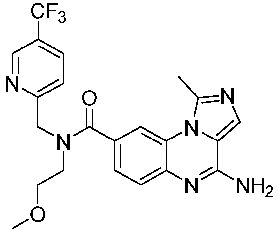 | 4-amino-*N*-(2-methoxyethyl)-1-methyl-*N*-((5-(trifluoromethyl)pyridin-2-yl)methyl)imidazo[1,5-*a*]quinoxaline-8-carboxamide | **459** |
| 225 | 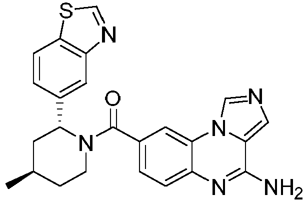 | (4-aminoimidazo[1,5-*a*]quinoxalin-8-yl)((2*R*,4*R*)-2-(benzo[*d*]thiazol-5-yl)-4-methylpiperidin-1-yl)methanone | **443** |
| 226 | 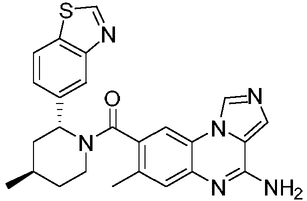 | (4-amino-7-methylimidazo[1,5-*a*]quinoxalin-8-yl)((2*R*,4*R*)-2-(benzo[*d*]thiazol-5-yl)-4-methylpiperidin-1-yl)methanone | **457** |
| 227 | 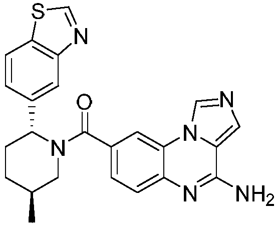 | (4-aminoimidazo[1,5-*a*]quinoxalin-8-yl)((2*R*,5*S*)-2-(benzo[d]thiazol-5-yl)-5-methylpiperidin-1-yl)methanone | 443 |
| 228 | 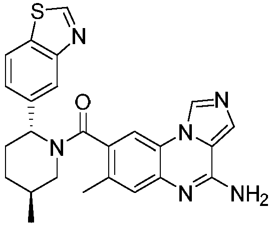 | (4-amino-7-methylimidazo[1,5-*a*]quinoxalin-8-yl)((2*R*,5*S*)-2-(benzo[d]thiazol-5-yl)-5-methylpiperidin-1-yl)methanone | 457 |
| 229 | 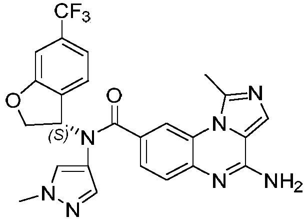 | (*S*)-4-amino-1-methyl-*N*-(1-methyl-1*H*-pyrazol-4-yl)-N-(6-(trifluoromethyl)-2,3-dihydrobenzofuran-3-yl)imidazo[1,5-*a*]quinoxaline-8-carboxamide | **508** |
| 230 | 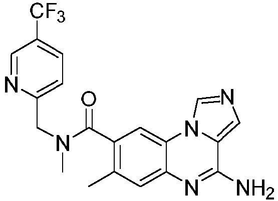 | 4-amino-*N*,7-dimethyl-*N*-((5-(trifluoromethyl)pyridin-2-yl)methyl)imidazo[1,5-*a*]quinoxaline-8-carboxamide | **415** |
| 231 | 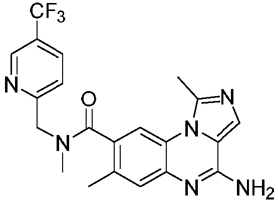 | 4-amino-*N*,1,7-trimethyl-*N*-((5-(trifluoromethyl)pyridin-2-yl)methyl)imidazo[1,5-*a*]quinoxaline-8-carboxamide | **429** |
| 232 | 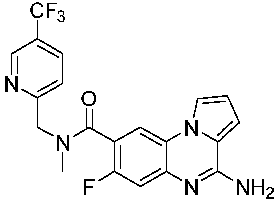 | 4-amino-7-fluoro-*N*-methyl-*N-*((5-(trifluoromethyl)pyridin-2-yl)methyl)pyrrolo[1,2-*a*]quinoxaline-8-carboxamide | **418** |
| 233 | 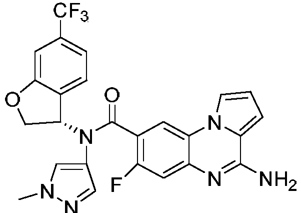 | (*S*)-4-amino-7-fluoro-*N*-(1-methyl-1*H*-pyrazol-4-yl)-N-(6-(trifluoromethyl)-2,3-dihydrobenzofuran-3-yl)pyrrolo[1,2-*a*]quinoxaline-8-carboxamide | **511** |

¹H NMR data of the compounds prepared in the above examples are as follows:

| **Example No.** | **¹H NMR** |
|---|---|
| **3** | ¹H NMR (400 MHz, DMSO-*d*₆) δ 9.15 (s, 1H), 8.93-8.71 (m, 3H), 8.38 (s, 1H), 8.10 (s, 1H), 7.92 (s, 1H), 7.71-7.28 (m, 6H), 5.84-5.40 (m, 1H), 4.93 (d, *J* = 17.0 Hz, 1H), 4.57 (d, *J =* 16.9 Hz, 1H), 1.63 (d, *J =* 6.9 Hz, 3H). |
| **14a** | ¹H NMR (400 MHz, MeOH-*d*₄) δ 9.35 (s, 1H), 8.98-8.93 (m, 1H), 8.53 (d, *J* = 1.6 Hz, 1H), 8.39 (s, 1H), 8.12 *(dd, J =* 8.4, 2.4 Hz, 1H), 7.85-7.68 (m, 3H), 5.43 (s, 1H), 5.02 (d, *J =* 11.2 Hz, 1H), 4.27-4.15 (m, 1H), 4.01 (dd, *J* = 12.4, 3.8 Hz, 1H), 3.88 (dd, *J* = 11.7, 3.5 Hz, 1H), 3.72 (d, *J =* 11.5 Hz, 1H), 0.87 (d, *J=* 7.0 Hz, 3H). |
| **22** | ¹H NMR (400 MHz, DMSO-*d*₆) δ 9.21 (d, *J* = 3.7 Hz, 1H), 8.46-8.18 (m, 1H), 7.91 (d, *J =* 3.7 Hz, 1H), 7.69-7.50 (m, 3H), 7.54-7.26 (m, 4H), 5.78-5.33 (m, 1H), 3.20-2.75 (m, 4H), 2.40-2.10 (m, 2H), 1.15-0.81 (m, 1H), 053-0.23(m, 2H), 0.14-0.06 (m, 2H). |
| **30** | ¹H NMR (400 MHz, DMSO-*d*₆) δ 9.07 (s, 1H), 8.19 (d, *J =* 1.9 Hz, 1H), 7.88 (s, 1H), 7.76 (d, *J =* 7.8 Hz, 1H), 7.48 (s, 1H), 7.46 (s, 2H), 7.37-7.29 (m, 2H), 7.24 (d, *J* = 8.4 Hz, 1H), 7.09 (s, 1H), 6.99 (s, 1H), 6.53-6.49 (m, 1H), 4.90 (t, *J* = 9.7 Hz, 1H), 4.68 (dd, *J* = 10.4, 4.1 Hz, 1H), 3.51 (s, 3H). |
| **30a** | ¹H NMR (400 MHz, DMSO-*d*₆) δ 9.07 (s, 1H), 8.19 (d, *J =* 1.9 Hz, 1H), 7.88 (s, 1H), 7.76 (d, *J =* 7.8 Hz, 1H), 7.48 (s, 1H), 7.46 (s, 2H), 7.37-7.29 (m, 2H), 7.24 (d, *J* = 8.4 Hz, 1H), 7.09 (s, 1H), 6.99 (s, 1H), 6.59-6.44 (m, 1H), 4.90 (t, *J* = 9.7 Hz, 1H), 4.68 (dd, *J* = 10.4, 4.1 Hz, 1H), 3.51 (s, 3H). |
| **34a** | ¹H NMR (400 MHz, MeOH-*d*₄) δ 9.09 (s, 1H), 8.24 (s, 1H), 8.04 (d, *J =* 7.9 Hz, 1H), 7.93 (s, 1H), 7.66 (d, *J* = 7.9 Hz, 1H), 7.57 (d, *J* = 8.3 Hz, 1H), 7.51 (s, 1H), 5.63-5.53 (m, 1H), 3.52-3.32 (m, 2H), 3.17-2.45 (m, 4H), 1.24-1.01 (m, 1H), 0.51 (d, *J* = 8.2 Hz, 2H), 0.17-0.03 (m, 2H). |
| **34b** | ¹H NMR (400 MHz, MeOH-*d*₄) δ 9.08 (s, 1H), 8.23 (s, 1H), 8.04 (d, *J* = 7.9 Hz, 1H), 7.93 (s, 1H), 7.65 (d, *J* = 7.9 Hz, 1H), 7.61-7.39 (m, 2H), 5.56 (t, *J* = 9.4 Hz, 1H), 3.52-3.34 (m, 2H), 3.25-2.91 (m, 2H), 2.78-2.47 (m, 2H), 1.14-1.01 (m, 1H), 0.60-0.42 (m, 2H), 0.25-0.02 (m, 2H). |
| **144** | ¹H NMR (400 MHz, MeOH-*d*₄) δ 9.06 (s, 1H), 8.88 (s, 1H), 8.26 (t, *J =* 1.2 Hz, 1H), 8.12 (d, *J* = 8.1 Hz, 1H), 7.94-7.88 (m, 1H), 7.74 (s, 1H), 7.58-7.44 (m, 2H), 5.09 (s, 2H), 3.54-3.35 (m, 2H), 1.15-0.98 (m, 1H), 0.55-0.41 (m, 2H), 0.25-0.06 (m, 2H). |
| **145** | ¹H NMR (400 MHz, DMSO-*d*₆) δ 9.43 (s, 1H), 9.30 (s, 1H), 8.91 (s, 1H), 8.38-8.32 (m, 2H), 8.15 (d, *J* = 8.4 Hz, 1H), 7.71 (d, *J* = 8.6 Hz, 1H), 7.66-7.42 (m, 3H), 4.88 (s, 2H), 3.12-3.03 (m, 1H), 1.23 *(d, J=* 6.8 Hz, 3H), 1.04-0.93 (m, 1H), 0.48-0.38 (m, 2H), 0.28-0.18 (m, 2H). |
| **146** | ¹H NMR (400 MHz, MeOH-*d*₄) δ 8.99 (s, 1H), 8.85-8.76 (m, 1H), 8.20 (s, 1H), 8.08-7.98 (m, 1H), 7.84 (s, 1H), 7.62-7.32 (m, 3H), 4.87 (s, 2H), 3.10 (s, 3H). |
| **147** | ¹H NMR (400 MHz, DMSO-*d*₆) δ 9.16 (s, 1H), 9.03-8.86 (m, 1H), 8.24 (s, 1H), 8.17 (d, *J =* 8.3 Hz, 1H), 7.91 (s, 1H), 7.54 (d, *J =* 8.2 Hz, 1H), 7.49-7.30 (m, 4H), 4.93 (s, 2H), 4.45 (s, 1H), 2.14 (t, *J =* 10.2 Hz, 2H), 2.03-1.96 (m, 2H), 1.61-1.35 (m, 2H). |
| **148** | ¹H NMR (400 MHz, MeOH-*d*₄) δ 9.16 (s, 1H), 8.83 (d, *J =* 2.4 Hz, 1H), 8.33 (s, 1H), 8.26 (s, 1H), 7.93 (s, 1H), 7.62-7.48 (m, 2H), 7.30-7.20 (m, 1H), 4.87 (s, 2H), 4.27 (q, *J =* 9.1 Hz, 2H). |
| **149** | ¹H NMR (400 MHz, MeOH-*d*₄) δ 8.84 (s, 1H), 8.78 (s, 1H), 8.11 (s, 1H), 8.02 (d, *J = 8.3* Hz, 1H), 7.81 (s, 1H), 7.61 (s, 1H), 7.46 (s, 1H), 7.27 (dd, *J* = 27.6, 19.1 Hz, 3H), 5.14 (s, 2H), 3.59 (s, 3H). |
| **154** | ¹H NMR (400 MHz, DMSO-*d*₆) δ 9.20 (s, 1H), 8.93 (s, 1H), 8.21-8.05 (m, 2H), 7.92 (s, 1H), 7.78 (d, *J =* 8.4 Hz, 1H), 7.57-7.25 (m, 4H), 4.94-4.79 (m, 2H), 3.04-2.97 (m, 1H), 1.81-1.71 (m, 1H), 1.66-1.59 (m, 1H), 1.01-0.92 (m, 1H), 0.84 (t, *J* = 7.4 Hz, 3H), 0.55-0.36 (m, 2H), 0.22-0.06 (d, *J =* 29.0 Hz, 2H). |
| **156** | ¹H NMR (400 MHz, DMSO-*d*₆) δ 9.19 (s, 1H), 8.33 (s, 1H), 8.29-8.18 (m, 1H), 8.12-8.00 (m, 1H), 7.95 (s, 1H), 7.61-7.22 (m, 4H), 5.19 (s, 2H), 3.40-3.32 (m, 2H), 1.10-0.96 (m, 1H), 0.46-0.28 (m, 2H), 0.27-0.00 (m, 2H). |
| **158** | ¹H NMR (400 MHz, DMSO-*d*₆) δ 9.37-9.05 (m, 1H), 8.67 (s, 1H), 8.36 (s, 1H), 8.11-7.82 (m, 3H), 7.69-7.35 (m, 4H), 5.01-4.72 (m, 2H), 3.29-3.20 (m, 2H), 1.11-0.91 (m, 1H), 0.49-0.29 (m, 2H), 0.24-0.00 (m, 2H). |
| **163** | ¹H NMR (400 MHz, DMSO-*d*₆) δ 9.18-8.97 (m, 1H), 8.97-8.85 (m, 1H), 8.35-8.22 (m, 1H), 8.20-8.04 (m, 1H), 7.93-7.84 (m, 1H), 7.64-7.42 (m, 3H), 7.29-7.09 (m, 1H), 5.10-4.70(m, 2H), 3.41 (s, 1H), 3.20 (d, *J* = 6.8 Hz, 1H), 1.13-0.85 (m, 1H), 0.50-0.26 (m, 2H), 0.24--0.04 (m, 2H). |
| **164** | ¹H NMR (400 MHz, MeOH-*d*₄) δ 9.30-9.08 (m, 1H), 8.89 (t, *J* = 3.3 Hz, 1H), 8.40-8.31 (m, 1H), 8.31-8.19 (m, 1H), 8.19-7.99 (m, 1H), 7.79-7.41 (m, 1H), 7.56-7.46 (m, 1H), 5.13 (d, *J =* 3.7 Hz, 1H), 4.69 (s, 1H), 3.80-3.33 (m, 1H), 3.19 (s, 1H), 2.47 (s, 3H), 1.24-0.90 (m, 1H), 0.65-0.37 (m, 2H), 0.35-0.07 (m, 2H). |
| **165** | ¹H NMR (400 MHz, DMSO-*d*₆) δ 9.19 (s, 0.6H), 9.00-8.95 (m, 1H), 8.89 (s, 0.4H), 8.36 (s, 0.6H), 8.27 (dd, *J* = 8.1, 2.4 Hz, 0.6H), 8.12(s, 0.4H), 8.08(dd, *J* = 8.1, 2.4 Hz, 0.4H), 7.94 (s, 0.6H), 7.87(s, 0.4H), 7.76 (d, *J* = 8.3 Hz, 0.6H), 7.67-7.40 (m, 3.4H), 5.21-4.53 (m, 2H), 3.80-3.71 (m, 0.4H), 3.23-3.06 (m, 1.6H), 1.15-0.85 (m, 1H), 0.55-0.23 (m, 2H), 0.19-0.08 (m, 2H). |
| **166** | ¹H NMR (400 MHz, MeOH-*d*₄) δ 8.91 (d, *J =* 29.8 Hz, 1H), 8.43-8.23 (m, 2H), 8.19-8.09 (m, 1H), 7.73 (s, 2H), 7.69-7.55 (m, 1H), 5.16-4.95(m, 2H), 3.50-3.34 (m, 2H), 3.13-2.91 (m, 3H), 1.19-0.96 (m, 1H), 0.57-0.41 (m, 2H), 0.30-0.01 (m, 2H). |
| **168** | ¹H NMR (400 MHz, MeOH-*d*₄) δ 8.86 (s, 0.6H), 8.84 (s, 0.4H), 8.24-8.12 (m, 1H), 8.06-8.02 (m, 1H), 7.84 (s, 0.6H), 7.78 (s, 0.4H), 7.70 (d, *J* = 8.3 Hz, 0.6H), 7.50 *(d, J=* 8.3 Hz, 0.4H), 7.28 (d, *J* = 10.9 Hz, 0.6H), 7.22 (d, *J* = 10.9 Hz, 0.4H), 5.13 (s, 1.2H), 4.90 (s, 0.8H), 3.56-3.34 (m, 2H), 3.03 (s, 1.8H), 2.81 (s, 1.2H), 1.23-0.90 (m, 1H), 0.61-0.39 (m, 2H), 0.28-0.04 (m, 2H). |
| **169** | ¹H NMR (400 MHz, DMSO-*d*₆) δ 8.94 (s, 1H), 8.21 (dd, *J* = 40.4, 8.3 Hz, 1H), 7.90 (s, 0.5H), 7.79 (s, 0.5H), 7.73-7.67 (m, 1.5H), 7.54-7.48 (m, 0.5H), 7.32 (d, *J = 14.1* Hz, 1H), 7.20 (d, *J* = 25.3 Hz, 2H), 5.11-4.50 (m, 2H), 3.84 (s, 1H), 3.15 (s, 1H), 2.95 (s, 1.5H), 2.59 (s, 1.5H), 2.33 (s, 3H), 1.19-1.09 (m, 0.5H), 0.96-0.84 (m, 0.5H), 0.55-0.20 (m, 3H), -0.01-0.08 (m, 1H). |
| **179** | ¹H NMR (400 MHz, DMSO-*d*₆) δ 9.49 (s, 1H), 9.43 (s, 1H), 8.60 (s, 1H), 8.53 (s, 1H), 8.40 (s, 1H), 7.74-7.53 (m, 5H), 4.95-4.64 (m, 3H), 4.22-4.07 (m, 2H), 2.75 (s, 3H). |
| **181a** | ¹H NMR (400 MHz, CDCl₃) δ 8.64 (s, 1H), 7.99 (d, *J =* 1.8 Hz, 1H), 7.85 (d, *J* = 7.5 Hz, 1H), 7.76 (s, 1H), 7.63 (d, *J =* 8.4 Hz, 1H), 7.45 (dd, *J* = 8.2, 1.7 Hz, 1H), 7.34 (d, *J =* 7.5 Hz, 1H), 6.09 (s, 1H), 5.49 (br s, 2H), 4.97 (t, *J =* 10.0 Hz, 1H), 4.88 (dd, *J =* 10.4, 4.9 Hz, 1H), 3.34 (dd, *J =* 15.0, 7.0 Hz, 1H), 3.20 (dd, *J =* 15.0, 6.5 Hz, 1H), 0.75 (s, 1H), 0.52-0.35 (m, 2H), -0.07-0.14 (m, 2H). |
| **181b** | ¹H NMR (400 MHz, CDCl₃) δ 8.64 (s, 1H), 7.99 (d, *J* = 1.8 Hz, 1H), 7.85 (d, *J* = 7.5 Hz, 1H), 7.76 (s, 1H), 7.63 (d, *J =* 8.4 Hz, 1H), 7.45 (dd, *J* = 8.2, 1.7 Hz, 1H), 7.34 (d, *J =* 7.5 Hz, 1H), 6.16-6.01 (m, 1H), 5.80-5.20 (br s, 2H), 4.97 (t, *J =* 10.0 Hz, 1H), 4.88 (dd, *J =* 10.4, 4.9 Hz, 1H), 3.34 (dd, *J* = 15.0, 7.0 Hz, 1H), 3.20 (dd, *J =* 15.0, 6.5 Hz, 1H), 0.75 (s, 1H), 0.53-0.35 (m, 2H), -0.02--0.19 (m, 2H). |
| **203** | ¹H NMR (400 MHz, MeOH-*d*₄) δ 9.08 (s, 1H), 8.89 (s, 1H), 8.28 (s, 1H), 8.14-8.05 (m, 1H), 7.94 (s, 1H), 7.70-7.44 (m, 3H), 5.50-5.30 (m, 1H), 3.45-3.35 (m, 2H), 1.85 (s, 3H), 1.06-0.93 (m, 1H), 0.47-0.34 (m, 2H), 0.21-0.06 (m, 2H). |
| **204** | ¹H NMR (400 MHz, MeOH-*d*₄) δ 9.07 (s, 1H), 8.28 (s, 1H), 8.07 (d, *J =* 7.5 Hz, 1H), 7.94 (s, 1H), 7.57 (s, 2H), 7.44 (d, *J* = 7.5 Hz, 1H), 6.39 (brs, 2H), 4.81 (dd, *J =* 10.5, 4.6 Hz, 1H), 2.88 (s, 3H). |
| **205a** | ¹H NMR (400 MHz, MeOH-*d*₄) δ 8.93 (s, 1H), 8.17-8.13 (m, 2H), 7.91 (s, 1H), 7.44 (s, 1H), 7.43-7.40 (m, 2H), 7.34 (d, *J* = 8.5 Hz, 1H), 7.13 (s, 1H), 6.60-6.53 (m, 1H), 5.02-4.95 (m, 1H), 4.87-4.83 (m, 1H), 3.60 (s, 3H). |
| **206a** | ¹H NMR (400 MHz, MeOH-*d*₄) δ 8.90 (s, 1H), 8.17 (d, *J =* 7.5 Hz, 1H), 7.92 (s, 1H), 7.87 (s, 1H), 7.49-7.41 (m, 2H), 7.20 (s, 1H), 7.11 (s, 1H), 6.67 (d, *J =* 8.6 Hz, 1H), 5.01 (t, *J =* 9.8 Hz, 1H), 4.86-4.83 (m, 1H), 3.54 (s, 3H), 2.40 (s, 3H). |
| **207a** | ¹H NMR (400 MHz, MeOH-*d*₄) δ 8.91 (s, 1H), 7.90 (s, 1H), 7.86 (s, 1H), 7.74 (d, *J = 7.8* Hz, 1H), 7.33-7.26 (m, 2H), 7.17 (s, 1H), 6.99 (s, 1H), 6.96 (s, 1H), 6.75-6.66 (m, 1H), 4.88-4.83 (m, 1H), 4.78-4.73 (m, 1H), 3.50 (s, 3H), 2.39 (s, 3H). |
| **208a** | ¹H NMR (400 MHz, MeOH-*d*₄) δ 8.91 (s, 1H), 8.11 (d, *J* = 1.8 Hz, 1H), 7.89 (s, 1H), 7.71 (d, *J* = 7.8 Hz, 1H), 7.44-7.37 (m, 1H), 7.32 (d, *J* = 8.4 Hz, 1H), 7.29-7.25 (m, 1H), 7.25 (s, 1H), 7.03 (s, 1H), 6.94 (s, 1H), 6.62-6.56 (m, 1H), 4.81-4.75 (m, 1H), 4.62 (s, 1H), 3.84 (q, *J =* 7.3 Hz, 2H), 1.05 (t, *J =* 7.3 Hz, 3H). |
| **209a** | ¹H NMR (400 MHz, MeOH-*d*₄) δ 8.93 (s, 1H), 8.11 (d, *J =* 1.8 Hz, 1H), 7.90 (s, 1H), 7.71 (d, *J =* 7.8 Hz, 1H), 7.44-7.37(m, 1H), 7.34 (d, *J =* 8.5 Hz, 1H), 7.32-7.27 (m, 1H), 7.26 (s, 1H), 7.01 (d, *J =* 0.8 Hz, 1H), 6.95 (s, 1H), 6.62-6.51 (m, 1H), 4.79- 4.73 (m, 1H), 4.61 (s, 1H), 3.39-3.32 (m, 1H), 0.86-0.77 (m, 2H), 0.70-0.57 (m, 2H). |
| **210a** | ¹H NMR (400 MHz, DMSO-*d*₆) δ 9.04 (s, 1H), 8.24 (s, 1H), 7.90 (s, 1H), 7.71 (d, *J = 7.8* Hz, 1H), 7.57 (s, 2H), 7.41 (s, 1H), 7.35 (d, *J =* 7.9 Hz, 1H), 7.25 (s, 1H), 7.16-7.03 (m, 1H), 6.92 (s, 1H), 6.66 (dd, *J =* 9.3, 3.9 Hz, 1H), 4.92 *(dd, J =* 10.5, 9.1 Hz, 1H), 4.63 (dd, *J* = 10.4, 3.9 Hz, 1H), 3.47 (s, 3H). |
| **211a** | ¹H NMR (400 MHz, DMSO-*d*₆) δ 9.01 (s, 1H), 8.24 (s, 1H), 8.14 (d, *J =* 7.5 Hz, 1H), 7.90 (s, 1H), 7.61-7.48 (m, 4H), 7.26 (s, 1H), 7.07 (s, 1H), 6.64-6.52 (m, 1H), 5.02 (t, *J =* 9.7 Hz, 1H), 4.70 (dd, *J =* 10.4, 4.2 Hz, 1H), 3.50 (s, 3H). |
| **212** | ¹H NMR (400 MHz, DMSO-*d*₆) δ 9.21-8.94 (m, 1H), 8.42-7.61 (m, 6H), 7.55-7.41(m, 1H), 5.44-4.80 (m, 2H), 3.24-3.10 (m, 2H), 1.24-0.86 (m, 1H), 0.59-0.21 (m, 2H), 0.16-0.09 (d, *J* = 9.8 Hz, 2H). |
| **213** | ¹H NMR (400 MHz, DMSO-*d*₆) δ 9.12-8.87 (m, 1H), 8.30-7.80 (m, 4H), 7.38-7.25 (m, 3H), 5.22-4.91 (m, 2H), 3.18 (s, 2H), 2.30 (s, 3H), 1.24-0.86 (m, 1H), 0.48-0.01 (m, 4H). |
| **214** | ¹H NMR (400 MHz, DMSO-*d*₆) δ 9.19 (s, 1H), 8.33 (s, 1H), 8.15 (s, 1H), 7.92 (s, 1H), 7.87 (s, 1H), 7.48-7.43 (m, 4H), 7.37 (d, *J =* 7.8 Hz, 1H), 7.18 (d, *J* = 7.7 Hz, 1H), 7.10 (s, 1H), 6.31-5.63 (m, 1H), 4.84-4.58 (m, 2H), 3.85 (s, 3H), 2.67 (s, 3H). |
| **215** | ¹H NMR (400 MHz, MeOH-*d*₄) δ 8.95-8.78 (m, 1H), 8.19-8.13 (m, 1H), 8.10-8.01 (m, 1H), 7.98-7.90 (m, 1H), 7.85-7.71 (m, 1H), 7.23-7.06 (m, 1H), 5.27-4.96 (m, 2H), 3.51-3.23 (m, 2H), 1.12-0.80 (m, 1H), 0.49-0.27 (m, 2H), 0.24-0.04 (m, 2H). |
| **216** | ¹H NMR (400 MHz, MeOH-*d*₄) δ 8.92 (s, 1H), 8.12 (d, *J* = 1.7 Hz, 1H), 7.89 (s, 1H), 7.74 (d, *J =* 7.8 Hz, 1H), 7.41 (dd, *J* = 8.4, 1.8 Hz, 1H), 7.33 (d, *J* = 8.5 Hz, 1H), 7.29 (d, *J* = 7.6 Hz, 2H), 7.08 (s, 1H), 6.95 (s, 1H), 6.63 (d, *J* = 8.3 Hz, 1H), 4.87-4.84 (m, 1H), 4.80-4.75 (m, 1H). |
| **217** | ¹H NMR (400 MHz, DMSO-*d*₆) δ 9.04 (s, 1H), 8.16 (s, 1H), 7.86 (s, 1H), 7.77 (d, *J* = 7.8 Hz, 1H), 7.67 (s, 1H), 7.46-7.21 (m, 4H), 7.11 (s, 1H), 7.01 (s, 1H), 6.74-6.53 (m, 2H), 5.12-5.00 (m, 2H), 4.91 (t, *J* = 9.7 Hz, 1H), 4.76-4.68 (m, 1H), 2.89-2.82 (m, 2H), 0.43-0.31 (m, 2H), 0.25 (s, 9H). |
| **218** | ¹H NMR (400 MHz, DMSO-*d*₆) δ 9.04 (s, 1H), 8.21 (d, *J =* 6.4 Hz, 1H), 7.89 (s, 1H), 7.69 (d, *J* = 7.8 Hz, 1H), 7.57 (s, 1H), 7.43 (s, 1H), 7.34 (d, *J* = 7.8 Hz, 1H), 7.11 (s, 1H), 6.99 (d, *J* = 10.9 Hz, 1H), 6.88 (s, 1H), 6.65-6.56 (m, 1H), 4.90 (t, *J* = 9.8 Hz, 1H), 4.66-4.59 (m, 1H), 3.49 (s, 3H). |
| **219** | ¹H NMR (400 MHz, DMSO-*d*₆) δ 9.03 (s, 1H), 8.21 (d, *J =* 6.4 Hz, 1H), 8.13 (d, *J = 7.5* Hz, 1H), 7.90 (s, 1H), 7.58 (d, *J* = 10.1 Hz, 2H), 7.52 (d, *J* = 7.5 Hz, 1H), 7.05 (s, 1H), 7.00 (d, *J* = 11.0 Hz, 1H), 6.54 (d, *J* = 7.4 Hz, 1H), 5.00 (t, *J* = 9.8 Hz, 1H), 4.70 (dd, *J* = 10.4, 4.2 Hz, 1H), 3.52 (s, 3H). |
| **224** | ¹H NMR (400 MHz, DMSO-*d*₆) δ 8.97 (s, 1H), 8.25-7.91 (m, 2H), 7.79 (s, 1H), 7.62 (s, 1H), 7.45 (s, 2H), 7.29 (s, 2H), 4.89 (s, 2H), 3.65-3.45(m, 4H), 3.15 (s, 3H), 2.95 (s, 3H). |
| **225** | ¹H NMR (400 MHz, MeOH-*d*₄) δ 9.24 (s, 1H), 8.90 (s, 1H), 8.11-8.07 (m, 1H), 8.05 (d, *J* = 8.4 Hz, 1H), 8.00 (s, 1H), 7.90 (s, 1H), 7.57-7.43 (m, 3H), 5.41-5.34 (m, 1H), 4.10-3.97 (m, 1H), 3.75-3.62 (m, 1H), 2.30-2.20 (m, 1H), 2.20-2.07 (m, 1H), 2.07-1.95 (m, 1H), 1.95-1.82 (m, 1H), 1.42-1.30 (m, 1H), 1.00 (d, *J* = 6.7 Hz, 3H). |
| **226** | ¹H NMR (400 MHz, MeOH-*d*₄) δ 9.40-8.90 (m, 2H), 8.25-7.72 (m, 4H), 7.72-7.45 (m, 1H), 7.39 (s, 1H), 5.65-5.00 (m, 1H), 3.80-3.40 (m, 2H), 2.55-2.27 (m, 3H), 2.27-1.81 (m, 4H), 1.50-1.20 (m, 1H), 1.01 (s, 3H). |
| **227** | ¹H NMR (400 MHz, MeOH-*d*₄) δ 9.29 (s, 1H), 9.06 (s, 1H), 8.23 (d, *J =* 1.7 Hz, 1H), 8.13 (d, *J =* 8.5 Hz, 1H), 8.10 (s, 1H), 7.92 (s, 1H), 7.60-7.49 (m, 3H), 5.78 (s, 1H), 3.88 (s, 1H), 3.44-3.35 (m, 1H), 2.45-2.30 (m, 2H), 2.01-1.85 (m, 2H), 1.54-1.43 (m, 1H), 1.11 (d, *J* = 6.7 Hz, 3H). |
| **228** | ¹H NMR (400 MHz, MeOH-*d*₄) δ 9.40-8.97 (m, 2H), 8.25-7.75 (m, 4H), 7.70-7.35 (m, 2H), 6.20-4.30 (m, 1H), 3.55-2.60 (m, 2H), 2.60-2.38 (m, 3H), 2.38-2.23 (m, 2H), 2.10-1.77 (m, 2H), 1.55-1.37 (m, 1H), 1.35-1.00 (m, 3H). |
| **229** | ¹H NMR (400 MHz, DMSO-*d*₆) δ 8.01 (s, 1H), 7.83-7.69 (m, 2H), 7.47-7.37 (m, 2H), 7.37-7.23 (m, 4H), 7.08 (s, 1H), 6.92 (s, 1H), 6.59-6.47 (m, 1H), 4.88 (t, *J =* 9.8 Hz, 1H), 4.71 (d, *J* = 10.1 Hz, 1H), 3.53 (s, 3H), 2.83 (s, 3H). |
| **230** | ¹H NMR (400 MHz, CDCl₃) δ 8.91-8.85 (m, 1H), 8.58-8.36 (m, 1H), 8.02-7.87 (m, 1H), 7.81-7.71 (m, 1H), 7.71-7.63 (m, 1H), 7.61-7.20 (m, 2H), 5.55 (br s, 2H), 5.10-4.50 (m, 2H), 3.20-2.92 (m, 3H), 2.45-2.37 (m, 3H). |
| **231** | ¹H NMR (400 MHz, MeOH-*d*₄) δ 8.91 (d, *J =* 12.7 Hz, 1H), 8.19-8.13 (m, 0.5H), 8.09-8.00 (m, 0.5H), 8.04 (s, 0.5H), 7.93 (s, 0.5H), 7.78 (d, *J* = 23.7 Hz, 1H), 7.70-7.63 (m, 0.5H), 7.46-7.36 (m, 1.5H), 5.01 (s, 1H), 4.76-4.63 (m, 1H), 3.17 (s, 1H), 3.04 (s, 3H), 2.75 (s, 1H), 2.40 (d, *J =* 6.2 Hz, 3H). |
| **232** | ¹H NMR (400 MHz, CDCl₃) δ 8.90-8.81 (m, 1H), 8.03-7.89 (m, 1H), 7.89-7.69 (m, 2H), 7.64-7.32 (m, 1H), 7.32-7.21 (m, 1H), 6.85-6.72 (m, 2H), 5.38 (br s, 2H), 5.05-4.65 (m, 2H), 3.19-3.05 (m, 3H). |
| **233** | ¹H NMR (400 MHz, DMSO-*d*₆) δ 8.19 (s, 1H), 8.05 (d, *J=* 6.4 Hz, 1H), 7.71 (d, *J* = 7.8 Hz, 1H), 7.41 (s, 1H), 7.34 (d, *J* = 8.0 Hz, 1H), 7.24 (s, 2H), 7.10 (s, 1H), 7.06 (d, *J* = 3.1 Hz, 1H), 6.94 (d, *J* = 10.9 Hz, 1H), 6.89 (s, 1H), 6.78-6.70 (m, 1H), 6.65-6.57(m, 1H), 4.90 (t, *J =* 9.8 Hz, 1H), 4.64 (dd, *J =* 10.5, 4.0 Hz, 1H), 3.48 (s, 3H). |

### Biological Test Evaluation

### I. Test on Inhibition of Proliferation of Human Colon Cancer HCT116 Cells

1. HCT 116 MTAP-knockout cells and HCT 116 wild-type MTAP cells were plated in 96-well flat-bottom plates and cultured overnight at 37 °C and 5% CO₂ in McCoy's 5A medium containing 10% fetal bovine serum + 1% penicillin-streptomycin.
2. The next day, compounds were each dissolved in DMSO, diluted successively with DMSO and medium and then transferred to cell plates; the final concentration of each compound was 10 µM, 4-fold dilution was performed to obtain 9 concentration points, and a DMSO control was provided.
3. Cells not treated with compound were subjected to CellTiter-Glo Luminescent Cell Viability Assay (Promega) to detect the cell viability, and reference was made to the kit instructions for the operation; the plates were then placed on an EnVision Multilabel Reader to detect luminescence signals.
4. Meanwhile, the cell plates containing cells treated with compounds were cultured continuously at 37 °C and 5% CO₂ for 6 days.
5. Then, the cell viability was also detected by CellTiter-Glo.
6. Finally, dose response curves were plotted using the four parameter dose response curve module of GraphPad Prism v 9.2.0 software and proliferation inhibition IC₅₀ (unit: nM) was calculated. The test results are as follows:

### Table 1. Biological test results

| **Example No.** | **IC₅₀ of HCT116 wild-type cells (nM)** | **IC₅₀ of HCT116 MTAP-knockout cells (nM)** | **Selectivity** | **Example No.** | **IC₅₀ of HCT116 wild-type cells (nM)** | **IC₅₀ of HCT116 MTAP-knockout cells (nM)** | **Selectivity** |
|---|---|---|---|---|---|---|---|
| **1** | **1498** | **29** | **51.7** | **205a** | **NT** | **313** | **/** |
| **2** | **207** | **5** | **41.4** | **206a** | **2235** | **41** | **54.5** |
| **3** | **166** | **8** | **20.8** | **207a** | **70** | **2** | **35** |
| **14a** | **2059** | **55** | **37.4** | **208a** | **523** | **5** | **104.6** |
| **22** | **5484** | **154** | **35.6** | **210a** | **59** | **1** | **59** |
| **30** | **725** | **12** | **60.4** | **211a** | **1328** | **19** | **69.9** |
| **30a** | **381** | **4** | **95.3** | **212** | **383** | **8** | **47.9** |
| **34a** | **NT** | **225** | **/** | **213** | **1319** | **15** | **87.9** |
| **34b** | **NT** | **1816** | **/** | **214** | **>10000** | **188** | **>53.1** |
| **144** | **2057** | **22** | **93.5** | **215** | **314** | **8** | **39.3** |
| **145** | **1075** | **24** | **44.8** | **216** | **1081** | **11** | **98.3** |
| **146** | **>10000** | **224** | **>44.6** | **217** | **546** | **41** | **13.3** |
| **147** | **4988** | **115** | **43.4** | **218** | **47** | **2** | **23.5** |
| **148** | **>10000** | **202** | **>49.5** | **219** | **257** | **3** | **85.7** |
| **149** | **7206** | **50** | **144.1** | **224** | **NT** | **323** | **/** |
| **154** | **406** | **22** | **18.5** | **225** | **>10000** | **499** | **20** |
| **156** | **1607** | **10** | **160.7** | **226** | **>10000** | **322** | **>31** |
| **158** | **NT** | **68** | **/** | **227** | **3445** | **12** | **283** |
| **163** | **330** | **6** | **55** | **228** | **5316** | **102** | **52.1** |
| **164** | **706** | **8** | **88.3** | **229** | **934** | **16** | **58.4** |
| **165** | **358** | **11** | **32.5** | **230** | **7572** | **108** | **70.1** |
| **167** | **2711** | **74** | **36.6** | **231** | **>10000** | **174** | **>57.5** |
| **168** | **7912** | **111** | **71.3** | **232** | **NT** | **2237** | **/** |
| **169** | **1877** | **35** | **53.6** | **233** | **564** | **17** | **33.2** |
| **179** | **3721** | **67** | **55.5** | **Comparative compound 1** | **42.7** | **16.3** | **2.6** |
| **181a** | **5103** | **330** | **15.5** | **Comparative compound 2** | **78.3** | **35.5** | **2.2** |
| **181b** | **NT** | **3126** | **/** | **Comparative compound 3** | **96** | **6** | **16** |
| **203** | **NT** | **155** | **/** | **Comparative compound 4** | **85** | **6** | **14.2** |
| **204** | **NT** | **238** | / | | | | |
| | 1. "NT" is an abbreviation of "Not Tested" and means that the detection has not been performed yet. | | | | | | |
| | 2. The comparative compound has the following structure (falling within the scope of claims of WO2022115377A1): | | | | | | |
| Notes | 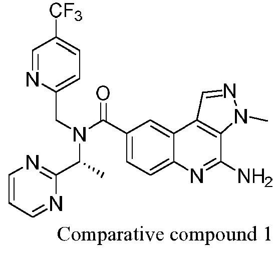 | | | 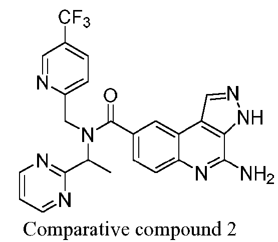 | | | |
| | 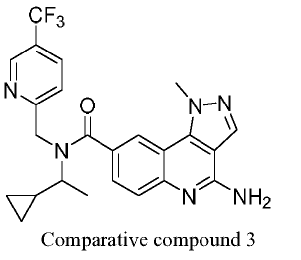 | | | 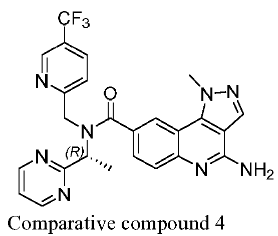 | | | |

From the biological activity data of the compounds of specific examples, the series of compounds of the present invention, at the cellular level, have strong inhibitory effect on the proliferation of human colon cancer HCT116 MTAP-knockout cells and have high selectivity for wild-type cells.

All documents mentioned in the present invention are incorporated as references, just as each document is individually cited as a reference. In addition, it should be understood that various modifications or changes may be made by those skilled in the art after reading the above disclosure of the present invention, and these equivalent forms also fall within the scope defined by the claims appended hereto.

## Claims

1. A compound of formula (I), a stereoisomer or pharmaceutically acceptable salt thereof: 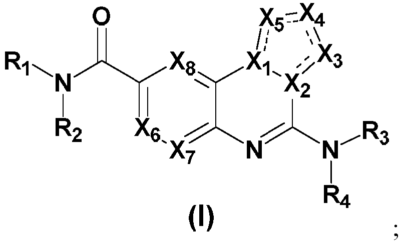
wherein, "- - -" **is a double bond or a single bond;**
X₁ is C or N, and X₂ is C or N, **provided that at least one of X₁ and X₂ is N;**
X₃ is CR₅ or N, X₄ is CR₆ or N, X₅ is CR₇ or N,
X₆ is CR₈ or N, X₇ is CR₉ or N, X₈ is CR₁₀ or N;
**R₁** is -(CR₁₁R₁₂)ₘ-R, or **R₁** and **R₂,** together with a nitrogen atom to which they are directly attached, form a **ring B,** wherein the **ring B** is a 4-10 membered nitrogen-containing heterocyclyl or 5-10 membered nitrogen-containing heteroaryl and optionally further substituted with one or more substituents selected from the group consisting of R, deuterium, halogen, cyano, nitro, azido, C₁₋₁₀ alkyl, C₂₋₁₀ alkenyl, C₂₋₁₀ alkynyl, =O, =S, -C₀₋₈ alkyl-SF₅, -C₀₋₈ alkyl-O-S(O)₂R₁₃, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, -C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(O)NR₁₆R₁₇ and -C₀₋₈ alkyl-N(R₁₆)-C(O)R₁₅;
**R is -ring A-(Ra)n,**
**ring A** is selected from the group consisting of C₃₋₁₂ cycloalkyl, 4-10 membered heterocyclyl, C₆₋₁₀ aryl and 5-10 membered heteroaryl, wherein the C₃₋₁₂ cycloalkyl or 4-10 membered heterocyclyl is optionally further fused to C₆₋₁₀ aryl or 5-10 membered heteroaryl, and the C₆₋₁₀ aryl or 5-10 membered heteroaryl is optionally further fused to 5-10 membered heteroaryl, C₃₋₁₂ cycloalkyl or 4-10 membered heterocyclyl,
**each Rₐ** is independently selected from the group consisting of hydrogen, deuterium, halogen, cyano, nitro, azido, C₁₋₁₀ alkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, -C₀₋₈ alkyl-SF₅, -C₀₋₈ alkyl-O-S(O)₂R₁₃, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, - C₀₋₈ alkyl-S-C(O)R₁₅, -C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(O)NR₁₆R₁₇ and -C₀₋₈ alkyl-N(R₁₆)-C(O)R₁₅, or, when n ≥ 2, two Rₐ on the same carbon, together with a carbon atom to which they are directly attached, form C(O), the above groups are independently optionally more further substituted with one or more substituents selected from the group consisting of deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, =O, =S, -C₀₋₈ alkyl-SF₅, -C₀₋₈ alkyl-O-S(O)₂R₁₃, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, -C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(O)NR₁₆R₁₇ and -C₀₋₈ alkyl-N(R₁₆)-C(O)R₁₅;
**R₂** is selected from the group consisting of hydrogen, deuterium, C₁₋₁₀ alkyl, C₂₋₁₀ alkenyl, C₂₋₁₀ alkynyl, C₃₋₁₂ cycloalkyl, 3-12 membered heterocyclyl, C₆₋₁₀ aryl, 5-10 member heteroaryl, -S(O)ᵣR₁₃, -O-R₁₄, -C(O)OR₁₄, -C(O)R₁₅ and -C(O)NR₁₆R₁₇, wherein the C₃₋₁₂ cycloalkyl or 3-12 membered heterocyclyl is optionally further fused to C₆₋₁₀ aryl or 5-10 membered heteroaryl, and the C₆₋₁₀ aryl or 5-10 membered heteroaryl is optionally further fused to C₃₋₁₂ cycloalkyl or 4-10 membered heterocyclyl, the above groups are independently optionally further substituted with one or more substituents selected from the group consisting of deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, halogen-substituted C₃₋₆ cycloalkyl, halogen-substituted 3-6 membered heterocyclyl, halogen-substituted C₆₋₈ aryl, halogen-substituted 5-8 membered heteroaryl, C₁₋₄ alkyl-substituted C₃₋₆ cycloalkyl, C₁₋₄ alkyl-substituted 3-6 membered heterocyclyl, C₁₋₄ alkyl-substituted C₆₋₈ aryl, C₁₋₄ alkyl-substituted 5-8 membered heteroaryl, (2-(trimethylsilyl)ethoxy)methyl, =O, =S, -C₀₋₈ alkyl-SF₅, -C₀₋₈ alkyl-O-S(O)₂R₁₃, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, - C₀₋₈ alkyl-S-C(O)R₁₅, -C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(O)NR₁₆R₁₇ and -C₀₋₈ alkyl-N(R₁₆)-C(O)R₁₅;
**R₃ and R₄** are each independently selected from the group consisting of hydrogen, deuterium, hydroxy, C₁₋₁₀ alkyl, C₂₋₁₀ alkenyl, C₂₋₁₀ alkynyl, C₃₋₁₂ cycloalkyl and 3-12 membered heterocyclyl, or R₃ and R₄, together with a nitrogen atom to which they are directly attached, form 4-10 membered nitrogen-containing heterocyclyl or 5-10 membered nitrogen-containing heteroaryl, the above groups are independently optionally further substituted with one or more substituents selected from the group consisting of deuterium, halogen, cyano, hydroxy, =O, =S, C₁₋₁₀ alkyl, C₁₋₁₀ haloalkyl, C₁₋₁₀ deuterioalkyl, C₂₋₁₀ alkenyl, C₂₋₁₀ alkynyl, C₁₋₁₀ alkoxy, C₃₋₁₂ cycloalkyl, C₃₋₁₂ cycloalkyloxy, 3-12 membered heterocyclyl, 3-12 membered heterocyclyloxy and -NR₁₆R₁₇;
**R₅, R₆ and R₇** are each independently selected from the group consisting of hydrogen, deuterium, halogen, cyano, nitro, azido, C₁₋₁₀ alkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, -C₀₋₈ alkyl-SF₅, -C₀₋₈ alkyl-O-S(O)₂R₁₃, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, -C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(O)NR₁₆R₁₇ and -C₀₋₈ alkyl-N(R₁₆)-C(O)R₁₅, the above groups are independently optionally more further substituted with one or more substituents selected from the group consisting of deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, =O, =S, -C₀₋₈ alkyl-SF₅, -C₀₋₈ alkyl-O-S(O)₂R₁₃, -C₀₋₈ alkylS(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, - C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(O)NR₁₆R₁₇ and -C₀₋₈ alkyl-N(R₁₆)-C(O)R₁₅;
**R₈, R₉ and R₁₀** are each independently selected from the group consisting of hydrogen, deuterium, halogen, cyano, nitro, azido, C₁₋₁₀ alkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, -C₀₋₈ alkyl-SF₅, -C₀₋₈ alkyl-O-S(O)₂R₁₃, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, -C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(O)NR₁₆R₁₇ and -C₀₋₈ alkyl-N(R₁₆)-C(O)R₁₅, the above groups are independently optionally more further substituted with one or more substituents selected from the group consisting of deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, =O, =S, -C₀₋₈ alkyl-SF₅, -C₀₋₈ alkyl-O-S(O)₂R₁₃, -C₀₋₈ alkylS(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, - C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(O)NR₁₆R₁₇ and -C₀₋₈ alkyl-N(R₁₆)-C(O)R₁₅;
**each R₁₁ and each R₁₂** are independently selected from the group consisting of hydrogen, deuterium, halogen, cyano, nitro, azido, C₁₋₁₀ alkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, -C₀₋₈ alkyl-SF₅, -C₀₋₈ alkyl-O-S(O)₂R₁₃, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, -C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(O)NR₁₆R₁₇ and -C₀₋₈ alkyl-N(R₁₆)-C(O)R₁₅, or R₁₁ and R₁₂, together with a carbon atom to which they are directly attached, form C₃₋₁₂ cycloalkyl, 4-10 membered heterocyclyl, C₆₋₁₀ aryl or 5-10 membered heteroaryl, the above groups are independently optionally more further substituted with one or more substituents selected from the group consisting of deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, =O, =S, -C₀₋₈ alkyl-SF₅, -C₀₋₈ alkyl-O-S(O)₂R₁₃, -C₀₋₈ alkylS(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, - C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(O)NR₁₆R₁₇ and -C₀₋₈ alkyl-N(R₁₆)-C(O)R₁₅;
each R₁₃ is independently selected from the group consisting of hydrogen, deuterium, hydroxy, C₁₋₁₀ alkyl, C₂₋₁₀ alkenyl, C₃₋₁₂ cycloalkyl, 3-12 membered heterocyclyl, C₆₋₁₀ aryl, 5-10 membered heteroaryl and -NR₁₆R₁₇, the above groups are independently optionally further substituted with one or more substituents selected from the group consisting of deuterium, halogen, hydroxy, =O, C₁₋₁₀ alkyl, C₁₋₁₀ alkoxy, C₃₋₁₂ cycloalkyl, C₃₋₁₂ cycloalkyloxy, 3-12 membered heterocyclyl, 3-12 membered heterocyclyloxy, C₆₋₁₀ aryl, C₆₋₁₀ aryloxy, 5-10 membered heteroaryl, 5-10 membered heteroaryloxy and -NR₁₆R₁₇;
each R₁₄ is independently selected from the group consisting of hydrogen, deuterium, C₁₋₁₀ alkyl, C₂₋₁₀ alkenyl, C₃₋₁₂ cycloalkyl, 3-12 membered heterocyclyl, C₆₋₁₀ aryl and 5-10 membered heteroaryl, the above groups are independently optionally further substituted with one or more substituents selected from the group consisting of deuterium, halogen, hydroxy, =O, cyano, C₁₋₁₀ alkyl, C₁₋₁₀ alkoxy, C₃₋₁₂ cycloalkyl, C₃₋₁₂ cycloalkyloxy, 3-12 membered heterocyclyl, 3-12 membered heterocyclyloxy, C₆₋₁₀ aryl, C₆₋₁₀ aryloxy, 5-10 membered heteroaryl, 5-10 membered heteroaryloxy and -NR₁₆R₁₇;
each R₁₅ is independently selected from the group consisting of hydrogen, deuterium, hydroxy, C₁₋₁₀ alkyl, C₁₋₁₀ alkoxy, C₂₋₁₀ alkenyl, C₂₋₁₀ alkynyl, C₃₋₁₂ cycloalkyl, C₃₋₁₂ cycloalkyloxy, 3-12 membered heterocyclyl, 3-12 membered heterocyclyloxy, C₆₋₁₀ aryl, C₆₋₁₀ aryloxy, 5-10 membered heteroaryl, 5-10 membered heteroaryloxy and -NR₁₆R₁₇, the above groups are independently optionally further substituted with one or more substituents selected from the group consisting of deuterium, halogen, hydroxy, =O, cyano, C₁₋₁₀ alkyl, C₁₋₁₀ alkoxy, C₃₋₁₂ cycloalkyl, C₃₋₁₂ cycloalkyloxy, 3-12 membered heterocyclyl, 3-12 membered heterocyclyloxy, C₆₋₁₀ aryl, C₆₋₁₀ aryloxy, 5-10 membered heteroaryl, 5-10 membered heteroaryloxy and -NR₁₆R₁₇;
each R₁₆ and each R₁₇ are independently selected from the group consisting of hydrogen, deuterium, hydroxy, C₁₋₁₀ alkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, sulfinyl, sulfonyl, methylsulfonyl, isopropylsulfonyl, cyclopropylsulfonyl, p-toluenesulfonyl, aminosulfonyl, dimethylaminosulfonyl and C₁₋₁₀ alkanoyl, the above groups are independently optionally further substituted with one or more substituents selected from the group consisting of deuterium, halogen, hydroxy, =O, C₁₋₁₀ alkyl, C₂₋₁₀ alkenyl, C₂₋₁₀ alkynyl, C₁₋₁₀ haloalkyl, C₁₋₁₀ deuterioalkyl, C₁₋₁₀ alkoxy, C₃₋₁₂ cycloalkyl, C₃₋₁₂ cycloalkyloxy, 3-12 membered heterocyclyl, 3-12 membered heterocyclyloxy, C₆₋₁₀ aryl, C₆₋₁₀ aryloxy, 5-10 membered heteroaryl, 5-10 membered heteroaryloxy, amino, mono-C₁₋₁₀ alkyl-substituted amino, di-C₁₋₁₀ alkyl-substituted amino and C₁₋₁₀ alkanoyl, or
R₁₆ and R₁₇, together with a nitrogen atom to which they are directly attached, form 4-10 membered heterocyclyl or 5-10 membered heteroaryl, the 4-10 membered heterocyclyl or 5-10 membered heteroaryl is optionally further substituted with one or more substituents selected from the group consisting of deuterium, halogen, hydroxy, =O, C₁₋₁₀ alkyl, C₂₋₁₀ alkenyl, C₂₋₁₀ alkynyl, C₁₋₁₀ haloalkyl, C₁₋₁₀ deuterioalkyl, C₁₋₁₀ alkoxy, C₃₋₁₂ cycloalkyl, C₃₋₁₂ cycloalkyloxy, 3-12 membered heterocyclyl, 3-12 membered heterocyclyloxy, C₆₋₁₀ aryl, C₆₋₁₀ aryloxy, 5-10 membered heteroaryl, 5-10 membered heteroaryloxy, amino, mono-C₁₋₁₀ alkyl-substituted amino, di-C₁₋₁₀alkyl-substituted amino and C₁₋₁₀ alkanoyl;
**m is 0, 1 or 2;**
**n is 0, 1, 2, 3, 4, 5 or 6; and**
each r is independently 0, 1 or 2.

2. The compound of formula (I), the stereoisomer or pharmaceutically acceptable salt thereof according to claim 1, wherein, **R₃ and R₄** are each independently selected from the group consisting of hydrogen, deuterium, hydroxy, C₁₋₄ alkyl, C₂₋₄ alkenyl, C₂₋₄ alkynyl, C₃₋₆ cycloalkyl and 3-6 membered heterocyclyl, the above groups are independently optionally further substituted with one or more substituents selected from the group consisting of deuterium, halogen, cyano, hydroxy, =O, =S, C₁₋₄ alkyl, C₁₋₄ haloalkyl, C₁₋₄ deuterioalkyl, C₂₋₄ alkenyl, C₂₋₄ alkynyl, C₁₋₄ alkoxy, C₃₋₆ cycloalkyl, C₃₋₆ cycloalkyloxy, 3-6 membered heterocyclyl, 3-6 membered heterocyclyloxy and -NR₁₆R₁₇;
wherein, R₁₆ and R₁₇ are defined as in claim 1;
**preferably, R₃ and R₄** are each independently selected from the group consisting of hydrogen, deuterium, hydroxy, C₁₋₄ alkyl, C₁₋₄ haloalkyl, C₁₋₄ deuterioalkyl, C₂₋₄ alkenyl, C₂₋₄ alkynyl, C₃₋₆ cycloalkyl and 3-6 membered heterocyclyl.

3. The compound of formula (I), the stereoisomer or pharmaceutically acceptable salt thereof according to claim 1, wherein, **R₅, R₆ and R₇** are each independently selected from the group consisting of hydrogen, deuterium, halogen, cyano, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -C₀₋₄ alkyl-SF₅, -C₀₋₄ alkyl-O-S(O)₂R₁₃, -C₀₋₄ alkyl-S(O)ᵣR₁₃, -C₀₋₄ alkyl-O-R₁₄, -C₀₋₄ alkyl-C(O)OR₁₄, -C₀₋₄ alkyl-C(O)SR₁₄, -C₀₋₄ alkyl-S-C(O)R₁₅, -C₀₋₄ alkyl-C(O)R₁₅, -C₀₋₄ alkyl-O-C(O)R₁₅, -C₀₋₄ alkyl-P(O)(R₁₅)₂, -C₀₋₄ alkyl-NR₁₆R₁₇, -C₀₋₄ alkyl-C(O)NR₁₆R₁₇ and -C₀₋₄ alkyl-N(R₁₆)-C(O)R₁₅, the above groups are independently optionally more further substituted with one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -C₀₋₄ alkyl-SF₅, -C₀₋₄ alkyl-O-S(O)₂R₁₃, -C₀₋₄ alkyl-S(O)ᵣR₁₃, - C₀₋₄ alkyl-O-R₁₄, -C₀₋₄ alkyl-C(O)OR₁₄, -C₀₋₄ alkyl-C(O)SR₁₄, -C₀₋₄ alkyl-S-C(O)R₁₅, -C₀₋₄ alkyl-C(O)R₁₅, -C₀₋₄ alkyl-O-C(O)R₁₅, -C₀₋₄ alkyl-P(O)(R₁₅)₂, -C₀₋₄ alkyl-NR₁₆R₁₇, -C₀₋₄ alkyl-C(O)NR₁₆R₁₇ and -C₀₋₄ alkyl-N(R₁₆)-C(O)R₁₅;
wherein, R₁₃, R₁₄, R₁₅, R₁₆, R₁₇ and r are defined as in claim 1;
**preferably, R₅, R₆ and R₇** are each independently selected from the group consisting of hydrogen, deuterium, halogen, cyano, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -SF₅, -O-S(O)₂R₁₃, -S(O)ᵣR₁₃, -OR₁₄, -C(O)OR₁₄, -C(O)SR₁₄, -S-C(O)R₁₅, -C(O)R₁₅, -O-C(O)R₁₅, -P(O)(R₁₅)₂, -NR₁₆R₁₇, - C(O)NR₁₆R₁₇ and -N(R₁₆)-C(O)R₁₅, the above groups are independently optionally more further substituted with one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -SF₅, -O-S(O)₂R₁₃, -S(O)ᵣR₁₃, -O-R₁₄, -C(O)OR₁₄, -C(O)SR₁₄, -S-C(O)R₁₅, -C(O)R₁₅, -O-C(O)R₁₅, - P(O)(R₁₅)₂, -NR₁₆R₁₇, -C(O)NR₁₆R₁₇ and -N(R₁₆)-C(O)R₁₅;
wherein, R₁₃, R₁₄, R₁₅, R₁₆, R₁₇ and r are defined as in claim 1.

4. The compound of formula (I), the stereoisomer or pharmaceutically acceptable salt thereof according to claim 1, wherein, **R₈, R₉ and R₁₀** are each independently selected from the group consisting of hydrogen, deuterium, halogen, cyano, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -C₀₋₄ alkyl-SF₅, -C₀₋₄ alkyl-O-S(O)₂R₁₃, -C₀₋₄ alkyl-S(O)ᵣR₁₃, -C₀₋₄ alkyl-O-R₁₄, -C₀₋₄ alkyl-C(O)OR₁₄, -C₀₋₄ alkyl-C(O)SR₁₄, -C₀₋₄ alkyl-S-C(O)R₁₅, -C₀₋₄ alkyl-C(O)R₁₅, -C₀₋₄ alkyl-O-C(O)R₁₅, -C₀₋₄ alkyl-P(O)(R₁₅)₂, -C₀₋₄ alkyl-NR₁₆R₁₇, -C₀₋₄ alkyl-C(O)NR₁₆R₁₇ and -C₀₋₄ alkyl-N(R₁₆)-C(O)R₁₅, the above groups are independently optionally more further substituted with one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -C₀₋₄ alkyl-SF₅, -C₀₋₄ alkyl-O-S(O)₂R₁₃, -C₀₋₄ alkyl-S(O)ᵣR₁₃, - C₀₋₄ alkyl-O-R₁₄, -C₀₋₄ alkyl-C(O)OR₁₄, -C₀₋₄ alkyl-C(O)SR₁₄, -C₀₋₄ alkyl-S-C(O)R₁₅, -C₀₋₄ alkyl-C(O)R₁₅, -C₀₋₄ alkyl-O-C(O)R₁₅, -C₀₋₄ alkyl-P(O)(R₁₅)₂, -C₀₋₄ alkyl-NR₁₆R₁₇, -C₀₋₄ alkyl-C(O)NR₁₆R₁₇ and -C₀₋₄ alkyl-N(R₁₆)-C(O)R₁₅;
wherein, R₁₃, R₁₄, R₁₅, R₁₆, R₁₇ and r are defined as in claim 1;
**preferably, R₈, R₉ and R₁₀** are each independently selected from the group consisting of hydrogen, deuterium, halogen, cyano, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -SF₅, -O-S(O)₂R₁₃, -S(O)ᵣR₁₃, -OR₁₄, -C(O)OR₁₄, -C(O)SR₁₄, -S-C(O)R₁₅, -C(O)R₁₅, -O-C(O)R₁₅, -P(O)(R₁₅)₂, -NR₁₆R₁₇, - C(O)NR₁₆R₁₇ and -N(R₁₆)-C(O)R₁₅, the above groups are independently optionally more further substituted with one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -SF₅, -O-S(O)₂R₁₃, -S(O)ᵣR₁₃, -O-R₁₄, -C(O)OR₁₄, -C(O)SR₁₄, -S-C(O)R₁₅, -C(O)R₁₅, -O-C(O)R₁₅, - P(O)(R₁₅)₂, -NR₁₆R₁₇, -C(O)NR₁₆R₁₇ and -N(R₁₆)-C(O)R₁₅;
wherein, R₁₃, R₁₄, R₁₅, R₁₆, R₁₇ and r are defined as in claim 1.

5. The compound of formula (I), the stereoisomer or pharmaceutically acceptable salt thereof according to claim 1, wherein, **each R₁₁ and each R₁₂** are independently selected from the group consisting of hydrogen, deuterium, halogen, cyano, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -C₀₋₄ alkyl-SF₅, -C₀₋₄ alkyl-O-S(O)₂R₁₃, -C₀₋₄ alkyl-S(O)ᵣR₁₃, -C₀₋₄ alkyl-O-R₁₄, -C₀₋₄ alkyl-C(O)OR₁₄, -C₀₋₄ alkyl-C(O)SR₁₄, -C₀₋₄ alkyl-S-C(O)R₁₅, -C₀₋₄ alkyl-C(O)R₁₅, -C₀₋₄ alkyl-O-C(O)R₁₅, -C₀₋₄ alkyl-P(O)(R₁₅)₂, -C₀₋₄ alkyl-NR₁₆R₁₇, -C₀₋₄ alkyl-C(O)NR₁₆R₁₇ and -C₀₋₄ alkyl-N(R₁₆)-C(O)R₁₅, or, R₁₁ and R₁₂, together with a carbon atom to which they are directly attached, form C₃₋₆ cycloalkyl, 4-8 membered heterocyclyl, C₆₋₈ aryl or 5-8 membered heteroaryl, the above groups are independently optionally more further substituted with one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -C₀₋₄ alkyl-SF₅, -C₀₋₄ alkyl-O-S(O)₂R₁₃, -C₀₋₄ alkyl-S(O)ᵣR₁₃, -C₀₋₄ alkyl-OR₁₄, -C₀₋₄ alkyl-C(O)OR₁₄, -C₀₋₄ alkyl-C(O)SR₁₄, -C₀₋₄ alkyl-S-C(O)R₁₅, -C₀₋₄ alkyl-C(O)R₁₅, -C₀₋₄ alkyl-O-C(O)R₁₅, -C₀₋₄ alkyl-P(O)(R₁₅)₂, -C₀₋₄ alkyl-NR₁₆R₁₇, -C₀₋₄ alkyl-C(O)NR₁₆R₁₇ and -C₀₋₄ alkyl-N(R₁₆)-C(O)R₁₅;
wherein, R₁₃, R₁₄, R₁₅, R₁₆, R₁₇ and r are defined as in claim 1;
**preferably, each R₁₁ and each R₁₂** are independently selected from the group consisting of hydrogen, deuterium, halogen, cyano, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -SF₅, -O-S(O)₂R₁₃, -S(O)ᵣR₁₃, -OR₁₄, -C(O)OR₁₄, -C(O)SR₁₄, -S-C(O)R₁₅, -C(O)R₁₅, -O-C(O)R₁₅, -P(O)(R₁₅)₂, -NR₁₆R₁₇, - C(O)NR₁₆R₁₇ and -N(R₁₆)-C(O)R₁₅, the above groups are independently optionally more further substituted with one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -SF₅, -O-S(O)₂R₁₃, -S(O)ᵣR₁₃, -O-R₁₄, -C(O)OR₁₄, -C(O)SR₁₄, -S-C(O)R₁₅, -C(O)R₁₅, -O-C(O)R₁₅, - P(O)(R₁₅)₂, -NR₁₆R₁₇, -C(O)NR₁₆R₁₇ and -N(R₁₆)-C(O)R₁₅;
wherein, R₁₃, R₁₄, R₁₅, R₁₆, R₁₇ and r are defined as in claim 1.

6. The compound of formula (I), the stereoisomer or pharmaceutically acceptable salt thereof according to claim 1, wherein, **R₁** is -(CR₁₁R₁₂)ₘ-R, **or R₁** and **R₂,** together with a nitrogen atom to which they are directly attached, form a **ring B,** the ring B is 4-10 membered nitrogen-containing heterocyclyl or 5-10 membered nitrogen-containing heteroaryl and optionally further substituted with one or more substituents selected from the group consisting of R, deuterium, halogen, cyano, C₁₋₄ alkyl, C₂₋₄ alkenyl, C₂₋₄ alkynyl, =O, =S, -C₀₋₄ alkyl-SF₅, -C₀₋₄ alkyl-O-S(O)₂R₁₃, -C₀₋₄ alkyl-S(O)ᵣR₁₃, -C₀₋₄ alkyl-O-R₁₄, -C₀₋₄ alkyl-C(O)OR₁₄, -C₀₋₄ alkyl-C(O)SR₁₄, - C₀₋₄ alkyl-S-C(O)R₁₅, -C₀₋₄ alkyl-C(O)R₁₅, -C₀₋₄ alkyl-O-C(O)R₁₅, -C₀₋₄ alkyl-P(O)(R₁₅)₂, -C₀₋₄ alkyl-NR₁₆R₁₇, -C₀₋₄ alkyl-C(O)NR₁₆R₁₇ and -C₀₋₄ alkyl-N(R₁₆)-C(O)R₁₅;
**R is -ring A-(Rₐ)ₙ, ring A is selected from** the group consisting of C₃₋₆ cycloalkyl, 4-8 membered heterocyclyl, C₆₋₈ aryl and 5-8 membered heteroaryl, wherein the C₃₋₆ cycloalkyl or 4-8 membered heterocyclyl is optionally further fused to C₆₋₈ aryl or 5-8 membered heteroaryl, and the C₆₋₈ aryl or 5-8 membered heteroaryl is optionally fused to 5-8 membered heteroaryl, C₃₋₆ cycloalkyl or 4-8 membered heterocyclyl,
**each Rₐ** is independently selected from the group consisting of hydrogen, deuterium, halogen, cyano, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -C₀₋₄ alkyl-SF₅, -C₀₋₄ alkyl-O-S(O)₂R₁₃, -C₀₋₄ alkyl-S(O)ᵣR₁₃, -C₀₋₄ alkyl-O-R₁₄, -C₀₋₄ alkyl-C(O)OR₁₄, -C₀₋₄ alkyl-C(O)SR₁₄, -C₀₋₄ alkyl-S-C(O)R₁₅, -C₀₋₄ alkyl-C(O)R₁₅, -C₀₋₄ alkyl-O-C(O)R₁₅, -C₀₋₄ alkyl-P(O)(R₁₅)₂, -C₀₋₄ alkyl-NR₁₆R₁₇, -C₀₋₄ alkyl-C(O)NR₁₆R₁₇ and -C₀₋₄ alkyl-N(R₁₆)-C(O)R₁₅, the above groups are independently optionally more further substituted with one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -C₀₋₄ alkyl-SF₅, -C₀₋₄ alkyl-O-S(O)₂R₁₃, -C₀₋₄ alkyl-S(O)ᵣR₁₃, -C₀₋₄ alkyl-O-R₁₄, -C₀₋₄ alkyl-C(O)OR₁₄, -C₀₋₄ alkyl-C(O)SR₁₄, -C₀₋₄ alkyl-S-C(O)R₁₅, -C₀₋₄ alkyl-C(O)R₁₅, -C₀₋₄ alkyl-O-C(O)R₁₅, -C₀₋₄ alkyl-P(O)(R₁₅)₂, -C₀₋₄ alkyl-NR₁₆R₁₇, -C₀₋₄ alkyl-C(O)NR₁₆R₁₇ and -C₀₋₄ alkyl-N(R₁₆)-C(O)R₁₅;
**R₂** is selected from the group consisting of hydrogen, deuterium, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, - S(O)ᵣR₁₃, -O-R₁₄, -C(O)OR₁₄, -C(O)R₁₅ and -C(O)NR₁₆R₁₇, the above groups are independently optionally further substituted with one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, halogen-substituted C₃₋₆ cycloalkyl, halogen-substituted 3-6 membered heterocyclyl, halogen-substituted C₆₋₈ aryl, halogen-substituted 5-8 membered heteroaryl, C₁₋₄ alkyl-substituted C₃₋₆ cycloalkyl, C₁₋₄ alkyl-substituted 3-6 membered heterocyclyl, C₁₋₄ alkyl-substituted C₆₋₈ aryl, C₁₋₄ alkyl-substituted 5-8 membered heteroaryl, (2-(trimethylsilyl)ethoxy)methyl, =O, =S, -C₀₋₄ alkyl-SF₅, - C₀₋₄ alkyl-O-S(O)₂R₁₃, -C₀₋₄ alkyl-S(O)ᵣR₁₃, -C₀₋₄ alkyl-O-R₁₄, -C₀₋₄ alkyl-C(O)OR₁₄, -C₀₋₄ alkyl-C(O)SR₁₄, -C₀₋₄ alkyl-S-C(O)R₁₅, -C₀₋₄ alkyl-C(O)R₁₅, -C₀₋₄ alkyl-O-C(O)R₁₅, -C₀₋₄ alkyl-P(O)(R₁₅)₂, -C₀₋₄ alkyl-NR₁₆R₁₇, -C₀₋₄ alkyl-C(O)NR₁₆R₁₇ and -C₀₋₄ alkyl-N(R₁₆)-C(O)R₁₅;
wherein, R₁₁, R₁₂, R₁₃, R₁₄, R₁₅, R₁₆, R₁₇, m, n and r are defined as in claim 1.

7. The compound of formula (I), the stereoisomer or pharmaceutically acceptable salt thereof according to claim 1, wherein, each R₁₃ is independently selected from the group consisting of hydrogen, deuterium, hydroxy, C₁₋₄ alkyl, C₂₋₄ alkenyl, C₃₋₆ cycloalkyl, 3-6 membered heterocyclyl, C₆₋₈ aryl, 5-8 membered heteroaryl and -NR₁₆R₁₇, the above groups are independently optionally further substituted with one or more substituents selected from the group consisting of deuterium, halogen, hydroxy, =O, C₁₋₄ alkyl, C₁₋₄ alkoxy, C₃₋₆ cycloalkyl, C₃₋₆ cycloalkyloxy, 3-6 membered heterocyclyl, 3-6 membered heterocyclyloxy, C₆₋₈ aryl, C₆₋₈ aryloxy, 5-8 membered heteroaryl, 5-8 membered heteroaryloxy and -NR₁₆R₁₇;
each R₁₄ is independently selected from the group consisting of hydrogen, deuterium, C₁₋₄ alkyl, C₂₋₄ alkenyl, C₃₋₆ cycloalkyl, 3-6 membered heterocyclyl, C₆₋₈ aryl and 5-8 membered heteroaryl, the above groups are independently optionally further substituted with one or more substituents selected from the group consisting of deuterium, halogen, hydroxy, =O, cyano, C₁₋₄ alkyl, C₁₋₄ alkoxy, C₃₋₆ cycloalkyl, C₃₋₆ cycloalkyloxy, 3-6 membered heterocyclyl, 3-6 membered heterocyclyloxy, C₆₋₈ aryl, C₆₋₈ aryloxy, 5-8 membered heteroaryl, 5-8 membered heteroaryloxy and -NR₁₆R₁₇;
each R₁₅ is independently selected from the group consisting of hydrogen, deuterium, hydroxy, C₁₋₄ alkyl, C₁₋₄ alkoxy, C₂₋₄ alkenyl, C₂₋₄ alkynyl, C₃₋₆ cycloalkyl, C₃₋₆ cycloalkyloxy, 3-6 membered heterocyclyl, 3-6 membered heterocyclyloxy, C₆₋₈ aryl, C₆₋₈ aryloxy, 5-8 membered heteroaryl, 5-8 membered heteroaryloxy and -NR₁₆R₁₇, the above groups are independently optionally further substituted with one or more substituents selected from the group consisting of deuterium, halogen, hydroxy, =O, cyano, C₁₋₄ alkyl, C₁₋₄ alkoxy, C₃₋₆ cycloalkyl, C₃₋₆ cycloalkyloxy, 3-6 membered heterocyclyl, 3-6 membered heterocyclyloxy, C₆₋₈ aryl, C₆₋₈ aryloxy, 5-8 membered heteroaryl, 5-8 membered heteroaryloxy and -NR₁₆R₁₇;
each R₁₆ and each R₁₇ are independently selected from the group consisting of hydrogen, deuterium, hydroxy, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, sulfinyl, sulfonyl, methylsulfonyl, isopropylsulfonyl, cyclopropylsulfonyl, p-toluenesulfonyl, aminosulfonyl, dimethylaminosulfonyl and C₁₋₄ alkanoyl, the above groups are independently optionally further substituted with one or more substituents selected from the group consisting of deuterium, halogen, hydroxy, =O, C₁₋₄ alkyl, C₂₋₄ alkenyl, C₂₋₄ alkynyl, C₁₋₄ haloalkyl, C₁₋₄ deuterioalkyl, C₁₋₄ alkoxy, C₃₋₆ cycloalkyl, C₃₋₆ cycloalkyloxy, 3-6 membered heterocyclyl, 3-6 membered heterocyclyloxy, C₆₋₈ aryl, C₆₋₈ aryloxy, 5-8 membered heteroaryl, 5-8 membered heteroaryloxy, amino, mono-C₁₋₄ alkyl-substituted amino, di-C₁₋₄ alkyl-substituted amino and C₁₋₄ alkanoyl, or
R₁₆ and R₁₇, together with a nitrogen atom to which they are directly attached, form 4-8 membered heterocyclyl or 5-8 membered heteroaryl, the 4-8 membered heterocyclyl or 5-8 membered heteroaryl is optionally further substituted with one or more substituents selected from the group consisting of deuterium, halogen, hydroxy, =O, C₁₋₄ alkyl, C₂₋₄ alkenyl, C₂₋₄ alkynyl, C₁₋₄ haloalkyl, C₁₋₄ deuterioalkyl, C₁₋₄ alkoxy, C₃₋₆ cycloalkyl, C₃₋₆ cycloalkyloxy, 3-6 membered heterocyclyl, 3-6 membered heterocyclyloxy, C₆₋₈ aryl, C₆₋₈ aryloxy, 5-8 membered heteroaryl, 5-8 membered heteroaryloxy, amino, mono-C₁₋₄ alkyl-substituted amino, di-C₁₋₄ alkyl-substituted amino and C₁₋₄ alkanoyl.

8. The compound of formula (I), the stereoisomer or pharmaceutically acceptable salt thereof according to claim 1, wherein the compound of formula (I) is a compound having formula (II): 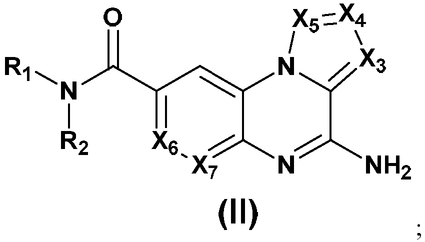 wherein, X₃ is CR₅ or N, X₄ is CR₆ or N, X₅ is CR₇ or N, X₆ is CR₈ or N, X₇ is CR₉ or N;
**R₁** is -(CR₁₁R₁₂)ₘ-R, or, **R₁** and **R₂,** together with a nitrogen atom to which they are directly attached, form a ring B, wherein the ring B is 4-10 membered nitrogen-containing heterocyclyl or 5-10 membered nitrogen-containing heteroaryl and optionally further substituted with one or more substituents selected from the group consisting of R, deuterium, halogen, cyano, C₁₋₄ alkyl, C₂₋₄ alkenyl, C₂₋₄ alkynyl, =O, =S, -SF₅, -O-S(O)₂R₁₃, -S(O)ᵣR₁₃, -O-R₁₄, -C(O)OR₁₄, -C(O)SR₁₄, -S-C(O)R₁₅, -C(O)R₁₅, -O-C(O)R₁₅, -P(O)(R₁₅)₂, -NR₁₆R₁₇, -C(O)NR₁₆R₁₇ and -N(R₁₆)-C(O)R₁₅;
**R** is 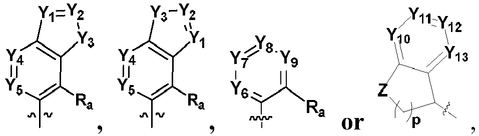
wherein, Y₁, Y₂, Y₄, Y₅, Y₆, Y₇, Y₈, Y₉, Y₁₀, Y₁₁, Y₁₂ and Y₁₃ are each independently CRₐ or N; Y₃ is -O-, -S- or -NRₐ₃-; Z is -O-, -S-, -CRₐ₁Rₐ₂-, -CRₐ₁Rₐ₂-O- or -NRₐ₃-; p is 0, 1, 2 or 3;
**each Rₐ** is independently selected from the group consisting of hydrogen, deuterium, halogen, cyano, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -SF₅, -O-S(O)₂R₁₃, -S(O)ᵣR₁₃, -O-R₁₄, -C(O)OR₁₄, -C(O)SR₁₄, -S-C(O)R₁₅, -C(O)R₁₅, -O-C(O)R₁₅, -P(O)(R₁₅)₂, -NR₁₆R₁₇, -C(O)NR₁₆R₁₇ and -N(R₁₆)-C(O)R₁₅, the above groups are independently optionally more further substituted with one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -SF₅, -O-S(O)₂R₁₃, -S(O)ᵣR₁₃, -O-R₁₄, -C(O)OR₁₄, -C(O)SR₁₄, -S-C(O)R₁₅, -C(O)R₁₅, -O-C(O)R₁₅, -P(O)(R₁₅)₂, -NR₁₆R₁₇, - C(O)NR₁₆R₁₇ and -N(R₁₆)-C(O)R₁₅;
**each Rₐ₁** and **each Rₐ₂** are independently selected from the group consisting of hydrogen, deuterium, halogen, cyano, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -SF₅, -O-S(O)₂R₁₃, -S(O)ᵣR₁₃, -O-R₁₄, - C(O)OR₁₄, -C(O)SR₁₄, -S-C(O)R₁₅, -C(O)R₁₅, -O-C(O)R₁₅, -P(O)(R₁₅)₂, -NR₁₆R₁₇, -C(O)NR₁₆R₁₇ and -N(R₁₆)-C(O)R₁₅, the above groups are independently optionally more further substituted with one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -SF₅, -O-S(O)₂R₁₃, -S(O)ᵣR₁₃, -O-R₁₄, -C(O)OR₁₄, -C(O)SR₁₄, -S-C(O)R₁₅, -C(O)R₁₅, -O-C(O)R₁₅, -P(O)(R₁₅)₂, -NR₁₆R₁₇, - C(O)NR₁₆R₁₇ and -N(R₁₆)-C(O)R₁₅;
**each Rₐ₃** is independently selected from the group consisting of hydrogen, deuterium, C₁₋₄ alkyl, C₃₋₆ cycloalkyl, 3-6 membered heterocyclyl, C₆₋₈ aryl, 5-8 membered heteroaryl, -SF₅, - C(O)OR₁₄, -C(O)SR₁₄, -C(O)R₁₅ and -C(O)NR₁₆R₁₇, the above groups are independently optionally more further substituted with one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -SF₅, -O-S(O)₂R₁₃, -S(O)ᵣR₁₃, -O-R₁₄, -C(O)OR₁₄, -C(O)SR₁₄, -S-C(O)R₁₅, -C(O)R₁₅, -O-C(O)R₁₅, -P(O)(R₁₅)₂, -NR₁₆R₁₇, -C(O)NR₁₆R₁₇ and -N(R₁₆)-C(O)R₁₅;
**each R₂** is independently selected from the group consisting of hydrogen, deuterium, C₁₋₄ alkyl, C₃₋₆ cycloalkyl, 3-6 membered heterocyclyl, C₆₋₈ aryl and 5-8 membered heteroaryl, the above groups are independently optionally further substituted with one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, halogen-substituted C₃₋₆ cycloalkyl, halogen-substituted 3-6 membered heterocyclyl, halogen-substituted C₆₋₈ aryl, halogen-substituted 5-8 membered heteroaryl, C₁₋₄ alkyl-substituted C₃₋₆ cycloalkyl, C₁₋₄ alkyl-substituted 3-6 membered heterocyclyl, C₁₋₄ alkyl-substituted C₆₋₈ aryl, C₁₋₄ alkyl-substituted 5-8 membered heteroaryl, (2-(trimethylsilyl)ethoxy)methyl, =O, =S, -SF₅, -O-S(O)₂R₁₃, -S(O)ᵣR₁₃, -O-R₁₄, -C(O)OR₁₄, - C(O)SR₁₄, -S-C(O)R₁₅, -C(O)R₁₅, -O-C(O)R₁₅, -P(O)(R₁₅)₂, -NR₁₆R₁₇, -C(O)NR₁₆R₁₇ and - N(R₁₆)-C(O)R₁₅;
**R₅**, **R₆ and R₇** are each independently selected from the group consisting of hydrogen, deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -SF₅, -O-S(O)₂R₁₃, -S(O)ᵣR₁₃, -O-R₁₄, -C(O)OR₁₄, -C(O)SR₁₄, -S-C(O)R₁₅, -C(O)R₁₅, -O-C(O)R₁₅, - P(O)(R₁₅)₂, -NR₁₆R₁₇, -C(O)NR₁₆R₁₇ and -N(R₁₆)-C(O)R₁₅;
**R₈ and R₉** are each independently selected from the group consisting of hydrogen, deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -SF₅, -O-S(O)₂R₁₃, -S(O)ᵣR₁₃, -O-R₁₄, -C(O)OR₁₄, -C(O)SR₁₄, -S-C(O)R₁₅, -C(O)R₁₅, -O-C(O)R₁₅, - P(O)(R₁₅)₂, -NR₁₆R₁₇, -C(O)NR₁₆R₁₇ and -N(R₁₆)-C(O)R₁₅;
**each R₁₁ and each R₁₂** are independently selected from the group consisting of hydrogen, deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -SF₅, -O-S(O)₂R₁₃, -S(O)ᵣR₁₃, -O-R₁₄, -C(O)OR₁₄, -C(O)SR₁₄, -S-C(O)R₁₅, -C(O)R₁₅, -O-C(O)R₁₅, - P(O)(R₁₅)₂, -NR₁₆R₁₇, -C(O)NR₁₆R₁₇ and -N(R₁₆)-C(O)R₁₅;
wherein, R₁₃, R₁₄, R₁₅, R₁₆, R₁₇, r and m are defined as in claim 1.

9. The compound of formula (I), the stereoisomer or pharmaceutically acceptable salt thereof according to claim 1, wherein the compound of formula (I) is a compound having formula (III₁) or formula (III₂): 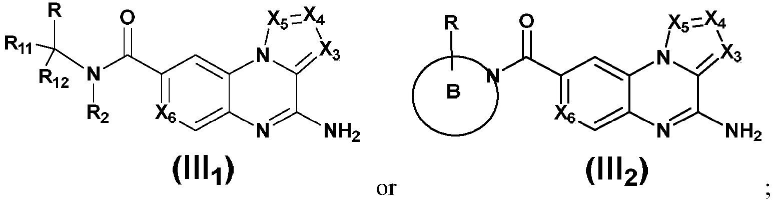
wherein, each X₃ is independently CR₅ or N, each X₄ is independently CR₆ or N, each X₅ is independently CR₇ or N, each X₆ is independently CR₈ or N;
in formula (III₂), ring B is 4-10 membered nitrogen-containing heterocyclyl, the 4-10 membered nitrogen-containing heterocyclyl is optionally further substituted with one or more substituents selected from the group consisting of hydrogen, deuterium, halogen, cyano, hydroxy, carboxyl, C₁₋₄ alkyl, C₁₋₄ haloalkyl, C₁₋₄ deuterioalkyl, C₁₋₄ alkoxy, C₂₋₄ alkenyl, C₂₋₄ alkynyl, C₃₋₆ cycloalkyl, 3-6 membered heterocyclyl, -SF₅ and -NR₁₆R₁₇;
**each R** is independently 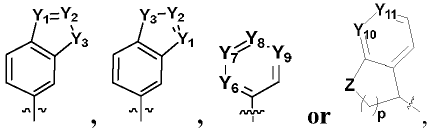
wherein, Y₁, Y₂, Y₆, Y₇, Y₈, Y₉, Y₁₀ and Y₁₁ are each independently CRₐ or N; Y₃ is -O- or - S-; Z is -O-, -S-, -CH₂- or -CH₂-O-; p is 0, 1, 2 or 3,
**each Rₐ** is independently selected from the group consisting of hydrogen, deuterium, halogen, cyano, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -SF₅, -O-R₁₄, -C(O)OR₁₄, -C(O)SR₁₄, -C(O)R₁₅, -O-C(O)R₁₅ and -NR₁₆R₁₇, the above groups are independently optionally more further substituted with one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -SF₅, -O-R₁₄, -C(O)OR₁₄, -C(O)R₁₅, - O-C(O)R₁₅ and -NR₁₆R₁₇;
**R₂** is selected from the group consisting of hydrogen, deuterium, C₁₋₄ alkyl, C₃₋₆ cycloalkyl, 3-6 membered heterocyclyl, C₆₋₈ aryl and 5-8 membered heteroaryl, the above groups are independently optionally further substituted with one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, halogen-substituted C₃₋₆ cycloalkyl, halogen-substituted 3-6 membered heterocyclyl, halogen-substituted C₆₋₈ aryl, halogen-substituted 5-8 membered heteroaryl, C₁₋₄ alkyl-substituted C₃₋₆ cycloalkyl, C₁₋₄ alkyl-substituted 3-6 membered heterocyclyl, C₁₋₄ alkyl-substituted C₆₋₈ aryl, C₁₋₄ alkyl-substituted 5-8 membered heteroaryl, (2-(trimethylsilyl)ethoxy)methyl, =O, =S, -SF₅, -OR₁₄, -C(O)OR₁₄, -C(O)R₁₅, -O-C(O)R₁₅ and -NR₁₆R₁₇;
**each R₅**, **each R₆ and each R₇** are independently selected from the group consisting of hydrogen, deuterium, halogen, cyano, hydroxy, carboxyl, C₁₋₄ alkyl, C₁₋₄ alkoxy, C₁₋₄ haloalkyl, C₁₋₄ haloalkoxy, C₁₋₄ deuterioalkyl, C₁₋₄ deuterioalkoxy, C₂₋₄ alkenyl, C₂₋₄ alkynyl, C₃₋₆ cycloalkyl, C₃₋₆ cycloalkyloxy, 3-6 membered heterocyclyl, 3-6 membered heterocyclyloxy, C₆₋₈ aryl, C₆₋₈ aryloxy, 5-8 membered heteroaryl, 5-8 membered heteroaryloxy, -SF₅ and -NR₁₆R₁₇;
**each R₈** is independently selected from the group consisting of hydrogen, deuterium, halogen, cyano, hydroxy, carboxyl, C₁₋₄ alkyl, C₁₋₄ alkoxy, C₁₋₄ haloalkyl, C₁₋₄ haloalkoxy, C₁₋₄ deuterioalkyl, C₁₋₄ deuterioalkoxy, C₂₋₄ alkenyl, C₂₋₄ alkynyl, C₃₋₆ cycloalkyl, C₃₋₆ cycloalkyloxy, 3-6 membered heterocyclyl, 3-6 membered heterocyclyloxy, C₆₋₈ aryl, C₆₋₈ aryloxy, 5-8 membered heteroaryl, 5-8 membered heteroaryloxy, -SF₅ and -NR₁₆R₁₇;
**each R₁₁ and each R₁₂** are independently selected from the group consisting of hydrogen, deuterium, halogen, cyano, hydroxy, carboxyl, C₁₋₄ alkyl, C₁₋₄ alkoxy, C₁₋₄ haloalkyl, C₁₋₄ haloalkoxy, C₁₋₄ deuterioalkyl, C₁₋₄ deuterioalkoxy, C₂₋₄ alkenyl, C₂₋₄ alkynyl, C₃₋₆ cycloalkyl, C₃₋₆ cycloalkyloxy, 3-6 membered heterocyclyl, 3-6 membered heterocyclyloxy, C₆₋₈ aryl, C₆₋₈ aryloxy, 5-8 membered heteroaryl, 5-8 membered heteroaryloxy, -SF₅ and -NR₁₆R₁₇;
wherein, R₁₄, R₁₅, R₁₆ and R₁₇ are defined as in claim 1.

10. The compound of formula (I), the stereoisomer or pharmaceutically acceptable salt thereof according to claim 9, wherein 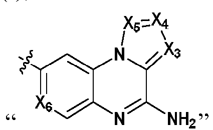 is selected from the group consisting of the following structures: 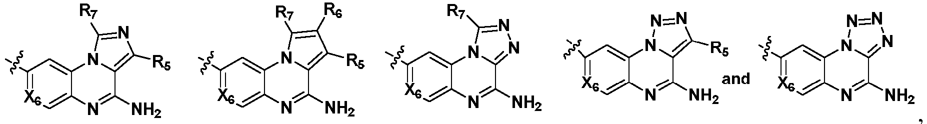
wherein, **each X₆** is independently CR₈ or N;
**each R₅**, **each R₆ and each R₇** are independently selected from the group consisting of hydrogen, deuterium, halogen, cyano, hydroxy, carboxyl, C₁₋₄ alkyl, C₁₋₄ alkoxy, C₁₋₄ haloalkyl, C₁₋₄ haloalkoxy, C₁₋₄ deuterioalkyl, C₁₋₄ deuterioalkoxy, C₂₋₄ alkenyl, C₂₋₄ alkynyl, C₃₋₆ cycloalkyl, C₃₋₆ cycloalkyloxy, 3-6 membered heterocyclyl, 3-6 membered heterocyclyloxy, C₆₋₈ aryl, C₆₋₈ aryloxy, 5-8 membered heteroaryl, 5-8 membered heteroaryloxy, -SF₅, amino, mono-C₁₋₄ alkyl-substituted amino and di-C₁₋₄ alkyl-substituted amino;
**each R₈** is independently selected from the group consisting of hydrogen, deuterium, halogen, cyano, hydroxy, carboxyl, C₁₋₄ alkyl, C₁₋₄ alkoxy, C₁₋₄ haloalkyl, C₁₋₄ haloalkoxy, C₁₋₄ deuterioalkyl, C₁₋₄ deuterioalkoxy, C₂₋₄ alkenyl, C₂₋₄ alkynyl, C₃₋₆ cycloalkyl, C₃₋₆ cycloalkyloxy, 3-6 membered heterocyclyl, 3-6 membered heterocyclyloxy, C₆₋₈ aryl, C₆₋₈ aryloxy, 5-8 membered heteroaryl, 5-8 membered heteroaryloxy, -SF₅, amino, mono-C₁₋₄ alkyl-substituted amino and di-C₁₋₄ alkyl-substituted amino.

11. The compound of formula (I), the stereoisomer or pharmaceutically acceptable salt thereof according to claim 1, wherein the compound of formula (I) is a compound having formula (IV₁₋₁), (IV₁₋₂), (IV₂₋₁) or (IV₂₋₂): 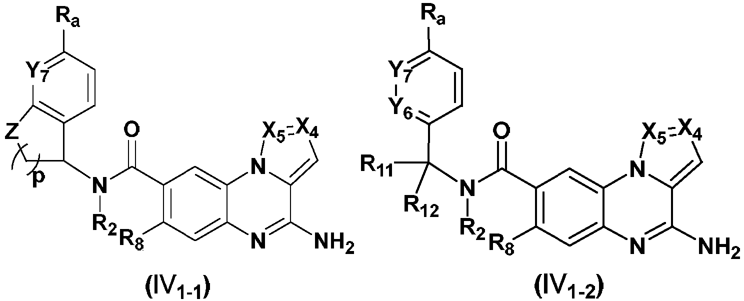 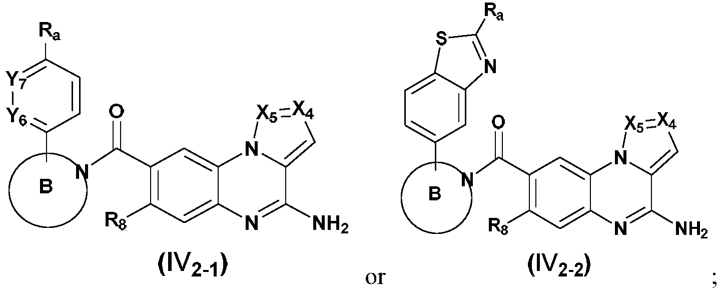
**wherein,** each X₄ is independently CR₆ or N; each X₅ is independently CR₇ or N;
each Y₆ and each Y₇ are independently CH or N; Z is O or CH₂; p is 1 or 2;
in formula (IV₂₋₁) or (IV₂₋₂), ring B, together with a substituent thereon, is selected from the following structures: 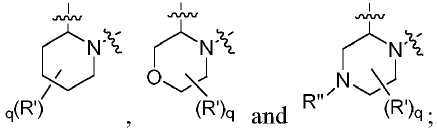 q is 0, 1 or 2;
**each R'** is independently selected from the group consisting of hydrogen, deuterium, fluoro, chloro, bromo, cyano, hydroxy, methyl, ethyl, n-propyl, isopropyl, trifluoromethyl, trideuteriomethyl, methoxy, ethoxy, cyclopropyl and -SF₅;
**each R"** is independently selected from the group consisting of hydrogen, deuterium, hydroxy, methyl, ethyl, *n*-propyl, isopropyl, trifluoromethyl, trideuteriomethyl and cyclopropyl;
**each R₂** is independently selected from the group consisting of hydrogen, deuterium, methyl, ethyl, n-propyl, isopropyl, *n*-butyl, isobutyl, cyclopropyl, cyclobutyl, bicyclo[1.1.1]pentyl, cyclohexyl, oxacyclobutyl, azacyclobutyl, tetrahydrofuryl, phenyl, pyrazolyl, imidazolyl, triazolyl, oxazolyl, thiazolyl, pyridyl, pyridazinyl, pyrimidinyl and triazinyl, the above groups are independently optionally further substituted with one or more substituents selected from the group consisting of deuterium, fluoro, chloro, bromo, cyano, methyl, ethyl, *n*-propyl, isopropyl, *n*-butyl, isobutyl, trifluoromethyl, difluoromethyl, trideuteriomethyl, dideuteriomethyl, vinyl, ethynyl, cyclopropyl, cyclobutyl, cyclopentyl, bicyclo[1.1.1]pentyl, cyclohexyl, oxacyclobutyl, azacyclopentyl, tetrahydrofuryl, phenyl, pyrazolyl, 2-(trimethylsilyl)ethoxy, methyl-substituted pyrazolyl, imidazolyl, triazolyl, oxazolyl, thiazolyl, pyridyl, fluoro-substituted pyridyl, pyridazinyl, pyrimidinyl, triazinyl, =O, =S, -SF₅, hydroxy, methoxy, ethoxy, cyclopropyloxy, cyclobutyloxy, amino, methylamino and dimethylamino;
**each Rₐ** is independently selected from the group consisting of hydrogen, deuterium, fluoro, chloro, bromo, cyano, methyl, ethyl, n-propyl, isopropyl, n-butyl, isobutyl, methoxy, ethoxy, trifluoromethyl, difluoromethyl, trideuteriomethyl, dideuteriomethyl, trifluoromethoxy, trideuteriomethoxy, vinyl, ethynyl, cyclopropyl, cyclobutyl, methyl-substituted cyclopropyl, methyl-substituted cyclobutyl, cyclopropyloxy, cyclobutyloxy, oxacyclobutyl, azacyclobutyl, tetrahydropyrrolyl, piperidinyl, methyl-substituted piperidinyl, phenyl, pyrazolyl, imidazolyl, triazolyl, oxazolyl, thiazolyl, pyrimidinyl, methyl-substituted pyrazolyl, -SF₅, hydroxy, amino, methylamino, dimethylamino, dimethylaminomethyl and dimethylaminoethyl;
**each R₆** is independently selected from the group consisting of hydrogen, deuterium, fluoro, chloro, bromo, cyano, hydroxy, methyl, ethyl, n-propyl, isopropyl, methoxy, trifluoromethyl, trifluoromethoxy, trideuteriomethyl, trideuteriomethoxy and cyclopropyl;
**each R₇** is independently selected from the group consisting of hydrogen, deuterium, fluoro, chloro, bromo, cyano, hydroxy, methyl, ethyl, n-propyl, isopropyl, methoxy, trifluoromethyl, trifluoromethoxy, trideuteriomethyl, trideuteriomethoxy and cyclopropyl;
**each R₈** is independently selected from the group consisting of hydrogen, deuterium, fluoro, chloro, bromo, cyano, hydroxy, methyl, ethyl, n-propyl, isopropyl, methoxy, trifluoromethyl, trifluoromethoxy, trideuteriomethyl, trideuteriomethoxy and cyclopropyl;
**R₁₁ and R₁₂** are each independently selected from the group consisting of hydrogen, deuterium, fluoro, chloro, bromo, cyano, methyl, trifluoromethyl, trideuteriomethyl and cyclopropyl.

12. The compound of formula (I), the stereoisomer or pharmaceutically acceptable salt thereof according to claim 11, wherein 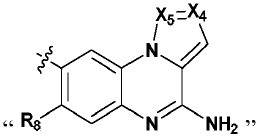 is 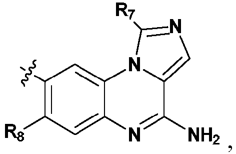 wherein,
**R₇** is selected from the group consisting of hydrogen, deuterium, fluoro, chloro, methyl, trifluoromethyl, trideuteriomethyl and cyclopropyl;
**R₈** is selected from the group consisting of hydrogen, deuterium, fluoro, chloro, bromo, cyano, methyl, trifluoromethyl, trideuteriomethyl and cyclopropyl.

13. The compound of formula (I), the stereoisomer or pharmaceutically acceptable salt thereof according to claim 11, wherein, 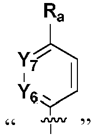 in the compound structures shown as formulas (IV₁₋₂) and (IV₂₋₁) is selected from the group consisting of 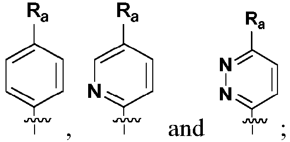 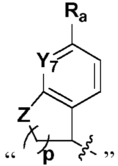 in the compound structure shown as formula (IV₁₋₁) is selected from the group consisting of 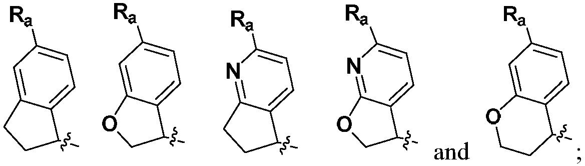 **each Rₐ** is independently selected from the group consisting of hydrogen, deuterium, fluoro, chloro, bromo, cyano, methyl, isopropyl, methoxy, trifluoromethyl, trideuteriomethyl, trifluoromethoxy, trideuteriomethoxy, cyclopropyl, pyrazolyl, methyl-substituted pyrazolyl and - SF₅.

14. The compound of formula (I), the stereoisomer or pharmaceutically acceptable salt thereof according to claim 11, wherein, **each R₂** is independently selected from the group consisting of hydrogen, deuterium, methyl, ethyl, n-propyl, isopropyl, cyclopropyl, cyclobutyl, bicyclo[1.1.1]pentyl, cyclohexyl, oxacyclobutyl, azacyclobutyl, tetrahydrofuryl, pyrazolyl, imidazolyl, thiazolyl, pyridyl and pyrimidinyl, the above groups are independently optionally further substituted with one or more substituents selected from the group consisting of deuterium, fluoro, chloro, bromo, cyano, methyl, ethyl, *n*-propyl, isopropyl, trifluoromethyl, difluoromethyl, trideuteriomethyl, dideuteriomethyl, cyclopropyl, cyclobutyl, cyclopentyl, bicyclo[1.1.1]pentyl, cyclohexyl, oxacyclobutyl, azacyclobutyl, tetrahydrofuryl, pyrazolyl, (2-(trimethylsilyl)ethoxy)methyl-substituted pyrazolyl, imidazolyl, thiazolyl, pyridyl, fluoro-substituted pyridyl, pyrimidinyl, methoxy, ethoxy and cyclopropyloxy.

15. The compound of formula (I), the stereoisomer or pharmaceutically acceptable salt thereof according to claim 11, wherein, **each R₂** is independently selected from the group consisting of hydrogen, deuterium, methyl, ethyl, trifluoromethyl, trifluoroethyl, trideuteriomethyl, ethyl, n-propyl, isopropyl, cyclopropyl, cyclobutyl and following substituent structures:

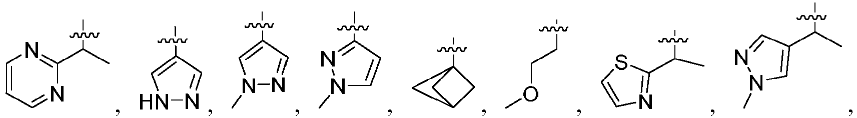

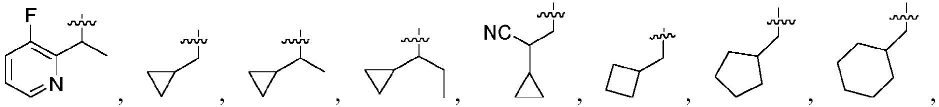

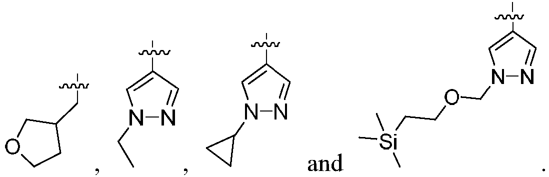

16. The compound of formula (I), the stereoisomer or pharmaceutically acceptable salt thereof according to claim 11, wherein the compound of formula (I) is a compound having formula (V₁₋₁₋₁) or formula (V₁₋₁₋₂): 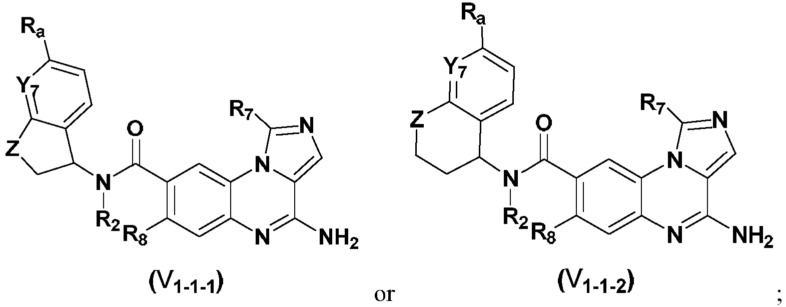
wherein, each Y₇ is independently CH or N; each Z is independently O or CH₂; each **Rₐ** is independently selected from the group consisting of hydrogen, deuterium, fluoro, chloro, bromo, cyano, methyl, methoxy, trifluoromethyl, trideuteriomethyl, trifluoromethoxy, trideuteriomethoxy, cyclopropyl, pyrazolyl, methyl-substituted pyrazolyl and -SF₅;
each **R₇** is independently selected from the group consisting of hydrogen, deuterium, fluoro, chloro, methyl, trifluoromethyl, trideuteriomethyl and cyclopropyl;
**each R₈** is independently selected from the group consisting of hydrogen, deuterium, fluoro, chloro, cyano, methyl, trifluoromethyl, trideuteriomethyl and cyclopropyl;
wherein R₂ is defined as in claim 11.

17. The compound of formula (I), the stereoisomer or pharmaceutically acceptable salt thereof according to claim 11, wherein the compound of formula (I) is a compound having formula (V₁₋₂₋₁) or formula (V₁₋₂₋₂): 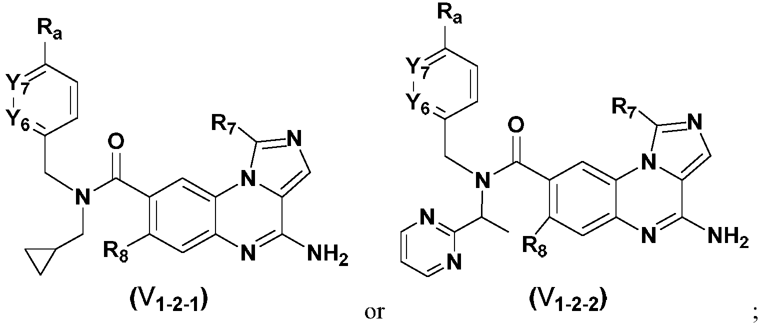
wherein, each Y₆ and each Y₇ are independently CH or N; **Rₐ** is selected from the group consisting of hydrogen, deuterium, fluoro, chloro, bromo, cyano, methyl, methoxy, trifluoromethyl, trideuteriomethyl, trifluoromethoxy, trideuteriomethoxy, cyclopropyl, pyrazolyl, methyl-substituted pyrazolyl and -SF₅;
**R₇** is selected from the group consisting of hydrogen, deuterium, fluoro, chloro, methyl, trifluoromethyl, trideuteriomethyl and cyclopropyl;
**R₈** is selected from the group consisting of hydrogen, deuterium, fluoro, chloro, cyano, methyl, trifluoromethyl, trideuteriomethyl and cyclopropyl.

18. The compound of formula (I), the stereoisomer or pharmaceutically acceptable salt thereof according to claim 11, wherein the compound of formula (I) is a compound having formula (V₂₋₁₋₁) or formula (V₂₋₁₋₂): 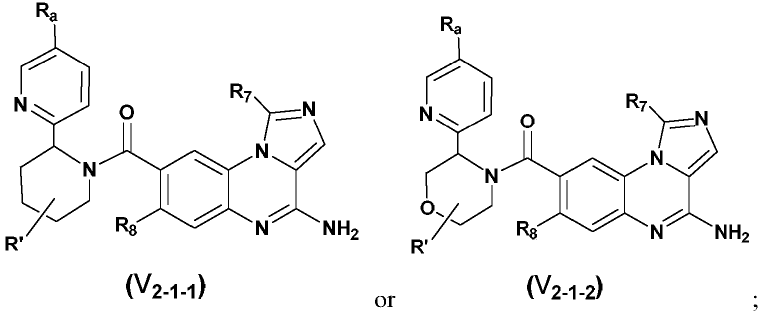
wherein, **each Rₐ** is independently selected from the group consisting of hydrogen, deuterium, fluoro, chloro, bromo, cyano, methyl, methoxy, trifluoromethyl, trideuteriomethyl, trifluoromethoxy, trideuteriomethoxy, cyclopropyl, pyrazolyl, methyl-substituted pyrazolyl and - SF₅;
**each R'** is independently selected from the group consisting of hydrogen, deuterium, fluoro, chloro, methyl, trifluoromethyl, trideuteriomethyl and cyclopropyl;
**each R₇** is independently selected from the group consisting of hydrogen, deuterium, fluoro, chloro, methyl, trifluoromethyl, trideuteriomethyl and cyclopropyl;
**each R₈** is independently selected from the group consisting of hydrogen, deuterium, fluoro, chloro, cyano, methyl, trifluoromethyl, trideuteriomethyl and cyclopropyl.

19. The compound of formula (I), the stereoisomer or pharmaceutically acceptable salt thereof according to claim 11, wherein the compound of formula (I) is a compound having formula (V₂₋₂₋₁) or formula (V₂₋₂₋₂): 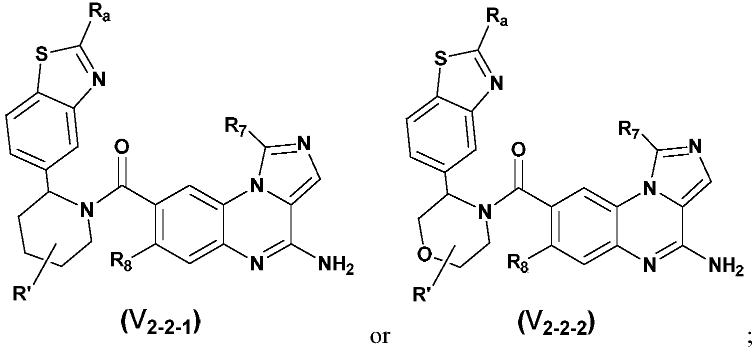
wherein, **each Rₐ** is independently selected from the group consisting of hydrogen, deuterium, fluoro, chloro, bromo, cyano, methyl, methoxy, trifluoromethyl, trideuteriomethyl, trifluoromethoxy, trideuteriomethoxy, cyclopropyl, pyrazolyl, methyl-substituted pyrazolyl, methyl-substituted piperidinyl, dimethylaminoethyl and -SF₅;
**each R'** is independently selected from the group consisting of hydrogen, deuterium, fluoro, chloro, methyl, trifluoromethyl, trideuteriomethyl and cyclopropyl;
**each R₇** is independently selected from the group consisting of hydrogen, deuterium, fluoro, chloro, methyl, trifluoromethyl, trideuteriomethyl and cyclopropyl;
**each R₈** is independently selected from the group consisting of hydrogen, deuterium, fluoro, chloro, cyano, methyl, trifluoromethyl, trideuteriomethyl and cyclopropyl.

20. The compound of formula (I), the stereoisomer or pharmaceutically acceptable salt thereof according to any one of claims 1-19, wherein the compound is selected from the group consisting of the following compounds:

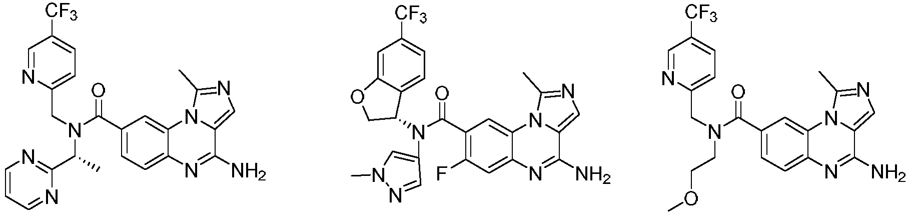

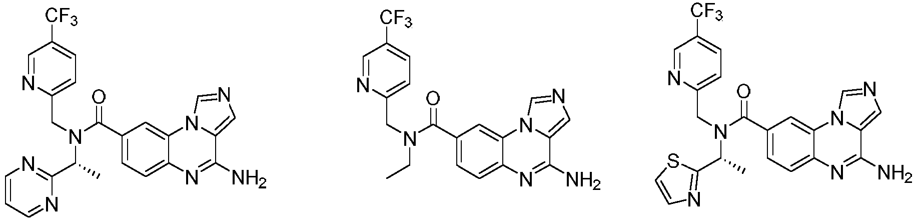

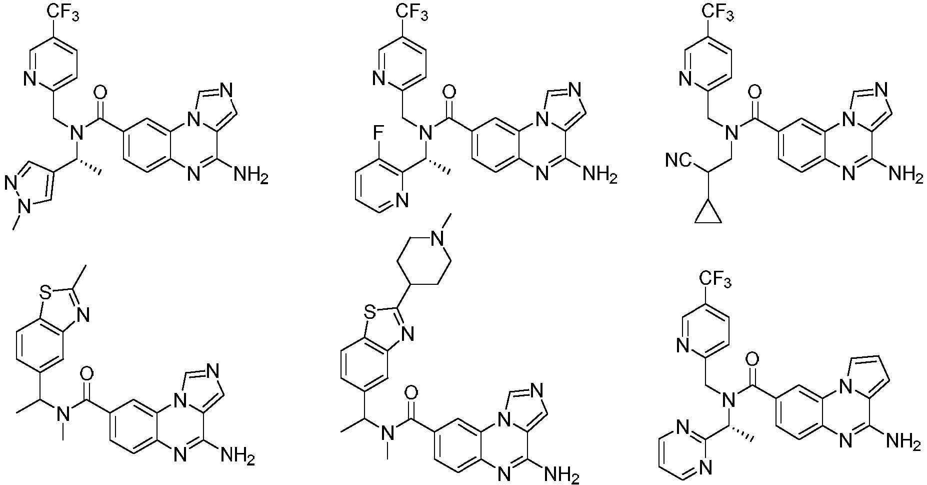

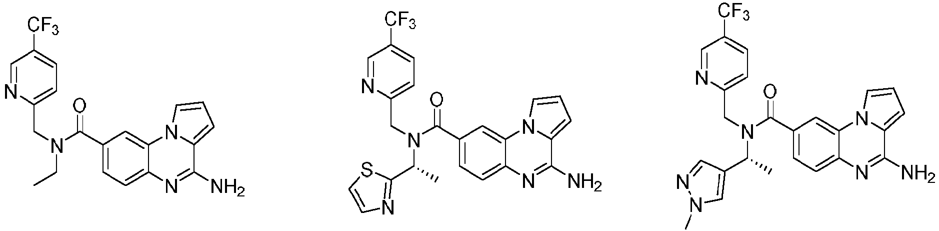

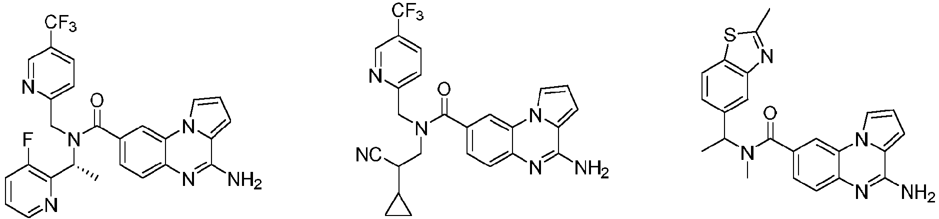

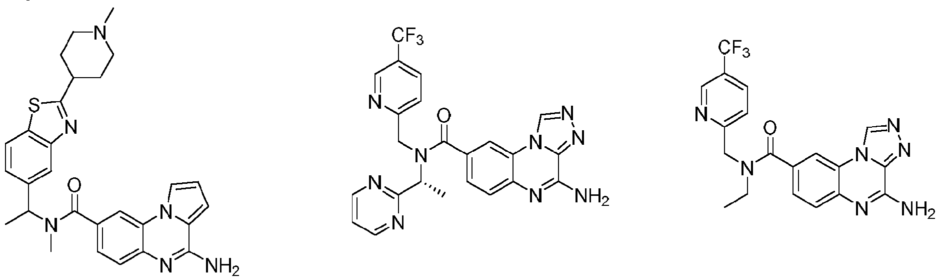

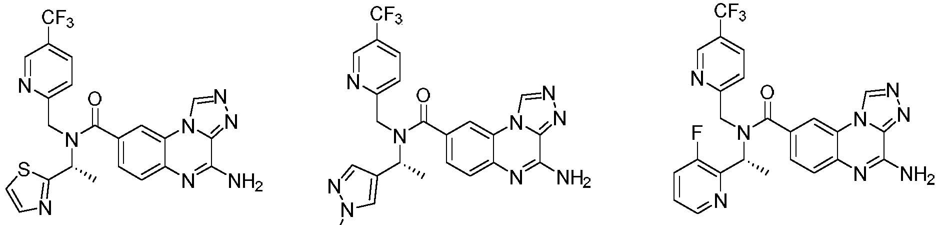

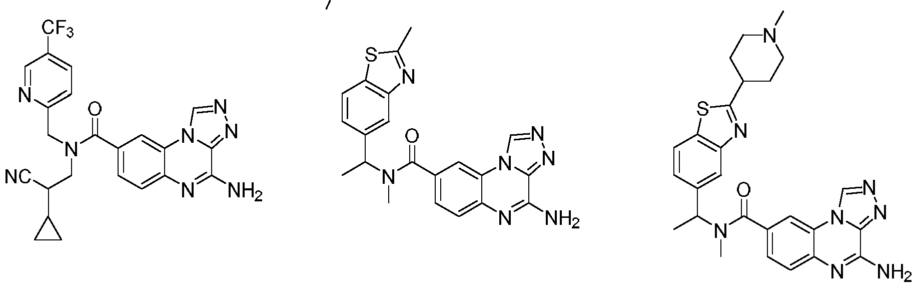

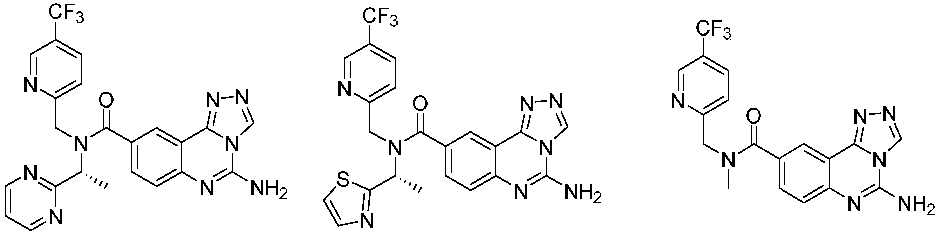

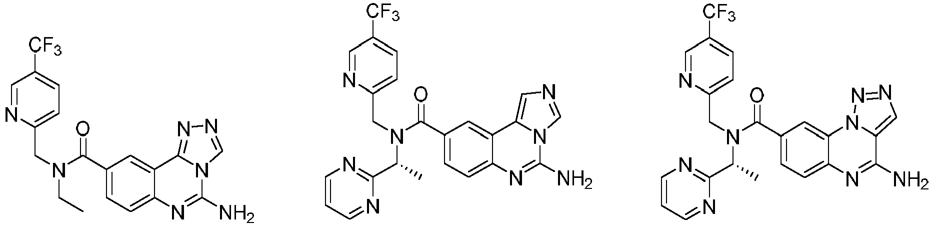

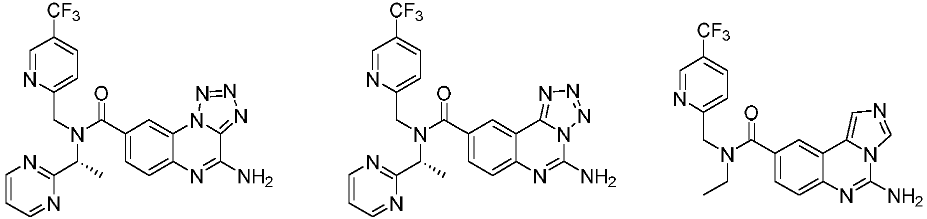

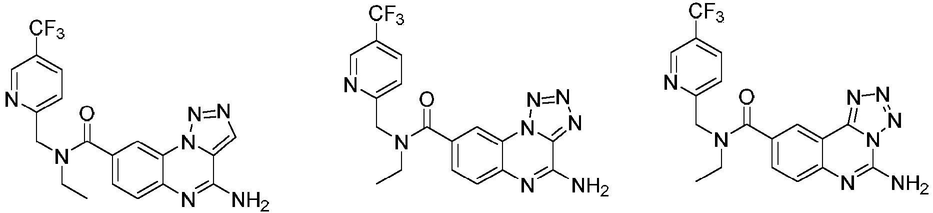

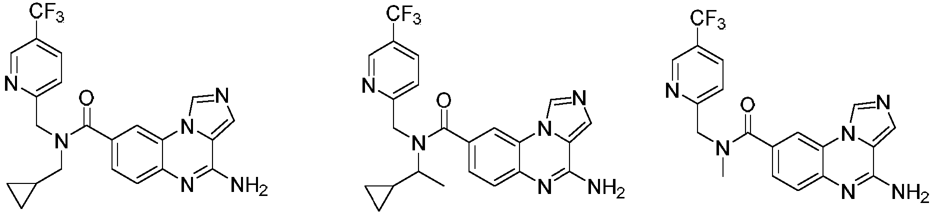

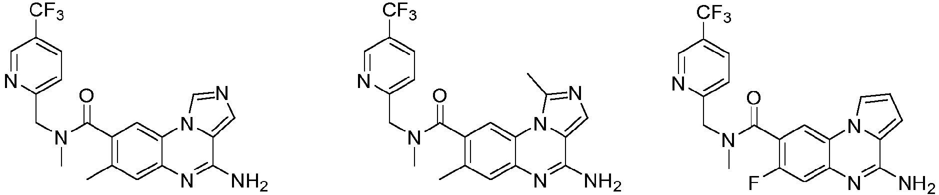

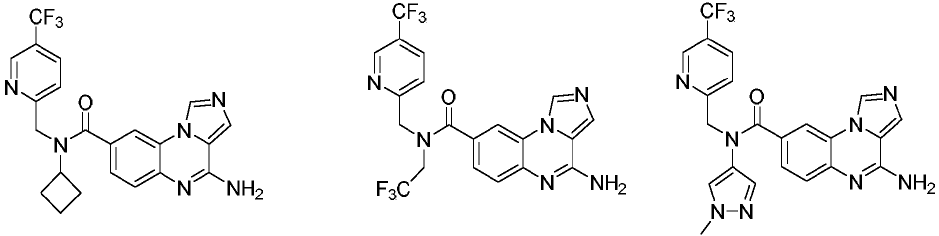

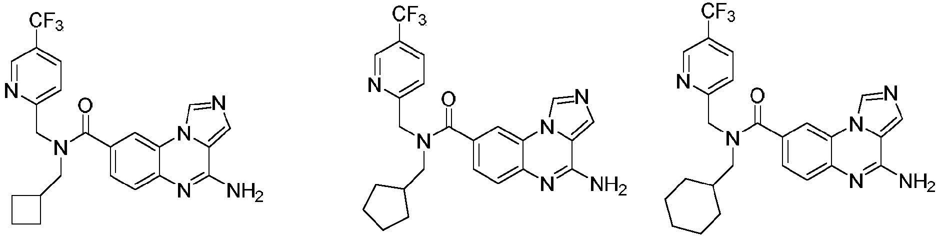

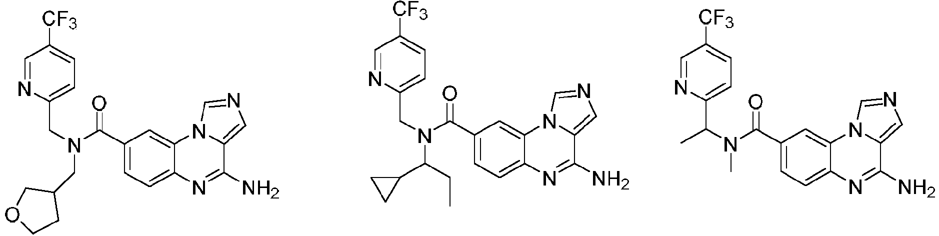

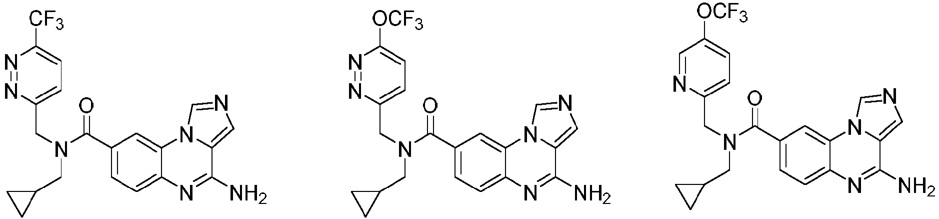

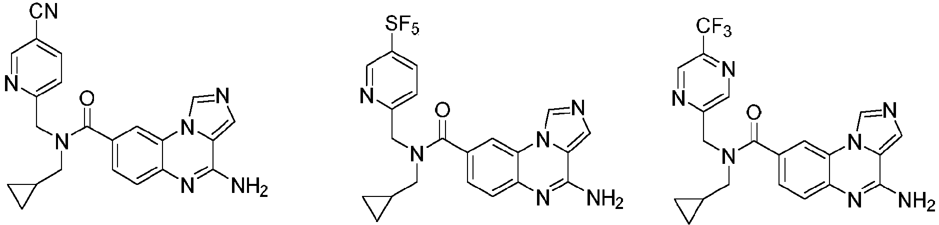

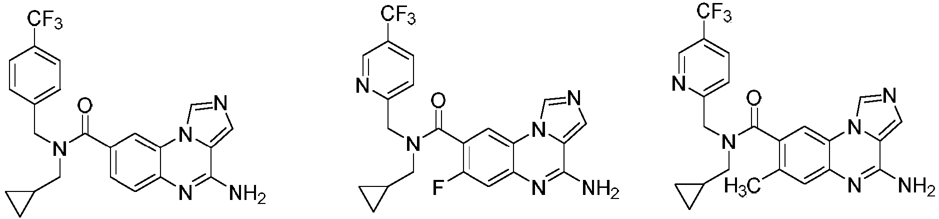

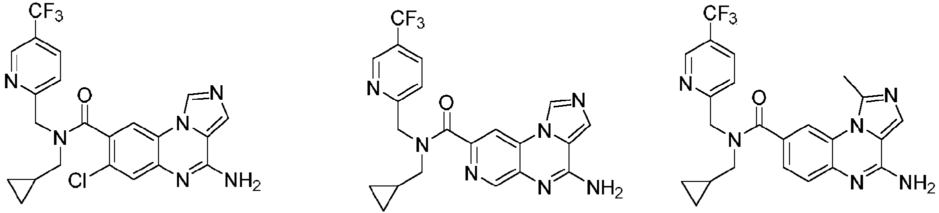

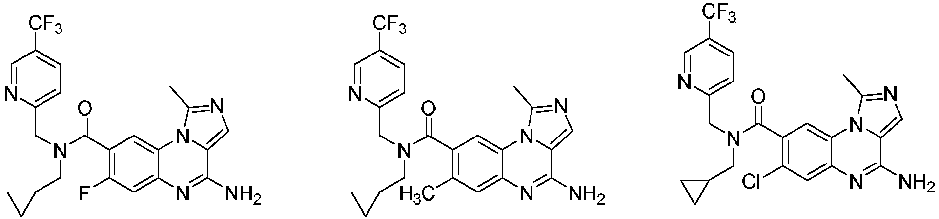

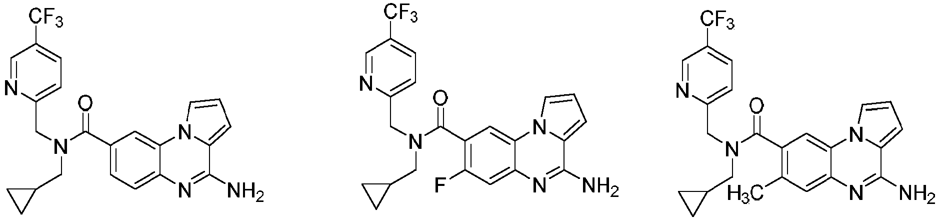

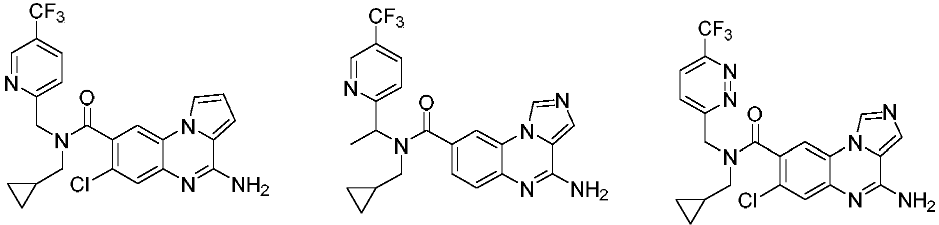

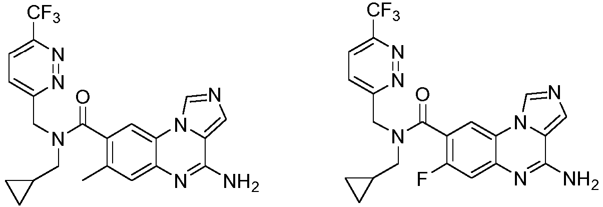

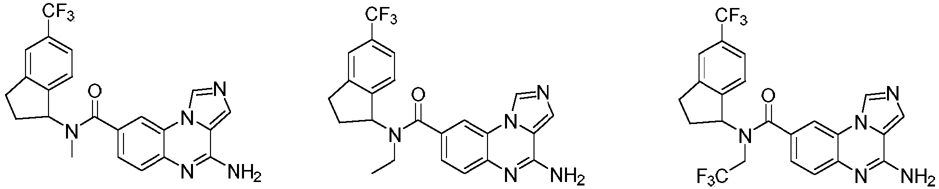

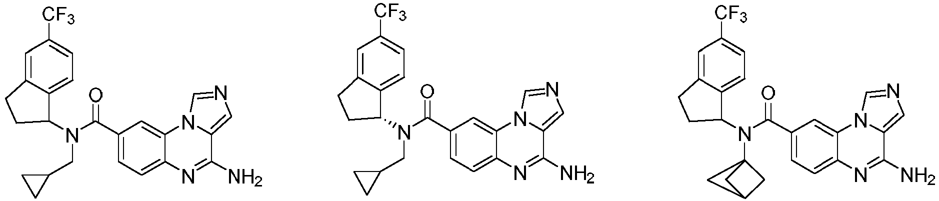

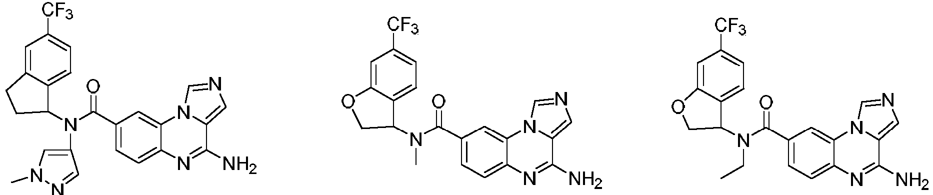

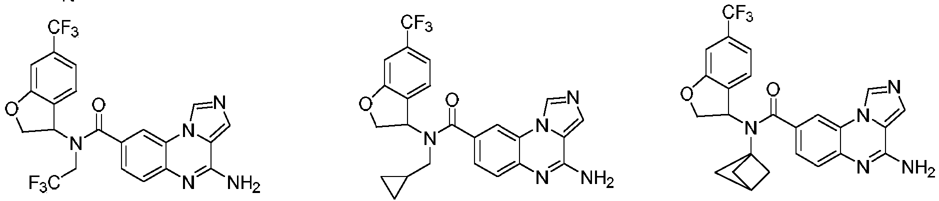

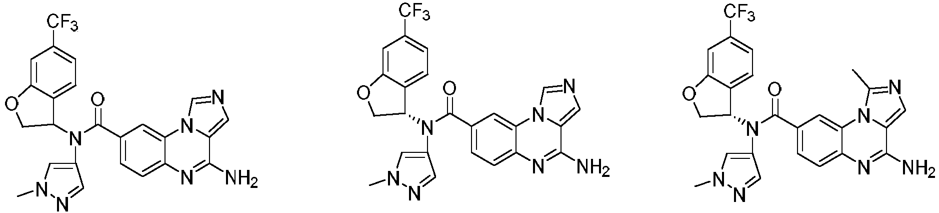

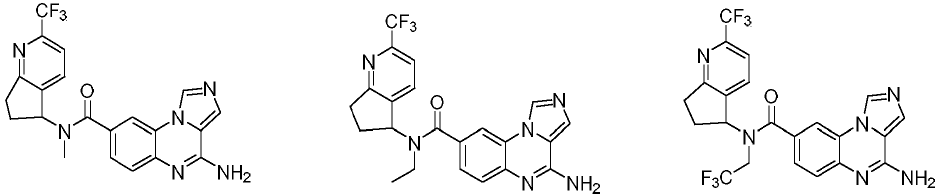

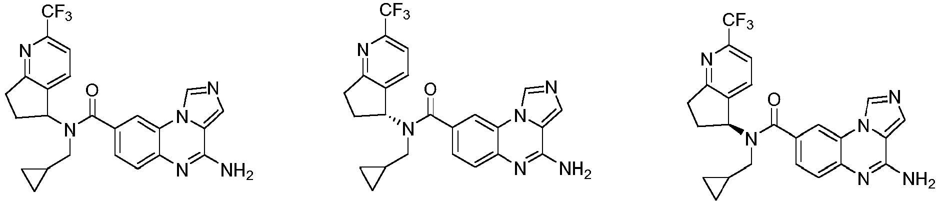

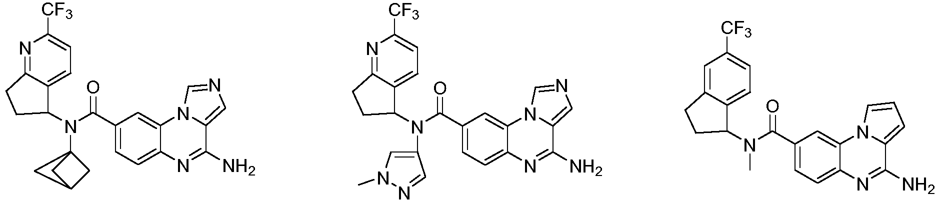

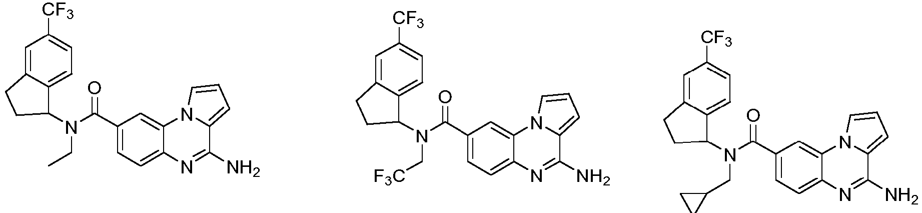

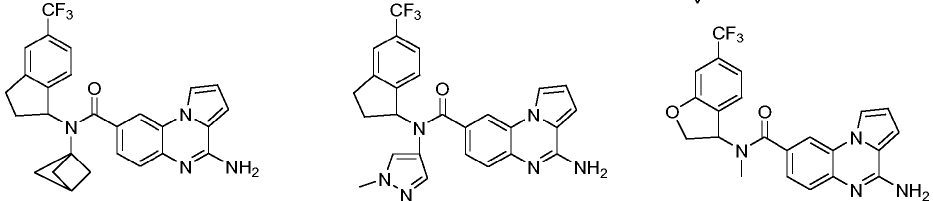

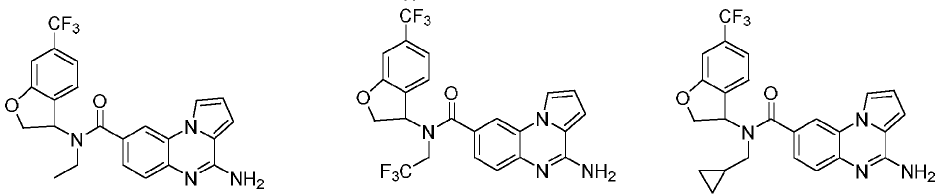

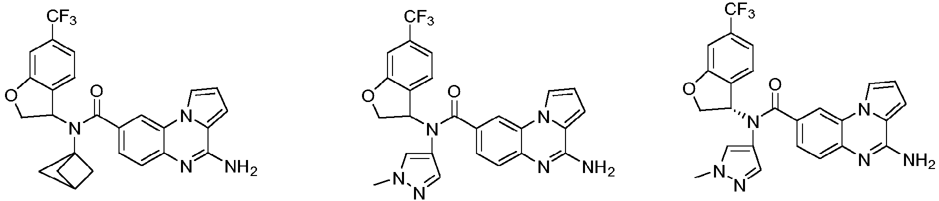

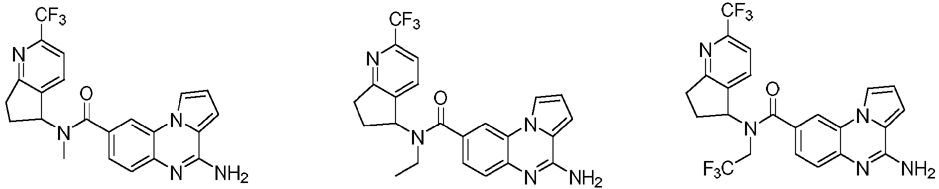

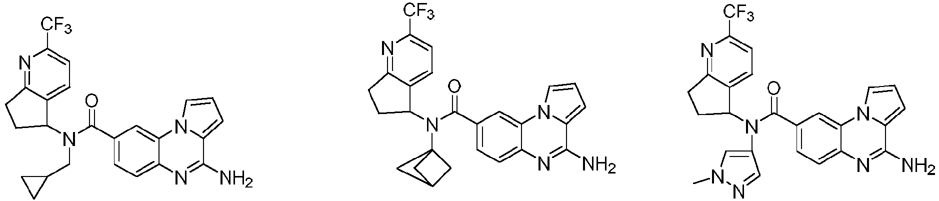

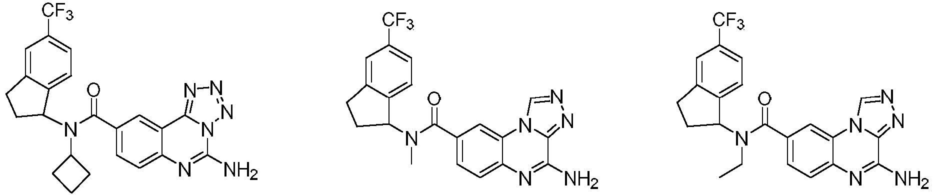

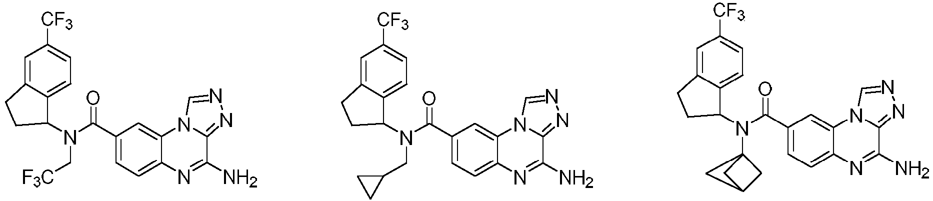

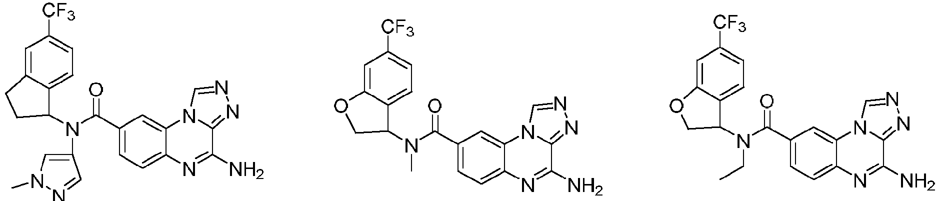

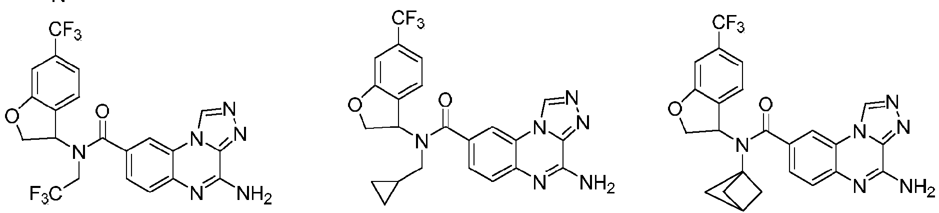

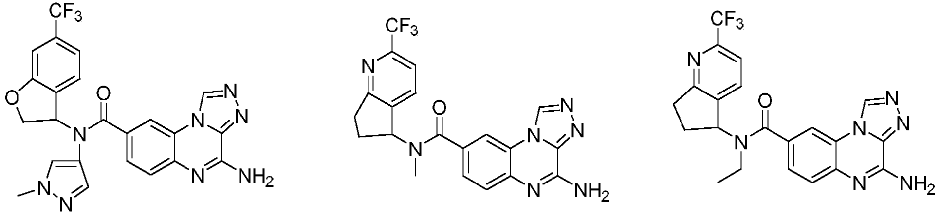

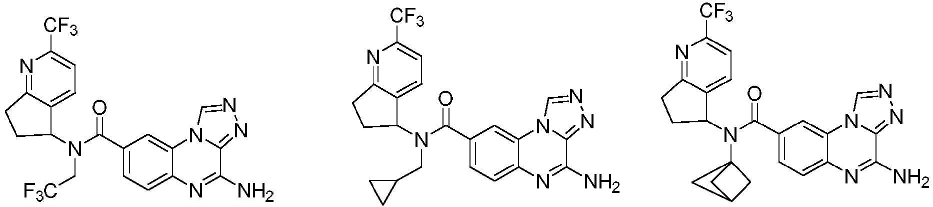

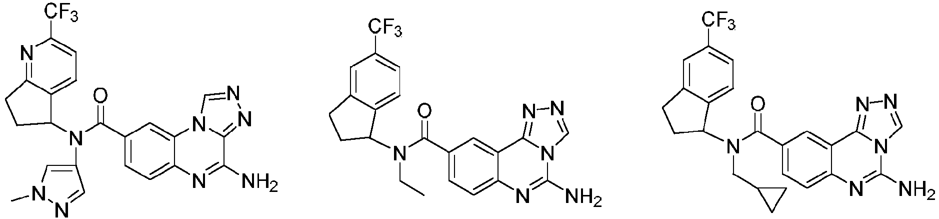

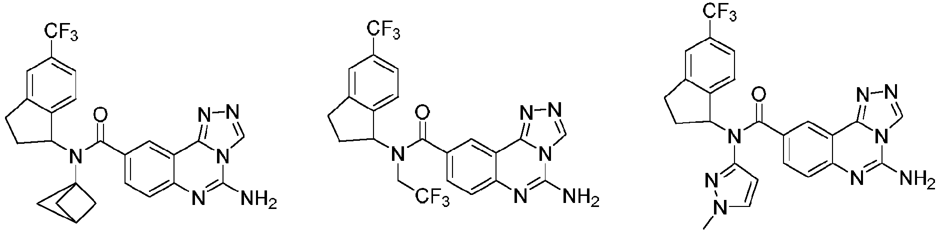

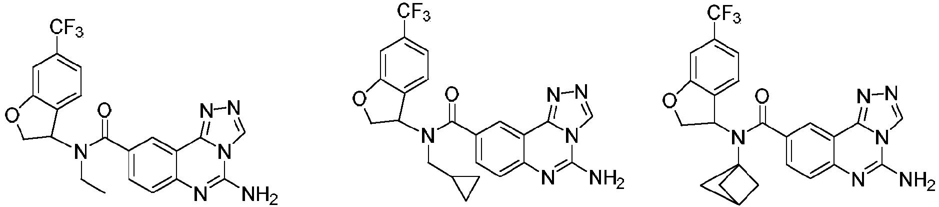

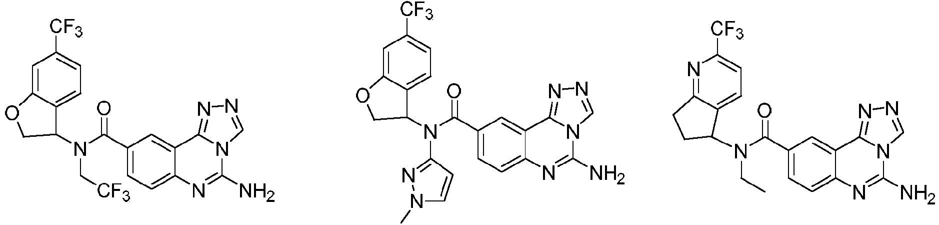

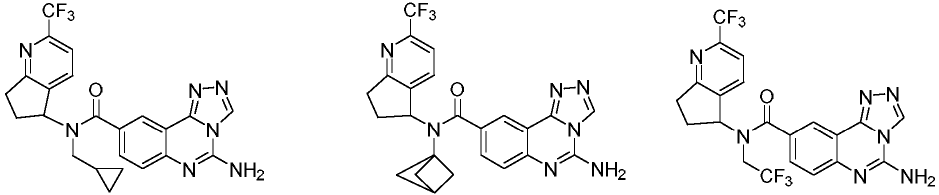

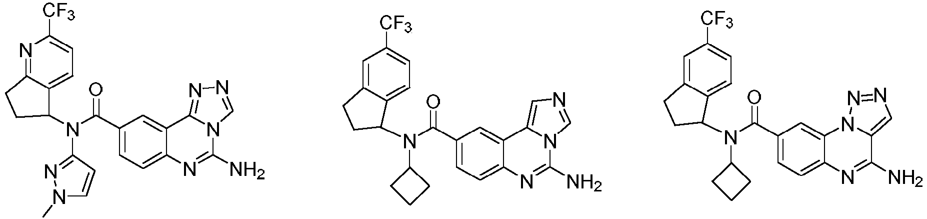

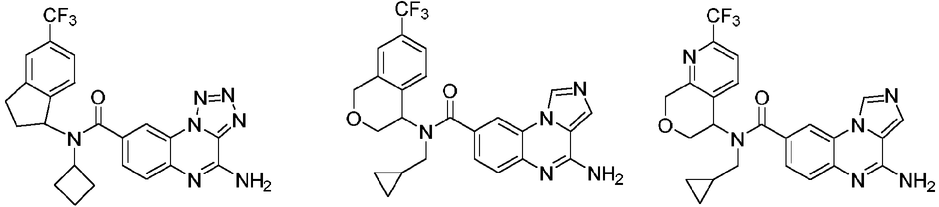

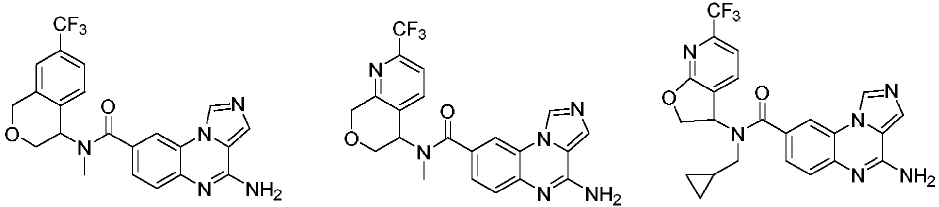

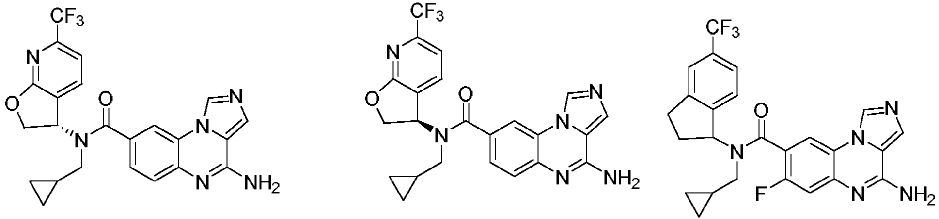

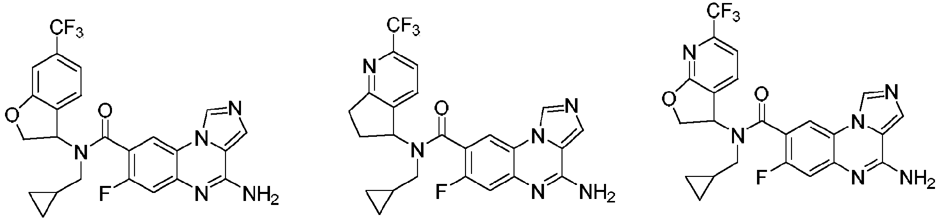

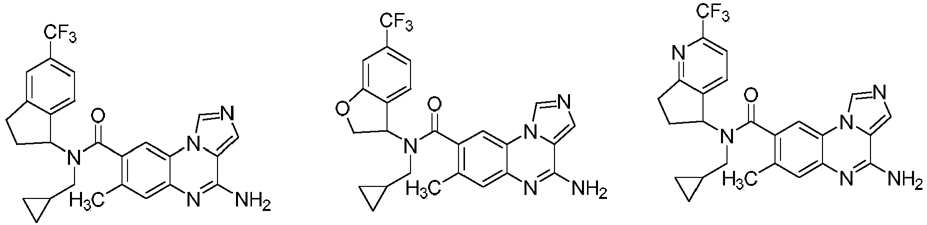

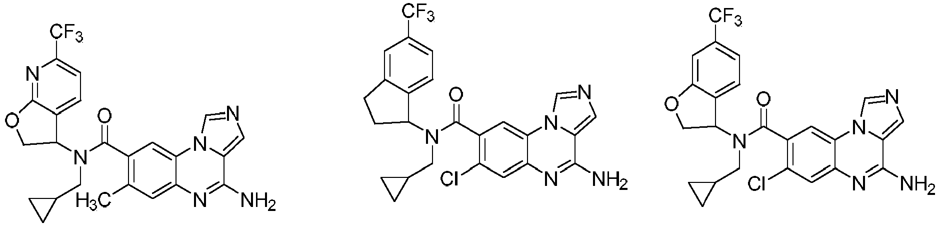

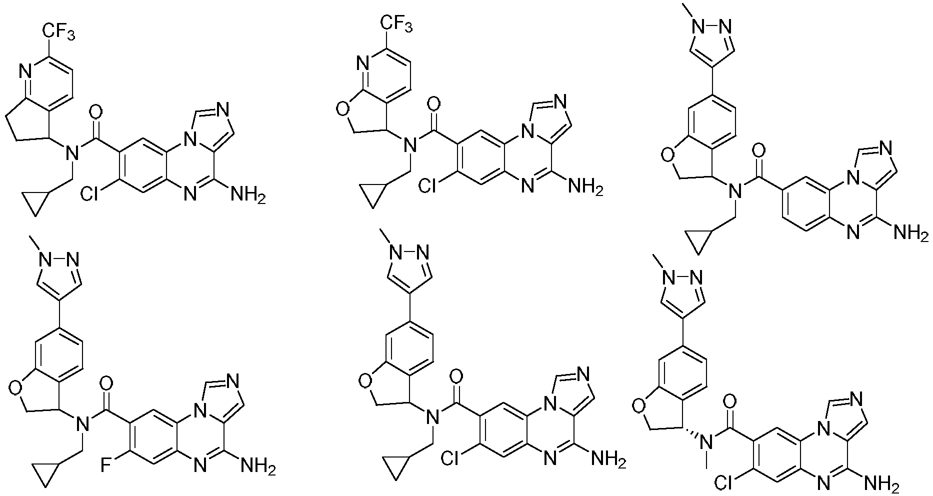

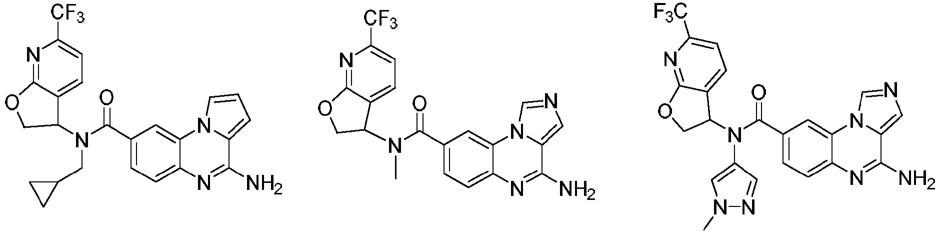

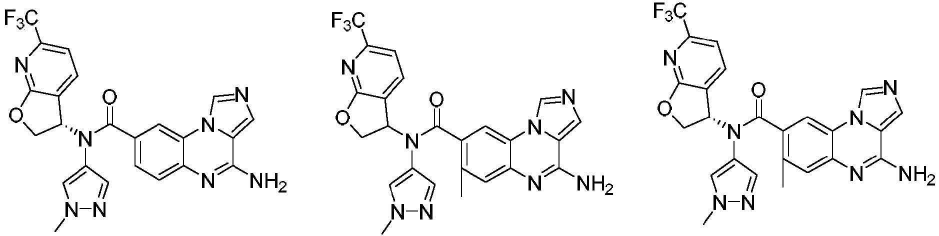

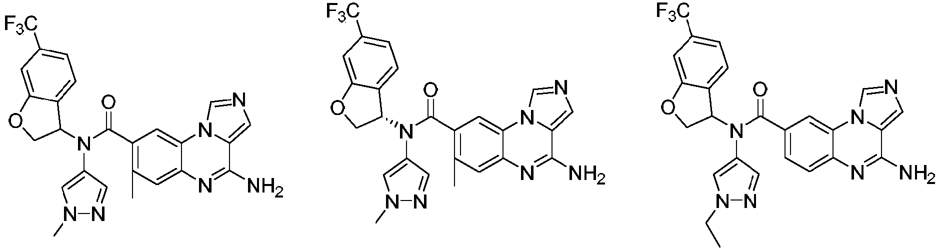

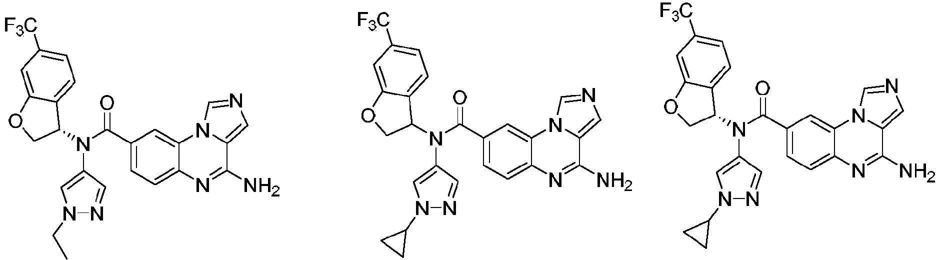

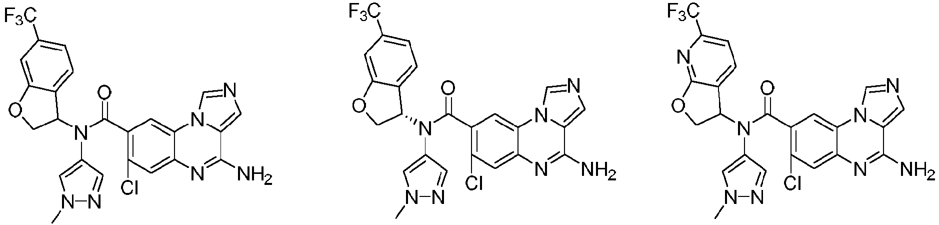

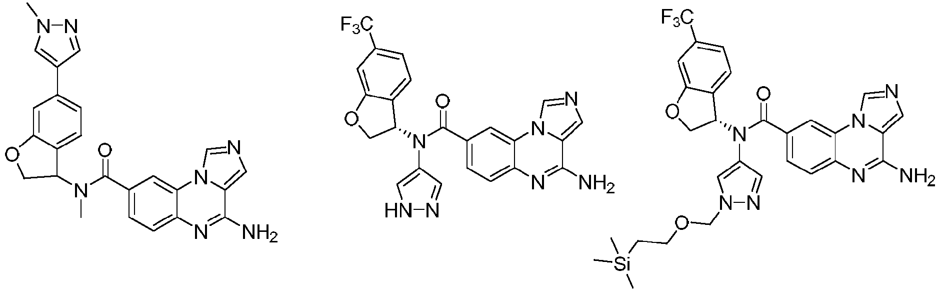

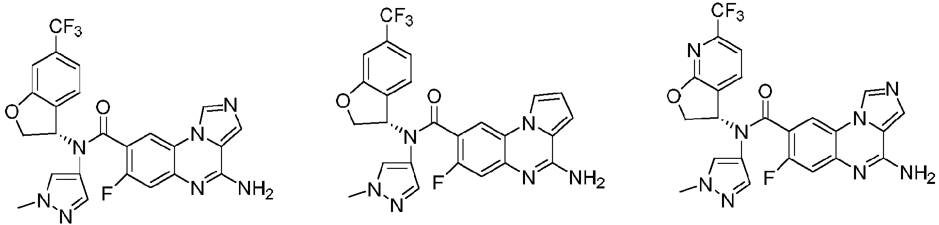

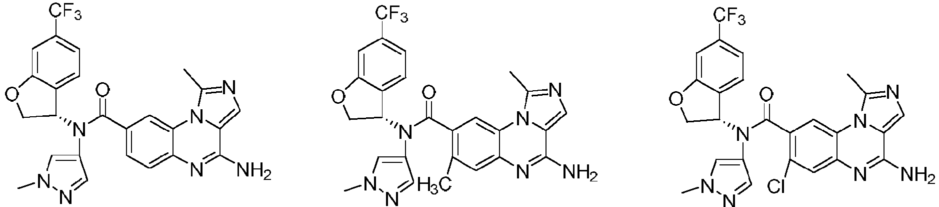

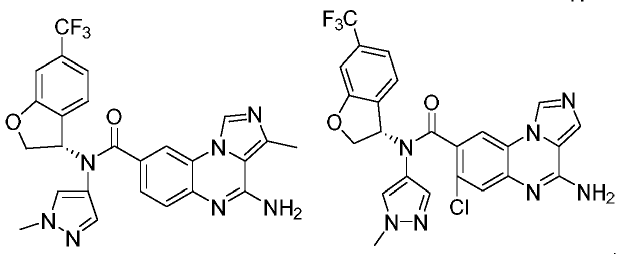

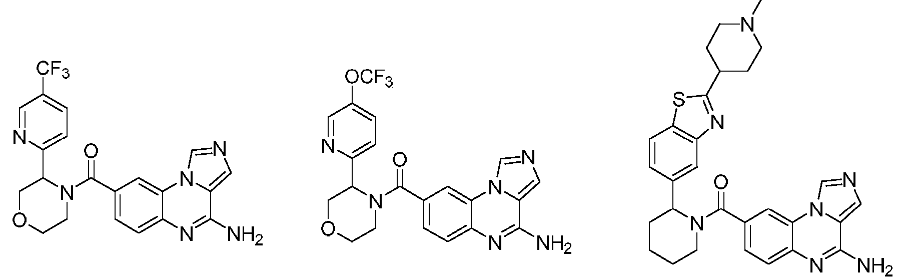

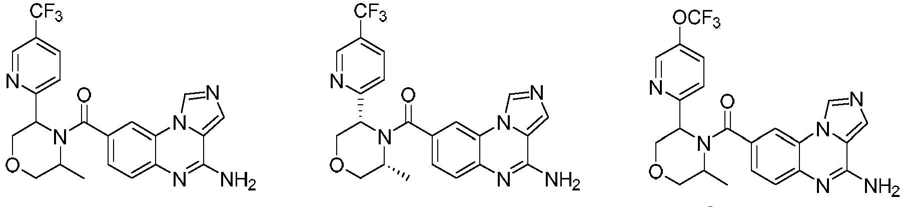

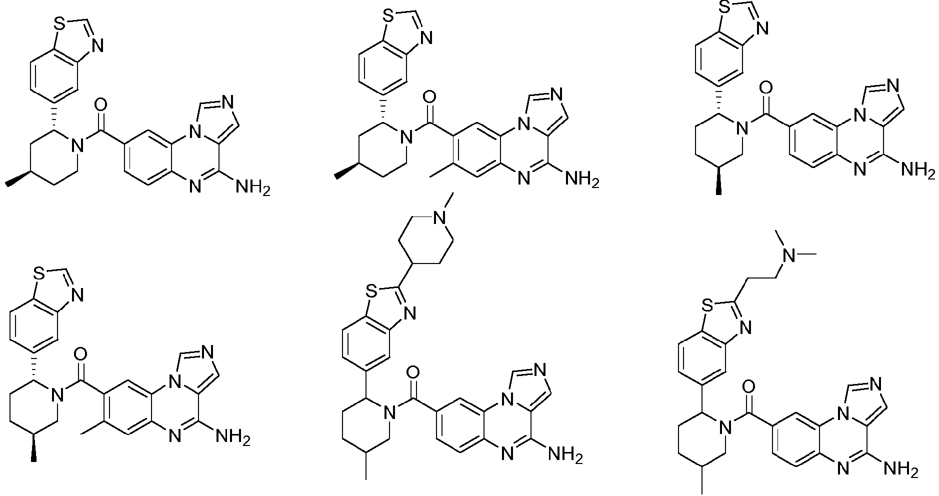

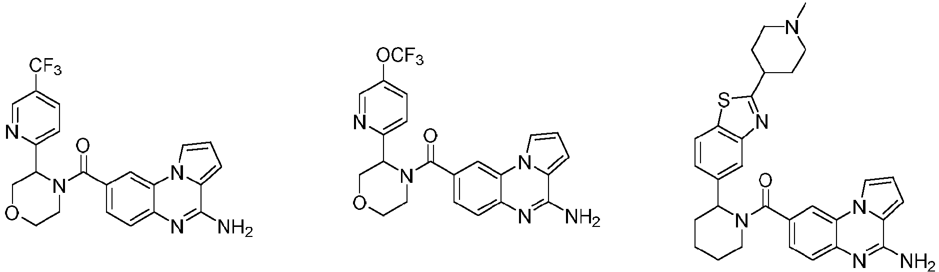

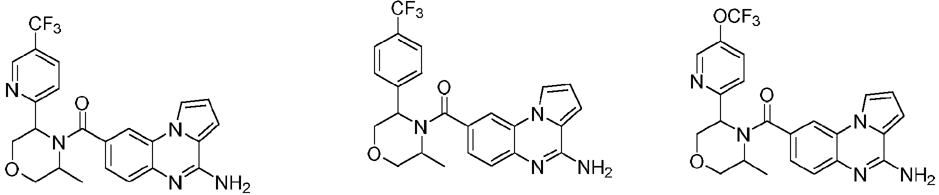

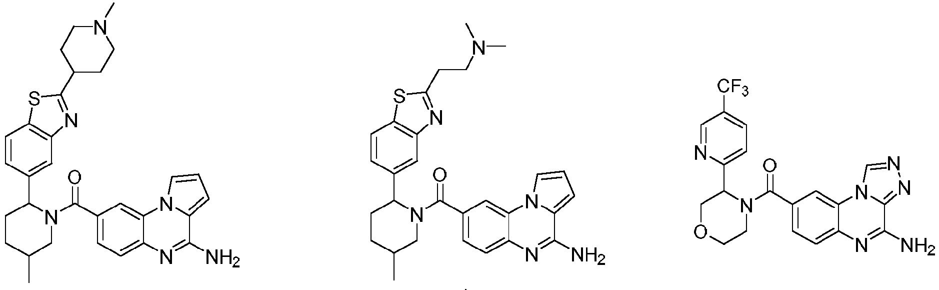

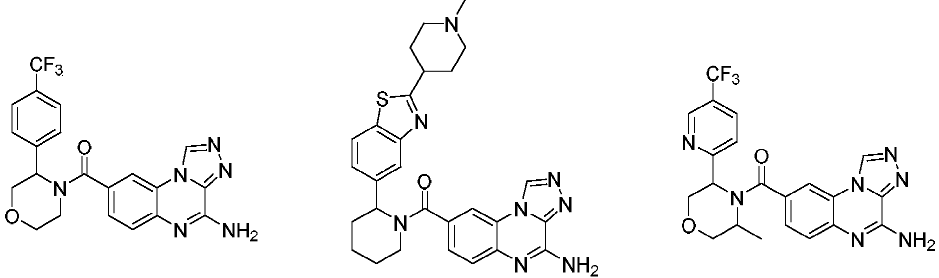

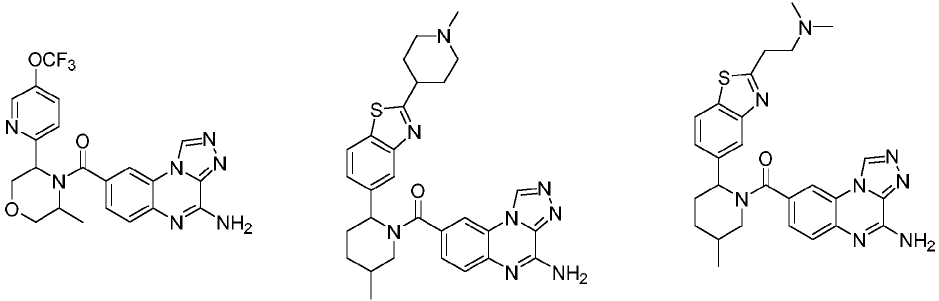

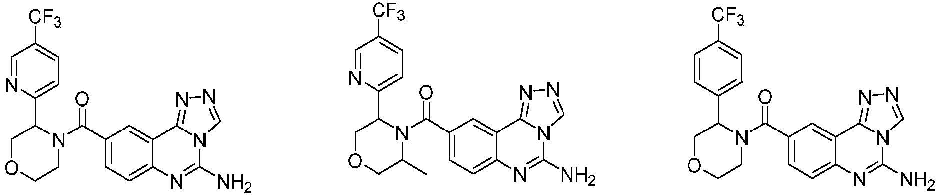

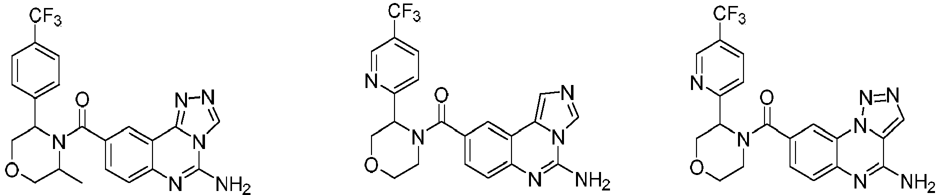

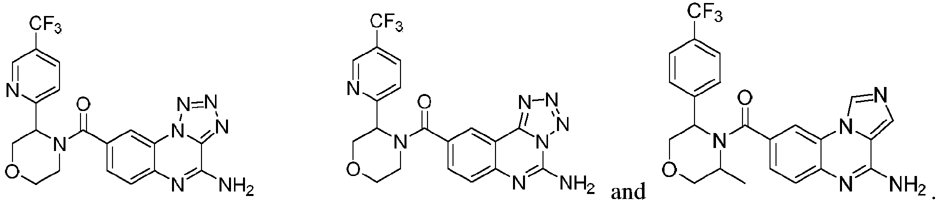

21. A pharmaceutical composition, comprising the compound of formula (I), the stereoisomer or pharmaceutically acceptable salt thereof according to any one of claims 1-20 and a pharmaceutically acceptable carrier.

22. Use of the compound of formula (I), the stereoisomer or pharmaceutically acceptable salt thereof according to any one of claims 1-20 in the preparation of a medicament for treating a MATP-related tumor.

23. The use according to claim 22, wherein the tumor is selected from the group consisting of cytoma, lymphoma, leukemia, osteoma, malignant teratoma, intraepithelial cancer, adenoma, fibroma, melanoma, fallopian tube cancer, bladder cancer, teratoma, embryonal carcinoma, choriocarcinoma, lipoma, liver cancer, cholangiocarcinoma, lung cancer, gastric cancer, hemangioma, gallbladder cancer, ampulla cancer, malignant melanoma, nevi, dysplastic nevi, myeloproliferative disease, Hodgkin's disease, chordoma, myxoma, rhabdomyoma, leiomyoma, hamartoma, mesothelioma, insulinoma, glucagonoma, gastrinoma, carcinoid tumor, vipoma, granuloma, xanthoma, osteitis deformans, ependymoma, schwanoma, congenital tumor, meningioma, glioma, skin cancer, head and neck cancer and sarcoma.

24. The use according to claim 22, wherein, the cytoma is selected from the group consisting of granulosa-thecal cell tumor, sertoli cell tumor, germ cell tumor, nephroblastoma, seminoma, hepatoblastoma, malignant fibrous histiocytoma, chondroblastoma, giant cell tumor, astrocytoma, medulloblastoma, glioblastoma multiforme, oligodendroglioma, retinoblastoma, squamous cell carcinoma, clear cell carcinoma, transitional cell carcinoma, interstitial cell carcinoma and basal cell carcinoma;
the lymphoma is malignant lymphoma or non-Hodgkin lymphoma;
the leukemia is acute and chronic myeloid leukemia, acute lymphoblastic leukemia or chronic lymphoblastic leukemia;
the osteoma is selected from the group consisting of osteochondroma, benign chondroma, osteoidosteoma, chondroma-like hamartoma, multiple myeloma and skull tumor;
the adenoma is selected from the group consisting of fibroadenoma, adenomatoid tumor, hepatocellular adenoma, bronchial adenoma, tubular adenoma, villous adenoma, breast cancer, pancreatic cancer, endometrial adenocarcinoma, prostate cancer, ductal adenocarcinoma and colorectal adenocarcinoma;
the fibroma is fibroma, chondromyxoid fibroma, neurofibroma or spinal neurofibroma;
the myeloproliferative disease is multiple myeloma or myelodysplastic syndrome;
the lung cancer is bronchogenic carcinoma or alveolar carcinoma;
the sarcoma is selected from the group consisting of fibrosarcoma, botryoid sarcoma, angiosarcoma, Kaposi's sarcoma, osteosarcoma, chondrosarcoma, Ewing's sarcoma, rhabdomyosarcoma, liposarcoma, leiomyosarcoma and meningosarcoma.

25. The compound of formula (I), the stereoisomer or pharmaceutically acceptable salt thereof according to any one of claims 1-20 for use as a PRMT5 inhibitor.
